# EUROPEAN PATENT SPECIFICATION

(11) **EP 4 291 552 B1**
(45) Date of publication and mention of the grant of the patent: **09.04.2025**
(21) Application number: 21706486.4
(22) Date of filing: 09.02.2021
(51) Int. Cl.: C07D 401/04, C07D 401/14, C07D 403/04, C07D 405/04, C07D 405/14, C07D 413/04, C07D 413/14, C07D 417/14, C07D 451/00, C07D 498/10, A61P 25/28, A61K 31/5355

(54) **MODULATORS OF COMPLEX I**
MODULATOREN DES KOMPLEX I
MODULATEURS DU COMPLEXE I

(43) Date of publication of application: 20.12.2023
(73) Proprietor: Boehringer Ingelheim International GmbH, 55216 Ingelheim am Rhein (DE)
(72) Inventor: GERLACH, Kai, 55216 Ingelheim am Rhein (DE); EICKMEIER, Christian, 55216 Ingelheim am Rhein (DE); KRIEGL, Jan, Magnus, 55216 Ingelheim am Rhein (DE); KUSSMAUL, Lothar, 55216 Ingelheim am Rhein (DE); RUDOLF, Klaus, 55216 Ingelheim am Rhein (DE); SCHMID, Bernhard, 55216 Ingelheim am Rhein (DE)
(74) Representative: Lutze, Oliver
(86) International application number: PCT/EP2021/053002
(87) International publication number: WO 2022/171265

(56) References cited:
- WO-A1-2018/162581
- US-A1- 2021 061 761

## Description

The present invention relates to compounds having formula (I) 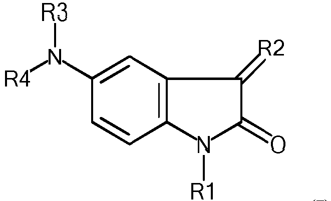 a method for their preparation, their pharmaceutical compositions, and these compounds for use in therapy. Unexpectedly, said compounds modulate the function of the mitochondrial complex I (NADH-quinone oxidoreductase) allowing the treatment or prevention of conditions having an association with Complex I NADH-quinone oxidoreductase mediated oxidative stress.

Oxidative stress reflects the imbalance between the generation and detoxification of Reactive Oxygen Species (ROS), which can cause toxic effects through the increased concentration of ROS, through disruption in cellular signaling and/or through damaging/oxidation of proteins, DNA or lipids. Oxidative stress is suspected to be important in many diseases.

A variety of enzymes generate reactive oxygen species (ROS) in cells. One major source of ROS is oxidative phosphorylation via Complex I. The enzyme is a protein complex, encoded by 39 nuclear and 7 mitochondrial genes which is expressed ubiquitously and transfers electrons from NADH to Ubiquinone, coupled to translocation of protons necessary for ATP synthesis.

Since NADH-oxidation is much faster than reduction of Ubiquinone, the enzyme is reduced under physiological conditions and electron leakage (i.e. production of the negatively charged superoxide O₂•⁻ radical) occurs at the NADH-binding site. In consequence a low efficiency of UQ reduction is coupled to increased O₂•⁻ formation, which can be observed in several diseases such as Leigh syndrome, LHON disease, AMD or Parkinson's disease among others. The cytotoxic O₂•⁻ generated by Complex I is detoxified mainly by the mitochondrial superoxide dismutase (SOD2), generating hydrogen peroxide which is detoxified by a variety of enzymes, such as Catalase, Glutathionperoxidase(s), Thioreredoxin(s) etc. Given the central importance of Complex I in oxidative phosphorylation and redox homeostasis, identification of agents that can inhibit ROS formation are of great interest as possible therapeutic agents.

WO 2018/162581 concerns desmethylanethole trithione derivatives for the treatment of diseases relating to reactive oxygen species of mitochondrial origin, such as neurogenerative diseases.

Terms not specifically defined herein should be given the meanings that would be given to them by one of skill in the art in light of the disclosure and the context. As used in the specification, however, unless specified to the contrary, the following terms have the meaning indicated and the following conventions are adhered to.

In the groups, radicals, or moieties defined below, the number of carbon atoms is often specified preceding the group, for example, C₁₋₆-alkyl means an alkyl group or radical having 1 to 6 carbon atoms. In general in groups like HO, H₂N, (O)S, (O)₂S, NC (cyano), HOOC, F₃C or the like, the skilled artisan can see the radical attachment point(s) to the molecule from the free valences of the group itself. For combined groups comprising two or more subgroups, the last named subgroup is the radical attachment point, for example, the substituent "aryl-C1-3-alkyl" means an aryl group which is bound to a C₁₋₃-alkyl-group, the latter of which is bound to the core or to the group to which the substituent is attached.

In case a compound of the present invention is depicted in form of a chemical name and as a formula in case of any discrepancy the formula shall prevail. An asterisk (*) may be used in sub-formulas to indicate the bond which is connected to the core molecule as defined.

The term "substituted" as used herein, means that any one or more hydrogens on the designated atom is replaced with a selection from the indicated group, provided that the designated atom's normal valence is not exceeded, and that the substitution results in a stable compound.

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

In general, substantially pure stereoisomers can be obtained according to synthetic principles known to a person skilled in the field, e.g. by separation of corresponding mixtures, by using stereochemically pure starting materials and/or by stereoselective synthesis. It is known in the art how to prepare optically active forms, such as by resolution of racemic forms or by synthesis, e.g. starting from optically active starting materials and/or by using chiral reagents.

Enantiomerically pure compounds of this invention or intermediates may be prepared via asymmetric synthesis, for example by preparation and subsequent separation of appropriate diastereomeric compounds or intermediates which can be separated by known methods (e.g. by chromatographic separation or crystallization) and/or by using chiral reagents, such as chiral starting materials, chiral catalysts or chiral auxiliaries.

Further, it is known to the person skilled in the art how to prepare enantiomerically pure compounds from the corresponding racemic mixtures, such as by chromatographic separation of the corresponding racemic mixtures on chiral stationary phases; or by resolution of a racemic mixture using an appropriate resolving agent, e.g. by means of diastereomeric salt formation of the racemic compound with optically active acids or bases, subsequent resolution of the salts and release of the desired compound from the salt; or by derivatization of the corresponding racemic compounds with optically active chiral auxiliary reagents, subsequent diastereomer separation and removal of the chiral auxiliary group; or by kinetic resolution of a racemate (e.g. by enzymatic resolution); by enantioselective crystallization from a conglomerate of enantiomorphous crystals under suitable conditions; or by (fractional) crystallization from a suitable solvent in the presence of an optically active chiral auxiliary.

The term halogen generally denotes fluorine, chlorine, bromine and iodine.

The term "alkyl", either apart or in combination with another radical, denotes an acyclic, saturated, branched or linear hydrocarbon radical with 1 to 6 C atoms. For example the term C₁₋₅-alkyl embraces the radicals H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-.

The term "cycloalkyl", either apart or in combination with another radical denotes a cyclic, saturated, unbranched hydrocarbon radical with 3 to 8 C atoms, preferably 3 to 5 C atoms. For example the term C₃₋₈-cycloalkyl includes cyclopropyl, cyclobutyl, cyclopentyl, cyclohexyl, cycloheptyl, and cyclooctyl and the term C₃₋₅-cycloalkyl cyclopropyl, cyclobutyl and cyclopentyl.

By the term "halo" added to an "alkyl" or "cycloalkyl" group (saturated or unsaturated) is such an alkyl or cycloalkyl group wherein one or more hydrogen atoms are replaced by a halogen atom selected from among fluorine, chlorine or bromine, preferably fluorine and chlorine, particularly preferred is fluorine. Examples include: H₂FC-, HF₂C-, F₃C-.

The term "aryl" as used herein, either apart or in combination with another radical, denotes a carbocyclic aromatic monocyclic group containing 6 carbon atoms which is optionally further fused to a second five- or six-membered, carbocyclic group which is optionally aromatic, saturated or unsaturated. Aryl includes, but is not limited to, phenyl, indanyl, indenyl, naphthyl, anthracenyl, phenanthrenyl, tetrahydronaphthyl and dihydronaphthyl.

The term "heterocyclyl" means a saturated or unsaturated mono- or polycyclic-ring systems including aromatic ring system containing one or more heteroatoms selected from N, O or S(O)r ,wherein r = 0, 1 or 2, consisting of 3 to 14 ring atoms, wherein none of the heteroatoms is part of the aromatic ring. The term "heterocyclyl" is intended to include all possible isomeric forms.

Thus, the term "heterocyclyl" includes the following exemplary structures which are not depicted as radicals as each form is optionally attached through a covalent bond to any atom so long as appropriate valences are maintained:

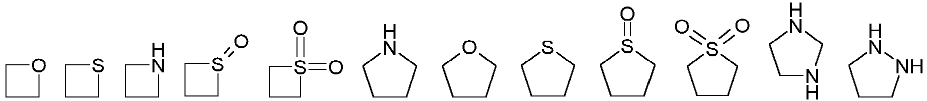

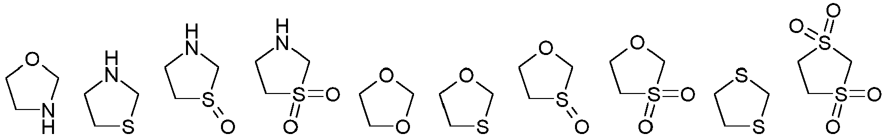

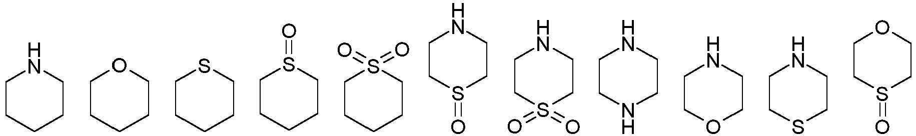

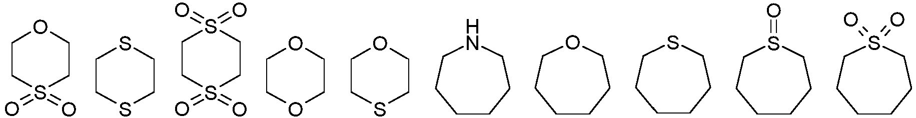

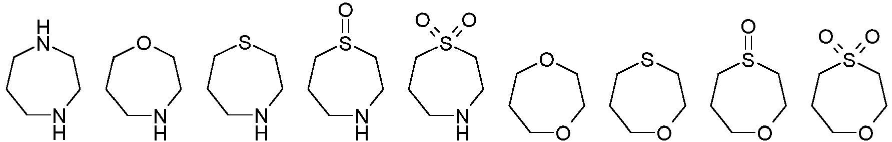

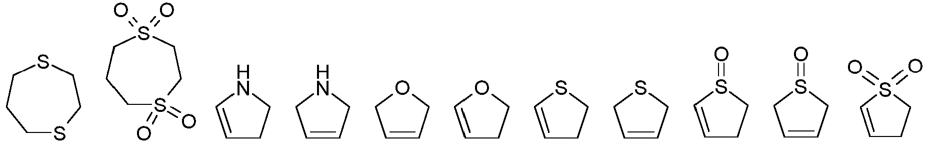

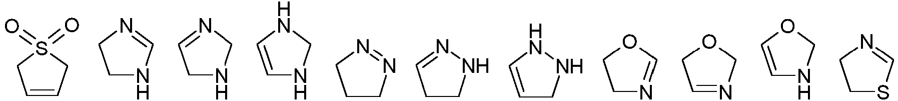

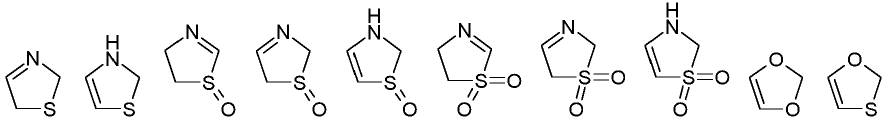

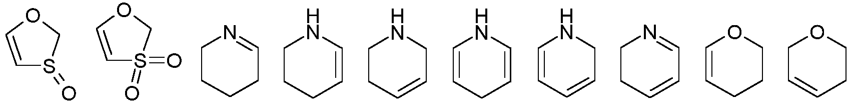

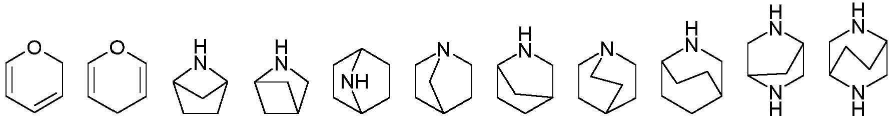

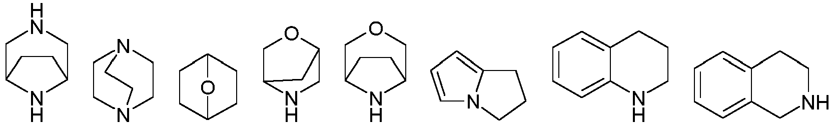

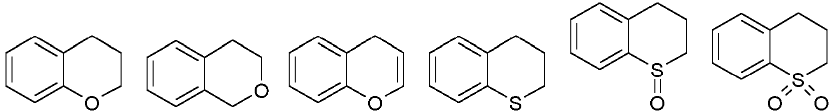

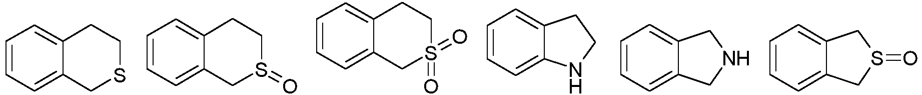

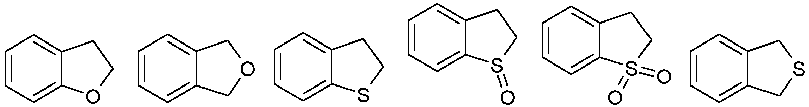

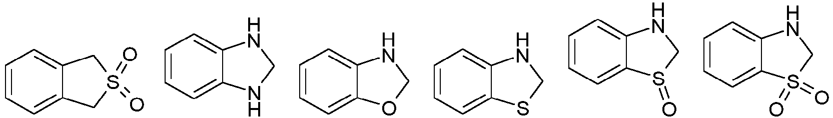

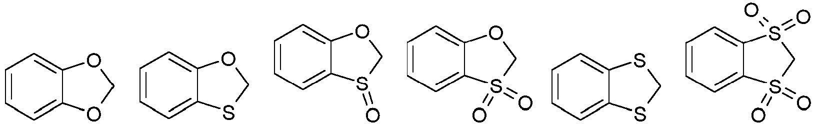

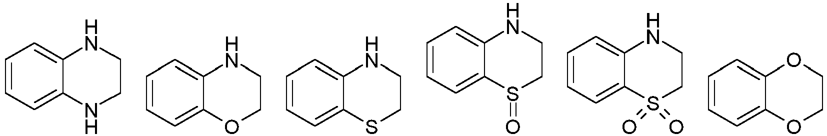

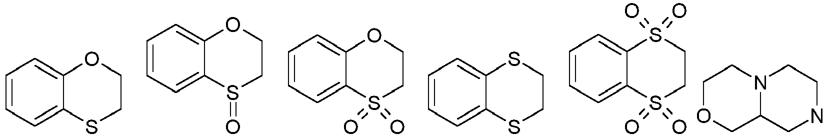

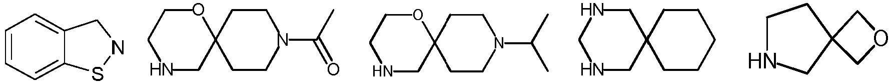

In particular, the term "heterocyclyl" includes the ring systems found in the exemplary compounds 1 to 175 and 1001-1178.

The term "heteroaryl" means a monocyclic-aromatic ring system containing one or more heteroatoms selected from N, O or S consisting of 5 to 6 ring atoms wherein at least one of the heteroatoms is part of the aromatic ring. The term "heteroaryl" is intended to include all the possible isomeric forms.

Thus, the term "heteroaryl" includes the following exemplary structures which are not depicted as radicals as each form is optionally attached through a covalent bond to any atom so long as appropriate valences are maintained:

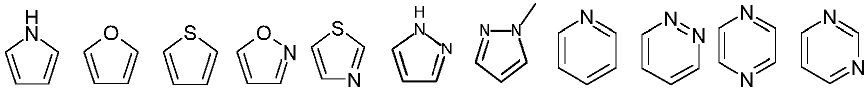

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salt" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methylbenzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, N-methyl-D-glucamine , potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention (e.g. trifluoro acetate salts) also comprise a part of the invention.

Many of the terms given above may be used repeatedly in the definition of a formula or group and in each case have one of the meanings given above, independently of one another.

A compound of the present invention or a salt thereof is described by formula (I) 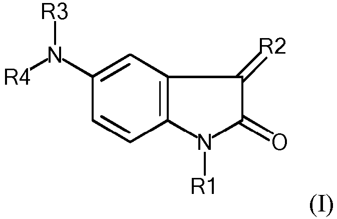 wherein:
**R1** is C₁₋₄-alkyl unsubstituted or substituted with MeO;
   unsubstituted tetrahydropyranyl, tetrahydrofuranyl, oxetanyl, dioxepanyl, pyrrolidinyl or piperidinyl; or
   pyrrolidinyl or piperidinyl with the nitrogen substituted by methyl, isopropyl, oxetanyl, ethoxcarbonyl, acetyl, or trifluoroacetyl;
**R2** is a 5-, 6- or 7-membered unsubstituted or substituted ring containing 1 or 2 heteroatoms selected from O or N, or an unsubstituted or substituted spirocyclic heterocyclyl group containing 1 to 3 heteroatoms selected from N or O consisting of 4 to 11 ring atoms, the ring or spirocyclic heterocyclyl group being bound in formula (I) by a C=C double bond, and
   in which ring or spirocyclic heterocyclyl group one N-atom can be substituted by methyl, isopropyl, acetyl, benzyloxycarbonyl, phenyl, oxetanyl or tetrahydropyranyl, and
   in which one or more C-atoms can be substituted by -methyl or -OH;
**R3** or **R4** are independently from one another
   hydrogen; C₁₋₆-alkyl, unsubstituted or substituted with one or more F, methoxy, C₃₋₈-cycloalkyl unsubstituted or substituted with one or more F; aryl; heteroaryl consisting of 5 to 6 ring atoms such as unsubstituted or substituted 5-pyrazolyl, or heterocyclyl consisting of 3 to 6 ring atoms, selected from the group consisting of oxetanyl, tetrahydropyranyl and pyrrolidinyl, said heterocyclyl being unsubstituted or substituted with C₁₋₆-alkyl, acetyl, tetrahydrofuranyl, oxetanyl; or hydroxyethylacetyl;
**or R3** and **R4** together with the attached N form a heterocyclyl ring selected from the group
   consisting of morpholinyl and pyrrolidinyl, both unsubstituted or substituted with C₁₋₆-alkyl, F, or hydroxyl.

Representative embodiments of **R1**in formula (I) are 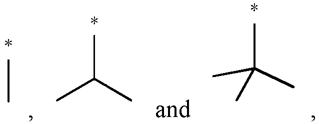 unsubstituted or substituted with MeO; 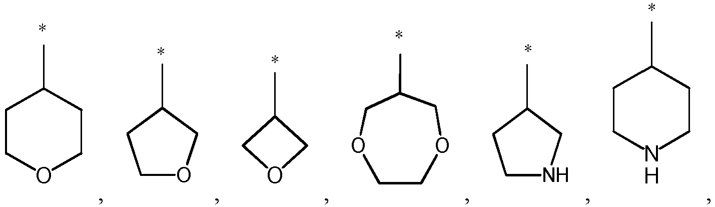 unsubstituted or the nitrogen substituted with

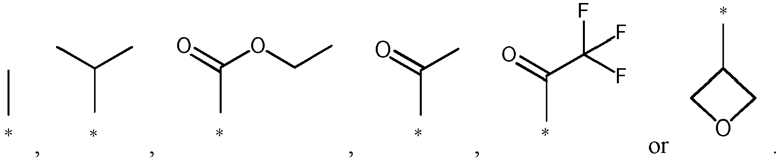

Representative embodiments of the structural element 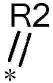 in formula (I) are

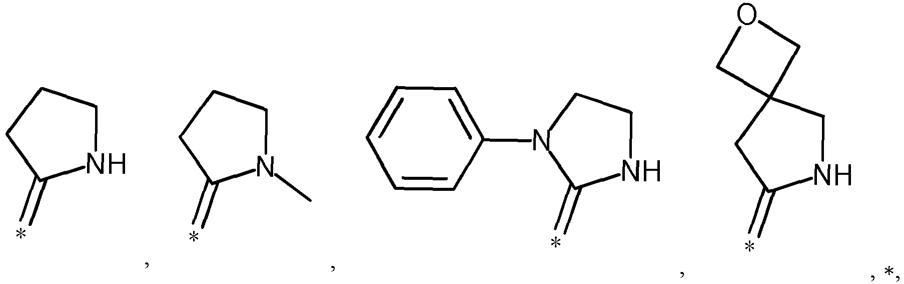

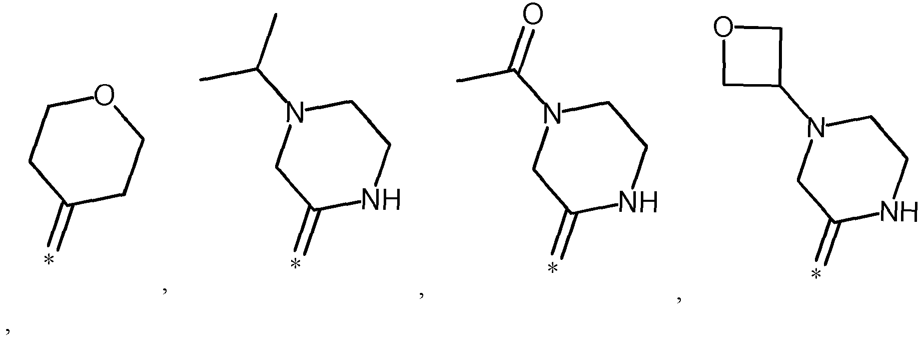

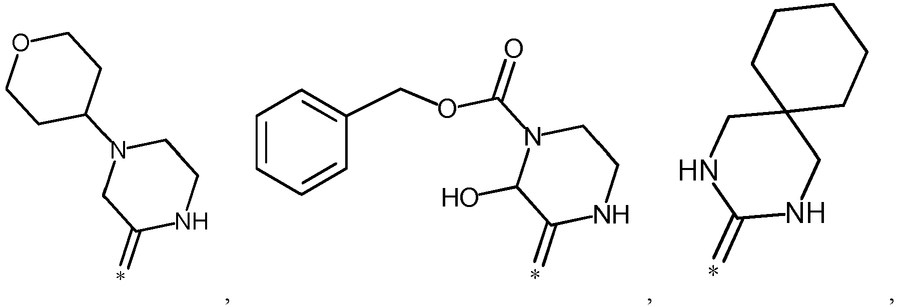

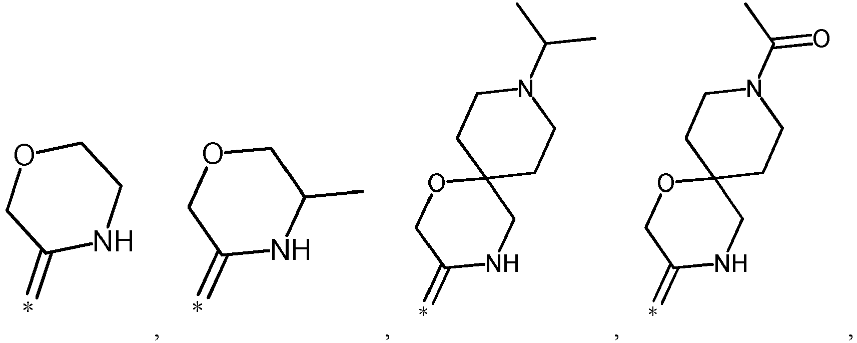

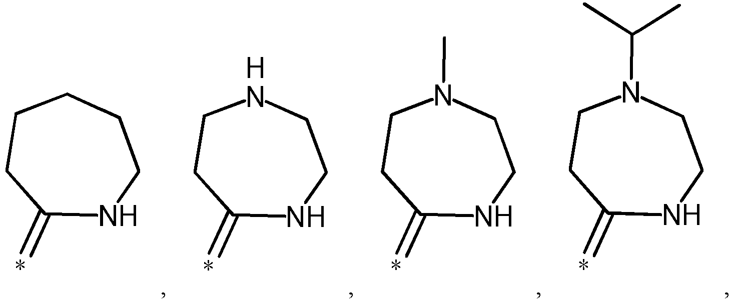

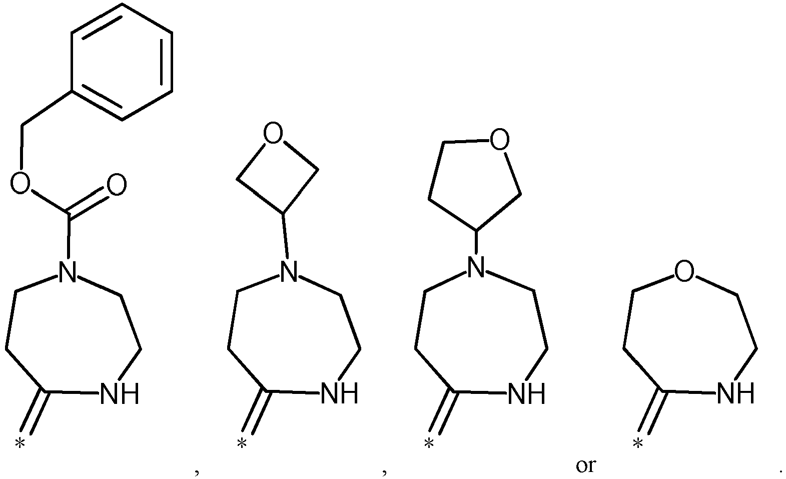

Representative embodiments of the amino group containing **R3** and **R4** in formula (I) are

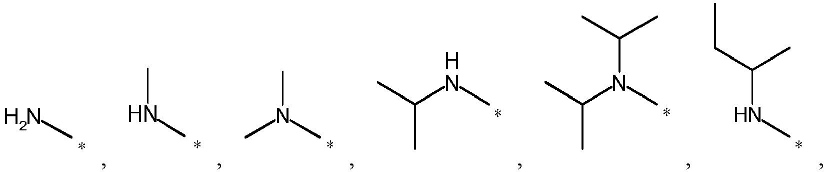

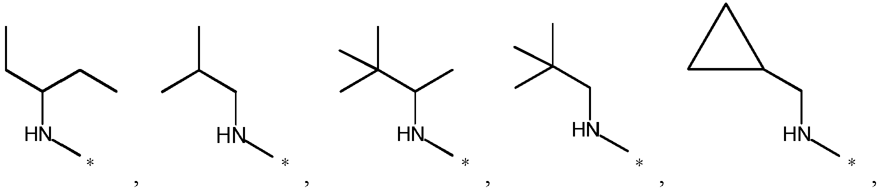

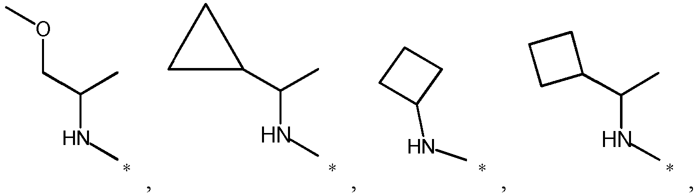

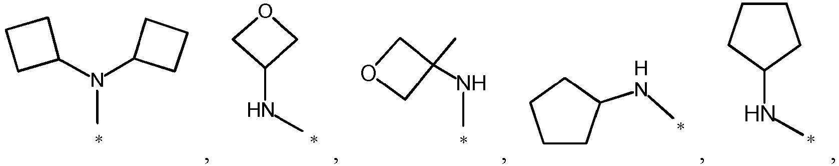

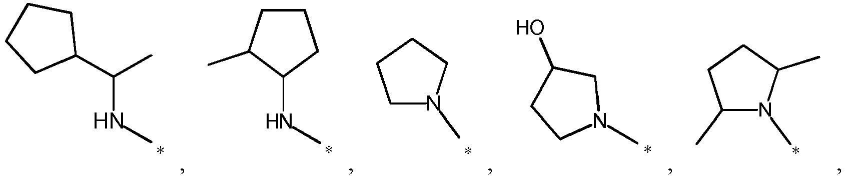

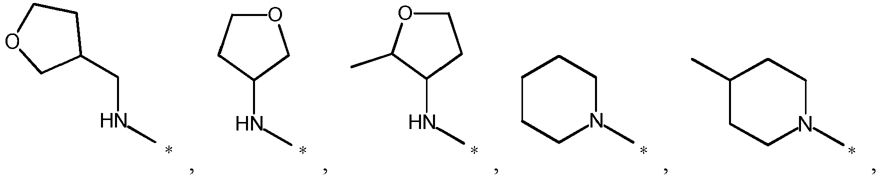

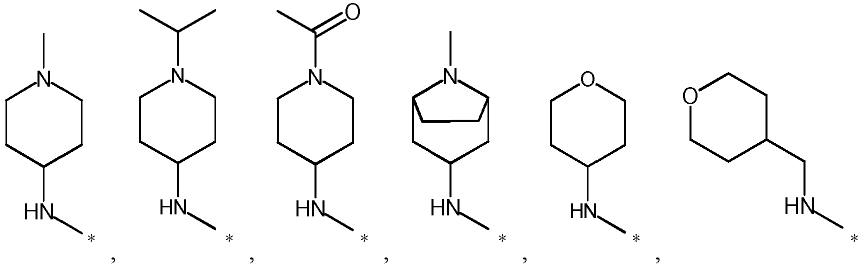

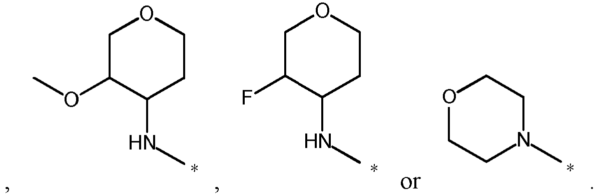

Individual embodiments of **R1** in formula (I) are

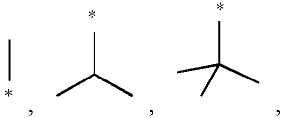

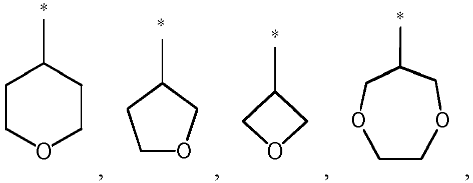

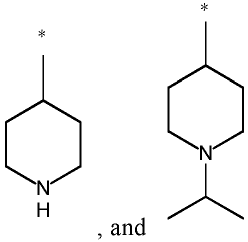

Preferred embodiments of **R1** in formula (I) are tetrahydropyranyl and dioxepanyl.

An individual embodiment of the structural element 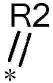 bound by a C=C double bond in formula (I) is a 6- or 7-membered ring containing 2 heteroatoms selected from O or N in which one or both N-atoms can be substituted by methyl, isopropyl, acetyl, benzyloxycarbonyl, phenyl, oxetanyl or tetrahydropyranyl, and in which one or more C-atoms can be substituted by -methyl or -OH

Other individual embodiments of 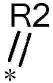 in formula (I) are

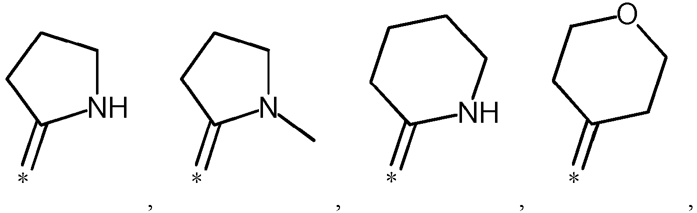

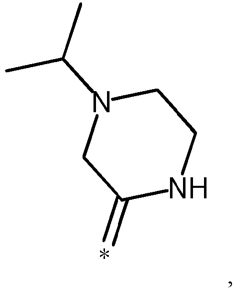

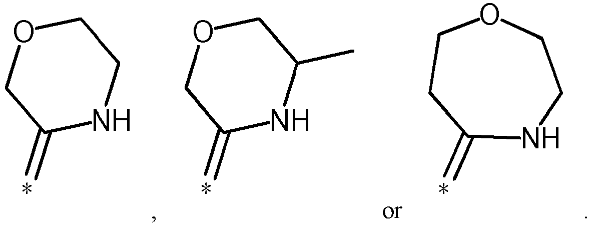

Individual embodiments of the amino group containing **R3** and **R4** in formula (I) encompass hydrogen as **R3** while **R4** is iso-propyl, cyclobutyl or cyclopentyl unsubstituted or substituted with fluorine (F).

Other individual embodiments of the amino group containing **R3** and **R4** in formula (I) are 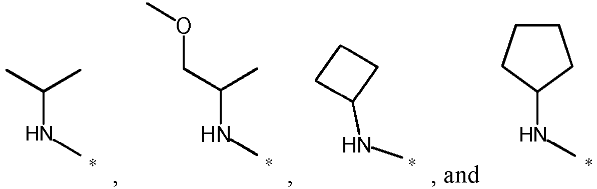 unsubstituted or substituted with one or more F.

Compounds according to the invention can be prepared with a method, wherein in a first step the compound (2-Fluoro-5-nitro-phenyl)-acetic acid (compound II) 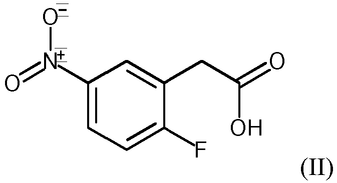 is reacted with an amine R1-NH₂ using an appropriate solvent like dimethyl acetamide, dimethyl formamide, N-methyl-pyrrolidinone, acetonitrile, DMSO, dichloromethane, toluene or the like at elevated temperature to form the compound 5-nitro-2,3-dihydro-1H-indol-2-one **(compound III)**

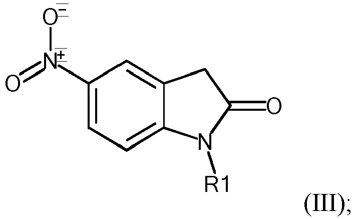

In the next step the 5-nitro-2,3-dihydro-1H-indol-2-one **(compound III)** is condensed at elevated temperatures in a microwave either without solvent or in a suitable solvent like piperidine with electrophiles suitable to result in a 5-nitro-3-ylidene-2,3-dihydro-1H-indol-2-one **(compound IV)**

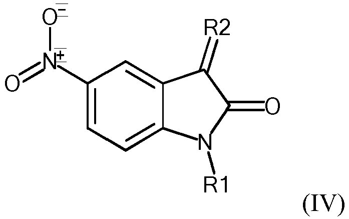

Suitable electrophiles can be iminoethers, ketones and acetals, e.g. 5-methoxy-3,6-dihydro-2H-oxazine, oxan-4-one, or 2,2-dimethoxy-1-methyl-pyrrolidine which are either commercially available or easily prepared from commercially available materials by those skilled in the art. E.g., iminoethers can be prepared from suitable amides via O-methylation with trimethyloxoniumtetrafluoroborate in a suitable solvent like methylene chloride.

In the next step the compound 5-nitro-3-ylidene-2,3-dihydro-1H-indol-2-one **(compound IV)** is reduced to a 5-amino-3-ylidene-2,3-dihydro-1H-indol-2-one **(compound V)**

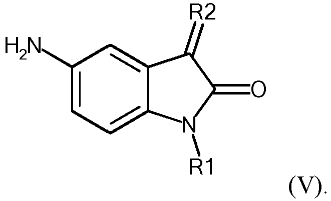

Said reductions can be achieved by catalytic hydrogenation using hydrogen gas under high pressure and a suitable catalyst like Raney-nickel in a suitable solvent like methanol.

To obtain a final compound of formula **(I)**, a compound of formula **(V)** can be reacted with suitable aldehydes or ketones and a reducing agent like sodium cyanaborohydride, sodium triacetoxyborohydride or the like in a suitable solvent like methanol with the addition of an organic acid like acetic acid, pTosOH or the like.

Alternatively an amine (V) may be reacted in a substitution reaction with suitable electrophiles carrying a leaving group like Cl-, Br-. I-, methylsulfonylester, trifluorosulfonylester, tolylsulfonylester or the like in a suitable solvent such as DMF or the like and in the presence of a suitable base such as potassium carbonate.

Of the above compounds it is possible to prepare salt forms which are also subject matter of the present invention, particularly pharmaceutically acceptable salts. Medicaments prepared thereof are another subject matter of the present invention.

A compound according to the invention can be used in a medicament or pharmaceutical composition for a human patient. Such a composition can be practiced on a human body to therapeutically treat or diagnose a disease.

Similarly, a compound according to the invention can be used in a medicament or pharmaceutical composition for an animal. Such a composition can be practiced on an animal body to therapeutically treat or diagnose an animal's disease.

In particular compounds of the present invention can be used for the manufacture of a pharmaceutical composition or medicament for the treatment or prevention of a condition mentioned below in a human being.

Suitable preparations for administering the compounds of formula 1 are apparent to those with ordinary skill in the art and include for example tablets, pills, capsules, suppositories, lozenges, troches, solutions, syrups, elixirs, sachets, injectables, inhalatives and powders etc. Suitable tablets are obtained, for example, by mixing one or more compounds according to formula I with known excipients, for example inert diluents, carriers, disintegrants, adjuvants, surfactants, binders and/or lubricants.

Such a pharmaceutical composition or medicament comprises a compound or pharmaceutically acceptable salt thereof according to the present invention in a therapeutically effective amount of 0.1 to 2000 mg.

In addition to a compound according to the present invention, such a medicament comprises a pharmaceutically acceptable carrier.

The present invention is directed to compounds useful in the treatment of a disease, disorder and condition wherein the inhibition of oxidative stress, as the lowering of ROS generated by Complex I is of therapeutic benefit. This includes but is not limited to the treatment and/or prevention of neurological or neurodegenerative or psychiatric conditions, and non-neuronal conditions such as cardiovascular diseases, ischemia-reperfusion injury, cancer and pulmonary and mitochondrial diseases.

**Neurological or neurodegenerative conditions** include e.g. Parkinson's disease, Alzheimer's disease (AD), Huntington's disease, amyotrophic lateral sclerosis (ALS), diseases involving retinal dysfunction like Retinopathy and age-related macular degeneration (AMD) and other brain disorders caused by trauma or other insults including aging.

**Mitochondrial diseases include** e.g. Leber's hereditary optic neuropathy (LHON), Leigh Syndrome, Myoclonic Epilepsy with Ragged Red Fibers (MERRF) , Mitochondrial myopathy, encephalomyopathy, lactic acidosis, stroke-like symptoms (MELAS) or Diabetes mellitus and deafness (DAD)
**Psychiatric conditions** include depressive disorders like major depression, major depressive disorder, psychiatric depression, dysthymia, and postpartum depression, and bipolar disorders), and fear-related disorders (e.g. post-traumatic stress disorder, panic disorder, agoraphobia, social phobias, generalized anxiety disorder, panic disorder, social anxiety disorder, obsessive compulsive disorder, and separation anxiety), chronic fatigue syndrome and Autism
**Pain disorders** include nociceptive pain, inflammatory pain, cancer pain, and neuropathic pain (e.g. cancer pain, osteoarthritic pain, rheumatoid arthritis pain, post-herpetic neuralgia, pain due to burns, and other indications). The pain can be chronic or acute.

**Non-neuronal conditions** include pulmonary diseases like chronic obstructive pulmonary disease (COPD), idiopathic pulmonary fibrosis (IPF) and other fibrotic diseases, nephropathy, proteinuric kidney disease, liver diseases such as hepatic dyslipidemia associated with cholestasis, non-alcoholic steatohepatitis (NASH), non-alcoholic fatty liver disease (NAFLD), itch, disorders associated with malfunction of the cardiovascular system or vascular permeability, e.g. heart failure, pulmonary arterial hypertension, acute respiratory distress syndrome (ARDS), maladaptive cardiac remodeling, disorders associated with maladaptive blood pressure control like hypertension or hypotension, infectious diseases like hepatitis and protozoal infections (including malaria, African sleeping sickness and Chagas disease), sarcopenia and other skeletal muscle diseases, disorders and other medical conditions such as diabetes, insulin resistance, metabolic syndrome and obesity.

The applicable daily dose of compounds of the present invention may vary from 0.1 to 2000 mg.

The actual pharmaceutically effective amount or therapeutic dose depends on factors known by those skilled in the art such as age and weight of the patient, route of administration and severity of disease. In any case, the drug substance is to be administered at a dose and in a manner which allows a pharmaceutically effective amount to be delivered that is appropriate to the patient's condition.

Suitable compositions for administering the compounds of the present invention can be produced by those with ordinary skill in the art and include for example tablets, pills, capsules, suppositories, lozenges, troches, solutions, syrups, elixirs, sachets, injectables, inhalatives, and powders. The content of a pharmaceutically active compounds may vary in the range from 0.1 to 95 wt.-%, preferably 5.0 to 90 wt.-% of the composition as a whole.

Suitable tablets can be obtained by mixing a compound of the present invention with known excipients, for example inert diluents, carriers, disintegrants, adjuvants, surfactants, binders and/or lubricants and compressing the resulting mixture to tablets.

The compounds of the present invention can be used as single active pharmaceutical ingredient or in combination with other active pharmaceutical ingredients known to be used in the art in connection with a treatment of any of the indications the treatment of which is in the focus of the present invention.

The compounds of the following table and their features are listed to illustrate the present invention, not to define its scope.

| **#** | **Formula** | **CI IC₅₀ / [µM]** | **HT22 IC₅₀ / [µM]** |
|---|---|---|---|
| 1 | 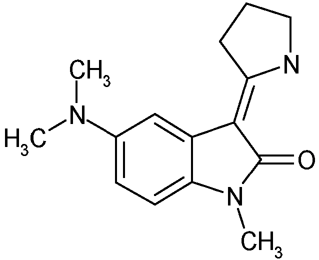 | 0.060 | 0.282 |
| 2 | 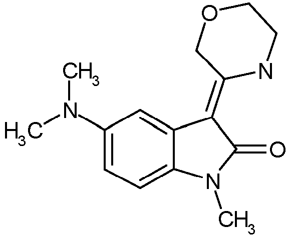 | 0.080 | 1.054 |
| 3 | 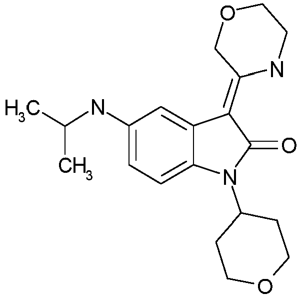 | 0.077 | 0.071 |
| 4 | 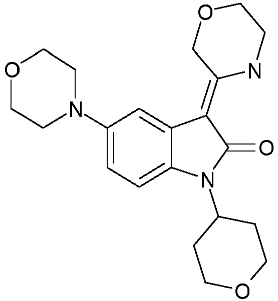 | 0.242 | 3.140 |
| 5 | 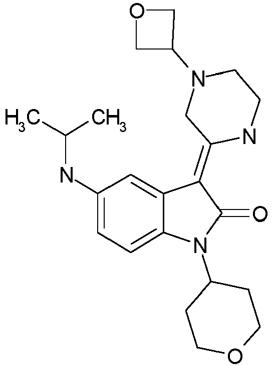 | 0.081 | 0.220 |
| 6 | 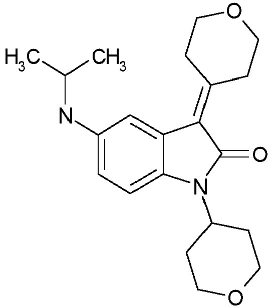 | 0.533 | 0.207 |
| 7 | 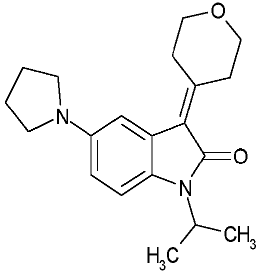 | 0.350 | 0.256 |
| 8 | 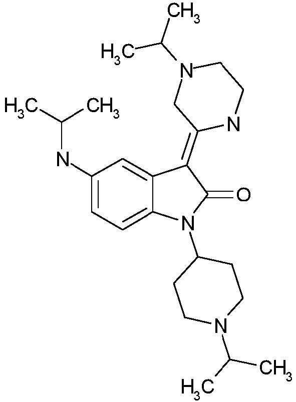 | 0.062 | 0.041 |
| 9 | 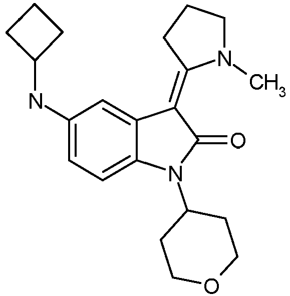 | 0.398 | 0.038 |
| 10 | 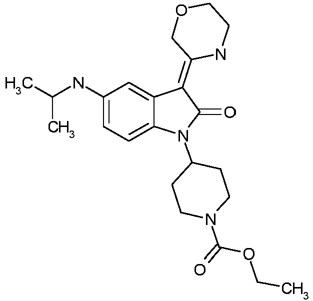 | 0.078 | 0.031 |
| 11 | 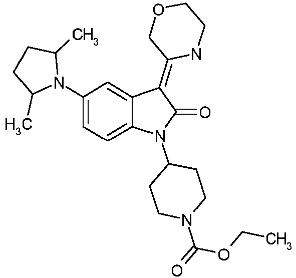 | 0.031 | 0.038 |
| 12 | 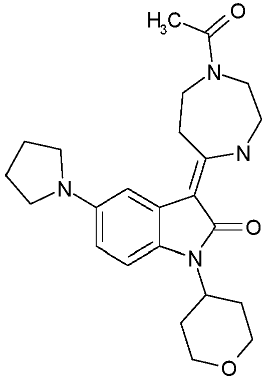 | 0.194 | 0.191 |
| 13 | 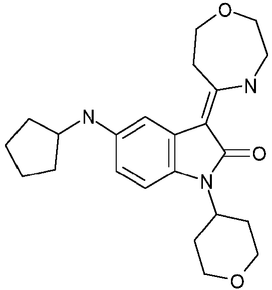 | 0.084 | 0.014 |
| 14 | 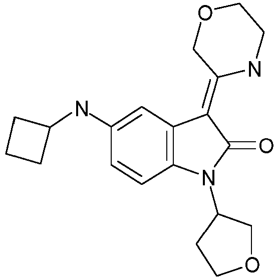 | 0.101 | 0.020 |
| 15 | 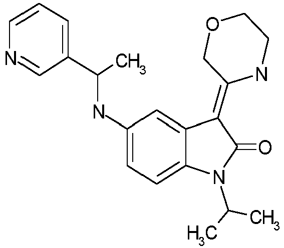 | 0.096 | 0.115 |
| 16 | 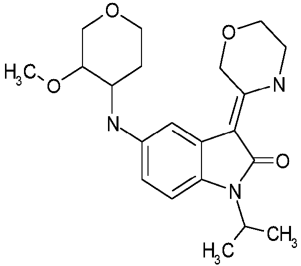 | 0.313 | 0.693 |
| 17 | 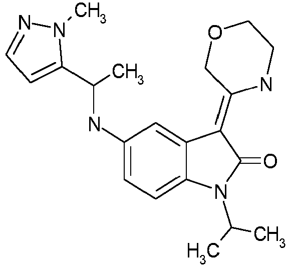 | 0.022 | 0.159 |
| 18 | 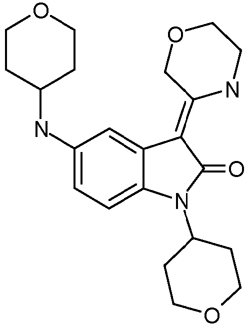 | 0.011 | 0.409 |
| 19 | 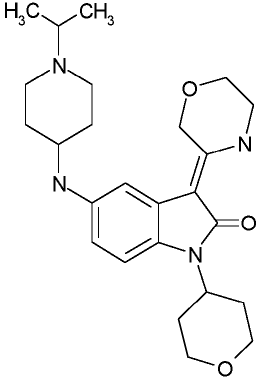 | 0.022 | 0.168 |
| 20 | 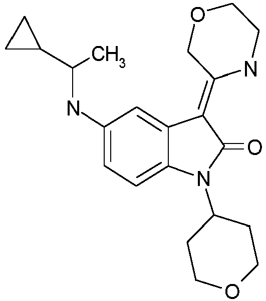 | 0.073 | 0.034 |
| 21 | 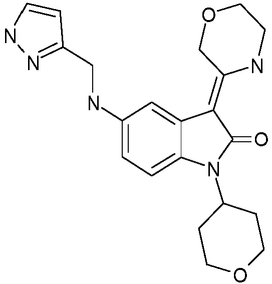 | 0.108 | 0.253 |
| 22 | 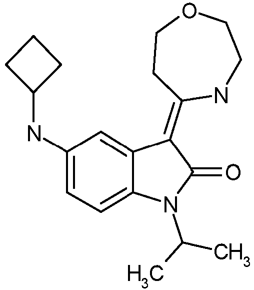 | 0.087 | 0.011 |
| 23 | 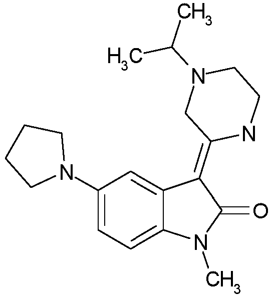 | 0.137 | 0.173 |
| 24 | 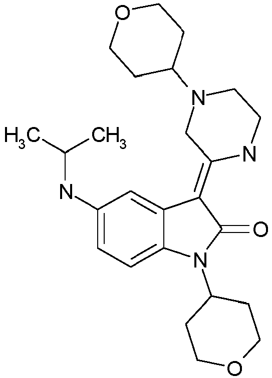 | 0.048 | 0.123 |
| 25 | 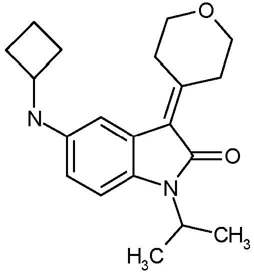 | 0.707 | 0.096 |
| 26 | 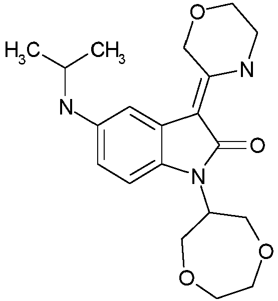 | 0.020 | 0.059 |
| 27 | 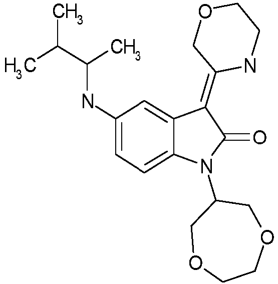 | 0.162 | 0.015 |
| 28 | 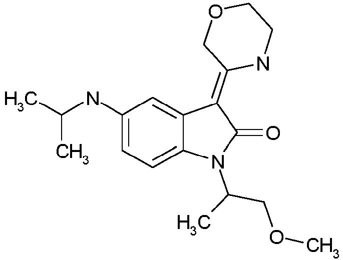 | 0.069 | 0.118 |
| 29 | 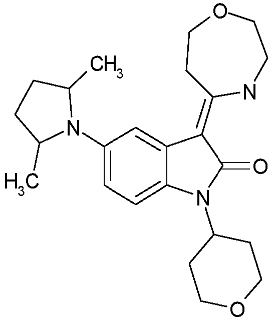 | 0.132 | 0.142 |
| 30 | 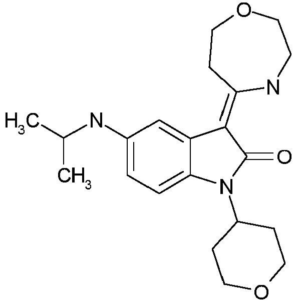 | 0.195 | 0.135 |
| 31 | 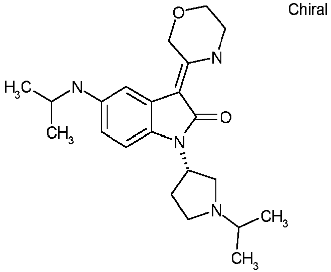 | 0.081 | 0.041 |
| 32 | 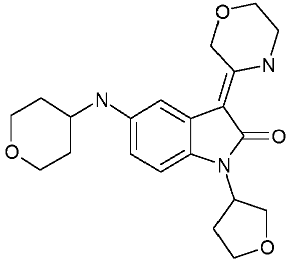 | 0.031 | 0.205 |
| 33 | 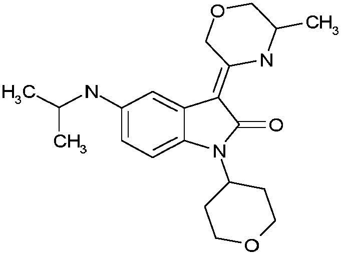 | 0.032 | 0.037 |
| 34 | 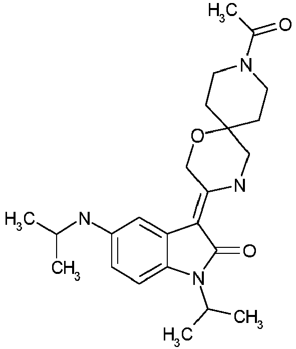 | 0.048 | 0.049 |
| 35 | 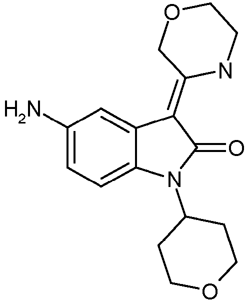 | 0.083 | 2.723 |
| 36 | 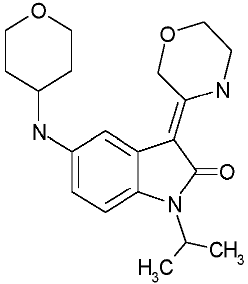 | 0.150 | 0.056 |
| 37 | 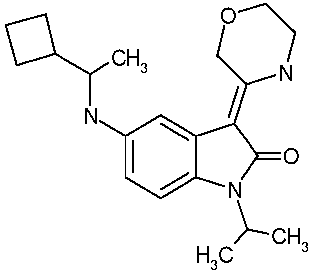 | 0.405 | 0.003 |
| 38 | 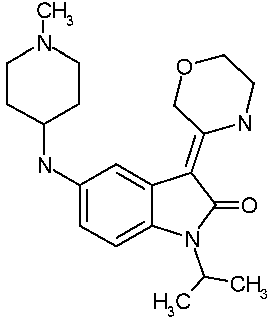 | 0.051 | 0.038 |
| 39 | 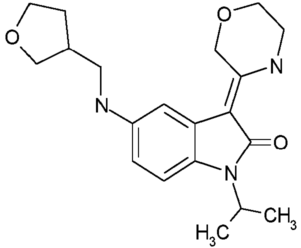 | 0.067 | 0.102 |
| 40 | 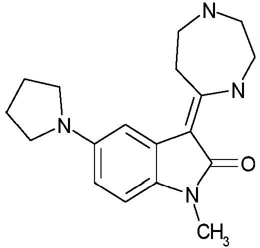 | 0.166 | 0.013 |
| 41 | 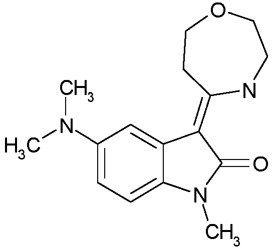 | 0.052 | 0.983 |
| 42 | 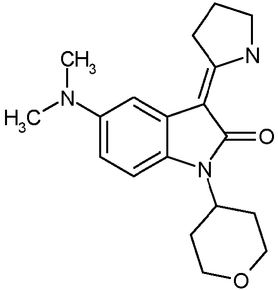 | 0.042 | 0.951 |
| 43 | 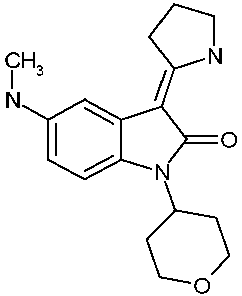 | 0.011 | 0.124 |
| 44 | 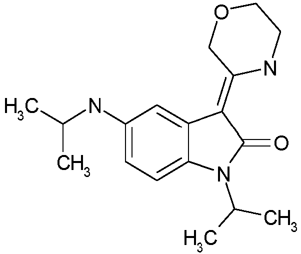 | 0.044 | 0.030 |
| 45 | 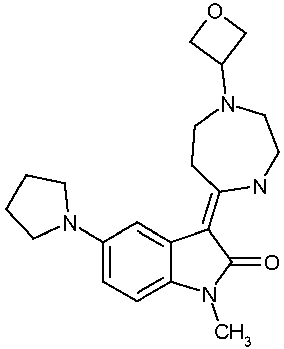 | 0.259 | 0.261 |
| 46 | 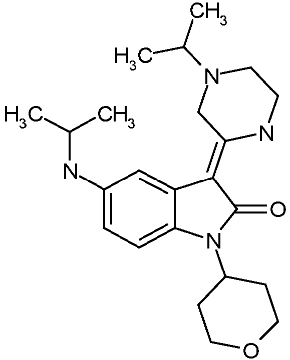 | 0.086 | 0.048 |
| 47 | 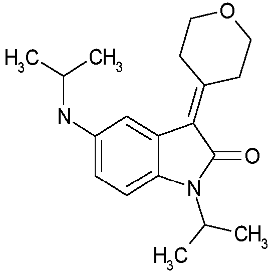 | 0.153 | 0.085 |
| 48 | 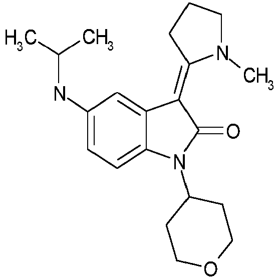 | 0.122 | 0.353 |
| 49 | 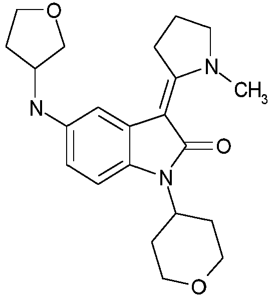 | 0.282 | 1.254 |
| 50 | 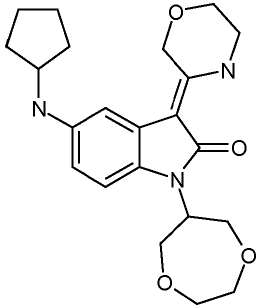 | 0.195 | 0.013 |
| 51 | 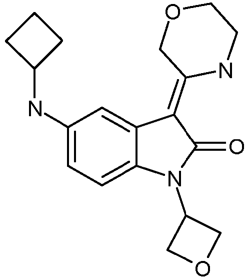 | 0.135 | 0.033 |
| 52 | 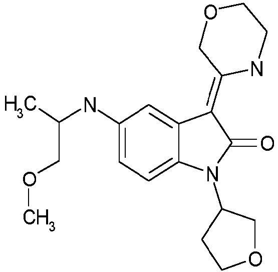 | 0.061 | 0.198 |
| 53 | 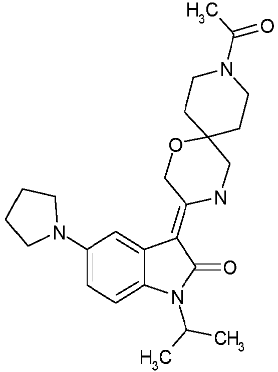 | 0.233 | 0.055 |
| 54 | 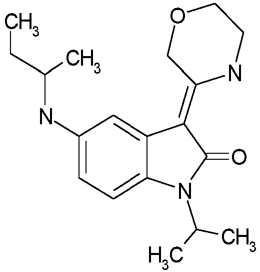 | 0.203 | 0.024 |
| 55 | 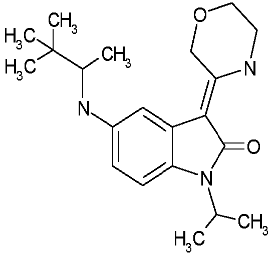 | 0.307 | 0.038 |
| 56 | 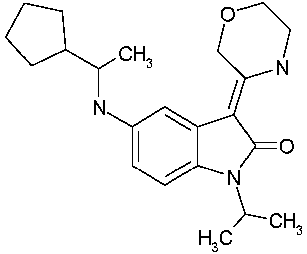 | 0.159 | 0.002 |
| 57 | 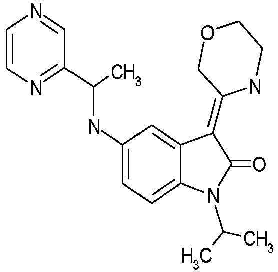 | 0.073 | 0.199 |
| 58 | 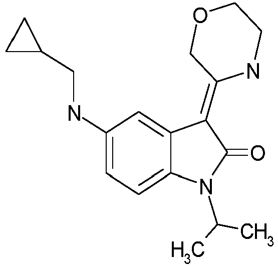 | 0.100 | 0.019 |
| 59 | 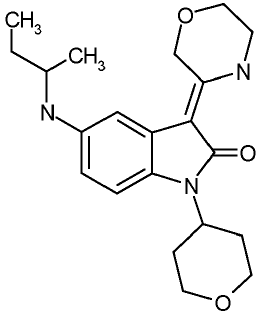 | 0.122 | 0.022 |
| 60 | 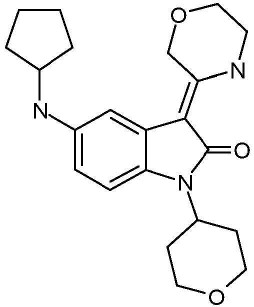 | 0.245 | 0.01 |
| 61 | 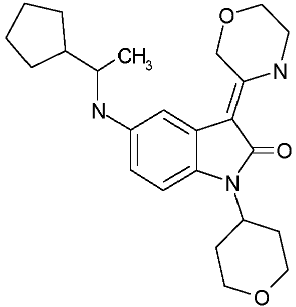 | 0.343 | 0.012 |
| 62 | 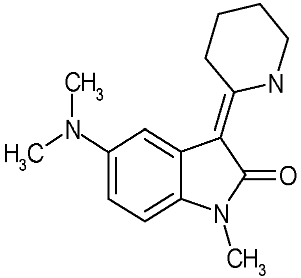 | 0.081 | 0.162 |
| 63 | 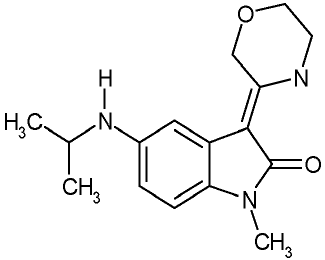 | 0.022 | 0.134 |
| 64 | 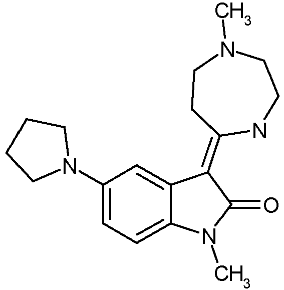 | 0.286 | 0.065 |
| 65 | 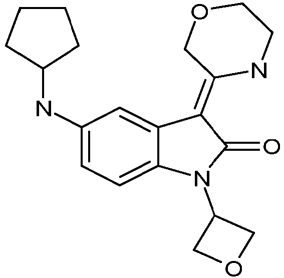 | 0.194 | 0.018 |
| 66 | 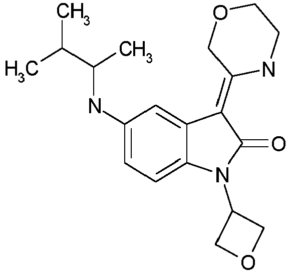 | 0.169 | 0.019 |
| 67 | 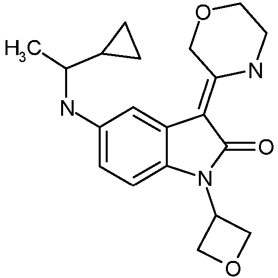 | 0.225 | 0.037 |
| 68 | 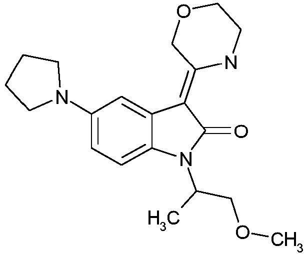 | 0.624 | 0.134 |
| 69 | 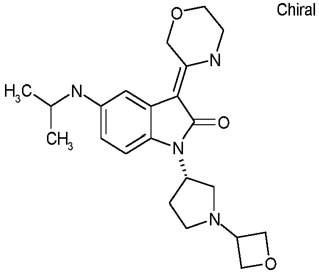 | 0.055 | 0.141 |
| 70 | 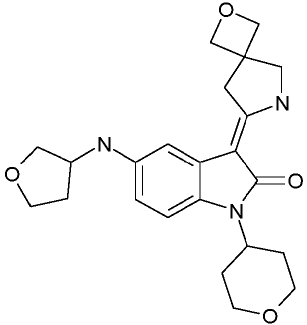 | 0.193 | 1.149 |
| 71 | 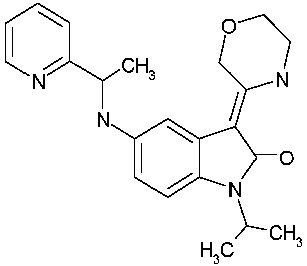 | 0.093 | 0.131 |
| 72 | 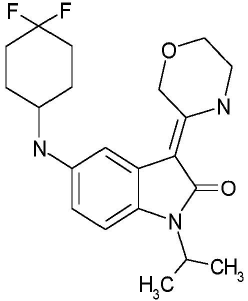 | 0.140 | 0.006 |
| 73 | 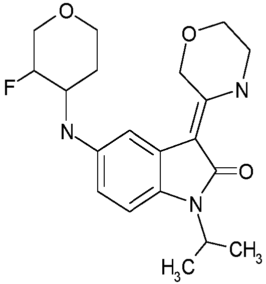 | 0.066 | 0.264 |
| 74 | 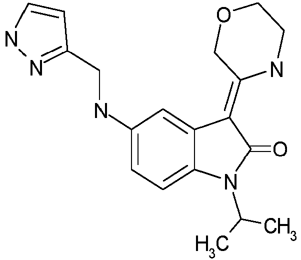 | 0.046 | 0.144 |
| 75 | 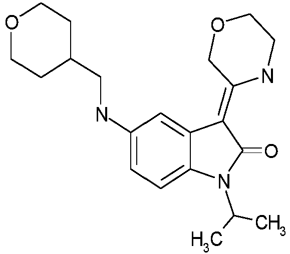 | 0.072 | 0.073 |
| 76 | 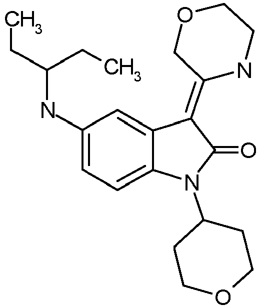 | 0.043 | 0.024 |
| 77 | 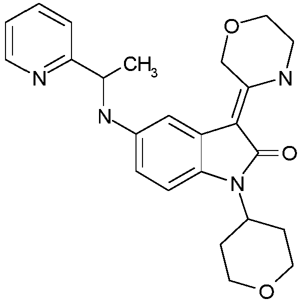 | 0.022 | 0.048 |
| 78 | 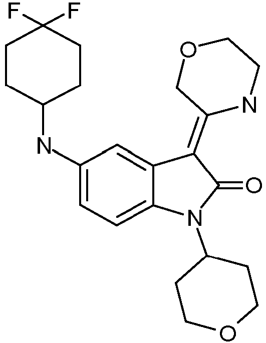 | 0.089 | 0.037 |
| 79 | 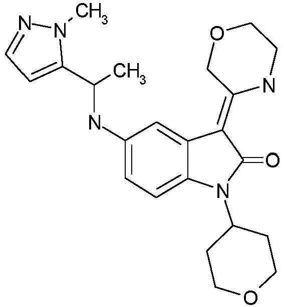 | 0.020 | 0.221 |
| 80 | 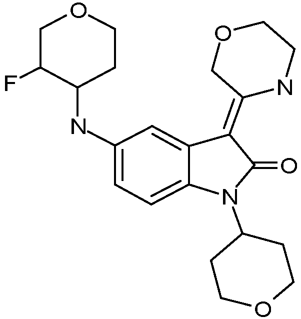 | 0.084 | 0.345 |
| 81 | 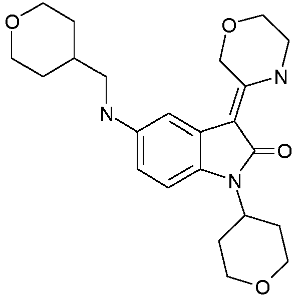 | 0.130 | 0.233 |
| 82 | 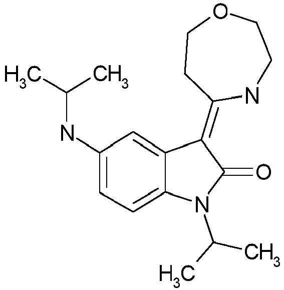 | 0.091 | 0.026 |
| 83 | 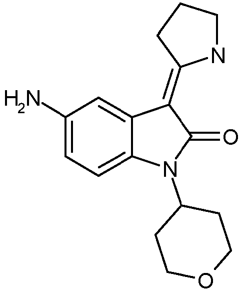 | 0.011 | 2.075 |
| 84 | 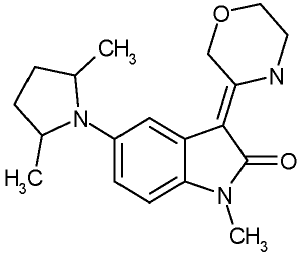 | 0.333 | 0.141 |
| 85 | 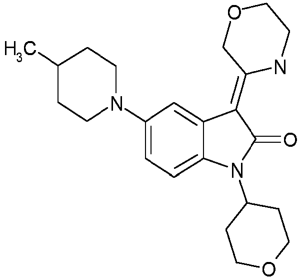 | 0.069 | 0.264 |
| 86 | 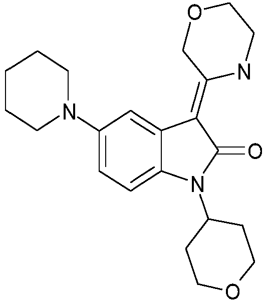 | 0.069 | 1.112 |
| 87 | 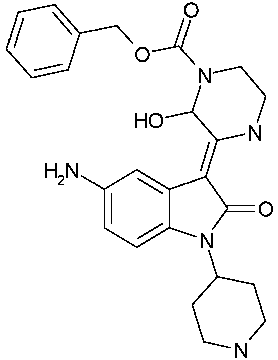 | 0.332 | 1.002 |
| 88 | 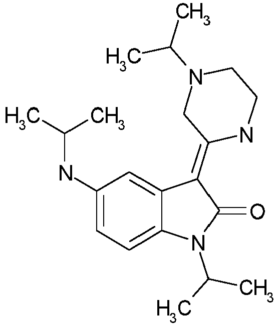 | 0.068 | 0.010 |
| 89 | 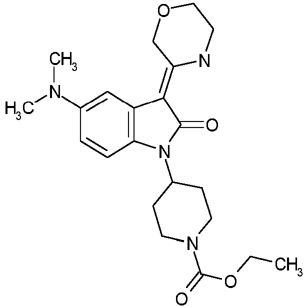 | 0.197 | 0.493 |
| 90 | 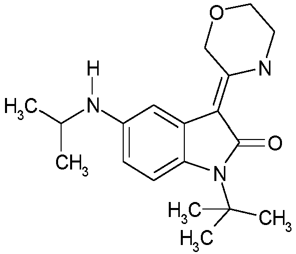 | 0.428 | 0.032 |
| 91 | 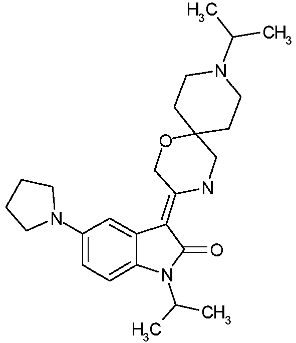 | 0.242 | 0.009 |
| 92 | 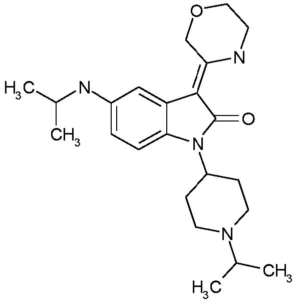 | 0.106 | 0.044 |
| 93 | 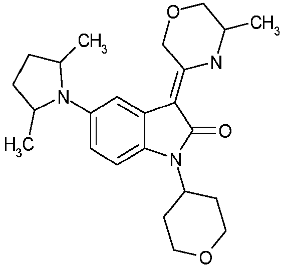 | 0.024 | 0.101 |
| 94 | 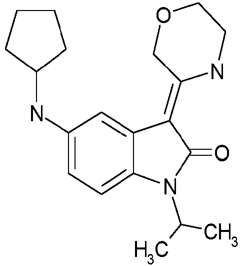 | 0.558 | 0.027 |
| 95 | 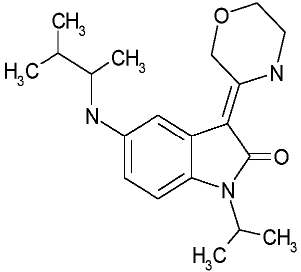 | 0.151 | 0.006 |
| 96 | 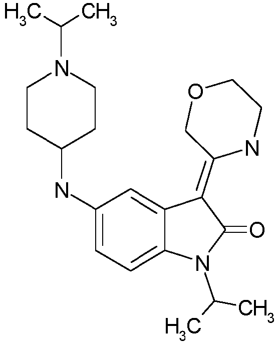 | 0.063 | 0.041 |
| 97 | 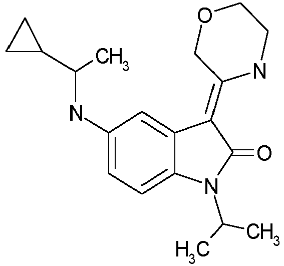 | 0.344 | 0.017 |
| 98 | 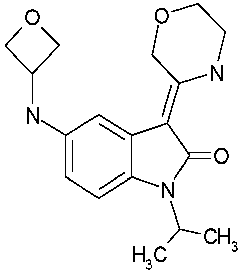 | 0.187 | 0.166 |
| 99 | 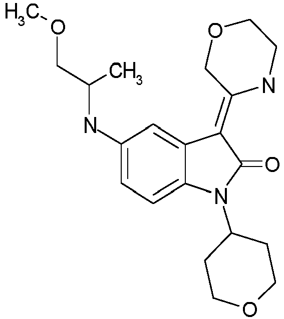 | 0.451 | 0.163 |
| 100 | 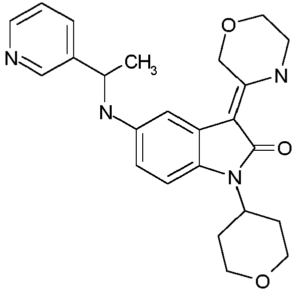 | 0.034 | 0.397 |
| 101 | 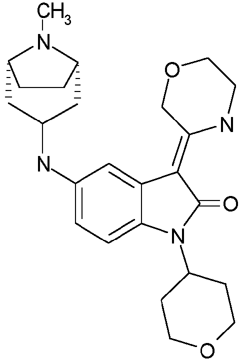 | 0.202 | 0.369 |
| 102 | 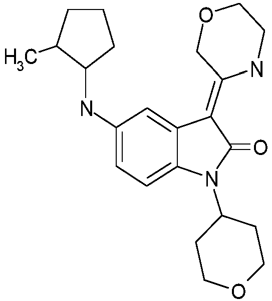 | 0.109 | 0.003 |
| 103 | 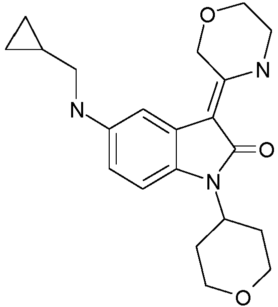 | 0.076 | 0.105 |
| 104 | 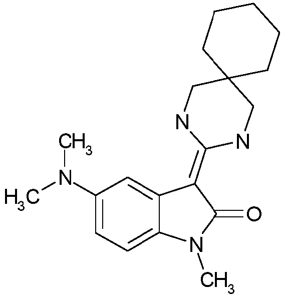 | 0.704 | |
| 105 | 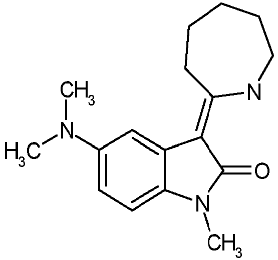 | 0.064 | 0.091 |
| 106 | 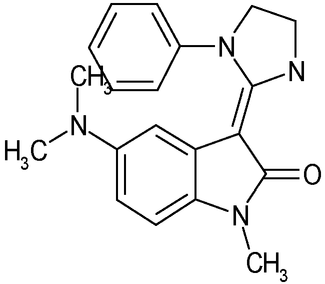 | 0.327 | 0.142 |
| 107 | 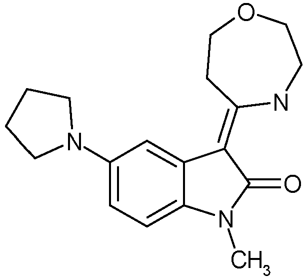 | 0.299 | 0.069 |
| 108 | 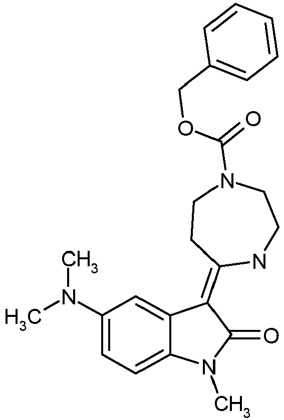 | 0.118 | 0.128 |
| 109 | 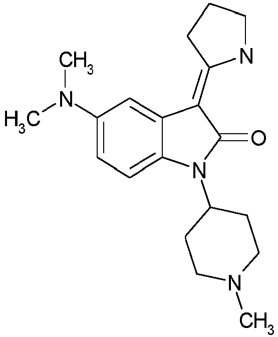 | 0.014 | 0.160 |
| 110 | 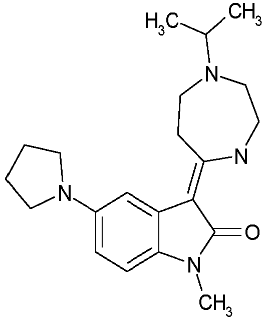 | 0.264 | 0.019 |
| 111 | 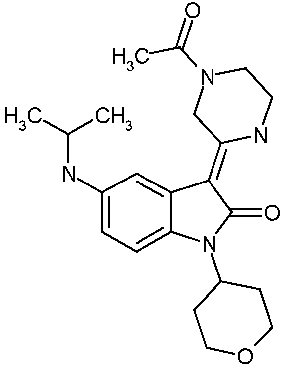 | 0.119 | 0.249 |
| 112 | 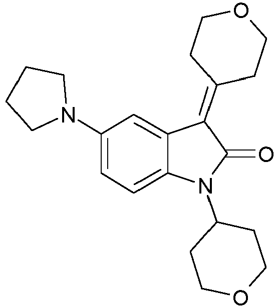 | 0.352 | 0.086 |
| 113 | 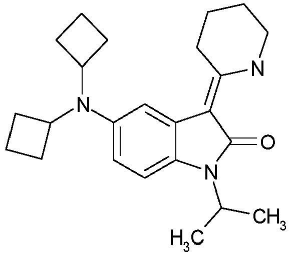 | 0.106 | 0.018 |
| 114 | 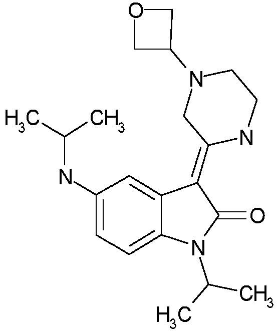 | 0.058 | 0.104 |
| 115 | 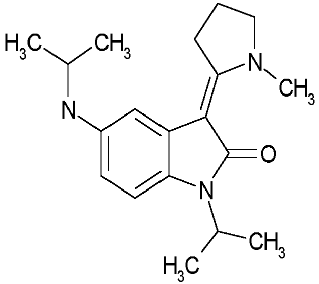 | 0.080 | 0.030 |
| 116 | 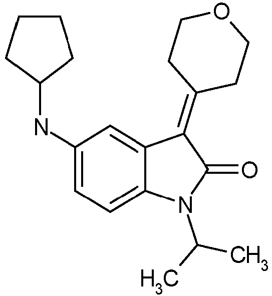 | 0.539 | 0.009 |
| 117 | 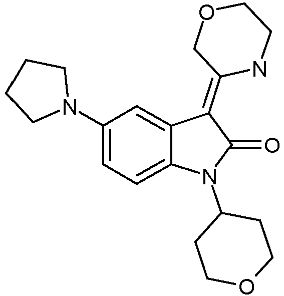 | 0.738 | 0.022 |
| 118 | 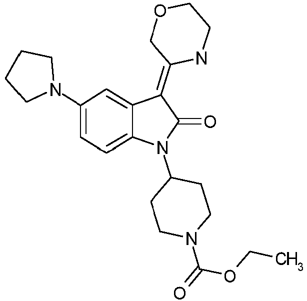 | 0.420 | 0.033 |
| 119 | 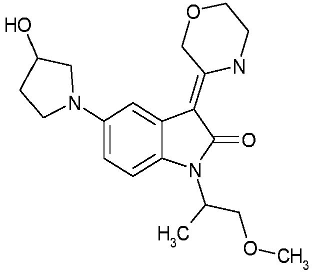 | 0.422 | 0.439 |
| 120 | 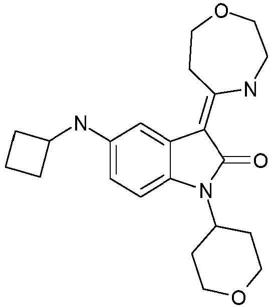 | 0.235 | 0.051 |
| 121 | 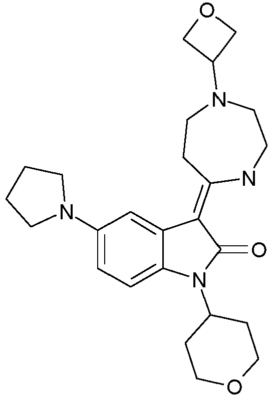 | 0.075 | 0.192 |
| 122 | 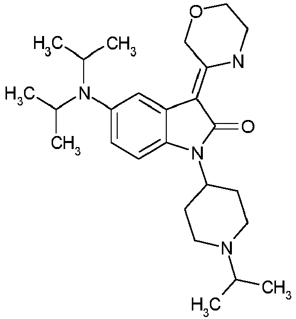 | 0.275 | 0.944 |
| 123 | 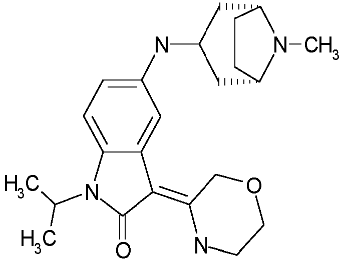 | 0.916 | 0.017 |
| 124 | 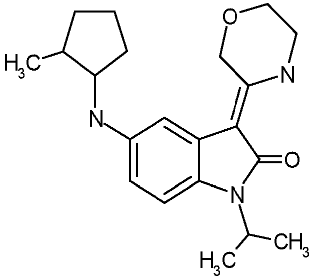 | 0.259 | 0.015 |
| 125 | 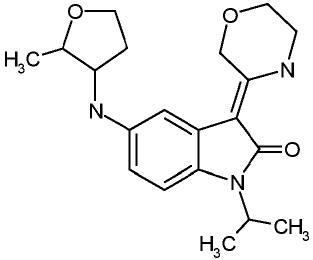 | 0.050 | 0.112 |
| 126 | 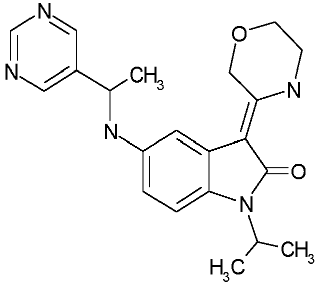 | 0.085 | 0.179 |
| 127 | 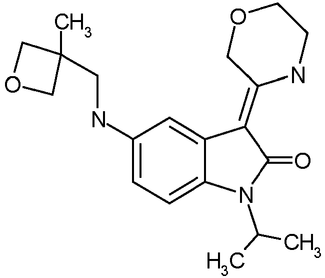 | 0.082 | 0.176 |
| 128 | 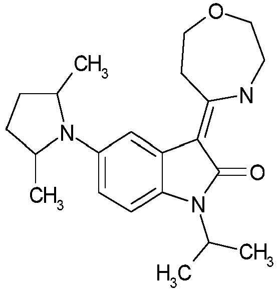 | 0.044 | 0.047 |
| 129 | 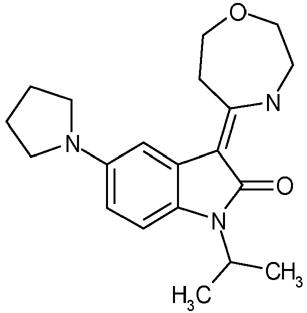 | 0.702 | 0.023 |
| 130 | 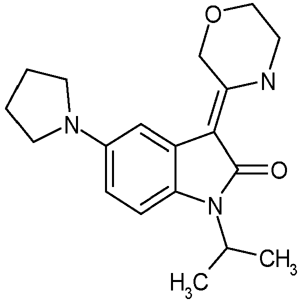 | 0.877 | 0.045 |
| 131 | 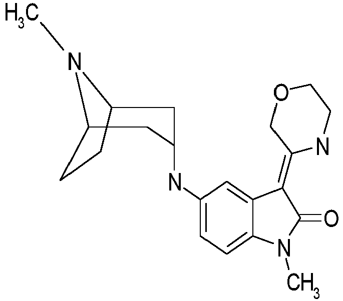 | 0.189 | 0.111 |
| 132 | 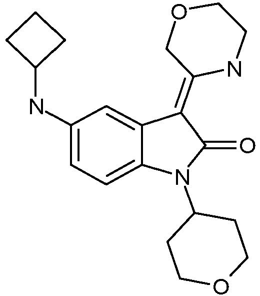 | 0.055 | 0.021 |
| 133 | 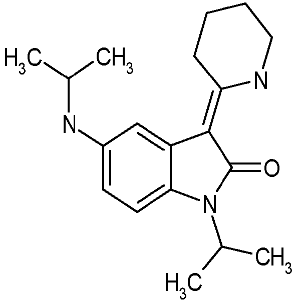 | 0.049 | 0.023 |
| 134 | 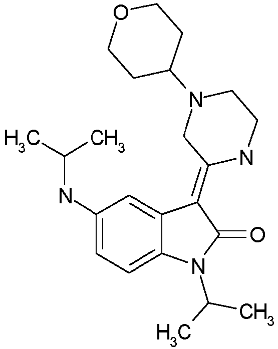 | 0.062 | 0.044 |
| 135 | 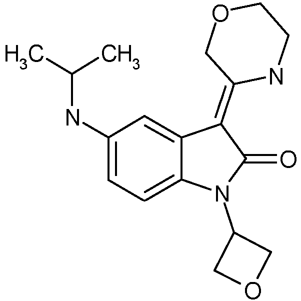 | 0.082 | 0.158 |
| 136 | 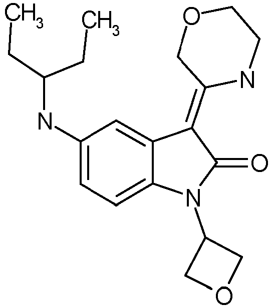 | 0.213 | 0.018 |
| 137 | 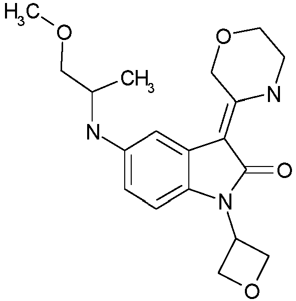 | 0.138 | 0.886 |
| 138 | 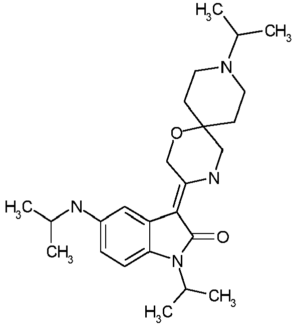 | 0.206 | 0.006 |
| 139 | 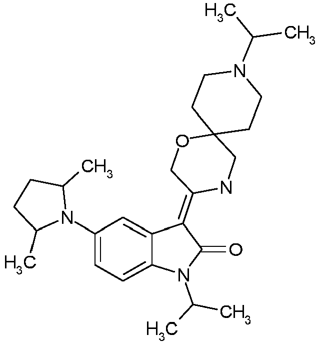 | 0.308 | 0.018 |
| 140 | 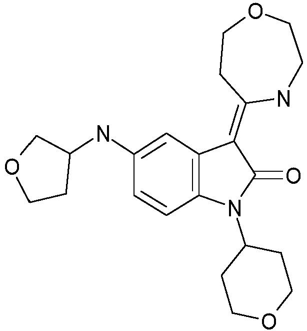 | 0.084 | 0.524 |
| 141 | 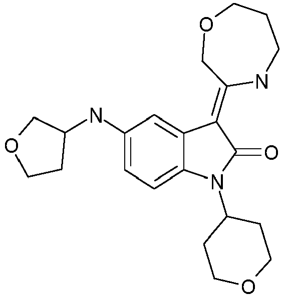 | 0.104 | 0.646 |
| 142 | 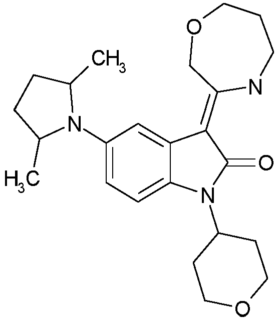 | 0.116 | 0.094 |
| 143 | 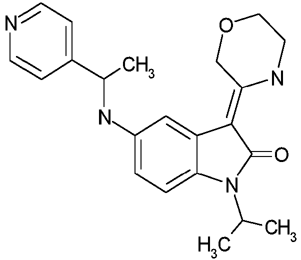 | 0.079 | 0.110 |
| 144 | 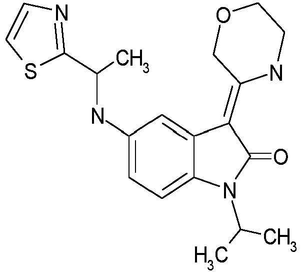 | 0.049 | 0.085 |
| 145 | 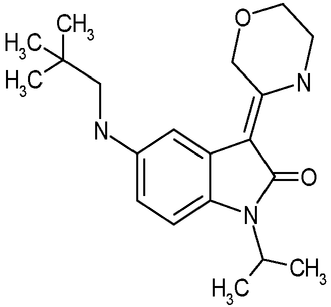 | 0.183 | 0.004 |
| 146 | 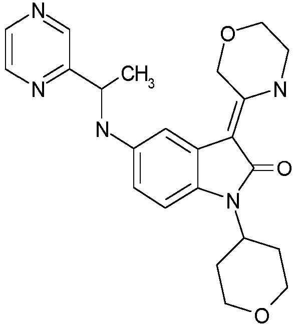 | 0.066 | 0.211 |
| 147 | 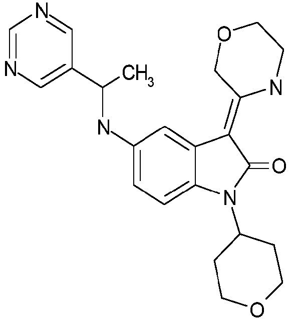 | 0.069 | 0.422 |
| 148 | 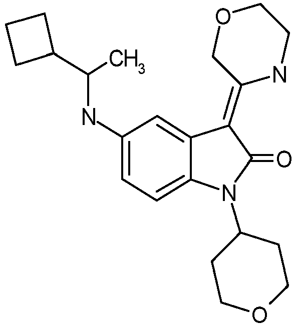 | 0.039 | 0.016 |
| 149 | 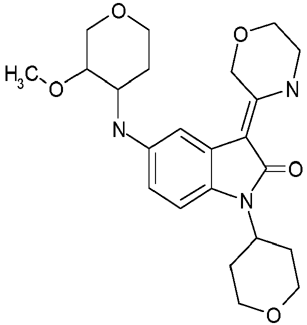 | 0.161 | 2.939 |
| 150 | 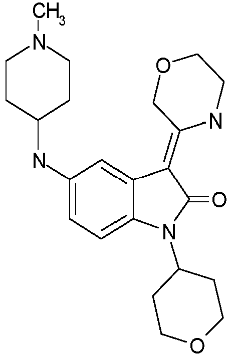 | 0.048 | 0.286 |
| 151 | 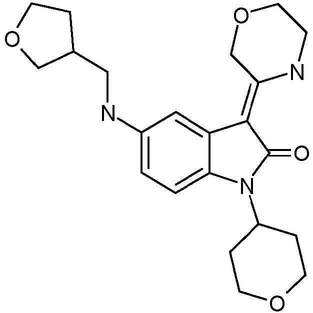 | 0.232 | 0.184 |
| 152 | 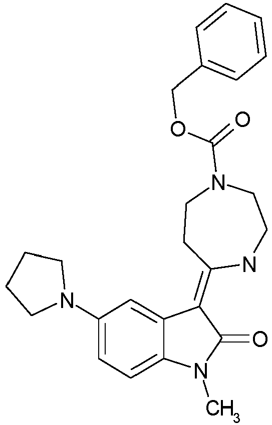 | 0.477 | 0.047 |
| 153 | 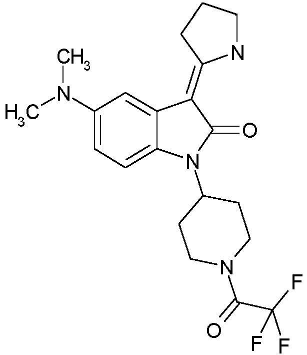 | 0.016 | 0.170 |
| 154 | 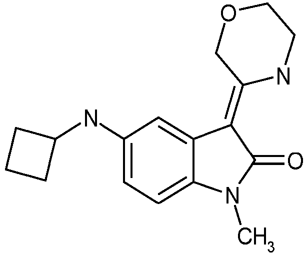 | 0.193 | 0.018 |
| 155 | 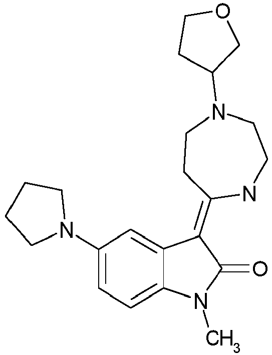 | 0.264 | 0.214 |
| 156 | 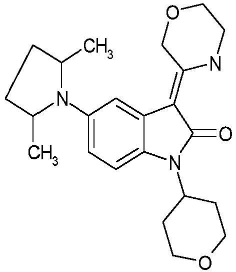 | 0.077 | 0.071 |
| 157 | 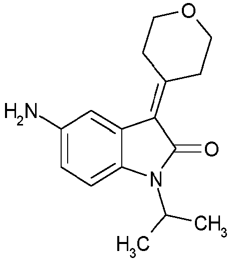 | 0.691 | 1.655 |
| 158 | 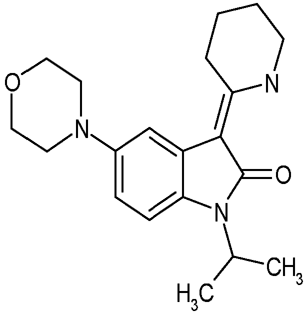 | 0.243 | 2.074 |
| 159 | 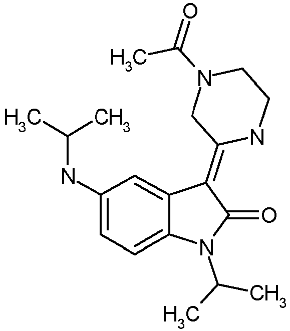 | 0.120 | 0.080 |
| 160 | 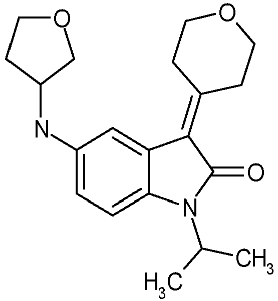 | 0.829 | 0.471 |
| 161 | 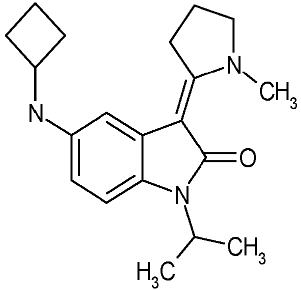 | 0.957 | 0.020 |
| 162 | 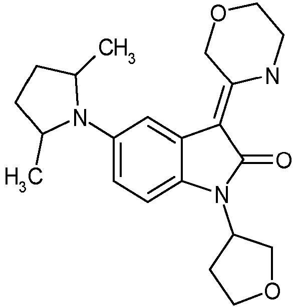 | 0.464 | 0.061 |
| 163 | 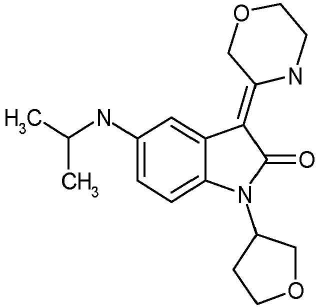 | 0.172 | 0.052 |
| 164 | 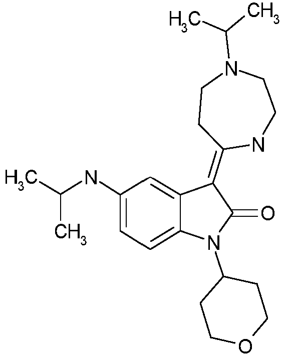 | 0.172 | 0.030 |
| 165 | 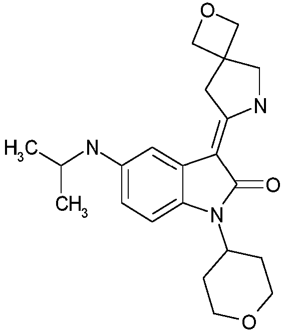 | 0.181 | 0.253 |
| 166 | 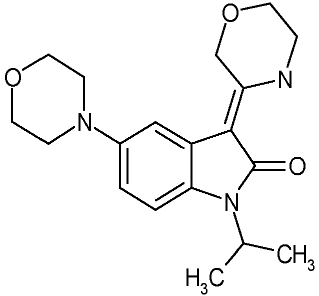 | 0.075 | 0.649 |
| 167 | 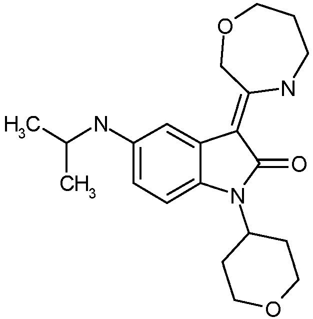 | 0.065 | 0.134 |
| 168 | 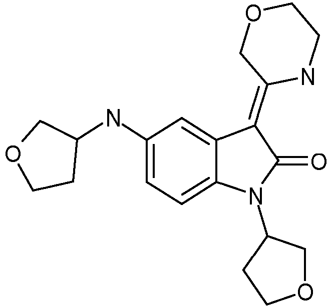 | 0.041 | 0.439 |
| 169 | 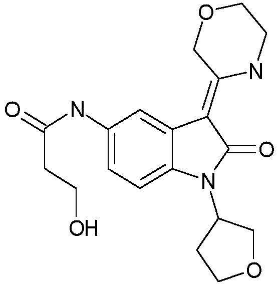 | 0.944 | 0.336 |
| 170 | 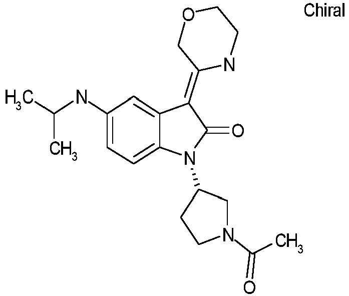 | 0.023 | 0.159 |
| 171 | 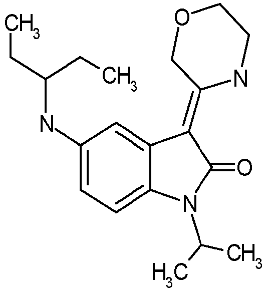 | 0.206 | 0.023 |
| 172 | 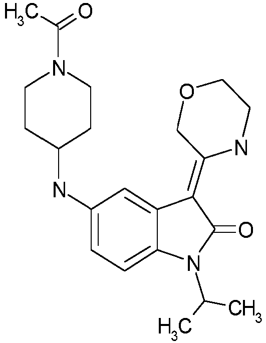 | 0.078 | 0.279 |
| 173 | 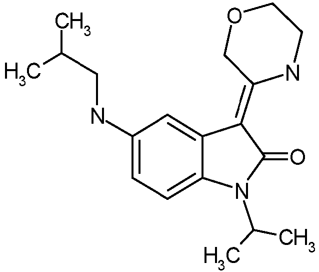 | 0.367 | 0.032 |
| 174 | 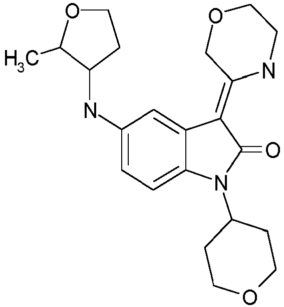 | 0.039 | 0.143 |
| 175 | 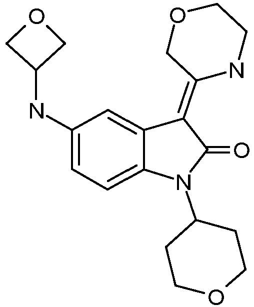 | 0.097 | 1.187 |

### Experimental Section

The following examples are intended to illustrate the invention, without restricting its scope.

As a rule, melting points, IR, ¹H-NMR and/or mass spectra have been obtained for the compounds prepared. Unless otherwise stated, Rf values were obtained using ready-made silica gel 60 F254 TLC plates (E-. Merck, Darmstadt, item no. 1.05714) without chamber saturation. The ratios given for the eluant refer to units by volume of the solvents in question. Chromatographic purification was done using silica gel supplied by E. Merck, Darmstadt (Silica gel 60, 0.040-0.063 mm), item no. 1.09385.2500).

The following abbreviations can be used in the following examples:
- BOC: tBuOC(O
- CH: Cyclohexane
- CM: Dichloromethane
- DIPEA: Diisopropylamine
- DMSO: Dimethylsulphoxide
- DMF: *N,N-Dimethylformamide*
- EA: Ethyl acetate
- ESI: Electrospray ionisation
- h: Hour(s)
- HPLC: High performance liquid chromatography
- M: Molar
- MeOH: Methanol
- EtOH: Ethanol
- min: Minute(s)
- mL: Milliliters
- µL: Microliters
- mmol: Millimoles
- µmol: Micromoles
- MPLC: Medium pressure liquid chromatography
- MS: Mass spectrometry
- NMP: *N*-Methyl-pyrrolidinone
- Pd/C: Palladium on charcoal
- PE: petroleum ether
- Rf: Retention factor
- Rt: Retention time
- sat.: Saturated
- Tert.: Tertiary
- TLC: Thin layer chromatography
- TFA: Trifluoroacetic acid
- THF: Tetrahydrofurane
- TBME: *tert*-Butyl methyl ether
- UPLC: Ultra performance liquid chromatography

All references to brine refer to a saturated aqueous solution of sodium chloride. Unless otherwise indicated, all temperatures are expressed in °C (degrees Centigrade). All reactions are conducted not under an inert atmosphere at room temperature unless otherwise noted.

### EXAMPLES:

### Example 1: HPLC/UPLC Methods

### Method A

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% NH₃]** | **% Solvent B [MeOH]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.75 | 0 | 100 | 4.0 | 60 |

### Method B

| **Device:** Waters Alliance with DAD and MS detector | | | | |
|---|---|---|---|---|
| **Column:** Waters SunFire C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.0 | 60 |
| 1.60 | 0 | 100 | 4.0 | 60 |
| 1.85 | 0 | 100 | 4.0 | 60 |
| 1.90 | 95 | 5 | 4.0 | 60 |

### Method C

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.8 | 60 |
| 1.60 | 0 | 100 | 4.8 | 60 |
| 1.85 | 0 | 100 | 4.8 | 60 |
| 1.90 | 95 | 5 | 4.8 | 60 |

### Method D

| **Device:** Waters Acquity with DAD and MS detector | | | | |
|---|---|---|---|---|
| **Column:** Waters SunFire C18, 2.1 x 20 mm, 2.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 99 | 1 | 1.3 | 60 |
| 0.15 | 99 | 1 | 1.3 | 60 |
| 1.10 | 0 | 100 | 1.3 | 60 |
| 1.25 | 0 | 100 | 1.3 | 60 |

### Method E

| **Device:** Waters Acquity with DAD and MS detector | | | | |
|---|---|---|---|---|
| **Column:** Supelco Ascentis Express C18, 2.1 x 50 mm, 2.7 µm | | | | |
| **Time [min]** | **% Solvent A [H₂O, 0.1% TFA]** | **% Solvent B [ACN, 0.08% TFA]** | **Flow rate [mL/min]** | **Temperature [°C]** |
| 0.00 | 95 | 5 | 1.5 | 60 |
| 0.70 | 1 | 99 | 1.5 | 60 |
| 0.80 | 1 | 99 | 1.5 | 60 |
| 0.81 | 95 | 5 | 1.5 | 60 |

### Method F

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH, 0.1% TFA]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.75 | 0 | 100 | 4.0 | 60 |
| 1.85 | 95 | 5 | 4.0 | 60 |

### Method G

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.0 | 60 |
| 1.60 | 0 | 100 | 4.0 | 60 |
| 1.85 | 0 | 100 | 4.0 | 60 |
| 1.90 | 95 | 5 | 4.0 | 60 |

### Method H

| **Device:** Waters Alliance with 2695 with PDA detector 2996 and micromass ZQ 2000 | | | | |
|---|---|---|---|---|
| **Column:** Microsorb C18, 4.6 x 20 mm, 5 µm | | | | |
| **Time** | **% Solvent A** | **% Solvent B** | **Flow rate** | **Temperature** |
| **[min]** | **[H₂O, 0.15% TFA]** | **[MeOH]** | **[mL/min]** | **[°C]** |
| 0.00 | 95 | 5 | 5.2 | rt |
| 0.25 | 95 | 5 | 5.2 | rt |
| 1.90 | 0 | 100 | 5.2 | rt |
| 2.05 | 0 | 100 | 5.2 | rt |
| 2.15 | 95 | 5 | 5.2 | rt |
| 2.25 | 95 | 5 | 5.2 | rt |
| 2.30 | 95 | 5 | 0.1 | rt |

### Method I

| **Device:** Waters Acquity with DAD and MS detector | | | | |
|---|---|---|---|---|
| **Column:** Waters SunFire C18, 2.1 x 30 mm, 2.5 µm | | | | |
| **Time [min]** | **% Solvent A [H₂O, 0.13% TFA]** | **% Solvent B [MeOH, 0.05% TFA]** | **Flow rate [mL/min]** | **Temperature [°C]** |
| 0.00 | 99 | 1 | 1.2 | 60 |
| 0.15 | 99 | 1 | 1.2 | 60 |
| 1.10 | 0 | 100 | 1.2 | 60 |
| 1.25 | 0 | 100 | 1.2 | 60 |

### Method J

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.90 | 0 | 100 | 4.0 | 60 |
| 2.00 | 95 | 5 | 4.0 | 60 |

### Method K

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.75 | 0 | 100 | 4.0 | 60 |
| 1.85 | 95 | 5 | 4.0 | 60 |

### Method L

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.9 | 60 |
| 1.60 | 0 | 100 | 4.9 | 60 |
| 2.20 | 95 | 5 | 4.9 | 60 |

### Method M

| **Device:** Waters Alliance with DAD and MS detector | | | | |
|---|---|---|---|---|
| **Column:** Waters XBridge C18, 4.6 x 30 mm, 3.5 µm | | | | |
| **Time [min]** | **% Solvent A [H₂O, 0.1% NH₃]** | **% Solvent B [MeOH, 0.1% NH₃]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.75 | 0 | 100 | 4.0 | 60 |

### Method N

| **Device:** Waters Alliance with DAD and MS detector | | | | |
|---|---|---|---|---|
| **Column:** Waters SunFire C18, 4.6 x 30 mm, 3.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 4.0 | 60 |
| 0.20 | 95 | 5 | 4.0 | 60 |
| 1.50 | 0 | 100 | 4.0 | 60 |
| 1.75 | 0 | 100 | 4.0 | 60 |
| 1.85 | 95 | 5 | 4.0 | 60 |

### Method O

| **Device:** Waters Acquity with DAD and MS detector | | | | |
|---|---|---|---|---|
| **Column:** Waters XBridge C18, 2.1 x 20 mm, 2.5 µm | | | | |
| **Time [min]** | **% Solvent A [H₂O, 0.1% TFA]** | **% Solvent B [MeOH]** | **Flow rate [mL/min]** | **Temperature [°C]** |
| 0.00 | 95 | 5 | 1.4 | 60 |
| 0.05 | 95 | 5 | 1.4 | 60 |
| 1.00 | 0 | 100 | 1.4 | 60 |
| 1.10 | 0 | 100 | 1.4 | 60 |

### Method P

| **Device:** Waters Acquity with DAD and MS detector | | | | |
|---|---|---|---|---|
| **Column:** Waters XBridge BEH C18, 2.1 x 30 mm, 1.7 µm | | | | |
| **Time [min]** | **% Solvent A [H₂O, 0.13% TFA]** | **% Solvent B [MeOH, 0.08% TFA]** | **Flow rate [mL/min]** | **Temperature [°C]** |
| 0.00 | 99 | 1 | 1.3 | 60 |
| 0.05 | 99 | 1 | 1.3 | 60 |
| 0.35 | 0 | 100 | 1.3 | 60 |
| 0.50 | 0 | 100 | 1.3 | 60 |

### Example 2: Synthesis of intermediates A1, A2, A4, A5, A7 to A9 and A11

### Intermediate A1:

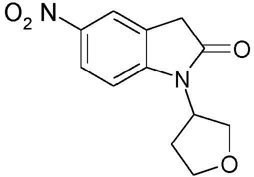

(2-Fluoro-5-nitro-phenyl)-acetic acid (4.60 g; 23.10 mmol) and tetrahydro-furan-3-ylamine (10.0 g; 114.78 mmol) in DMSO (20 mL) are stirred at 45°C over night. HCl (aq. solution; 2M; 92.4 mL; 184.80 mmol) is added. After stirring for 1.5 h at 45°C the resulting precipitate is filtered off, washed with water and dried.
MS (ESI⁺): m/z = 249 [M+H]⁺
HPLC (Method B): Rt = 1.0 min

The following intermediates were prepared in an analogous manner to intermediate A1:

| No. | Structure | Educt 1 | Educt 2 | Mass signal(s) | Revalue or Rₜ |
|---|---|---|---|---|---|
| | Comment | | | | |
| A2 | 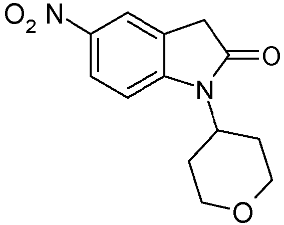 | (2-Fluoro-5-nitrophenyl)-acetic acid | Tetrahydropyran-4-ylamine | (M+H)⁺ 263 | 1.03 min (Method B) |
| | | | | | |
| A4 | 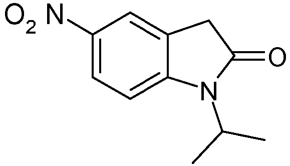 | (2-Fluoro-5-nitrophenyl)-acetic acid | Isopropylamine | (M+H)⁺ 221 | |
| | HCl (4M, aq. solution) is used instead of HCl (2M, aq. solution) | | | | |
| A5 | 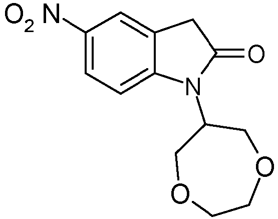 | (2-Fluoro-5-nitrophenyl)-acetic acid | 1,4-Dioxepan-6-amine | (M+H)⁺ 279 | 1.08 min (Method A) |
| | **Purification** by MPLC (DCM/MeOH; 0/0 -> 99/1) | | | | |
| A7 | 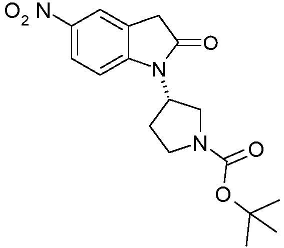 | (2-Fluoro-5-nitrophenyl)-acetic acid | *(S)*-3-Amino-pyrrolidine-1-carboxylic acid *tert*-butyl ester | (M+H)⁺ 346 | 1.34 min (Method A) |
| | | | | | |
| A8 | 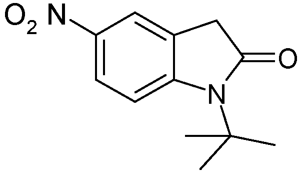 | (2-Fluoro-5-nitrophenyl)-acetic acid | *tert*-Butylamine | (M+H)⁺ = 235 | 0.77 min (Method O) |
| | Additional amine (6 eq.) is added. Mixture is stirred at 100°C over night | | | | |
| A9 | 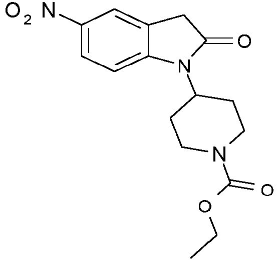 | (2-Fluoro-5-nitrophenyl)-acetic acid | 4-Amino-piperidine-1-carboxylic acid ethyl ester | (M+H)⁺ 334 | 1.24 min (Method B) |
| | | | | | |
| A11 | 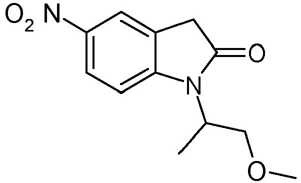 | (2-Fluoro-5-nitrophenyl)-acetic acid | 2-Methoxy-1-methyl-ethylamine | (M+H)⁺ 251 | 1.08 min (Method B) |
| | | | | | |

### Example 3: Synthesis of intermediate A3

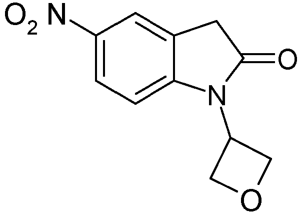

### Step 1:

(2-Fluoro-5-nitro-phenyl)-acetic acid (500 mg; 2.51 mmol) and oxetan-3-ylamine (936 mg; 12.81 mmol) in DMSO (2 mL) are stirred at 45°C over night. The mixture is purified by preparative HPLC (eluent A. water + 0.15 % conc. ammonia, eluent B: MeOH).
MS (ESI⁺): m/z = 251 [M-H]⁻
HPLC (Method A): Rt = 0.66 min

### Step 2:

Intermediate A3 Step 1 (462 mg; 0.92 mmol) and TBTU (0.71 g; 2.20 mmol) in DMF (8 mL) are stirred at room temperature over night. The resulting precipitate is filtered off and dried.
MS (ESI⁺): m/z = 235 [M+H]⁺
HPLC (Method A): Rt = 0.95 min

### Example 4: Synthesis of intermediate A6

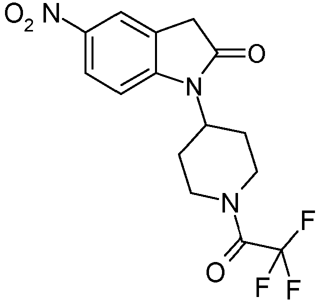

### Step 1:

1,3-Dihydro-1-(piperidin-4-yl)-(2H)-indol-2-one (5.51 g; 25.49 mmol) and TEA (7.16 mL; 50.99 mmol) in DCM (30 mL) are cooled in an ice bath. Trifluoroacetic anhydride (4.25 mL; 30.59 mmol) is added drop wise. The mixture is stirred at room temperature for 2 h. The mixture is diluted with NaHCO₃ (aq. solution; 9%; 20 mL). After the gas development has stopped, the mixture is further diluted with DCM and water. The organic layer is separated, dried and evaporated. The residue is purified by MPLC (DCM/MeOH; 1/0 -> 97/3).
MS (ESI⁺): m/z = 313 [M+H]⁺
HPLC (Method A): Rt = 1.25 min

### Step 2:

Intermediate A6 Step 1 (8.02 g; 25.68 mmol) is solved in conc. sulphuric acid (45 mL) and cooled to -5°C. A cooled mixture of conc. sulphuric acid (15 mL) and conc. nitric acid (1.80 mL; 28.25 mmol) is added drop wise. After stirring for 1 h at -5°C the mixture is poured on ice water. The resulting precipitate is filtered off and dried. The residue is taken up in DCM. The organic layer is washed with NaHCO₃ (aq. solution; 9%), separated, dried and evaporated.
MS (ESI⁺): m/z = 358 [M+H]⁺
HPLC (Method A): Rt = 1.22 min

### Example 5: Synthesis of intermediate A10

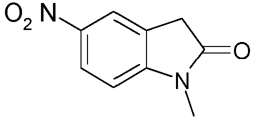

To a cooled mixture of sodium nitrate (12.71 g; 149.48 mmol) and conc. sulphuric acid (22.9 mL; 407.68 mmol) additional conc. sulphuric acid (40 mL) is added drop wise. 1-Methyl-1,3-dihydro-indol-2-one (20.0 g; 135.89 mmol) is taken up in conc. sulphuric acid (120 mL) and added drop wise to the cooled nitrosulphuric acid. The mixture is allowed to warm up to room temperature over night. The mixture is poured on ice water. The resulting precipitate is filtered off, washed with water and dried. The residue is taken up in DCM, washed with water and brine, separated, dried and evaporated.
MS (ESI⁺): m/z = 193 [M+H]⁺
HPLC (Method H): Rt = 0.90 min

### Example 6: Synthesis of intermediates B1, B2, B4, B6, B7, B9 and B10

The following intermediates are prepared according to the given references or are commercially available:

| **Name** | **Structure** | **Reference** |
|---|---|---|
| **B1** | 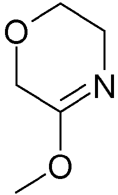 | WO2006/72350 |
| **B2** | 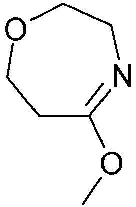 | WO2005/111029 |
| **B4** | 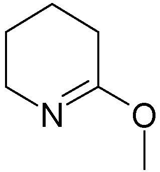 | WO2010/68520 |
| **B6** | 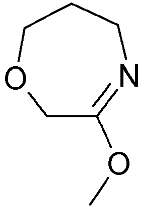 | WO2010/68520 |
| **B7** | 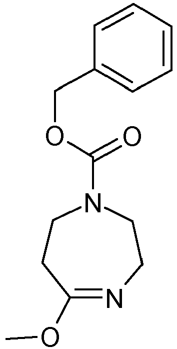 | EP1790641 |
| **B9** | 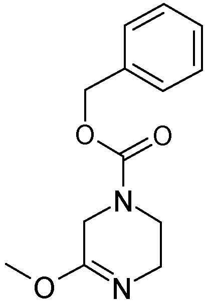 | WO2008/76356 |
| **B10** | 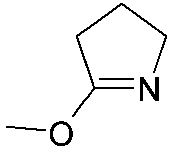 | WO2004/60376 |

### Example 7: Synthesis of intermediates B3, B5 and B8

### Intermediate B3:

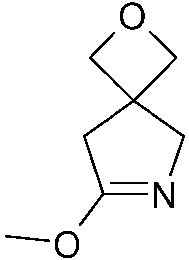

2-Oxa-6-aza-spiro[3.4]octan-7-one (1.00 g; 7.87 mmol) and trimethyloxonium tertafluoroborate (1.28 g; 8.65 mmol) in DCM (120 mL) are stirred at room temperature over night. The mixture is diluted with saturated NaHCO₃ solution until gas development stops. The organic layer is separated, dried and evaporated.
MS (ESI⁺): m/z = 142 [M+H]⁺

The following intermediates are prepared in an analogous manner to intermediate B3:

| No. | Structure | Educt 1 | Educt 2 | Mass signal(s) | Revalue or Rₜ |
|---|---|---|---|---|---|
| | Comment | | | | |
| B5 | 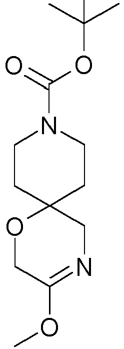 | 3-Oxo-1-oxa-4,9-diaza-spiro[5.5]und ecane-9-carboxylic acid tert-butyl ester | Trimethyloxon ium tertafluorobora te | (M+H)⁺ = 285 | |
| | | | | | |
| B8 | 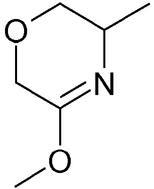 | 5-Methyl-morpholin-3-one | Trimethyloxon ium tertafluorobora te | | |
| | | | | | |

### Example 8: Synthesis of intermediates C1-C3, C5-C8, C10-C12, C14, C16, C18, C20-C25, C27-C29, C32-C36

### Intermediate C1:

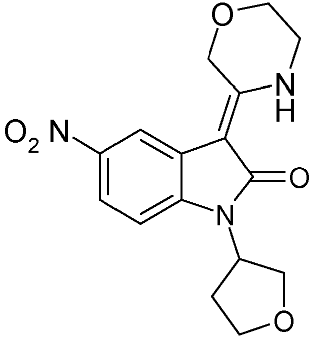

Intermediate A1 (700 mg; 2.82 mmol) and Intermediate B1 (714 mg; 6.20 mmol) are stirred at 130°C for 20 min in a microwave. The resulting precipitate is suspended in MeOH, filtered off and dried.
MS (ESI⁺): m/z = 332 [M+H]⁺
HPLC (Method B): Rt = 1.29 min

The following intermediates were prepared in an analogous manner to intermediate C1:

| No. | Structure | Educt 1 | Educt 2 | Mass signal(s) | Revalue or Rt |
|---|---|---|---|---|---|
| | Comment | | | | |
| C2 | 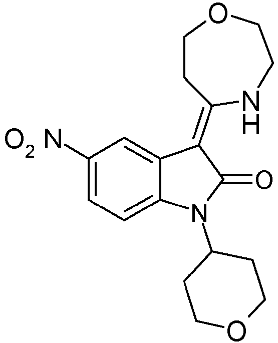 | A2 | B2 | (M+H)⁺ =360 | 1.29 min (Method B) |
| | | | | | |
| C3 | 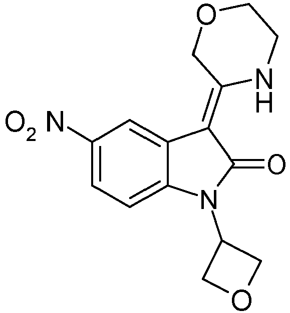 | A3 | B1 | (M+H)⁺ =318 | 1.19 min (Method A) |
| | Combined reaction time in microwave 2.5 h at 130°C | | | | |
| C5 | 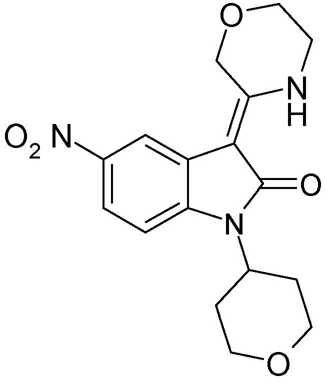 | A2 | B1 | (M+H)⁺ =346 | 0.84 min (Method D) |
| | After 20min additional B1 is added and the mixture is stirred for 20 min at 130°C | | | | |
| C6 | 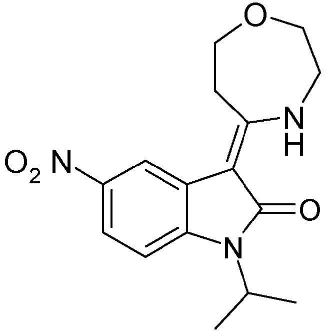 | A4 | B2 | (M+H)⁺ =318 | 1.31 min (Method N) |
| | | | | | |
| C7 | 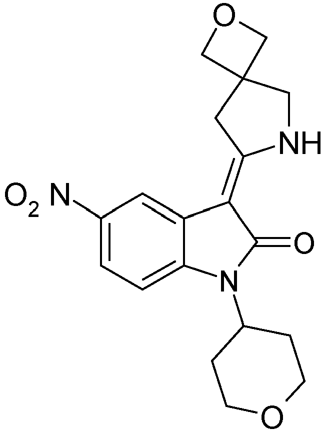 | A2 | B3 | (M+H)⁺ =372 | 1.22 min (Method B) |
| | The mixture is stirred for 40 min at 130°C and for 20 min at 140°C | | | | |
| C8 | 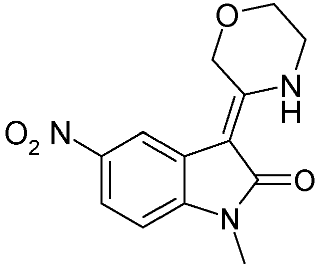 | 1-Methyl-5-nitro-1,3-dihydro-indol-2-one | B1 | (M+H)⁺ =276 | 0.58 min (Method O) |
| | | | | | |
| C10 | 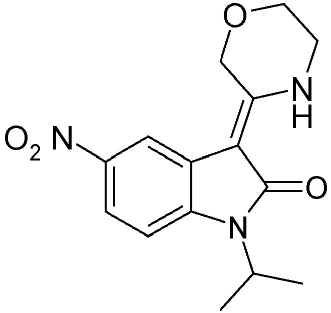 | A4 | B1 | (M+H)⁺ =304 | 1.24 min (Method C) |
| | The mixture is stirred for 40 min at 130°C and for 20 min at 140°C | | | | |
| C11 | 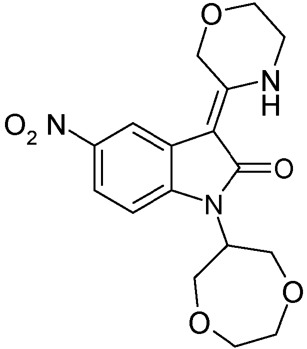 | A5 | B1 | (M+H)⁺ =362 | 1.27 min (Method A) |
| | Combined reaction time in microwave 165 min at 130°C | | | | |
| C12 | 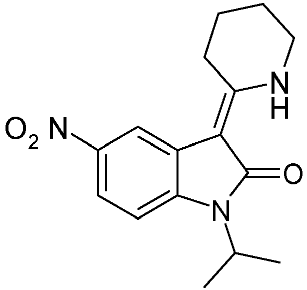 | A4 | B4 | (M+H)⁺ =302 | 1.45 min (Method A) |
| | | | | | |
| C14 | 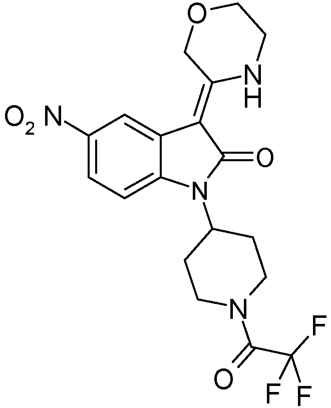 | A6 | B1 | (M+H)⁺ =441 | 1.41 min (Method B) |
| | Combined reaction time in microwave 40 min at 130°C | | | | |
| C16 | 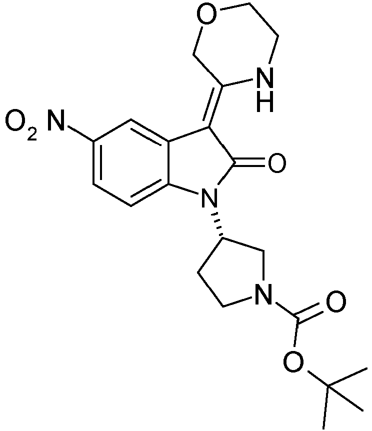 | A7 | B1 | (M+H)⁺ =431 | 1.46 min (Method A) |
| C18 | 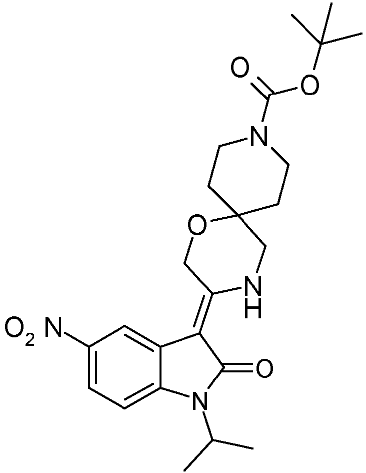 | A4 | B5 | (M+H)⁺ =473 | 1.65 min (Method B) |
| | | | | | |
| C20 | 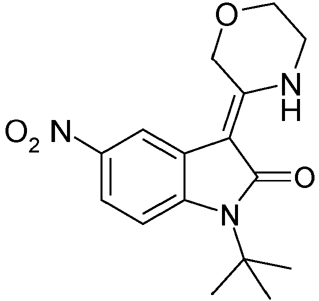 | A8 | B1 | (M+H)⁺ =318 | 1.45 min (Method A) |
| | Combined reaction time in microwave 4.5 h at 130°C. Purification by MPLC (CH/EA = 4/1) | | | | |
| C21 | 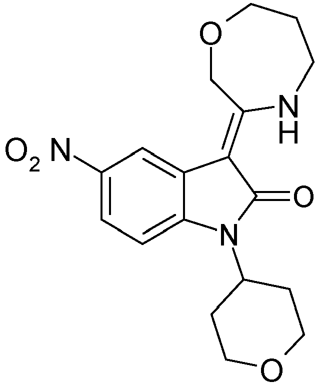 | A2 | B6 | (M+H)⁺ =360 | 1.31 min (Method B) |
| | | | | | |
| C22 | 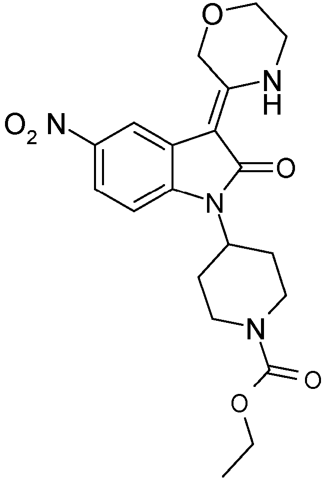 | A9 | B1 | (M+H)⁺ =417 | 1.43 min (Method B) |
| C23 | 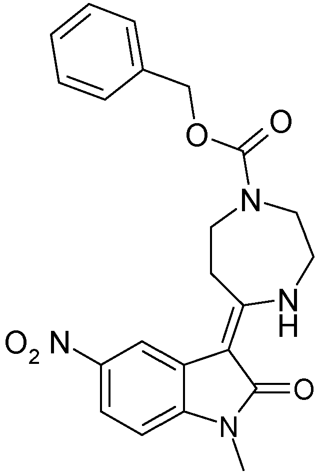 | A10 | B7 | (M+H)⁺ =423 | 1.64 min (Method H) |
| | | | | | |
| C24 | 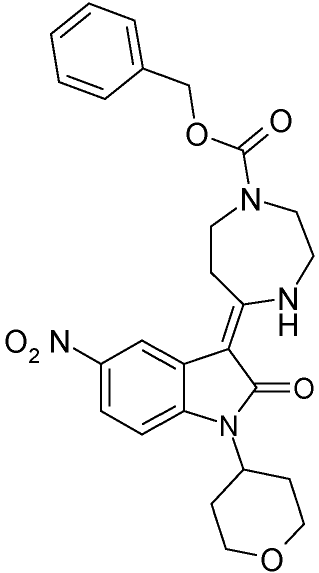 | A2 | B7 | (M+H)⁺ =493 | 1.5 min (Method B) |
| C25 | 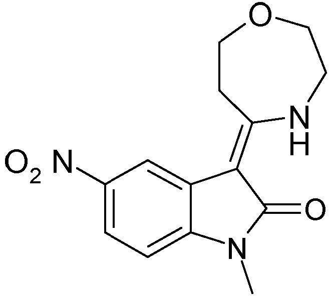 | A10 | B2 | (M+H)⁺ =290 | 1.29 min (Method H) |
| | | | | | |
| C27 | 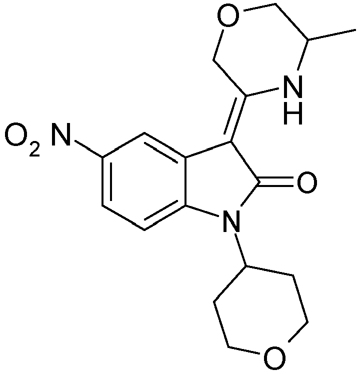 | A2 | B8 | (M+H)⁺ =260 | 1.36 min (Method B) |
| | Additional reaction time in microwave 30 min at 140°C. Purification by preparative HPLC (eluent A. water + 0.1 % conc. ammonia, eluent B: MeOH) | | | | |
| C28 | 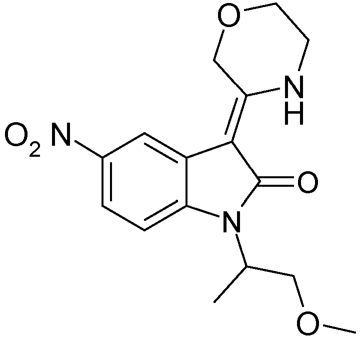 | A11 | B1 | (M+H)⁺ =334 | 1.32 min (Method B) |
| | Combined reaction time in microwave 40 min at 130°C | | | | |
| C29 | 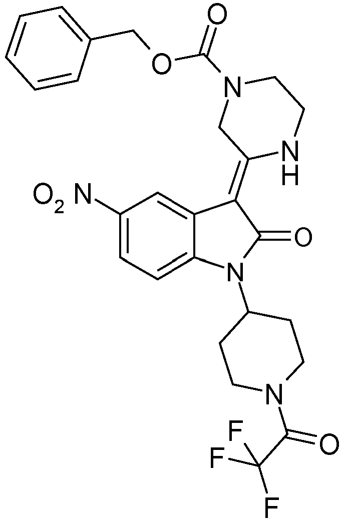 | A6 | B9 | (M+H)⁺ =574 | 0.98 min (Method B) |
| | Combined reaction time in microwave 40 min at 130°C | | | | |
| C32 | 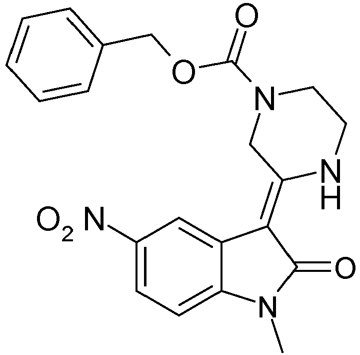 | A10 | B9 | (M+H)⁺ =409 | 1.45 min (Method A) |
| C33 | 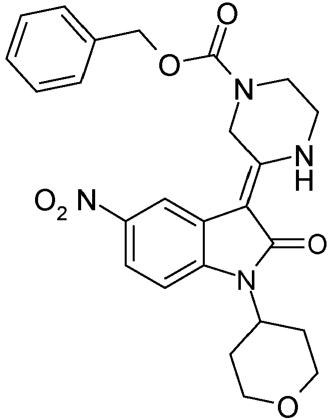 | A2 | B9 | (M+H)⁺ =479 | 1.47 min (Method A) |
| | | | | | |
| C34 | 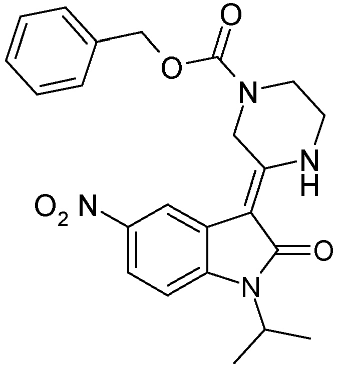 | A4 | B9 | (M+H)⁺ =437 | 1.54 min (Method A) |
| | | | | | |
| C35 | 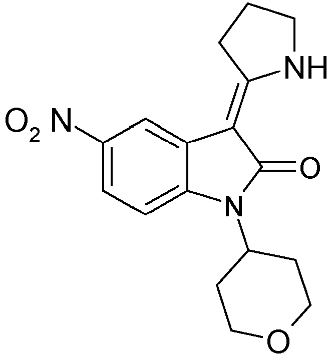 | A2 | B10 | (M+H)⁺ =330 | 1.16 min (Method L) |
| C36 | 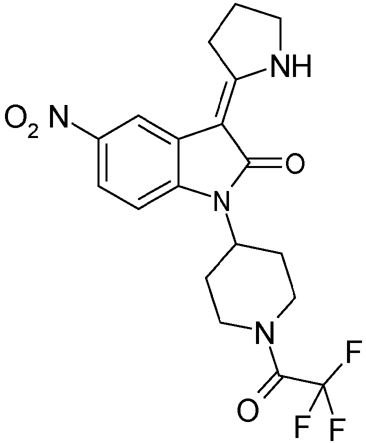 | A6 | B10 | (M+H)⁺ =425 | 1.28 min (Method K) |

### Example 9: Synthesis of intermediate C4 and C31:

### Intermediate C4:

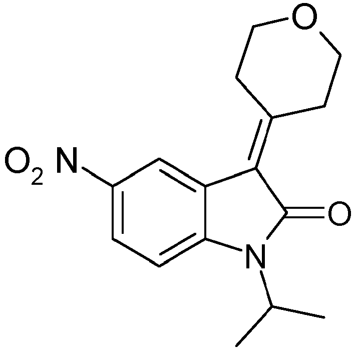

Intermediate A4 (1.50 g; 6.81 mmol) and tetrahydro-4H-pyran-4-one (13.0 mL; 140.76 mmol) in piperidine (1.36 mL; 13.62 mmol) are stirred at 100°C for 15 min in a microwave. The solvent is evaporated. The residue is stirred in TBME. The precipitate is filtered off and dried.
MS (ESI⁺): m/z = 303 [M+H]⁺
HPLC (Method A): Rt = 1.40 min

The following intermediates were prepared in an analogous manner to intermediate C4:

| No. | Structure | Educt 1 | Educt 2 | Mass signal(s) | Revalue or Rt |
|---|---|---|---|---|---|
| | Comment | | | | |
| C31 | 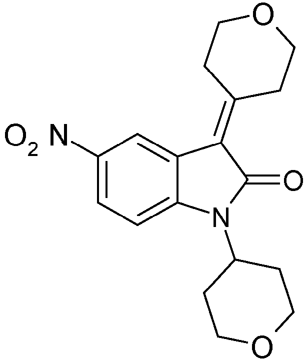 | A2 | Tetrahydro-4H-pyran-4-one | M⁺ = 344 | 1.32 min (Method A) |

### Example 10: Synthesis of intermediate C9 and C13

### Intermediate C9:

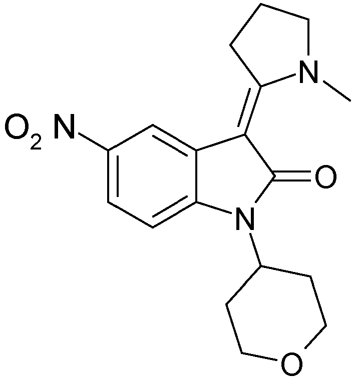

The following reaction is performed under a nitrogen atmosphere.

Intermediate A2 (225 mg; 1.24 mmol) and 2,2-dimethoxy-1-methyl-pyrrolidine (450 mg; 3.10 mmol) in chloroform (2.5 mL) are stirred at reflux for 3 h. Additional 2,2-dimethoxy-1-methyl-pyrrolidine (1.2 eq.) is added and the mixture is stirred at 65°C over night. The mixture is washed with sat. NaHCO₃ solution. The organic layer is separated, washed with brine, dried and evaporated. The residue is purified by preparative HPLC (eluent A. water + 0.15 % conc. ammonia, eluent B: MeOH).
MS (ESI⁺): m/z = 344 [M+H]⁺
HPLC (Method A): Rt = 1.26 min

The following intermediates were prepared in an analogous manner to intermediate C9:

| No. | Structure | Educt 1 | Educt 2 | Mass signal(s) | Revalue or Rt |
|---|---|---|---|---|---|
| | Comment | | | | |
| C13 | 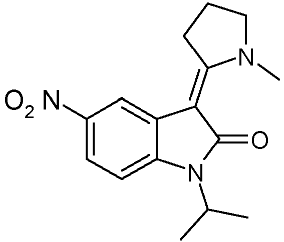 | A4 | 2,2-diemthoxy-1-methyl-pyrrolidine | (M+H)⁺ = 302 | 1.34 min (Method A) |

### Example 11: Synthesis of intermediate C15 and C37

### Intermediate C15:

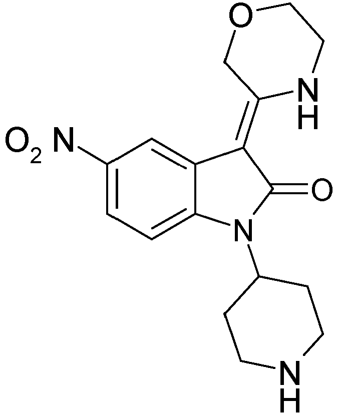

Intermediate C14 (81 mg; 0.16 mmol) in THF (5 mL) and potassium carbonate (51 mg; 0.37 mmol) in water (1.5 mL) are stirred at 40°C for 2 h. The mixture is diluted with brine and EA. The organic layer is separated, dried and evaporated.
MS (ESI⁺): m/z = 345 [M+H]⁺
HPLC (Method B): Rt = 0.9 min

The following intermediates were prepared in an analogous manner to intermediate C15:

| No. | Structure | Educt 1 | Mass signal(s) | Revalue or Rₜ |
|---|---|---|---|---|
| | Comment | | | |
| C37 | 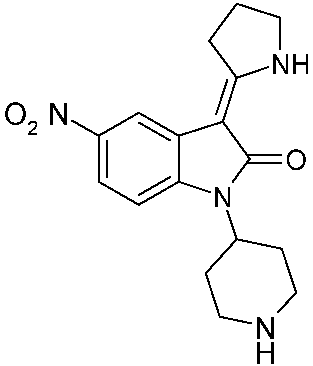 | C36 | (M+H)⁺ = 329 | 1.07 min (Method N) |
| | The residue is taken up in DCM/water. The precipitate is filtered off and dried. | | | |

### Example 12: Synthesis of intermediate C17

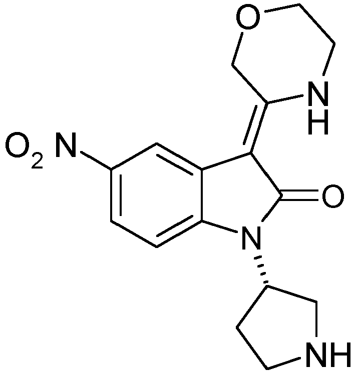

Intermediate C16 (800 mg; 1.86 mmol) in DCM/TFA (1/1; 15 mL) are stirred at room temperature for 1 h. The solvent is evaporated. The residue is taken up in DCM and washed with NaOH (aq. solution; 1M). The organic layer is separated, dried and evaporated.
MS (ESI⁺): m/z = 331 [M+H]⁺
HPLC (Method B): Rt = 0.91 min

### Example 13: Synthesis of intermediate C19

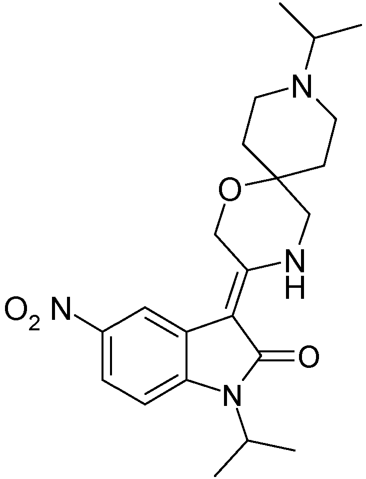

### Step 1:

Intermediate C18 (2.70 g; 5.71 mmol) in DCM/TFA (1/1; 30 mL) are stirred at room temperature for 1 h. The solvent is evaporated.
MS (ESI⁺): m/z = 373 [M+H]⁺
HPLC (Method B): Rt = 1.15 min

### Step 2:

Intermediate C19 Step 1 (600 mg; 1.23 mmol), acetone (448 µL; 6.17 mmol) and glacial acetic acid (182 µL; 3.33 mmol) in MeOH (20 mL) are stirred at room temperature for 1 h. Sodium cyanoborohydride (155 mg; 2.47 mmol) is stirred at room temperature for 2 h. Additional acetone (2 mL) is added. After stirring over night the mixture is diluted with sat. NaHCO₃ solution. The organic layer is separated, dried and evaporated.
MS (ESI⁺): m/z = 415 [M+H]⁺
HPLC (Method B): Rt = 1.12 min

### Example 14: Synthesis of intermediate C26

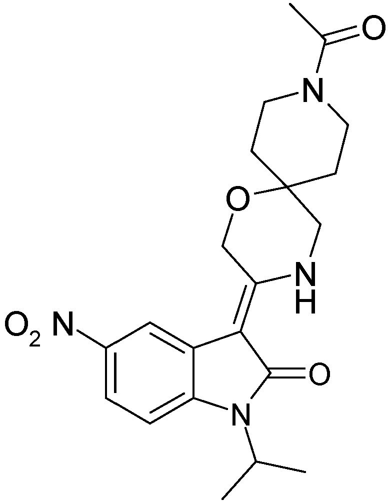

### Step 1:

Intermediate C18 (2.70 g; 5.71 mmol) in DCM/TFA (1/1; 30 mL) are stirred at room temperature for 1 h. The solvent is evaporated.
MS (ESI⁺): m/z = 373 [M+H]⁺
HPLC (Method B): Rt = 1.15 min

### Step 2:

Intermediate C26 Step 1 (300 mg; 0.62 mmol), acetic anhydride (87 µL; 0.93 mmol) and TEA (316 µL; 1.85 mmol) in DCM (7 mL) are stirred at room temperature for 1 h. The mixture is diluted with sat. NaHCO₃ solution and DCM. The organic layer is separated, dried and evaporated.
MS (ESI⁺): m/z = 415 [M+H]⁺
HPLC (Method B): Rt = 1.40 min

### Example 15: Synthesis of intermediate C30

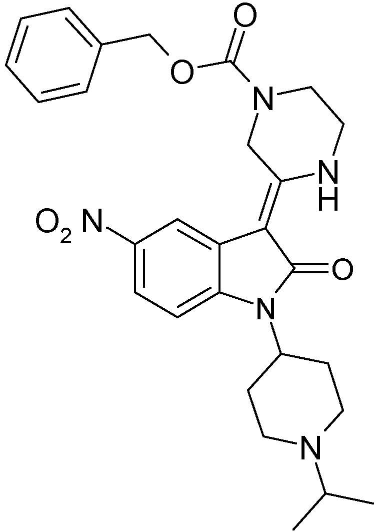

### Step 1:

Intermediate C29 (500 mg; 0.87 mmol) in THF (17 mL) and potassium carbonate (157 mg; 1.13 mmol) in water (12 mL) are stirred at 40°C for 5 h. The mixture is diluted with NaHCO₃ (aq. solution; 9%) and extracted with EA. The organic layer is washed with brine, separated, dried and evaporated. The residue is purified by preparative HPLC (eluent A. water + 0.15 % conc. ammonia, eluent B: MeOH).
MS (ESI⁺): m/z = 478 [M+H]⁺
HPLC (Method A): Rt = 1.46 min

### Step 2:

Intermediate C30 Step 1 (118 mg; 0.25 mmol), acetone (90 µL; 1.24 mmol) and glacial acetic acid (36 µL; 0.67 mmol) in MeOH (8 mL) are stirred at room temperature for 2 h. Sodium cyanoborohydride (31 mg; 0.49 mmol) is added and the mixture stirred is stirred at 40° C for 2 days. The mixture is diluted with NaHCO₃ (aq. solution; 9%) and DCM. The organic layer is separated, dried and evaporated. The residue is stirred in MeOH, filtered off and dried. The residue is purified by preparative HPLC (eluent A. water + 0.15 % conc. ammonia, eluent B: MeOH).
MS (ESI⁺): m/z = 520 [M+H]⁺
HPLC (Method A): Rt = 1.58 min

### Example 16: Synthesis of intermediate C38

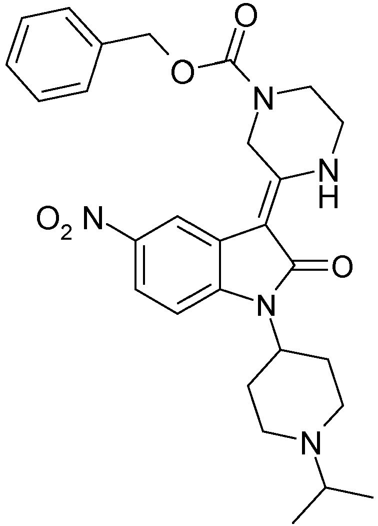

Intermediate C29 (203 mg; 0.35 mmol) in THF (7 mL) and potassium carbonate (64 mg; 0.46 mmol) in water (5 mL) are stirred at 40°C for 3 days. The mixture is diluted with NaHCO₃ (aq. solution; 9%) and extracted with EA. The organic layer is washed with brine, separated, dried and evaporated. The residue is purified by preparative HPLC (eluent A. water + 0.15 % conc. ammonia, eluent B: MeOH).
MS (ESI⁺): m/z = 494 [M+H]⁺
HPLC (Method A): Rt = 1.38 min

### Example 17: Synthesis of intermediates D1-D12, D15-D26, and D28-D31

### Intermediate D1:

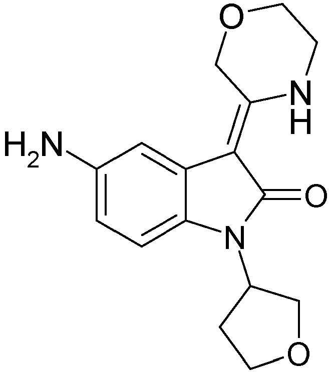

Intermediate C1 (862 mg; 2.60 mmol) and Raney-Nickel (150 mg) in MeOH (25 mL) and THF (50 mL) are hydrogenated in a Parr apparatus (rt; 50 psi; 4.5 h). The catalyst is filtered off and the solvent is evaporated.
MS (ESI⁺): m/z = 302 [M+H]⁺
HPLC (Method B): Rt = 0.67 min

The following intermediates were prepared in an analogous manner to intermediate D1:

| No. | Structure | Educt 1 | Mass signal(s) | Revalue or Rₜ |
|---|---|---|---|---|
| | Comment | | | |
| D2 | 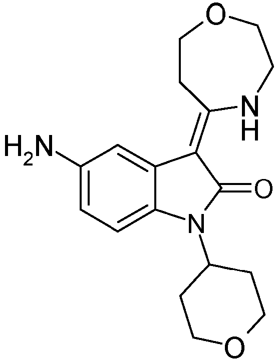 | C2 | (M+H)⁺ = 330 | 0.73 min (Method B) |
| | | | | |
| D3 | 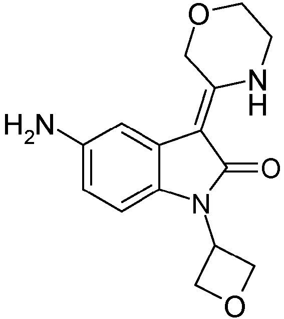 | C3 | (M+H)⁺ = 288 | 0.89 min (Method A) |
| | | | | |
| D4 | 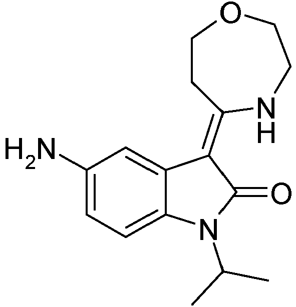 | C6 | (M+H)⁺ = 288 | 0.65 min (Method M) |
| | | | | |
| D5 | 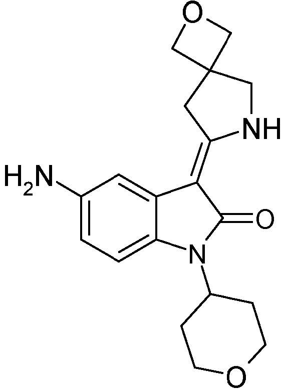 | C7 | (M+H)⁺ = 342 | 0.69 min (Method B) |
| | | | | |
| D6 | 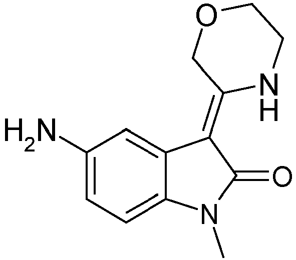 | C8 | (M+H)⁺ = 246 | 0.39 min (Method O) |
| | | | | |
| D7 | 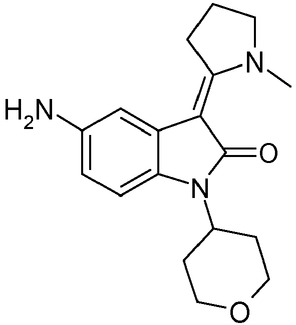 | C9 | (M+H)⁺ = 314 | 0.96 min (Method A) |
| | | | | |
| D8 | 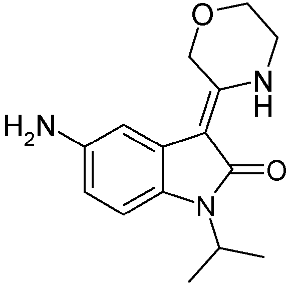 | C10 | (M+H)⁺ = 274 | 0.71 min (Method C) |
| | | | | |
| D9 | 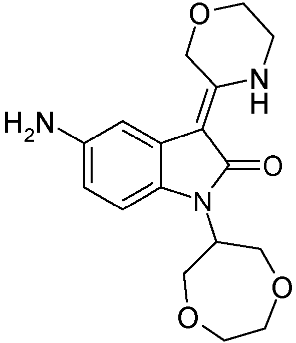 | C11 | (M+H)⁺ = 332 | 0.95 min (Method A) |
| | | | | |
| D10 | 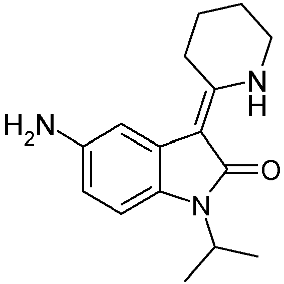 | C12 | (M+H)⁺ = 272 | 1.14 min (Method A) |
| | | | | |
| D11 | 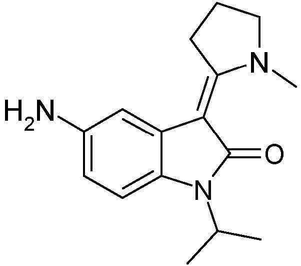 | C13 | (M+H)⁺ = 272 | 0.71 min (Method G) |
| | | | | |
| D12 | 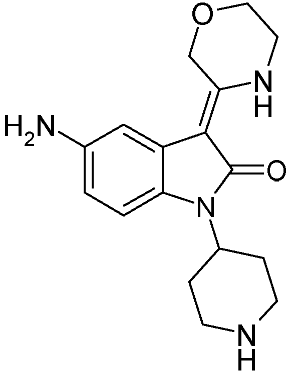 | C15 | (M+H)⁺ = 315 | |
| | | | | |
| D15 | 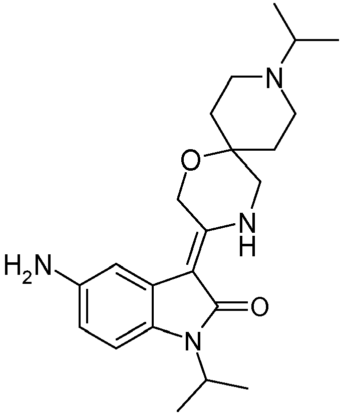 | C19 | (M+H)⁺ = 385 | 0.71 min (Method B) |
| | | | | |
| D16 | 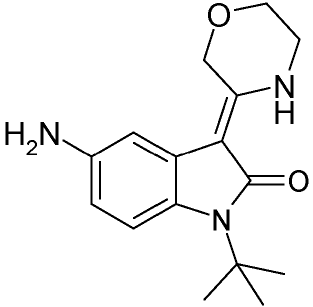 | C20 | (M+H)⁺ = 288 | 0.91 min (Method G) |
| | | | | |
| D17 | 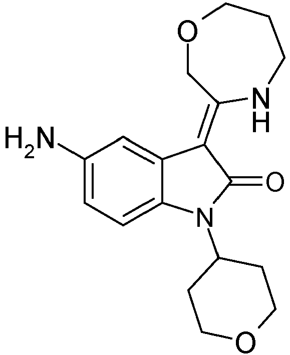 | C21 | (M+H)⁺ = 330 | 0.76 min (Method B) |
| | | | | |
| D18 | 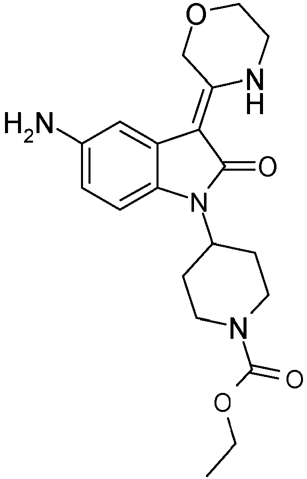 | C22 | (M+H)⁺ = 387 | 0.92 min (Method B) |
| | Purification by preparative HPLC ((eluent A. water + 0.1 % conc. ammonia, eluent B: MeOH) | | | |
| D19 | 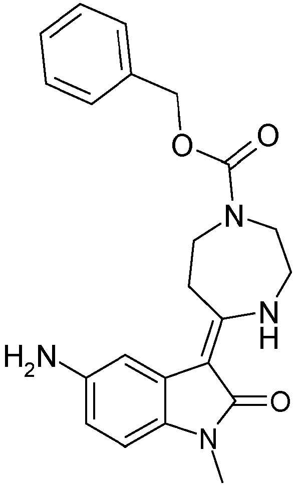 | C23 | (M+H)⁺ = 393 | 1.15 min (Method H) |
| | Purification by preparative HPLC (eluent A. water + 0.1 % conc. ammonia, eluent B: MeOH) | | | |
| D20 | 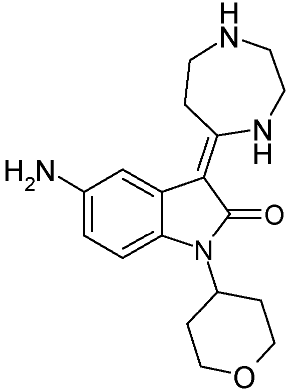 | C24 | (M+H)⁺ = 329 | 1.5 min (Method B) |
| | | | | |
| D21 | 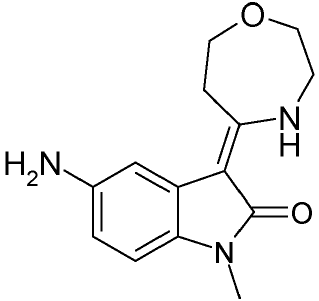 | C25 | (M+H)⁺ = 260 | 0.78 min (Method F) |
| | | | | |
| D22 | 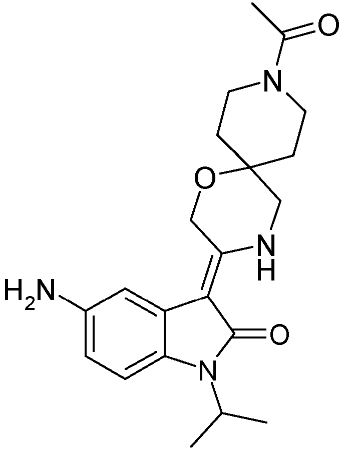 | C26 | (M+H)⁺ = 385 | 0.84 min (Method B) |
| | | | | |
| D23 | 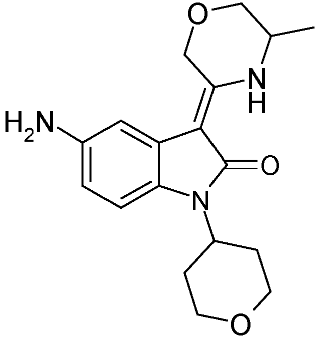 | C27 | (M+H)⁺ = 330 | 0.79 min (Method B) |
| | | | | |
| D24 | 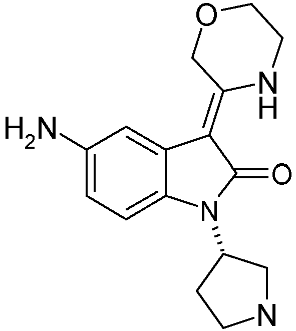 | C17 | (M+H)⁺ = 301 | 0.36 min (Method B) |
| | | | | |
| D25 | 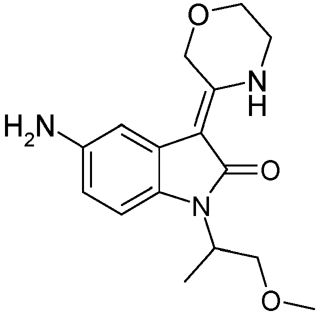 | C28 | (M+H)⁺ = 304 | 0.69 min (Method B) |
| | | | | |
| D26 | 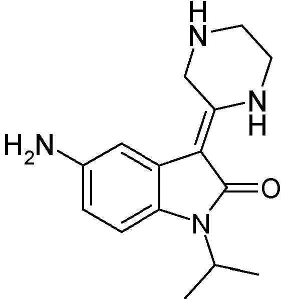 | C30 | (M+H)⁺ = 356 | 1.14 min (Method A) |
| | | | | |
| D28 | 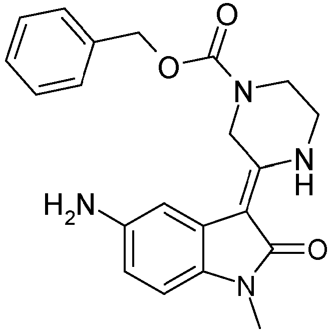 | C32 | (M+H)⁺ = 379 | 1.25 min (Method A) |
| | | | | |
| D29 | 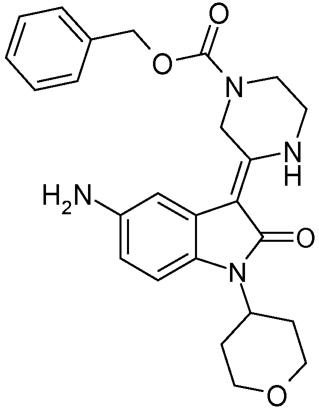 | C33 | (M+H)⁺ = 449 | 1.28 min (Method A) |
| | | | | |
| D30 | 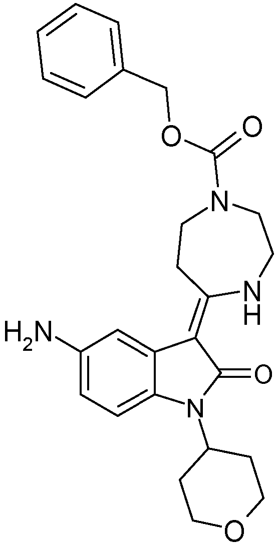 | C24 | (M+H)⁺ = 463 | 1.05 min (Method B) |
| | | | | |
| D31 | 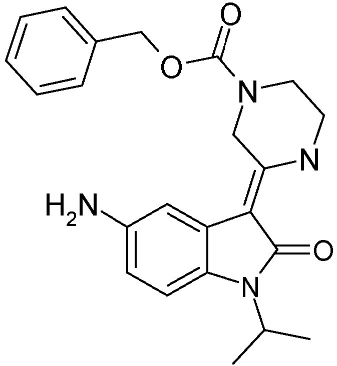 | C34 | (M+H)⁺ = 407 | 1.34 min (Method a) |
| | | | | |

### Example 18: Synthesis of intermediate D13

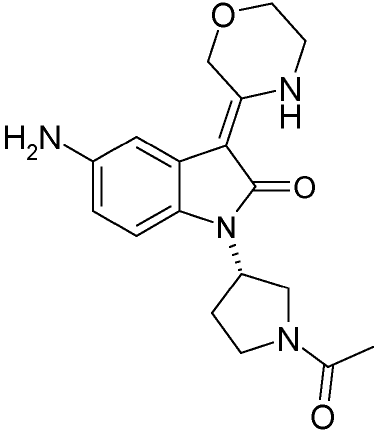

### Step 1:

Intermediate C17 (270 mg; 0.82 mmol), acetic anhydride (85 µL; 0.90 mmol) and DIPEA (423 µL; 2.46 mmol) in DCM (5 mL) are stirred at room temperature for 15 min. The solvent is evaporated.
MS (ESI⁺): m/z = 373 [M+H]⁺
HPLC (Method A): Rt = 1.17 min

### Step 2:

Intermediate D13 Step 1 (430 mg; 0.58 mmol) and Raney-Nickel (50 mg) in MeOH (5 mL) and THF (10 mL) are hydrogenated in a Parr apparatus (rt; 50 psi; 17 h). The catalyst is filtered off and the solvent is evaporated. The residue is purified by preparative HPLC (eluent A. water + 0.1 % conc. ammonia, eluent B: MeOH).
MS (ESI⁺): m/z = 343 [M+H]⁺

### Example 19: Synthesis of intermediate D14

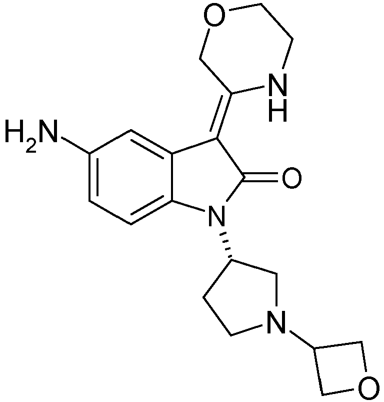

### Step 1:

Intermediate C17 (489 mg; 1.48 mmol), oxetan-3-one (160 mg; 2.22 mmol) and glacial acetic acid (218 µL; 4.00 mmol) in MeOH (20 mL) are stirred at room temperature for 1 h. Sodium cyanoborohydride (186 mg; 2.96 mmol) is added and the mixture is stirred at room temperature for 1 h. THF (5 mL) is added. After stirring over night additional sodium cyanoborohydride (186 mg; 2.96 mmol) is added. The mixture is diluted with water. The organic solvent is evaporated and the aqueous layer is extracted with DCM. The organic layer is separated, dried and evaporated. The residue is stirred in MeOH, filtered off and dried. The residue is purified by preparative HPLC (eluent A. water + 0.1 % conc. ammonia, eluent B: MeOH).
MS (ESI⁺): m/z = 387 [M+H]⁺
HPLC (Method A): Rt = 1.24 min

### Step 2:

Intermediate D14 Step 1 (164 mg; 0.42 mmol) and Raney-Nickel (50 mg) in MeOH (5 mL) and THF (10 mL) are hydrogenated in a Parr apparatus (rt; 50 psi; 4 h). The catalyst is filtered off and the solvent is evaporated.
MS (ESI⁺): m/z = 357 [M+H]⁺
HPLC (Method B): Rt = 0.43 min

### Example 20: Synthesis of intermediate D27

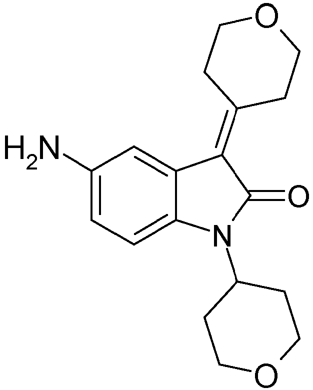

Intermediate C31 (448 mg; 1.30 mmol) and powdered iron (392 mg; 7.02 mmol) in water (14 mL) and ethanol (29 mL) are heated to reflux. Glacial acetic acid (0.79 mL; 13.78 mmol) is added drop wise and the mixture is stirred for 1 h. The organic solvent is evaporated and the residue is taken up in DCM and water. The mixture is alkalised with NaOH (aq. solution; 5 mL). The mixture is filtered through celite. The organic layer is separated, dried and evaporated. The residue is stirred in MeOH/ACN, filtered off and dried.
MS (ESI⁺): m/z = 315 [M+H]⁺
HPLC (Method A): Rt = 1.07 min

### Example 21: Synthesis of intermediate E1

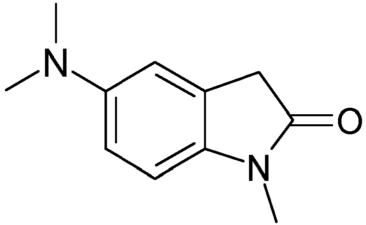

Intermediate A10 (11.0 g; 57.24 mmol) and Pd/C (10%; 1.0 g) in DCM (200 mL) are hydrogenated in a Parr apparatus (rt; 50 psi; 5 h). Additional Pd/C (10%; 1.0 g) and MeOH (100 mL) is added and the mixture is hydrogenated for 3 h. Formaldehyde (aq. solution; 37%; 22.70 mL; 304.90 mmol) is added and the mixture is stirred for 10 min without H₂-pressure and for further 3 h with H₂-pressure. The catalyst is filtered off and the solvent is evaporated. The residue is taken up in NaOH (aq. solution; 1M) and extracted with DCM. The organic layer is separated, dried and evaporated. The residue is purified by MPLC (DCM/MeOH = 98/2).
MS (ESI⁺): m/z = 191 [M+H]⁺
HPLC (Method P): Rt = 0.25 min

### Example 22: Synthesis of intermediates F1, F2 and F4

### Intermediate F1:

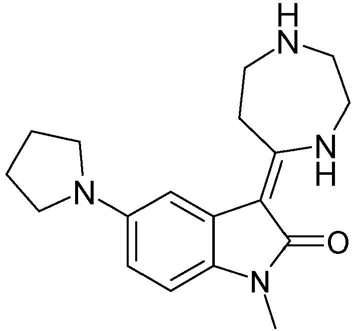

### Step 1:

Intermediate D19 (430 mg; 1.10 mmol), 1,4-diiodo-butane (145 µL; 1.10 mmol) and potassium carbonate (303 mg; 2.19 mmol) in DMF (12 mL) are stirred at 70°C for 2 h. After stirring over night at room temperature the mixture is diluted with NaHCO₃ (aq. solution; 9%) and extracted with EA. The organic layer is washed with brine, separated, dried and evaporated. The residue is purified by preparative HPLC (eluent A. water + 0.15 % conc. ammonia, eluent B: MeOH).
MS (ESI⁺): m/z = 447 [M+H]⁺
HPLC (Method A): Rt = 1.57 min

### Step 2:

Intermediate F1 Step 1(203 mg; 0.46 mmol) and Pd/C (10%; 20 mg) in MeOH (20 mL) and THF (10 mL) are hydrogenated in a Parr apparatus (rt; 50psi; 1 h). Additional Pd/C (10%) is added and the mixture is hydrogenated. The catalyst is filtered off and the solvent is evaporated.
MS (ESI⁺): m/z = 313 [M+H]⁺
HPLC (Method A): Rt = 1.33 min

The following intermediates were prepared in an analogous manner to intermediate F1:

| No. | Structure | Educt 1 | Educt 2 | Mass signal(s) | Revalue or Rₜ |
|---|---|---|---|---|---|
| | Comment | | | | |
| F2 | 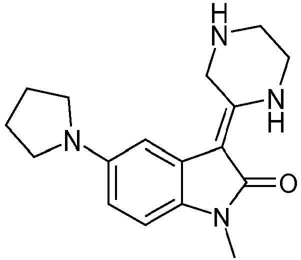 | D28 | 1,4-Diiodo-butane | (M+H)⁺ = 299 | |
| | | | | | |
| F4 | 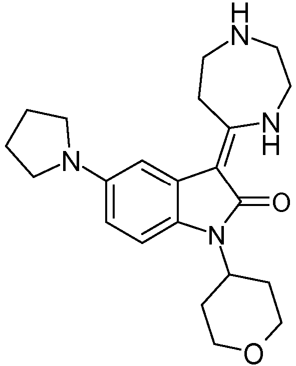 | D30 | 1,4-Diiodo-butane | (M+H)⁺ = 383 | 0.65 min (Method B) |
| | | | | | |

### Example 23: Synthesis of intermediates F3 and F5

### Intermediate F3:

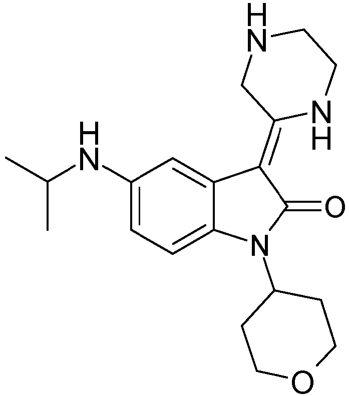

### Step 1:

Intermediate D29 (0.78 g; 1.74 mmol), acetone (631 µL; 8.70 mmol) and glacial acetic acid (256 µL; 4.70 mmol) in MeOH (20 mL) are stirred for 2h at room temperature. Sodium cyanoborohydride (219 mg; 3.48 mmol) is added and the mixture is stirred at room temperature over night. The mixture is diluted with NaHCO₃ (aq. solution; 9%) and extracted with DCM. The organic layer is separated, dried and evaporated. The residue is purified by preparative HPLC (eluent A. water + 0.15 % conc. ammonia, eluent B: MeOH).
MS (ESI⁺): m/z = 491 [M+H]⁺
HPLC (Method A): Rt = 1.49 min

### Step 2:

Intermediate F3 Step 1 (348 mg; 0.71 mmol) and Pd/C (10%; 35 mg) in MeOH (14 mL) and THF (10 mL) are hydrogenated in a Parr apparatus (rt; 50psi; 1.25 h). The catalyst is filtered off and the solvent is evaporated. The residue is purified by preparative HPLC (eluent A. water + 0.15 % conc. ammonia, eluent B: MeOH).
MS (ESI⁺): m/z = 357 [M+H]⁺
HPLC (Method A): Rt = 1.19 min

The following intermediate was prepared in an analogous manner to intermediate F3:

| No. | Structure | Educt 1 | Educt 2 | Mass signal(s) | Revalue or Rₜ |
|---|---|---|---|---|---|
| | Comment | | | | |
| F5 | 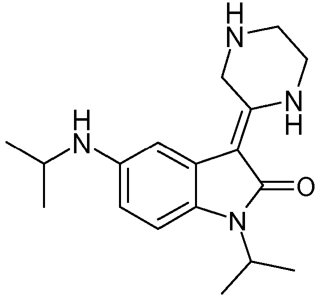 | D31 | Acetone | (M+H)⁺ = 315 | 1.29 min (Method A) |
| | | | | | |

### Example 24: Synthesis of intermediate G1

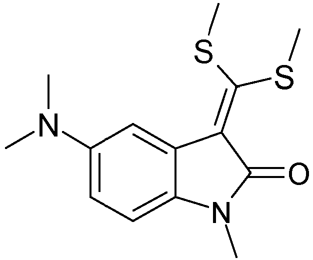

The following reaction is performed under an argon atmosphere.

Intermediate E1 (700 mg; 3.68 mmol) and carbon disulfide (0.24 mL; 4.05 mmol) in DMF (15 mL) are cooled in an ice bath. Sodium hydride (55% in mineral oil; 0.32 g; 7.36 mmol) is added and the mixture is stirred for 20 min. The mixture is allowed to warm up to room temperature. After 1 h of stirring at room temperature the mixture is poured on ice water. The resulting precipitate is filtered off, washed with water and dried.
MS (ESI⁺): m/z = 295 [M+H]⁺
HPLC (Method H): Rt = 1.05 min

### Example 25: Synthesis of compounds 1001 to 1123

### Compound 1001:

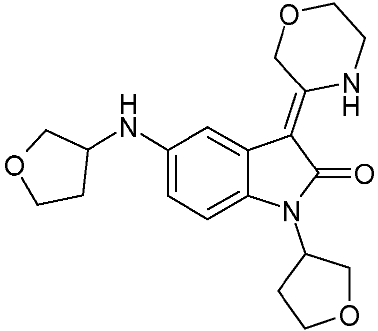

Intermediate D1 (150 mg; 0.50 mmol), dihydro-furan-3-one (58 µL; 0.75 mmol) and glacial acetic acid (73 µL; 1.34 mmol) in MeOH (3 mL) are stirred for 1 h at room temperature. Sodium cyanoborohydride (63 mg; 1.00 mmol) is added and the mixture is stirred for 1 h at room temperature. The mixture is purified by preparative HPLC (eluent A. water + 0.1 % conc. ammonia, eluent B: MeOH).
MS (ESI⁺): m/z = 372 [M+H]⁺
HPLC (Method A): Rt = 1.11 min

In analogy to the preparation of compound 1001 the following compounds are obtained:

| **Nr.** | **Structure** | **Educt 1** | **Educt 2** | **Mass signal(s)** | **Rₜ** |
|---|---|---|---|---|---|
| **1002** | 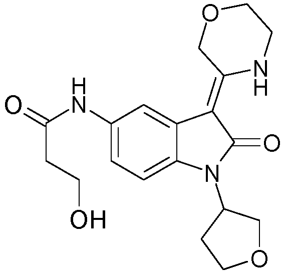 | D1 | 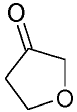 | (M+H)⁺ = 374 | 0.98 min (Method A) |
| **1003** | 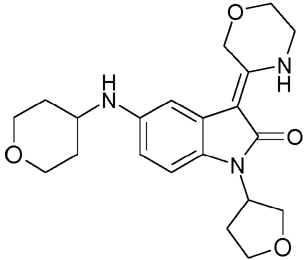 | D1 | 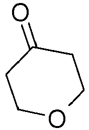 | (M+H)⁺ = 386 | 1.13 min (Method A) |
| **1004** | 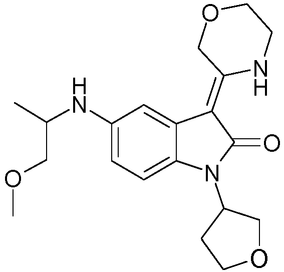 | D1 | 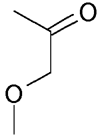 | (M+H)⁺ = 374 | 1.21 min (Method A) |
| **1005** | 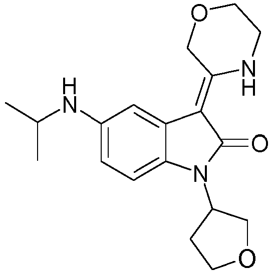 | D1 | 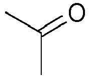 | (M+H)⁺ = 344 | 0.85 min (Method B) |
| **1006** | 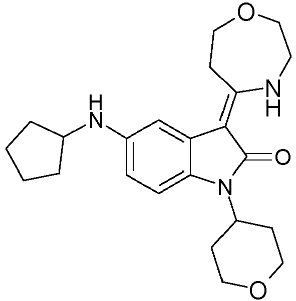 | D2 | 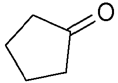 | (M+H)⁺ = 398 | 1.4 min (Method A) |
| **1007** | 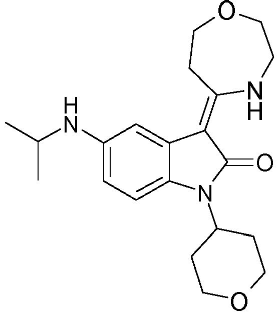 | D2 | 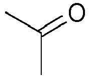 | (M+H)⁺ = 372 | 0.9 min (Method B) |
| **1008** | 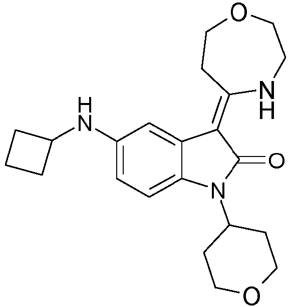 | D2 | 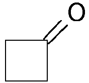 | (M+H)⁺ = 384 | 1.33 min (Method A) |
| **1009** | 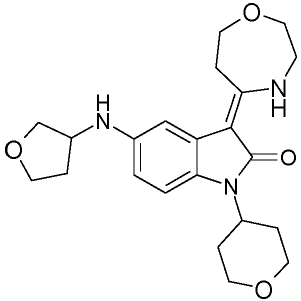 | D2 | 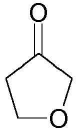 | (M+H)⁺ = 400 | 1.15 min (Method A) |
| **1010** | 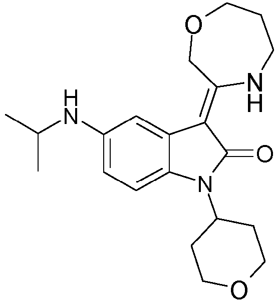 | D17 | 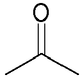 | (M+H)⁺ = 372 | 0.92 min (Method B) |
| **1011** | 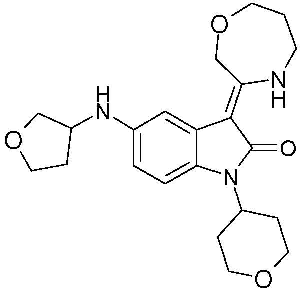 | D17 | 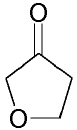 | (M+H)⁺ = 400 | 0.85 min (Method B) |
| **1012** | 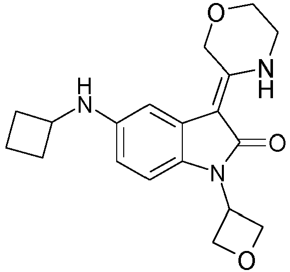 | D3 | 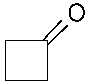 | (M+H)⁺ = 342 | 1.27 min (Method A) |
| **1013** | 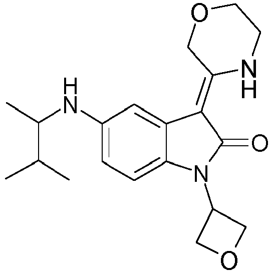 | D3 | 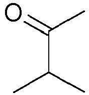 | (M+H)⁺ = 358 | 1.41 min (Method A) |
| **1014** | 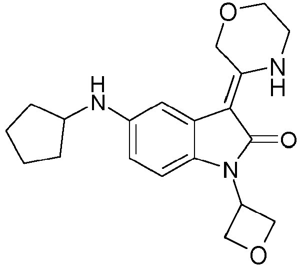 | D3 | 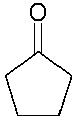 | (M+H)⁺ = 356 | 1.35 min (Method A) |
| **1015** | 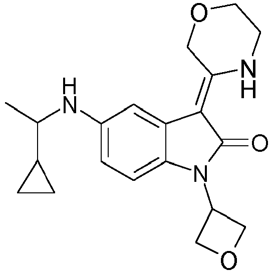 | D3 | 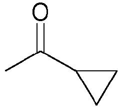 | (M+H)⁺ = 356 | 1.33 min (Method A) |
| **1016** | 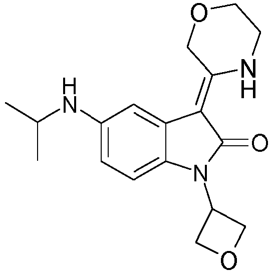 | D3 | 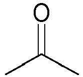 | (M+H)⁺ = 330 | 1.22 min (Method A) |
| **1017** | 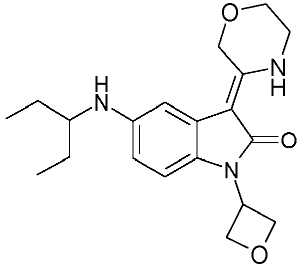 | D3 | 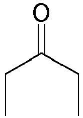 | (M+H)⁺ = 358 | 1.42 min (Method A) |
| **1018** | 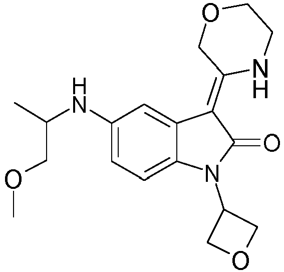 | D3 | 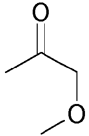 | (M+H)⁺ = 360 | 1.18 min (Method A) |
| **1019** | 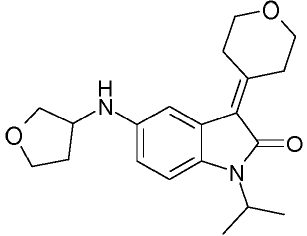 | Example 176 | 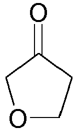 | (M+H)⁺ = 343 | 1.30 min (Method A) |
| **1020** | 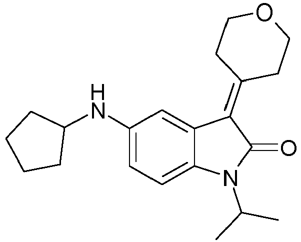 | Example 176 | 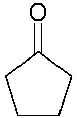 | (M+H)⁺ = 341 | 1.53 min (Method A) |
| **1021** | 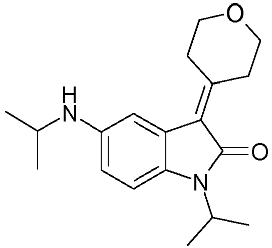 | Example 176 | 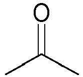 | (M+H)⁺ = 315 | 1.44 min (Method A) |
| **1022** | 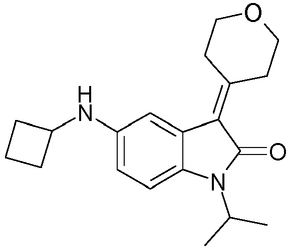 | Example 176 | 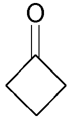 | (M+H)⁺ = 327 | 1.48 min (Method A) |
| **1023** | 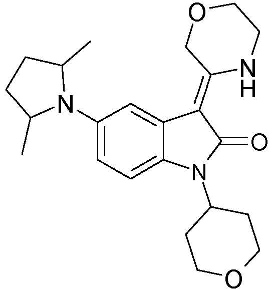 | Example 173 | 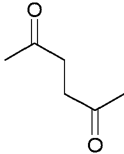 | (M+H)⁺ = 398 | 1.53 min (Method A) |
| **1024** | 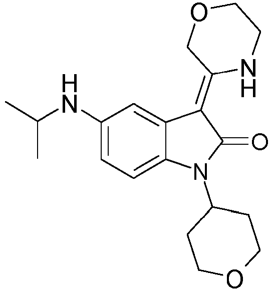 | Example 173 | 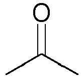 | (M+H)⁺ = 358 | 0.83 min (Method C) |
| **1025** | 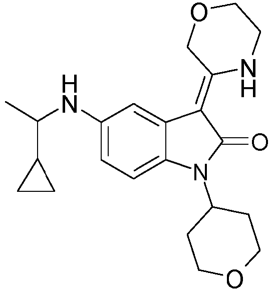 | Example 173 | 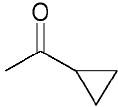 | (M+H)⁺ = 384 | 1.38 min (Method A) |
| **1026** | 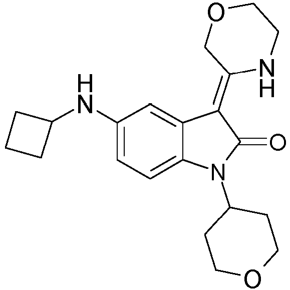 | Example 173 | 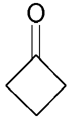 | (M+H)⁺ = 370 | 1.33 min (Method A) |
| **1027** | 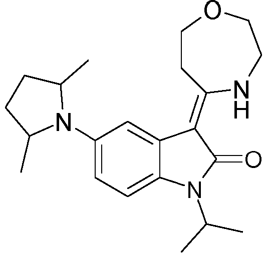 | D4 | 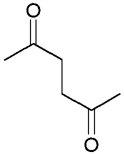 | (M+H)⁺ = 370 | 0.74 min (Method D) |
| **1028** | 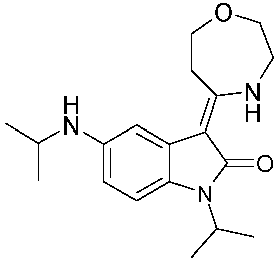 | D4 | 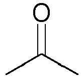 | (M+H)⁺ = 330 | 1.37 min (Method A) |
| **1029** | 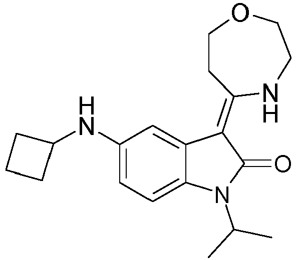 | D4 | 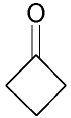 | (M+H)⁺ = 342 | 1.42 min (Method A) |
| **1030** | 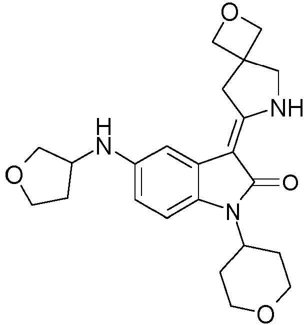 | D5 | 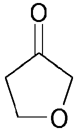 | (M+H)⁺ = 412 | 0.78 min (Method B) |
| **1031** | 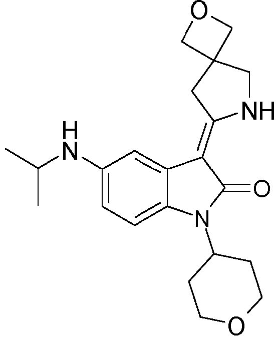 | D5 | 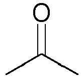 | (M+H)⁺ = 384 | 0.84 min (Method B) |
| **1032** | 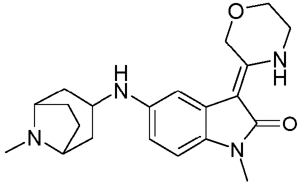 | D6 | 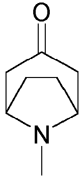 | (M+H)⁺ = 369 | |
| **1033** | 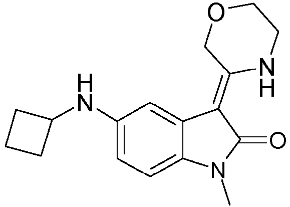 | D6 | 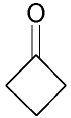 | (M+H)⁺ = 300 | 1.29 min (Method A) |
| **1034** | 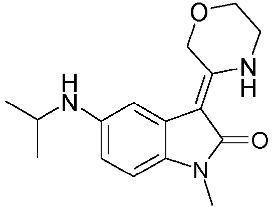 | D6 | 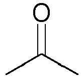 | (M+H)⁺ = 288 | 1.23 min (Method A) |
| **1035** | 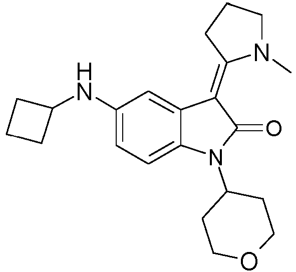 | D7 | 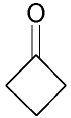 | (M+H)⁺ = 368 | 1.31 min (Method A) |
| **1036** | 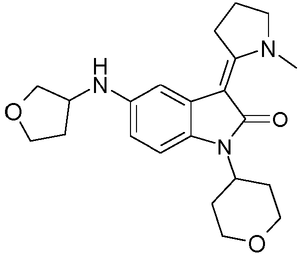 | D7 | 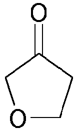 | (M+H)⁺ = 384 | 1.12 min (Method A) |
| **1037** | 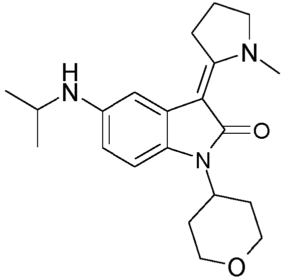 | D7 | 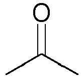 | (M+H)⁺ = 356 | 1.26 min (Method A) |
| **1038** | 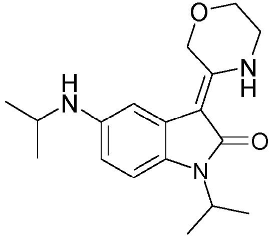 | D8 | 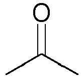 | (M+H)⁺ = 316 | 0.9 min (Method C) |
| **1039** | 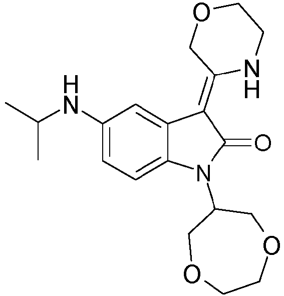 | D9 | 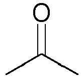 | (M+H)⁺ = 374 | 1.28 min (Method A) |
| **1040** | 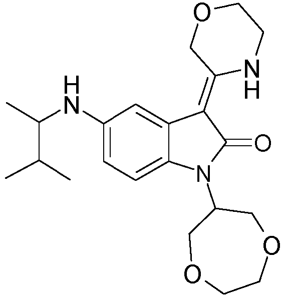 | D9 | 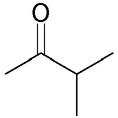 | (M+H)⁺ = 402 | 1.45 min (Method A) |
| **1041** | 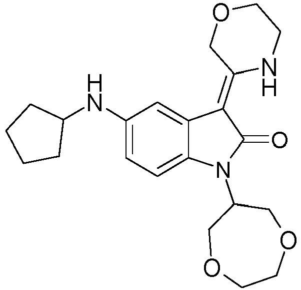 | D9 | 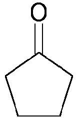 | (M+H)⁺ = 400 | 1.39 min (Method A) |
| **1042** | 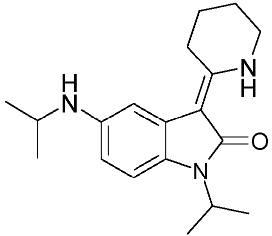 | D10 | 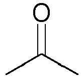 | (M+H)⁺ = 314 | 1.42 min (Method A) |
| **1043** | 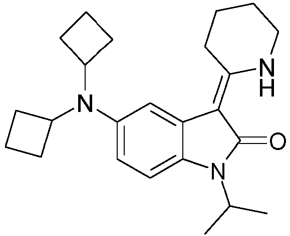 | D10 | 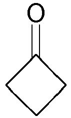 | (M+H)⁺ = 380 | 1.66 min (Method A) |
| **1044** | 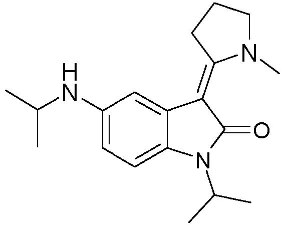 | D11 | 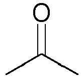 | (M+H)⁺ = 314 | 0.67 min (Method A) |
| **1045** | 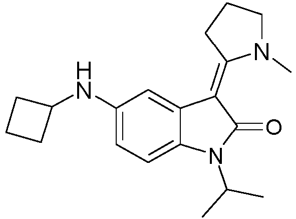 | D11 | 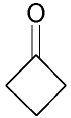 | (M+H)⁺ = 326 | 0.81 min (Method A) |
| **1046** | 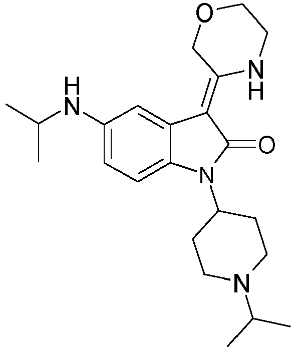 | D12 | 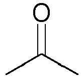 | (M+H)⁺ = 399 | 0.71 min (Method B) |
| | 7 eq. of acetone and 4 eq. of borohydride were used, due to double alkylation | | | | |
| **1047** | 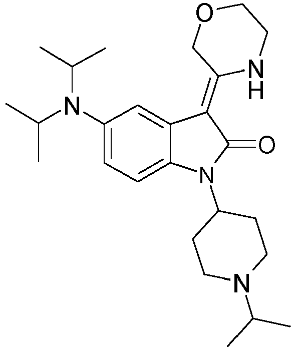 | D12 | 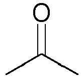 | (M+H)⁺ = 441 | 0.74 min (Method B) |
| | 7 eq. of acetone and 4 eq. of borohydride were used, due to triple alkylation | | | | |
| **1048** | 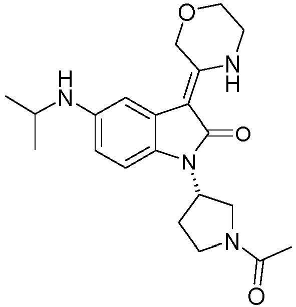 | D13 | 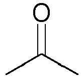 | (M+H)⁺ = 385 | 1.21 min (Method A) |
| **1049** | 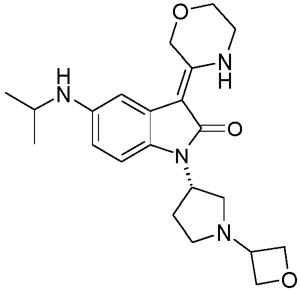 | D14 | 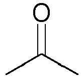 | (M+H)⁺ = 399 | 0.63 min (Method B) |
| **1050** | 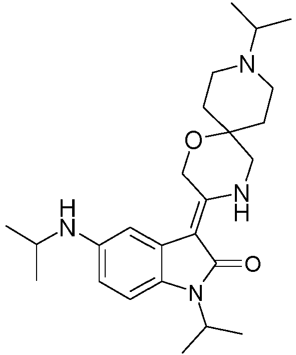 | D15 | 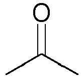 | (M+H)⁺ = 427 | 1.46 min (Method A) |
| **1051** | 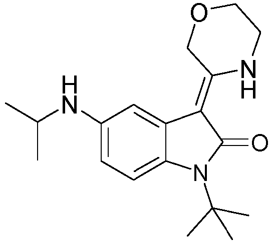 | D16 | 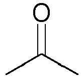 | (M+H)⁺ = 330 | 1.45 min (Method A) |
| **1052** | 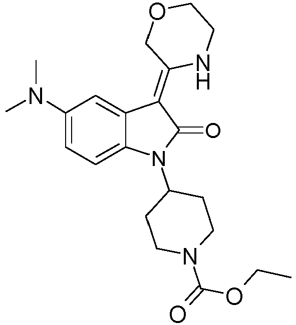 | D18 | 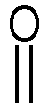 | (M+H)⁺ = 415 | 0.94 min (Method B) |
| **1053** | 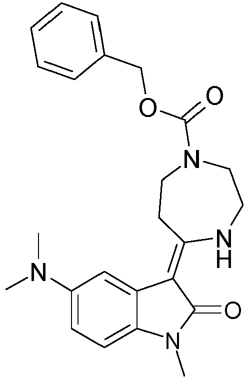 | D19 | 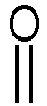 | (M+H)⁺ = 421 | 1.45 min (Method F) |
| **1054** | 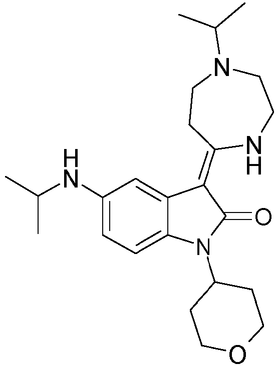 | D20 | 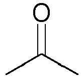 | (M+H)⁺ = 413 | 1.45 min (Method B) |
| | 5 eq. of acetone and 2 eq. of borohydride were used, due to double alkylation | | | | |
| **1055** | 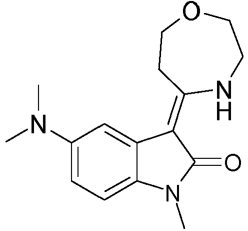 | D21 | 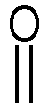 | (M+H)⁺ = 288 | 0.86 min (Method F) |
| **1056** | 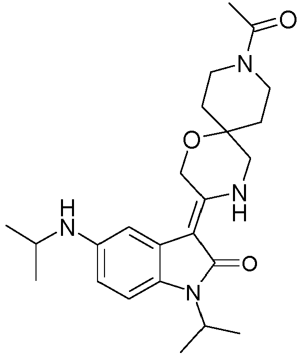 | D22 | 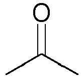 | (M+H)⁺ = 427 | 0.97 min (Method B) |
| **1057** | 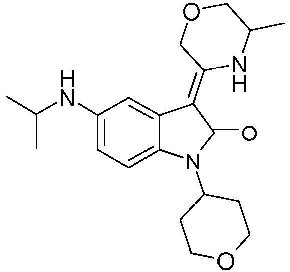 | D23 | 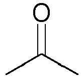 | (M+H)⁺ = 372 | 0.98 min (Method B) |
| **1058** | 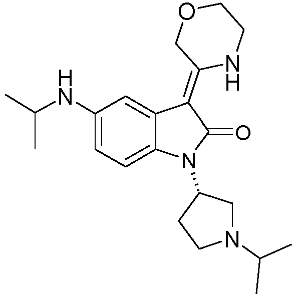 | D24 | 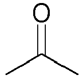 | (M+H)⁺ = 385 | 0.7 min (Method B) |
| | 5 eq. of acetone and 2 eq. of borohydride were used, due to double alkylation | | | | |
| **1059** | 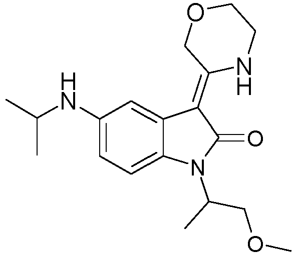 | D25 | 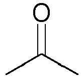 | (M+H)⁺ = 346 | 0.85 min (Method B) |
| **1060** | 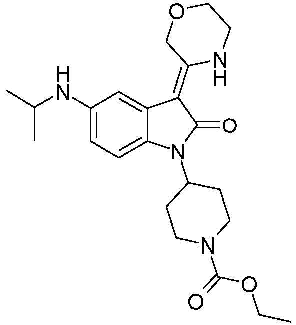 | D18 | 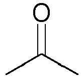 | (M+H)⁺ = 429 | 1.01 min (Method B) |
| **1061** | 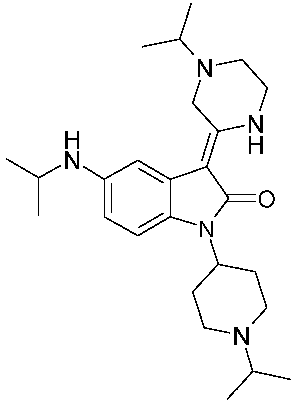 | D26 | 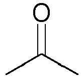 | (M+H)⁺ = 440 | 1.51 min (Method A) |
| **1062** | 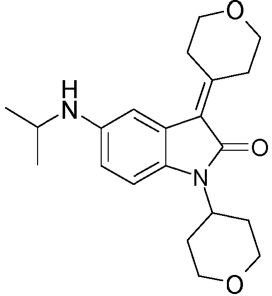 | D27 | 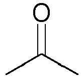 | (M+H)⁺ = 440 | 0.97 min (Method G) |
| **1063** | 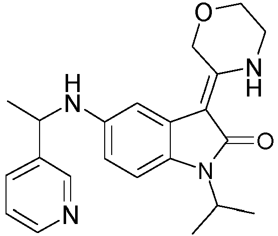 | D8 | 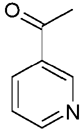 | (M+H)⁺ = 379 | 0.29 min (Method E) |
| **1064** | 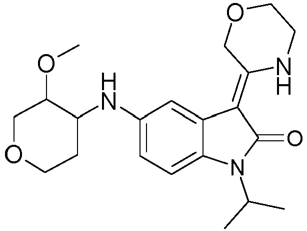 | D8 | 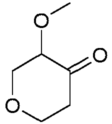 | (M+H)⁺ = 388 | 0.30 min (Method E) |
| **1065** | 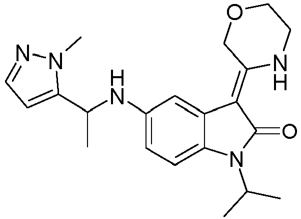 | D8 | 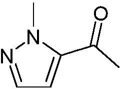 | (M+H)⁺ = 381 | 0.30 min (Method E) |
| **1066** | 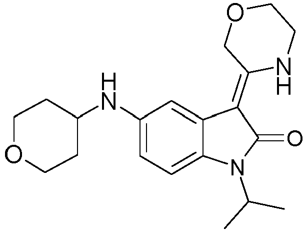 | D8 | 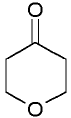 | (M+H)⁺ = 358 | 0.30 min (Method E) |
| **1067** | 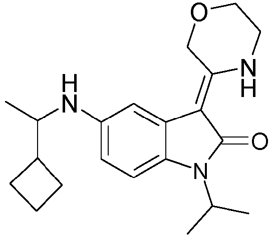 | D8 | 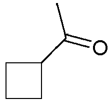 | (M+H)⁺ = 356 | 0.37 min (Method E) |
| **1068** | 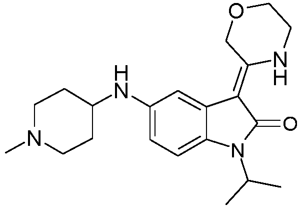 | D8 | 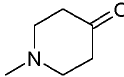 | (M+H)⁺ = 371 | 0.24 min (Method E) |
| **1069** | 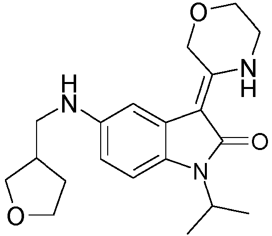 | D8 | 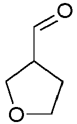 | (M+H)⁺ = 358 | 0.30 min (Method E) |
| **1070** | 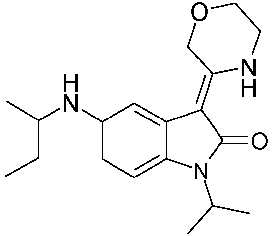 | D8 | 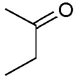 | (M+H)⁺ = 330 | 0.34 min (Method E) |
| **1071** | 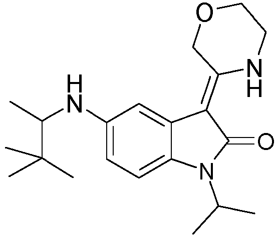 | D8 | 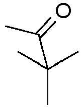 | (M+H)⁺ = 358 | 0.38 min (Method E) |
| **1072** | 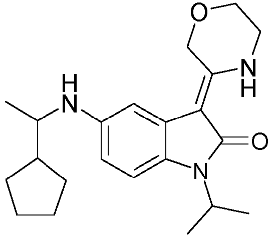 | D8 | 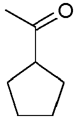 | (M+H)⁺ = 370 | 0.39 min (Method E) |
| **1073** | 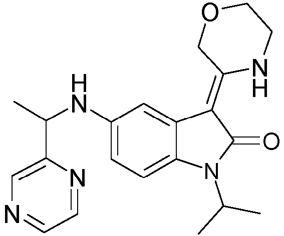 | D8 | 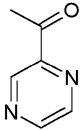 | (M+H)⁺ = 380 | 0.31 min (Method E) |
| **1074** | 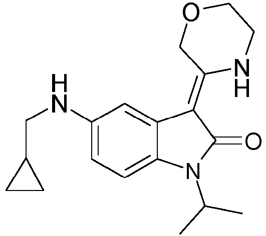 | D8 | 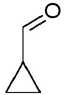 | (M+H)⁺ = 328 | 0.33 min (Method E) |
| **1075** | 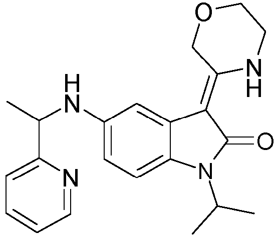 | D8 | 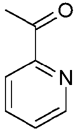 | (M+H)⁺ = 379 | 0.32 min (Method E) |
| **1076** | 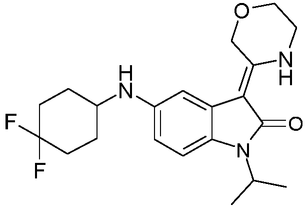 | D8 | 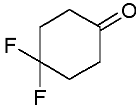 | (M+H)⁺ = 392 | 0.36 min (Method E) |
| **1077** | 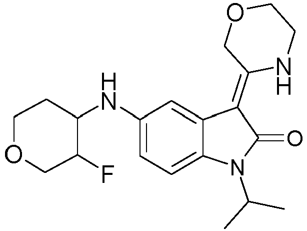 | D8 | 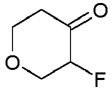 | (M+H)⁺ = 376 | 0.30 min (Method E) |
| **1078** | 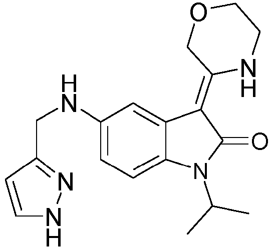 | D8 | 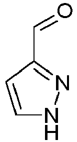 | (M+H)⁺ = 354 | 0.28 min (Method E) |
| **1079** | 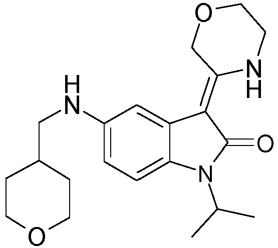 | D8 | 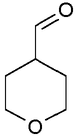 | (M+H)⁺ = 372 | 0.31 min (Method E) |
| **1080** | 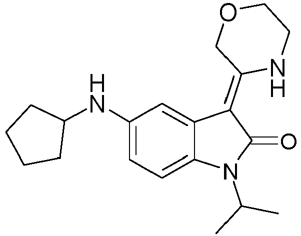 | D8 | 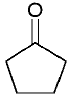 | (M+H)⁺ = 342 | 0.35 min (Method E) |
| **1081** | 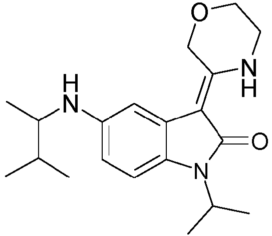 | D8 | 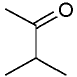 | (M+H)⁺ = 344 | 0.36 min (Method E) |
| **1082** | 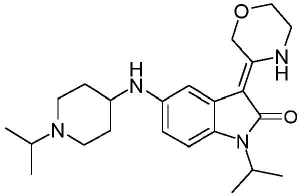 | D8 | 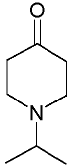 | (M+H)⁺ = 399 | 0.26 min (Method E) |
| **1083** | 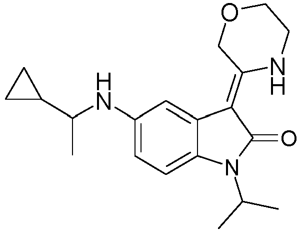 | D8 | 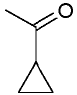 | (M+H)⁺ = 342 | 0.34 min (Method E) |
| **1084** | 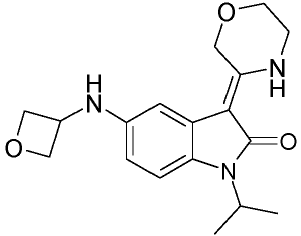 | D8 | 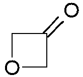 | (M+H)⁺ = 330 | 0.31 min (Method E) |
| **1085** | 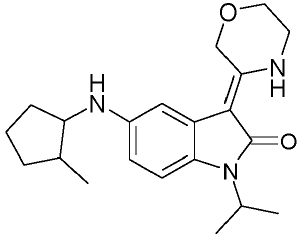 | D8 | 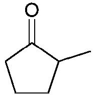 | (M+H)⁺ = 356 | 0.34 min (Method E) |
| **1086** | 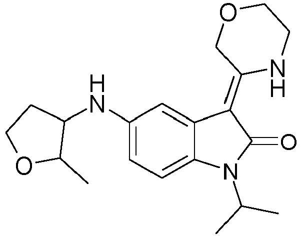 | D8 | 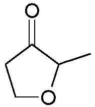 | (M+H)⁺ = 358 | 0.31 min (Method E) |
| **1087** | 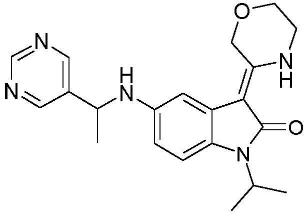 | D8 | 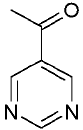 | (M+H)⁺ = 380 | 0.30 min (Method E) |
| **1088** | 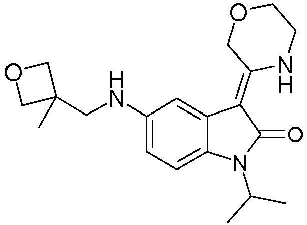 | D8 | 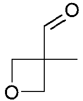 | (M+H)⁺ = 358 | 0.30 min (Method E) |
| **1089** | 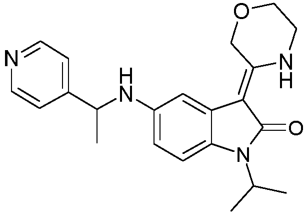 | D8 | 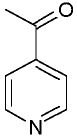 | (M+H)⁺ = 379 | 0.30 min (Method E) |
| **1090** | 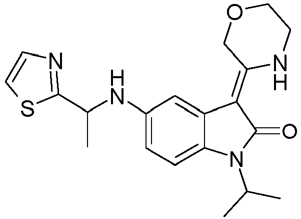 | D8 | 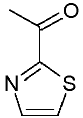 | (M+H)⁺ = 385 | 0.36 min (Method E) |
| **1091** | 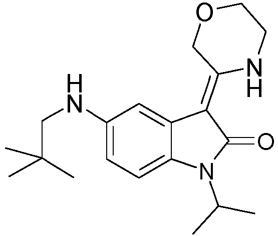 | D8 | 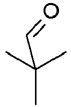 | (M+H)⁺ = 344 | 0.37 min (Method E) |
| **1092** | 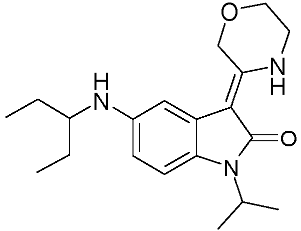 | D8 | 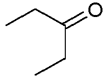 | (M+H)⁺ = 344 | 0.36 min (Method E) |
| **1093** | 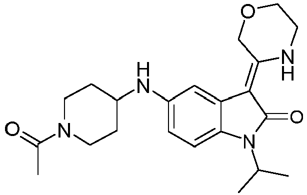 | D8 | 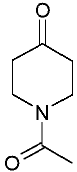 | (M+H)⁺ = 399 | 0.29 min (Method E) |
| **1094** | 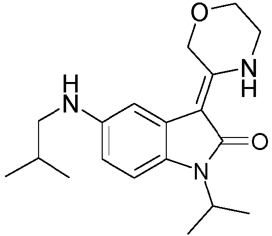 | D8 | 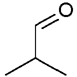 | (M+H)⁺ = 330 | 0.35 min (Method E) |
| **1095** | 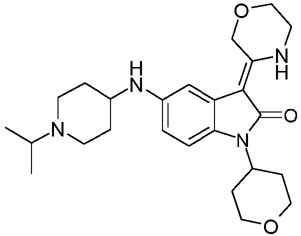 | Example 173 | 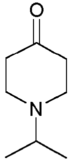 | (M+H)⁺ = 441 | 0.24 min (Method E) |
| **1096** | 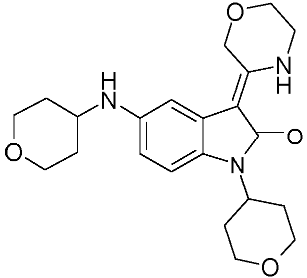 | Example 173 | 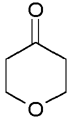 | (M+H)⁺ = 400 | 0.28 min (Method E) |
| **1097** | 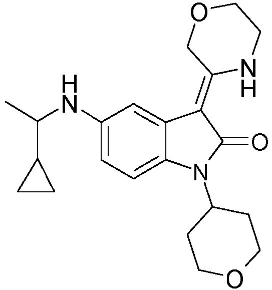 | Example 173 | 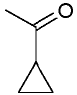 | (M+H)⁺ = 384 | 0.32 min (Method E) |
| **1098** | 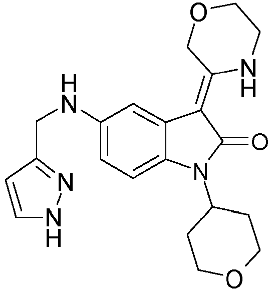 | Example 173 | 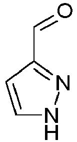 | (M+H)⁺ = 396 | 0.26 min (Method E) |
| **1099** | 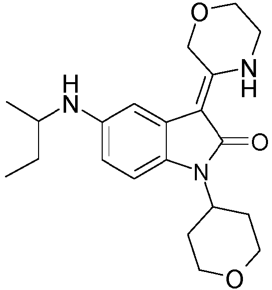 | Example 173 | 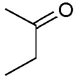 | (M+H)⁺ = 372 | 0.31 min (Method E) |
| **1100** | 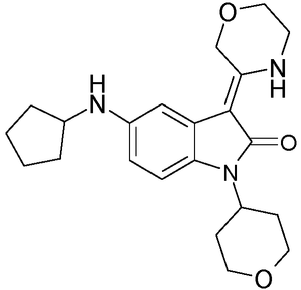 | Example 173 | 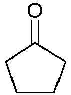 | (M+H)⁺ = 384 | 0.32 min (Method E) |
| **1101** | 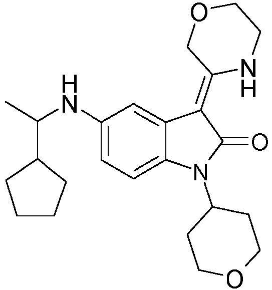 | Example 173 | 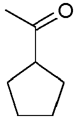 | (M+H)⁺ = 412 | 0.37 min (Method E) |
| **1102** | 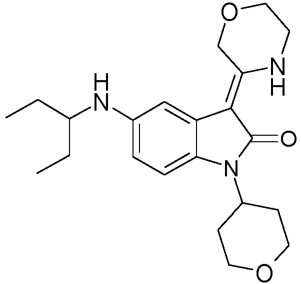 | Example 173 | 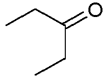 | (M+H)⁺ = 386 | 0.33 min (Method E) |
| **1103** | 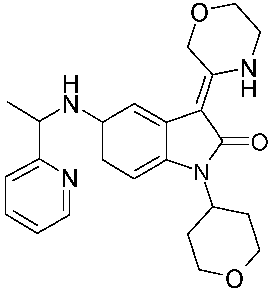 | Example 173 | 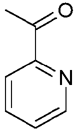 | (M+H)⁺ = 421 | 0.29 min (Method E) |
| **1104** | 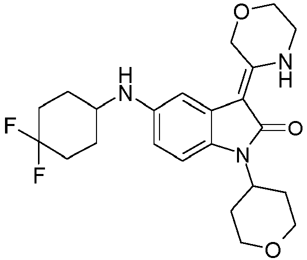 | Example 173 | 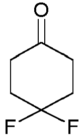 | (M+H)⁺ = 434 | 0.34 min (Method E) |
| **1105** | 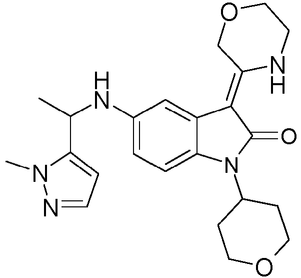 | Example 173 | 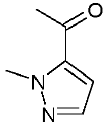 | (M+H)⁺ = 424 | 0.28 min (Method E) |
| **1106** | 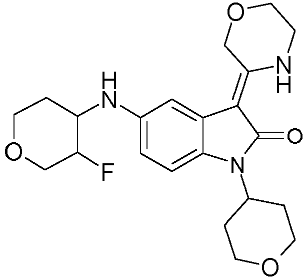 | Example 173 | 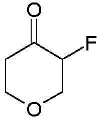 | (M+H)⁺ = 418 | 0.28 min (Method E) |
| **1107** | 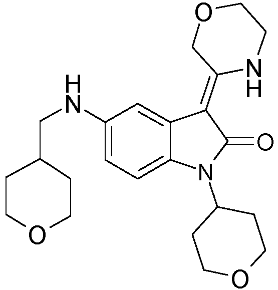 | Example 173 | 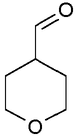 | (M+H)⁺ = 414 | 0.29 min (Method E) |
| **1108** | 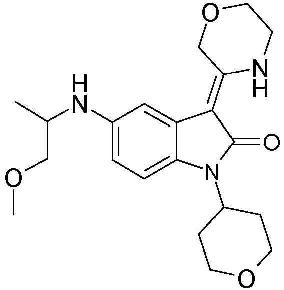 | Example 173 | 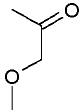 | (M+H)⁺ = 388 | 0.29 min (Method E) |
| **1109** | 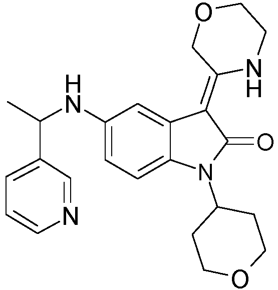 | Example 173 | 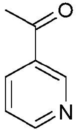 | (M+H)⁺ = 421 | 0.27 min (Method E) |
| **1110** | 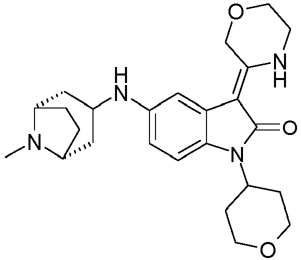 | Example 173 | 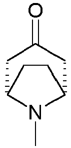 | (M+H)⁺ = 439 | 0.27 min (Method E) |
| **1111** | 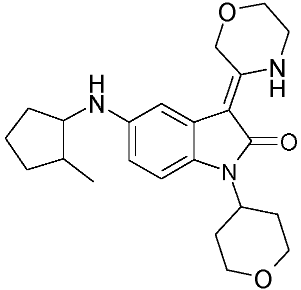 | Example 173 | 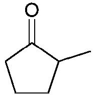 | (M+H)⁺ = 398 | 0.34 min (Method E) |
| **1112** | 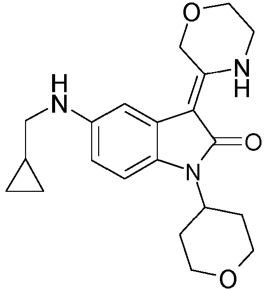 | Example 173 | 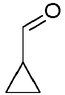 | (M+H)⁺ = 370 | 0.30 min (Method E) |
| **1113** | 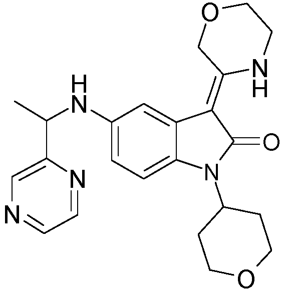 | Example 173 | 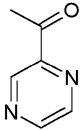 | (M+H)⁺ = 422 | 0.29 min (Method E) |
| **1114** | 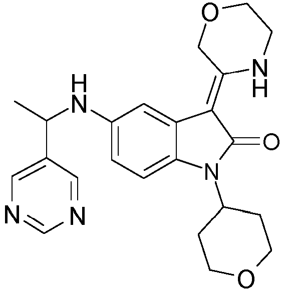 | Example 173 | 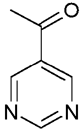 | (M+H)⁺ = 422 | 0.28 min (Method E) |
| **1115** | 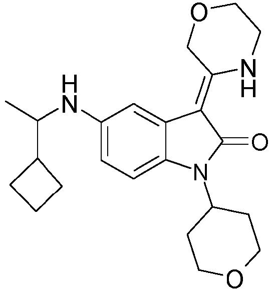 | Example 173 | 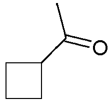 | (M+H)⁺ = 398 | 0.34 min (Method E) |
| **1116** | 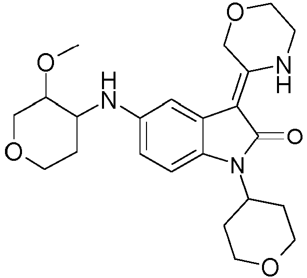 | Example 173 | 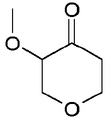 | (M+H)⁺ = 430 | 0.28 min (Method E) |
| **1117** | 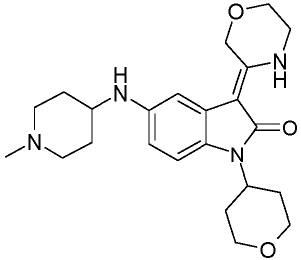 | Example 173 | 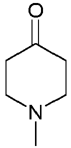 | (M+H)⁺ = 413 | 0.23 min (Method E) |
| **1118** | 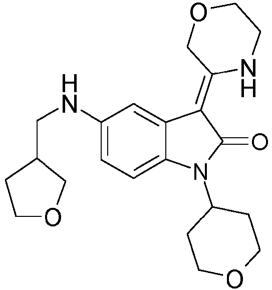 | Example 173 | 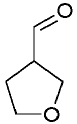 | (M+H)⁺ = 400 | 0.28 min (Method E) |
| **1119** | 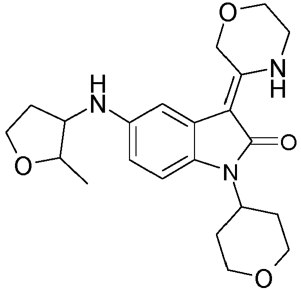 | Example 173 | 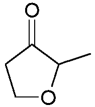 | (M+H)⁺ = 400 | 0.29 min (Method E) |
| **1120** | 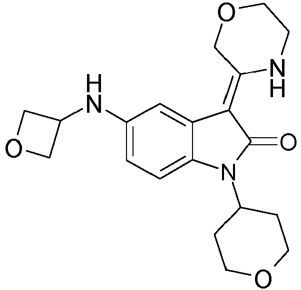 | Example 173 | 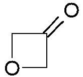 | (M+H)⁺ = 372 | 0.28 min (Method E) |
| **1121** | 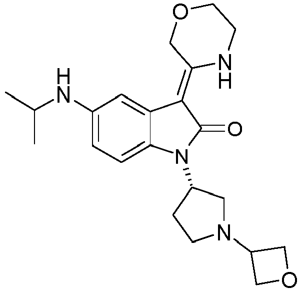 | D14 | 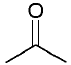 | (M+H)⁺= 399 | 0.63 min (Method B) |
| **1122** | 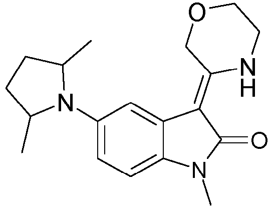 | D6 | 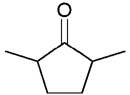 | (M+H)⁺= 328 | 1.52 min (Method A) |
| **1123** | 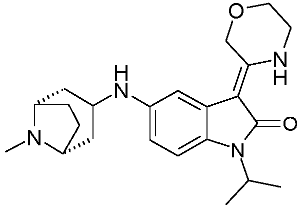 | D8 | 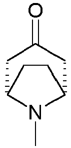 | (M+H)⁺= 397 | 0.29 min (Method E) |

### Example 26: Synthesis of compounds 1124 to 1147

### Compound 1124:

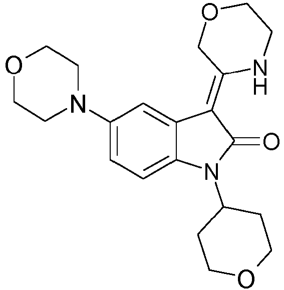

Compound 1173 (70 mg; 0.22 mmol), 2-iodo-2-(-2-iodo-ethoxy)-ethane (32 µL; 0.22 mmol) and potassium carbonate (61 mg; 0.44 mmol) in DMF (3 mL) are stirred for 2 h at 70°C. Additional 2-iodo-2-(2-thoxy)-ethane (0.22 mmol) and potassium carbonate (0.44 mmol) are added. The mixture is stirred at 70°C for 1.5 h and at room temperature for 3 days. The mixture is diluted with NaHCO₃ (aq. solution; 9%) and extracted with EA. The organic layer is washed with brine, separated, dried and evaporated. The residue is purified by preparative HPLC (eluent A: water + 0.1 % conc. Ammonia, eluent B: MeOH).
MS (ESI⁺): m/z = 386 [M+H]⁺
HPLC (Method A): Rₜ = 1.19 min

In analogy to the preparation of example 1124 the following compounds are obtained:

| **Nr.** | **Structure** | **Educt 1** | **Educt 2** | **Mass signal(s)** | **Rₜ** |
|---|---|---|---|---|---|
| **1125** | 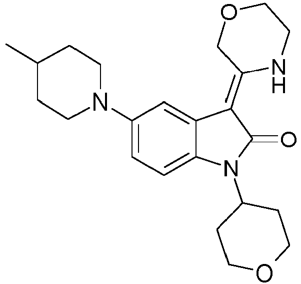 | Example 1173 | 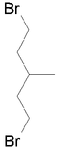 | (M+H)⁺= 398 | 1.51 min (Method A) |
| **1126** | 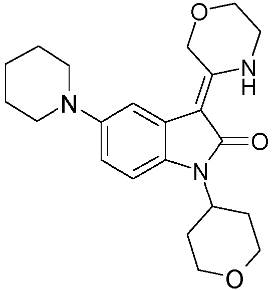 | Example 1173 | 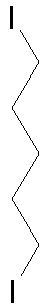 | (M+H)⁺= 384 | 1.43 min (Method A) |
| **1127** | 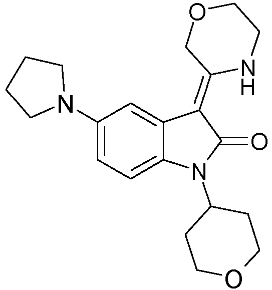 | Example 1173 | 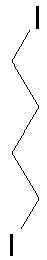 | (M+H)⁺= 370 | 0.83 min (Method G) |
| **1128** | 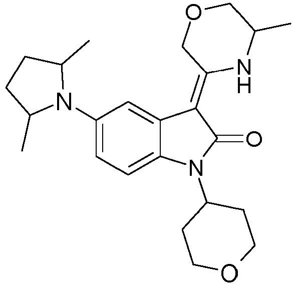 | D23 | 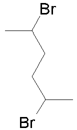 | (M+H)⁺= 412 | 1.58 min (Method A) |
| **1129** | 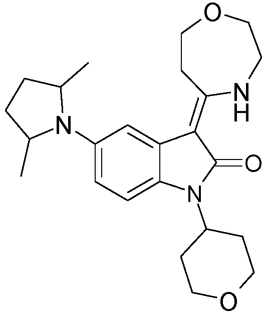 | D2 | 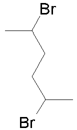 | (M+H)⁺= 412 | 0.91 min (Method B) |
| **1130** | 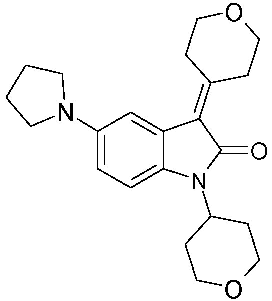 | D27 | 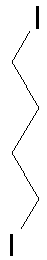 | (M+H)⁺= 369 | 1.51 min (Method A) |
| **1131** | 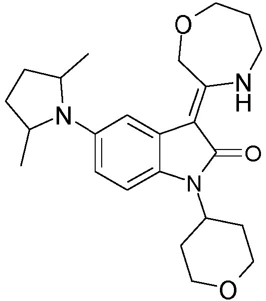 | D17 | 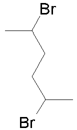 | (M+H)⁺= 412 | 1.54 min (Method A) |
| **1132** | 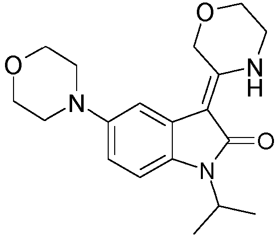 | D8 | 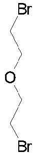 | (M+H)⁺= 342 | 1.29 min (Method A) |
| **1133** | 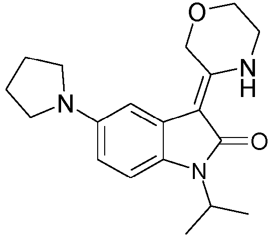 | D8 | 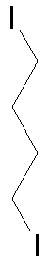 | (M+H)⁺= 328 | 0.83 min (Method C) |
| **1134** | 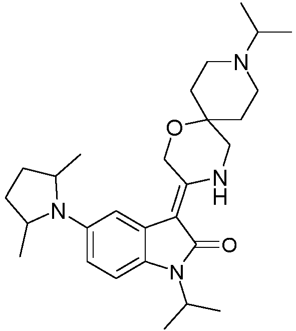 | D15 | 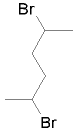 | (M+H)⁺= 467 | 1.71 min (Method A) |
| **1135** | 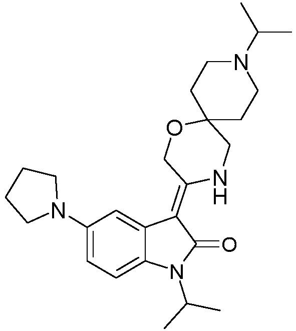 | D15 | 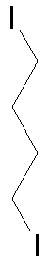 | (M+H)⁺= 439 | 1.67 min (Method A) |
| **1136** | 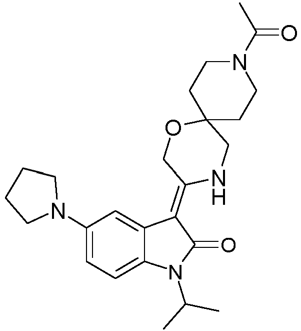 | D22 | 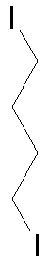 | (M+H)⁺= 439 | 0.93 min (Method B) |
| **1137** | 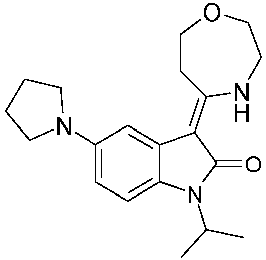 | D4 | 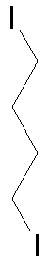 | (M+H)⁺= 342 | 0.72 min (Method D) |
| **1138** | 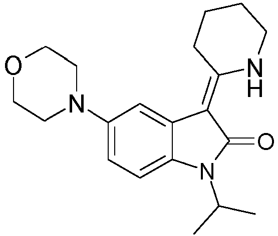 | D10 | 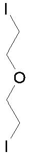 | (M+H)⁺= 342 | 1.36 min (Method A) |
| **1139** | 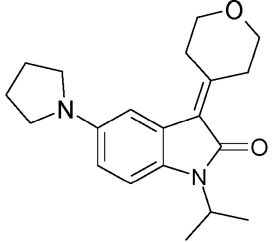 | Example 1177 | 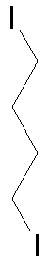 | (M+H)⁺= 327 | 1.58 min (Method A) |
| **1140** | 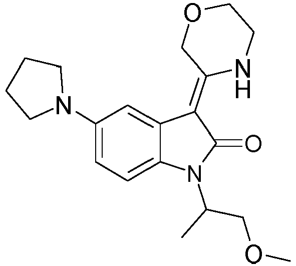 | D25 | 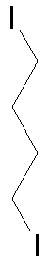 | (M+H)⁺= 358 | 0.81 min (Method B) |
| **1141** | 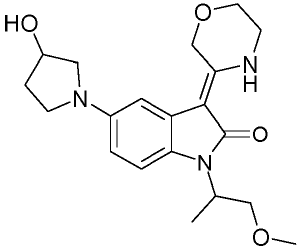 | D25 | 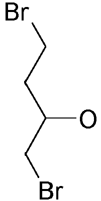 | (M+H)⁺= 374 | 0.8 min (Method B) |
| **1142** | 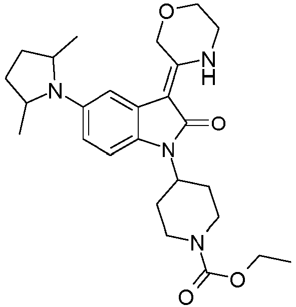 | D18 | 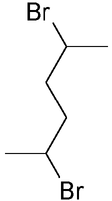 | (M+H)⁺= 442 | 1.05 min (Method B) |
| **1143** | 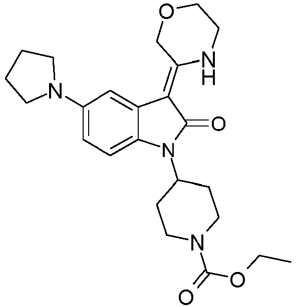 | D18 | 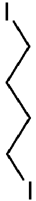 | (M+H)⁺= 441 | 0.99 min (Method B) |
| **1144** | 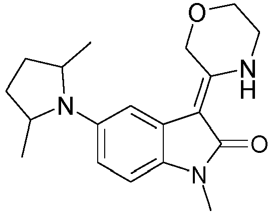 | D6 | 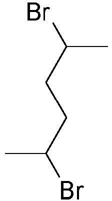 | (M+H)⁺= 328 | 1.52 min (Method A) |
| **1145** | 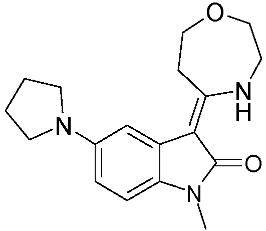 | D21 | 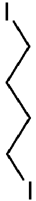 | (M+H)⁺= 314 | 0.92 min (Method F) |
| **1146** | 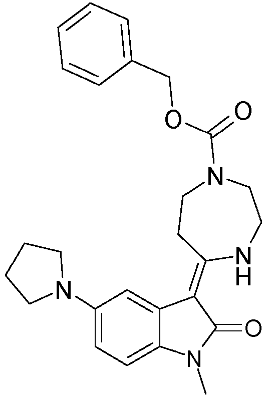 | D19 | 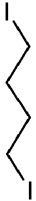 | (M+H)⁺= 447 | 1.17 min (Method F) |
| **1147** | 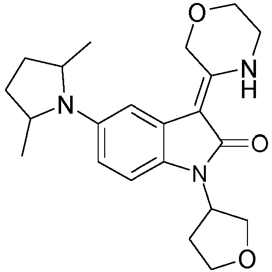 | D1 | 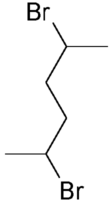 | (M+H)⁺= 384 | 0.87 min (Method B) |

### Example 27: synthesis of compounds 1148 to 1151

### Compound 1148:

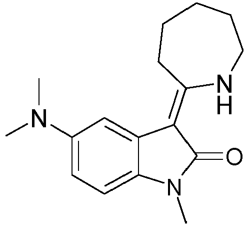

Intermediate E1 (200 mg; 1.05 mmol) and 7-methoxy-3,4,5,6-tetrahydro-2*H*-azepine (134 mg; 1.05 mmol) are stirred for 20 minutes in a microwave at 170°C. The residue is purified by preparative HPLC (eluent A: water + 0.1 % conc. Ammonia, eluent B: MeOH).
MS (ESI⁺): m/z = 286 [M+H]⁺
HPLC (Method H): Rₜ = 1.05 min

In analogy to the preparation of example 27 the following compounds are obtained:

| **Nr.** | **Structure** | **Educt 1** | **Educt 2** | **Mass signal(s)** | **Rₜ** |
|---|---|---|---|---|---|
| **1149** | 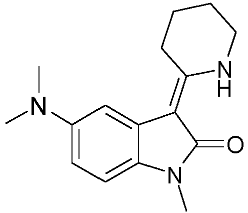 | E1 | 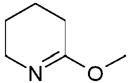 | (M+H)⁺= 272 | 0.98 min (Method H) |
| **1150** | 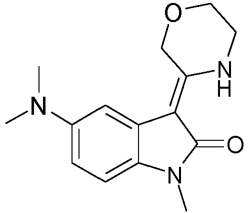 | E1 | 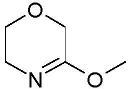 | (M+H)⁺= 274 | 0.43 min (Method I) |
| **1151** | 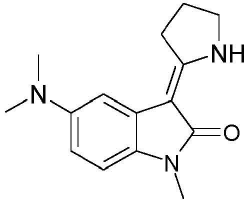 | E1 | 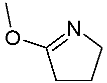 | (M+H)⁺= 258 | 0.89 min (Method H) |

### Example 28: synthesis of compounds 1152 to 1163

### Compound 1152:

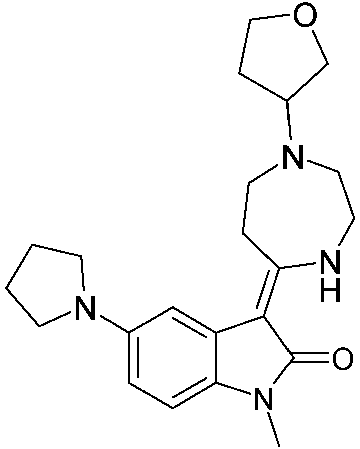

Intermediate F1 (30 mg; 0.10 mmol), 3-(2*H*)-furanone dihydrochloride (9 µL; 0.12 mmol) and glacial acetic acid (13 µL; 0.24 mmol) in MeOH (2 mL) are stirred at 50°C for 1 h. Sodium cyanoborohydride (12 mg; 0.19 mmol) is added and stirred at room temperature over night. The mixture is diluted with NaHCO₃ (aq. solution; 9%) and extracted with DCM. The organic layer is separated, dried and evaporated. The residue is purified by preparative HPLC (eluent A: water + 0.1 % conc. Ammonia, eluent B: MeOH).
MS (ESI⁺): m/z = 383 [M+H]⁺
HPLC (Method A): Rₜ = 1.42 min

In analogy to the preparation of example 1152 the following compounds are obtained:

| **Nr.** | **Structure** | **Educt 1** | **Educt 2** | **Mass signal(s)** | **Rₜ** |
|---|---|---|---|---|---|
| **1153** | 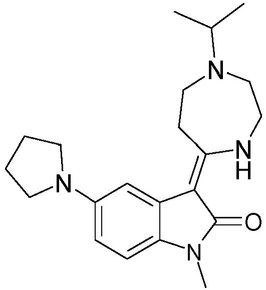 | F1 | 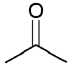 | (M+H)⁺= 355 | 1.54 min (Method A) |
| **1154** | 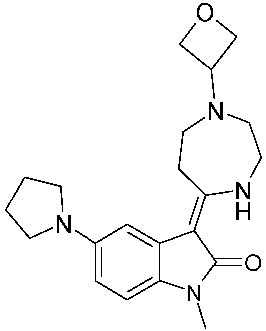 | F1 | 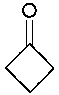 | (M+H)⁺= 369 | 1.38 min (Method A) |
| **1155** | 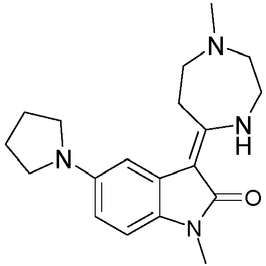 | F1 | 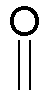 | (M+H)⁺= 327 | 1.42 min (Method A) |
| **1156** | 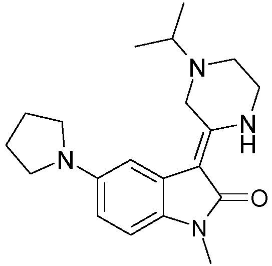 | F2 | 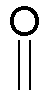 | (M+H)⁺= 341 | 1.53 min (Method A) |
| **1157** | 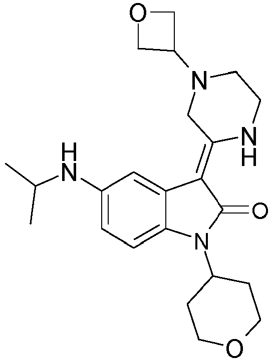 | F3 | 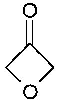 | (M+H)⁺= 413 | 1.24 min (Method A) |
| **1158** | 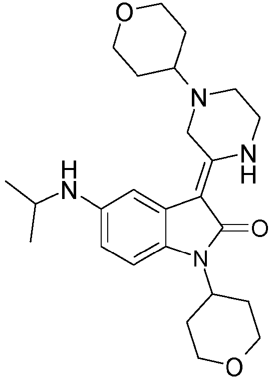 | F3 | 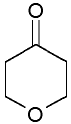 | (M+H)⁺= 441 | 1.30 min (Method A) |
| **1159** | 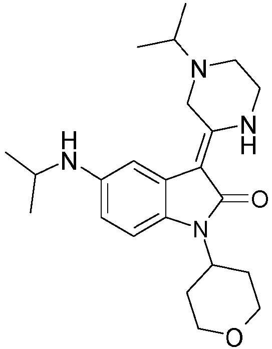 | F3 | 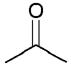 | (M+H)⁺= 399 | 1.38 min (Method A) |
| **1160** | 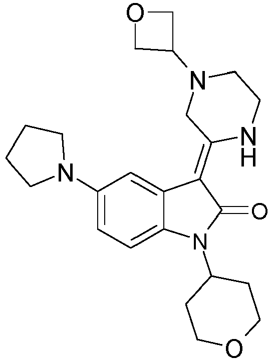 | F4 | 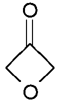 | (M+H)⁺ = 439 | 0.67 min (Method B) |
| **1161** | 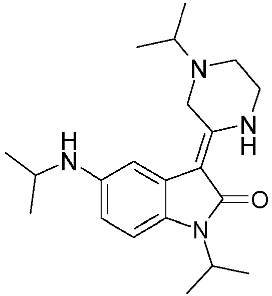 | F5 | 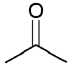 | (M+H)⁺= 357 | 1.45 min (Method A) |
| **1162** | 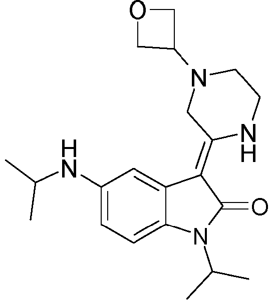 | F5 | 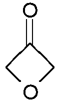 | (M+H)⁺= 371 | 1.34 min (Method A) |
| **1163** | 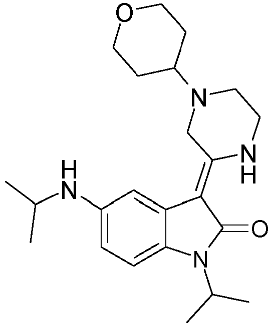 | F5 | 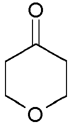 | (M+H)⁺= 399 | 1.38 min (Method A) |

### Example 29: synthesis of compounds 1164

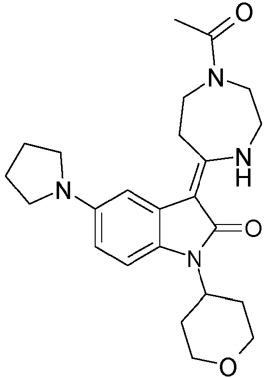

Intermediate F4 (100 mg; 0.26 mmol), acetic anhydride (37 µL; 0.39 mmol) and DIPEA (134 µL; 0.78 mmol) in DCM (3 mL) are stirred at room temperature for 1 h. The mixture is diluted with NaHCO₃ (aq. solution; 9%) and extracted with DCM. The organic layer is separated, dried and evaporated. The residue is taken up in MeOH and the precipitate is filtered off. The precipitate is purified by preparative HPLC (eluent A: water + 0.1 % conc. ammonia, eluent B: MeOH).
MS (ESI⁺): m/z = 425 [M+H]⁺
HPLC (Method B): Rₜ =0.84 min

### Example 30: synthesis of compound 1165 to 1166

### Compound 1165:

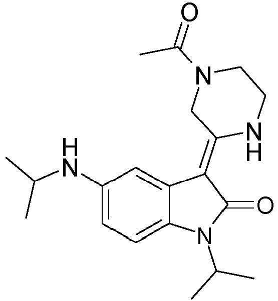

Intermediate F5 (30 mg; 0.10 mmol), acetyl chloride (6 µL; 0.09 mmol) and TEA (20 µL; 0.14 mmol) in THF (3 mL) are stirred at room temperature for 10 minutes. Additional acetyl chloride is added. The mixture is diluted with water and extracted with DCM. The organic layer is separated, dried and evaporated. The residue is purified by preparative HPLC (eluent A: water + 0.1 % TFA, eluent B: MeOH).
MS (ESI⁺): m/z = 357 [M+H]⁺
HPLC (Method D): Rₜ =0.62 min

In analogy to the preparation of compound 1165 the following compound is obtained:

| **Nr.** | **Structure** | **Educt 1** | **Educt 2** | **Mass signal(s)** | **Rₜ** |
|---|---|---|---|---|---|
| **1166** | 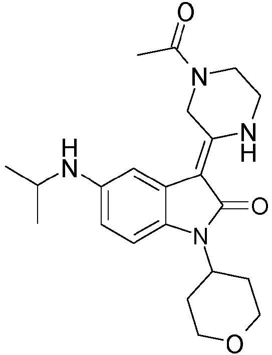 | F3 | 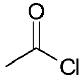 | (M+H)⁺= 399 | 0.88 min (Method J) |

### Example 31: synthesis of compounds 1167 to 1168

### Compound 1167:

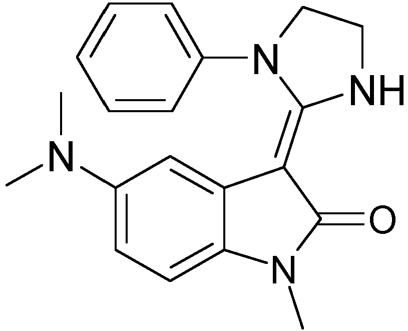

Intermediate G1 (90 mg; 0.31 mmol) and *N1*-phenyl-ethane-1,2-diamine (42 mg; 0.31 mmol) in *n*-butanol (2 mL) are stirred for 15 min in a microwave at 220°C. The solvent is evaporated and the residue is purified by preparative HPLC (eluent A: water + 0.1 % conc. ammonia, eluent B: MeOH).
MS (ESI⁺): m/z = 335 [M+H]⁺
HPLC (Method H): Rₜ = 1.02 min

In analogy to the preparation of example 1167 the following compounds are obtained:

| **Nr.** | **Structure** | **Educt 1** | **Educt 2** | **Mass signal(s)** | **Rₜ** |
|---|---|---|---|---|---|
| **1168** | 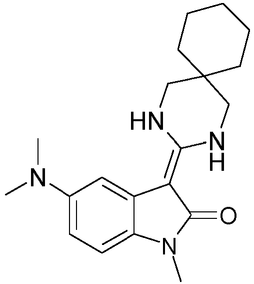 | G1 | 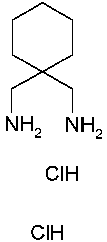 | (M+H)⁺= 341 | 1.16 min (Method J) |

### Example 32: synthesis of compound 1169 to 1171

### Compound 1169:

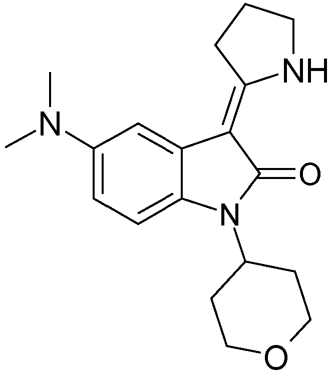

Intermediate C35 (100 mg; 0.26 mmol), formaldehyde (aq. solution; 37%; 0.19 mL; 2.58 mmol) and Raney-Nickel (50 mg) in MeOH (10 mL) are hydrogenated in a Parr apparatus (rt; 1.1 bar; 8 h). The catalyst is filtered off and the solvent is evaporated. The residue is stirred in MeOH, filtered off and dried.
MS (ESI⁺): m/z = 328 [M+H]⁺
HPLC (Method ?): Rₜ = 0.72 min

In analogy to the preparation of example 1169 the following compounds are obtained:

| **Nr.** | **Structure** | **Educt 1** | **Mass signal(s)** | **Rₜ** |
|---|---|---|---|---|
| **1170** | 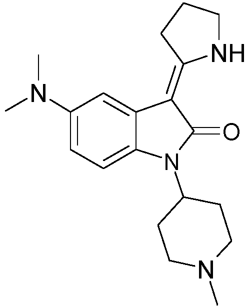 | C37 | (M+H)⁺ = 341 | 0.75 min (Method K) |
| | 15 eq. of formaldehyde used, due triple alkylation | | | |
| **1171** | 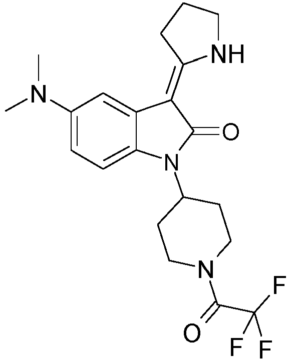 | C36 | (M+H)⁺ = 423 | 1.06 min (Method K) |

### Example 33: synthesis of compound 1172 to 1174

### Compound 1172:

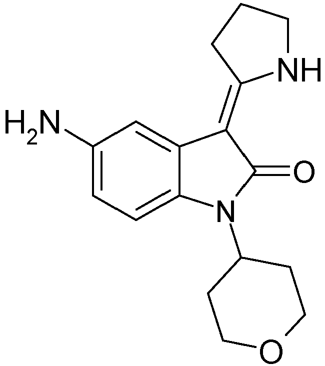

Intermediate C35 (200 mg; 0.52 mmol) and Raney-Nickel (50 mg) in MeOH (10 mL) are hydrogenated in a Parr apparatus (rt; 1.1 bar; 8 h). The catalyst is filtered off and the solvent is evaporated. The residue is purified by preparative HPLC (eluent A: water + 0.1 % conc. ammonia, eluent B: MeOH).
MS (ESI⁺): m/z = 300 [M+H]⁺
HPLC (Method L): Rₜ = 0.63 min

In analogy to the preparation of example 1172 the following compounds are obtained:

| **Nr.** | **Structure** | **Educt 1** | **Mass signal(s)** | **Rₜ** |
|---|---|---|---|---|
| **1173** | 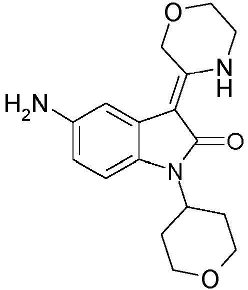 | C5 | (M+H)⁺ = 316 | 0.69 min (Method B) |
| **1174** | 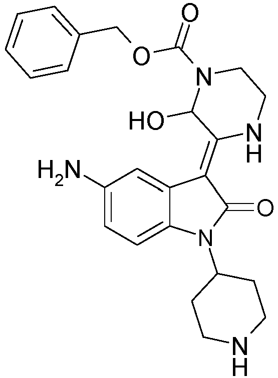 | C38 | (M+H)⁺ = 464 | 1.24 min (Method A) |

### Example 34: synthesis of compound 1175

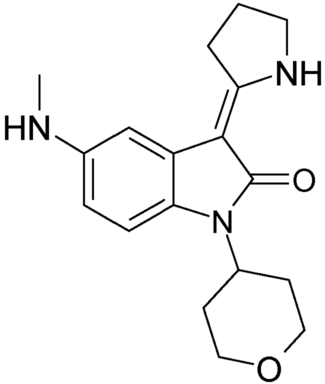

Intermediate C35 (200 mg; 0.52 mmol) and Raney-Nickel (50 mg) in MeOH (10 mL) are hydrogenated in a Parr apparatus (rt; 1.1 bar; 8 h). The catalyst is filtered off and the solvent is evaporated. The residue is purified by preparative HPLC (eluent A: water + 0.1 % conc. ammonia, eluent B: MeOH).
MS (ESI⁺): m/z = 314 [M+H]⁺
HPLC (Method M): Rₜ = 1.12 min

### Example 35: synthesis of compound 1176

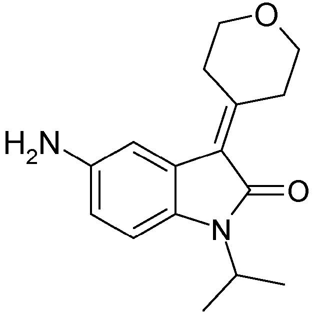

Intermediate C4 (1.78 g; 5.90 mmol) and powdered iron (1.78 g; 31.87 mmol) in water (56.5 mL) and ethanol (116 mL) are stirred at 80°C. Glacial acetic acid (3.58 mL; 62.55 mmol) are added drop wise and the mixture is stirred for 1 h at 80°C. The organic solvent is evaporated and the aq. layer is alkalised with NaOH (7 mL) and extracted with DCM. Iron is filtered off through celite. The organic layer is separated, dried and evaporated. The residue is purified by HPLC (eluent A: water + 0.15 % conc. ammonia, eluent B: MeOH).
MS (ESI⁺): m/z = 273 [M+H]⁺
HPLC (Method A): Rₜ = 1.16 min

### Example 36: synthesis of compound 1177

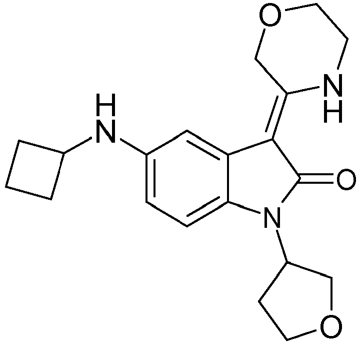

Intermediate D1 (150 mg; 0.50 mmol), bromo-cyclobutane (101 mg; 0.75 mmol) and potassium carbonate (138 mg; 1.00 mmol) in DMF (3 mL) are stirred at 70°C for 4 h. Additional bromo-cyclobutane (101 mg; 0.75 mmol) is added and the mixture is stirred at 80°C over night. Additional bromo-cyclobutane (101 mg; 0.75 mmol) is added. After stirring for 4 h at 90°C the mixture is purified by preparative HPLC (eluent A: water + 0.15 % conc. ammonia, eluent B: MeOH).
MS (ESI⁺): m/z = 356 [M+H]⁺
HPLC (Method B): Rₜ = 0.89 min

### Example 37: : synthesis of compound 1178

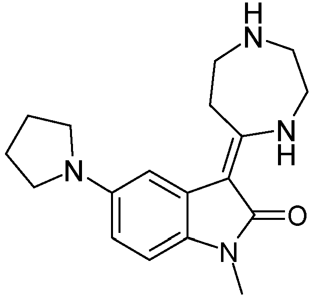

Example 146 (65 mg; 0.15 mmol) and Pd/C (10%; 10 mg) in MeOH (5 mL) is hydrogenated in a Parr apparatus (rt; 50 psi; 1 h). The catalyst is filtered off and the solvent is removed. The residue is purified by preparative HPLC (eluent A: water + 0.15 % conc. ammonia, eluent B: MeOH).
MS (ESI⁺): m/z = 313 [M+H]⁺
HPLC (Method A): Rₜ = 1.34 min

### Example 38: BIOLOGICAL ASSAYS

The biological activity of compounds is determined by the following methods:

### Assay A: Determination of Complex I mediated ROS-inhibition (CI)

Enzyme kinetic experiments permit the detection of ROS generated through Complex I. Herefore Complex I was purified from bovine heart (Sharpley et al. 2006 Biochemistry. 45(1):241-8.. First a subcellular fractionation was conducted to obtain a crude mitochondria fraction, followed by a hypotonoc lysis and differential centrifugation, froim which mitochondrial membranes were obtained. Solubilzation of mitochondrial membranes followed by an ion exchange chromatography and size exclusion chromatography resulted in enzyme preparations which contain Complex I with little contaminations of Complex IV.These preparations were used to study ROS-generation by Complex I, the substrate NADH (1 µM) and oxygen (ambient). The generated ROS are detected via the Oxiation of Amplex red in a coupled reaction containing Amplex Red and horse radish peroxidase

IC50 of a compound of the invention was estimated by testing the compound using a 8 point concentration-response experiment.

In 384-well microtiter plates 5 µl of test compound (final concentrations ranging from 0.01 nM to 30 µM, diluted in assay buffer and 1% DMSO final) or control was mixed with 5 µl of substrate mix (3 µM NADH, 10 µM AmplexRed, 1 mM Fructose 1,6 bis-phopshate and 1 mM AsO4). The enzymatic reaction was started by addition of 15 µl of enzyme mix (containing 20 µg/ml Complex I, 2 U/ml horse radish peroxidase, 1 U/ml Aldolase, 1 U/ml Trioseisomerase, 1 U/ml Glycerinaldehyd-3-phosphat-Dehydrogenase) and the generation of ROS was followed by measuring the increase in absorption at 557 nm every 53 seconds at room temperature for 12 minutes, followed by linear regression (slope analysis). To assess the potency of Compounds
IC50 values are calculated as 50% activity of Complex I by nonlinear regression curve fitting, using a 4-parameter sigmoidal dose-response model.

### Assay B: Determination of Cellular Protection (HT22)

To show selective pathway engagement in a cellular context, a mice neuroblastoma cells (HT-22) were depleted of the endogenous antioxidant glutathione resulting on oxidative stress on the mitochondrial and cellular level and cell death (Tan S, Sagara Y, Liu Y, Maher P, Schubert D. The regulation of reactive oxygen species production during programmed cell death. J Cell Biol. 1998;141:1423-1432). By incubation with high concentrations of Glutamate (5 mM) these cells are depleted of intracellular glutathione do to a inhibition of cystine uptake, which results in an accumulation of mitochondrial derived ROS and ultimately cell death.

In 384-well plates, 2000 HT-22 cells were seeded in 50 ul cell culture medium (DMEM containing 10% fetal calf serum and 1% penicillin/streptomycin) and were cultured for 24 hours, followed by the incubation with glutamate (to induce cell death) or vehicle (viable cells (100%) in presence of test compound (0.01-30 µM final) for 16 hours. Viability of the cells were assessed by adding 10% Alamar Blue reagent and incubation for 1h at 37°C, followed by measuring Fluorescence (excitation 530nm, emission 590nm.

To assess the potency of compounds, EC50 values were calculated by nonlinear regression curve fitting, using a 4-parameter sigmoidal dose-response model (see Complex I assay).

## Claims

1. A compound having formula (I) or a salt or hydrate or solvate thereof 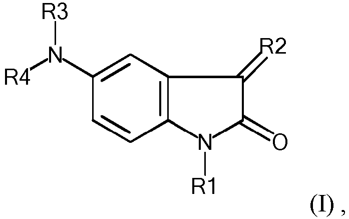 wherein:
**R1** is C₁₋₄-alkyl unsubstituted or substituted with MeO; tetrahydropyranyl, tetrahydrofuranyl, oxetanyl, dioxepanyl, pyrrolidinyl or piperidinyl; or pyrrolidinyl or piperidinyl with the nitrogen substituted by methyl, isopropyl, oxetanyl, ethoxycarbonyl, acetyl, or trifluoroacetyl;
**R2** is a 5-, 6- or 7-membered unsubstituted or substituted ring containing 1 or 2 heteroatoms selected from O or N or an unsubstituted or substituted spirocyclic heterocyclyl group containing 1 to 3 heteroatoms selected from N or O consisting of 4 to 11 ring atoms, the ring or spirocyclic heterocyclyl group being bound in formula (I) by a C=C double bond, and
in which ring or spirocyclic heterocyclyl group one N-atom can be substituted by methyl, isopropyl, acetyl, benzyloxycarbonyl, phenyl, oxetanyl or tetrahydropyranyl, and
in which one or more C-atoms can be substituted by -methyl or -OH.;
**R3** or **R4** are independently from one another
hydrogen; C₁₋₆-alkyl, unsubstituted or substituted with one or more F, methoxy, C₃₋₈-cycloalkyl unsubstituted or substituted with one or more F; aryl; heteroaryl consisting of 5 to 6 ring atoms; or heterocyclyl selected from the group consisting of oxetanyl, tetrahydropyranyl and pyrrolidinyl, said heterocyclyl being unsubstituted or substituted with C₁₋₆-alkyl, acetyl, tetrahydrofuranyl, oxetanyl or hydroxyethylacetyl;
or **R3** and **R4** together with the attached N form a heterocyclyl ring selected from the group consisting of morpholinyl and pyrrolidinyl both unsubstituted or substituted with C₁₋₆-alkyl, F, or hydroxyl.

2. The compound of claim 1 or a salt thereof, wherein **R1** is 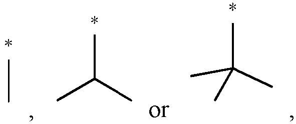 unsubstituted or substituted with MeO; 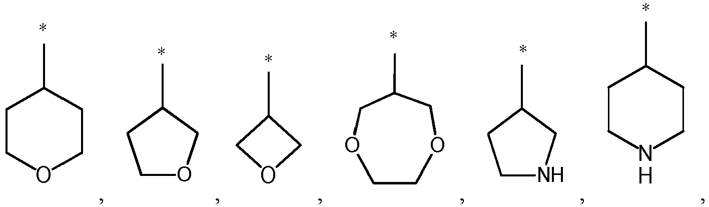 unsubstituted or the nitrogen substituted with

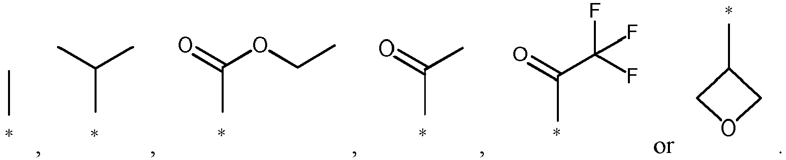

3. The compound of claim 1 or a salt thereof wherein **R1** is

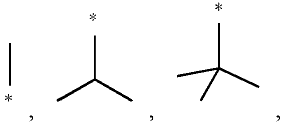

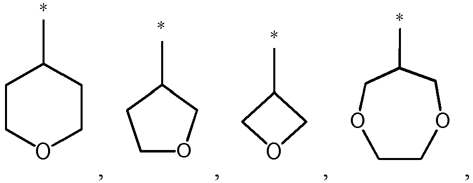

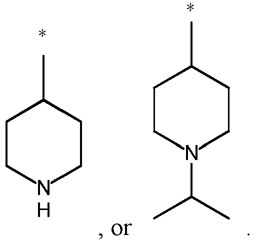

4. The compound of claim 2 or a salt thereof, wherein **R1** is tetrahydropyranyl, or dioxepanyl.

5. The compound of claim 1 or a salt thereof wherein **R2** is a 5-, 6- or 7-membered ring containing 1 or 2 heteroatoms selected from O or N bound in formula (1) by a C=C double bond,
in which one or both N-atoms can be substituted by methyl, isopropyl, acetyl, benzyloxycarbonyl, phenyl, oxetanyl or tetrahydropyranyl, and
in which one or more C-atoms can be substituted by -methyl or -OH.

6. The compound of claim 1 or a salt thereof, wherein the structural element 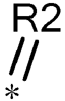 is

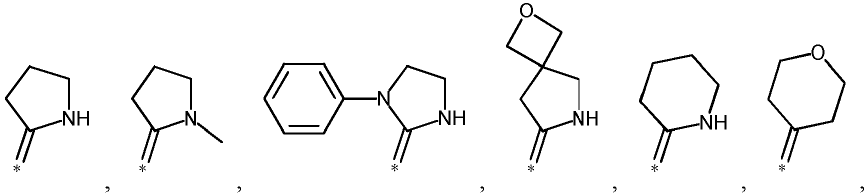

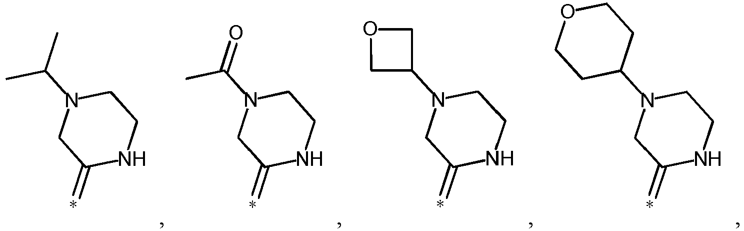

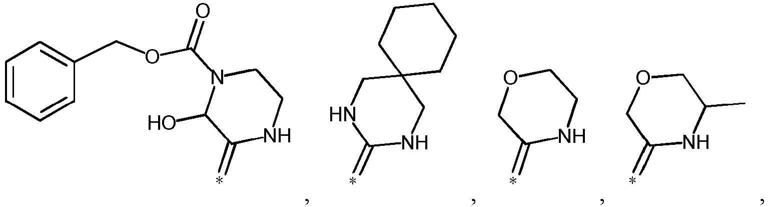

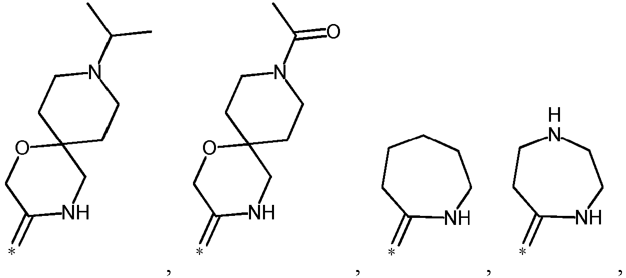

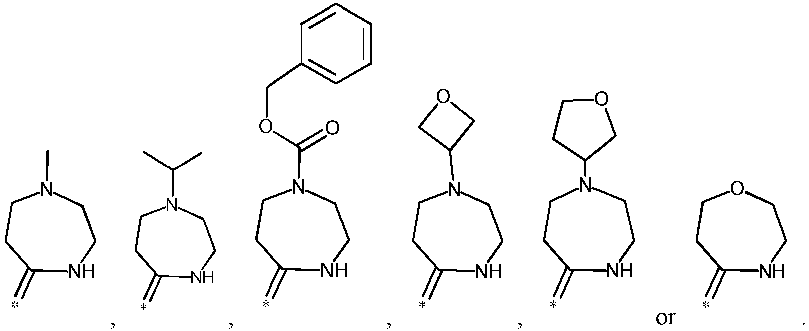

7. The compound of claim 1 or a salt thereof wherein the amino group containing **R3** and **R4** in formula (I) is

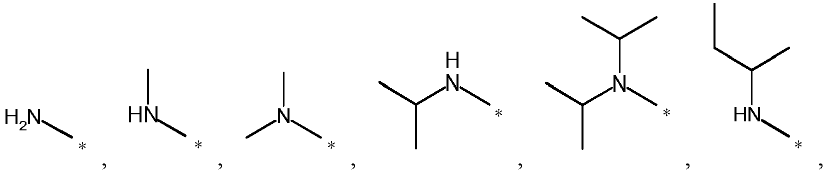

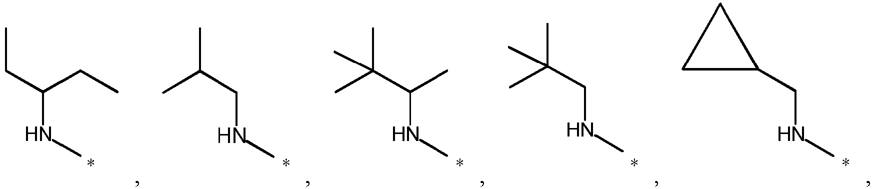

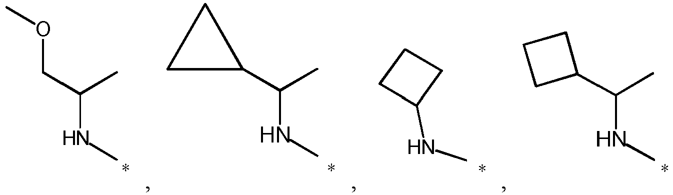

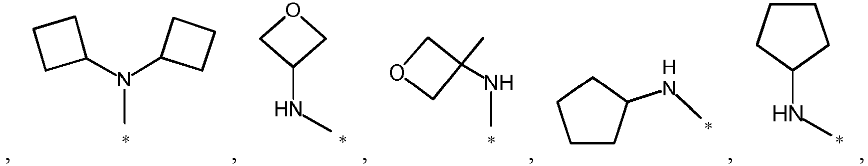

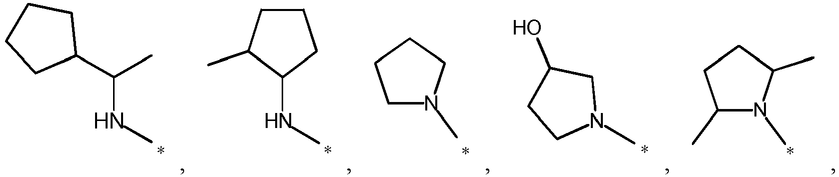

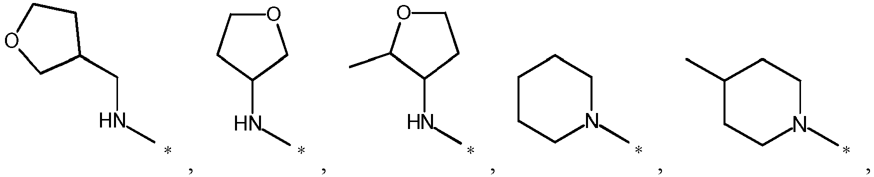

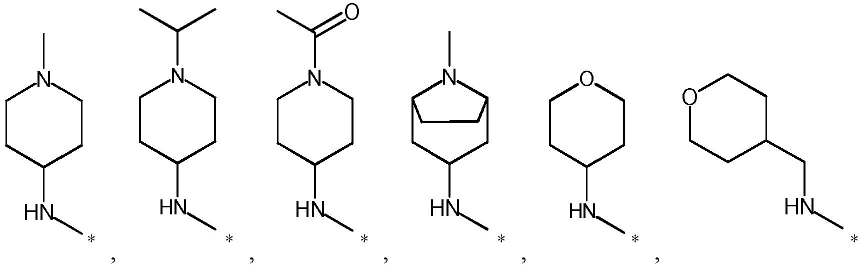

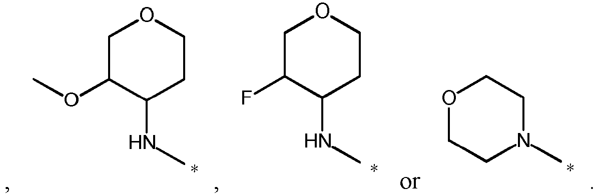

8. The compound of claim 1 or a salt thereof, wherein amino group containing **R3** and **R4** in formula (I) encompasses hydrogen as **R3** while **R4** is iso-propyl, cyclobutyl or cyclopentyl, unsubstituted or substituted with fluorine (F).

9. The compound of claim 1 or a salt thereof, wherein amino group containing **R3** and **R4** in formula (I) is 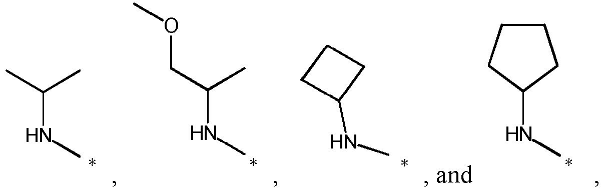 unsubstituted or substituted with F.

10. A salt of a compound of any one of claims 1-9.

11. The compound of claim 1 or a salt thereof wherein formula (I) is the formula of any one of compounds 1 to 175 and 1001-1178.
| **#** | **Formula** | **#** | **Formula** |
|---|---|---|---|
| 1 | 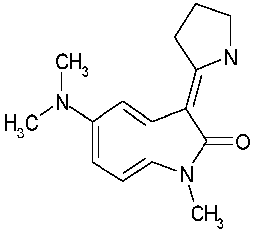 | 2 | 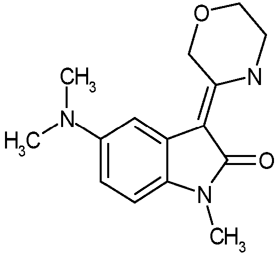 |
| 3 | 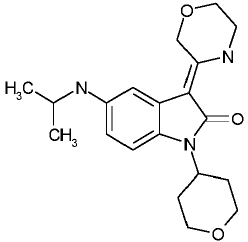 | 4 | 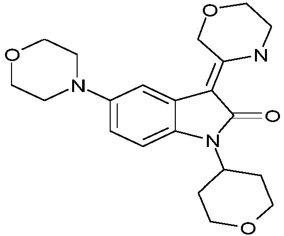 |
| 5 | 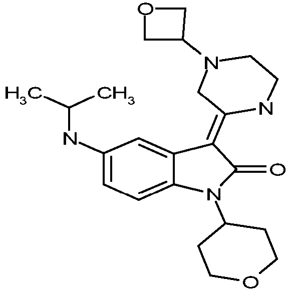 | 6 | 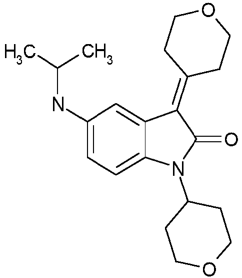 |
| 7 | 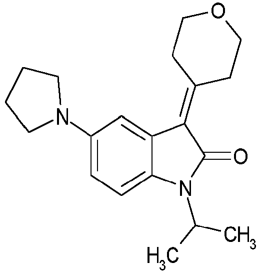 | 8 | 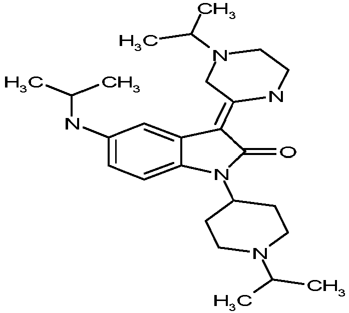 |
| 9 | 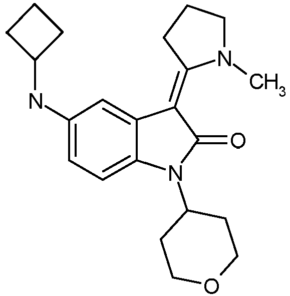 | 10 | 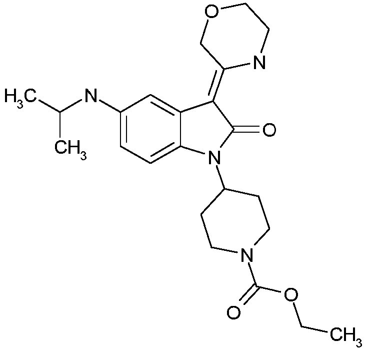 |
| 11 | 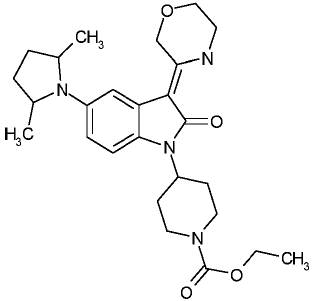 | 12 | 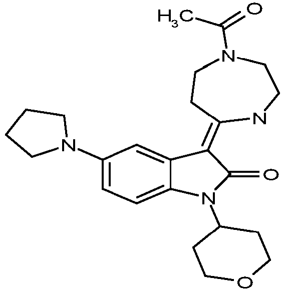 |
| 13 | 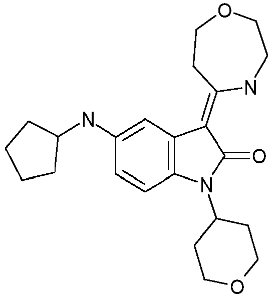 | 14 | 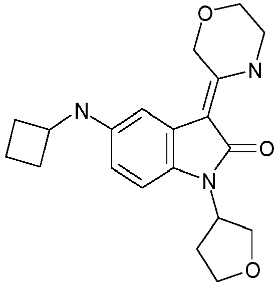 |
| 15 | 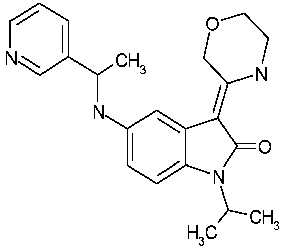 | 16 | 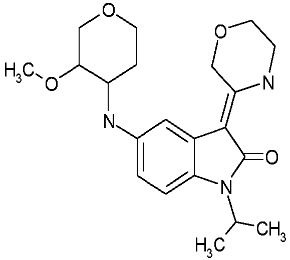 |
| 17 | 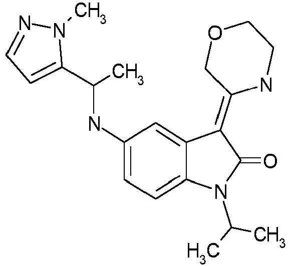 | 18 | 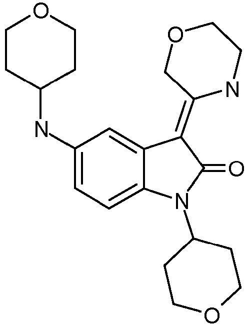 |
| 19 | 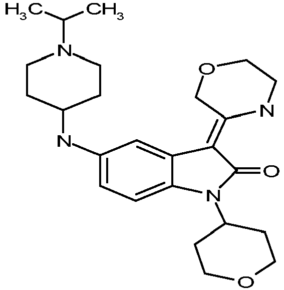 | 20 | 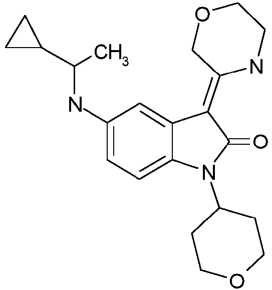 |
| 21 | 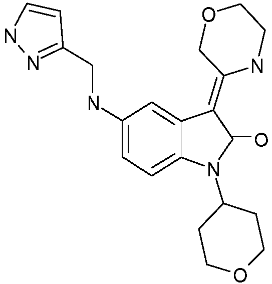 | 22 | 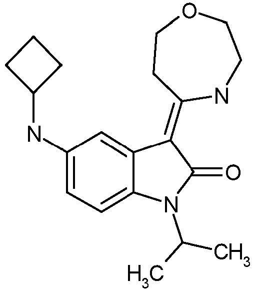 |
| 23 | 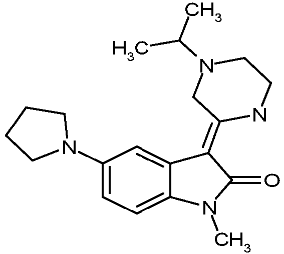 | 24 | 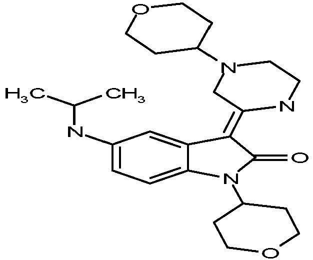 |
| 25 | 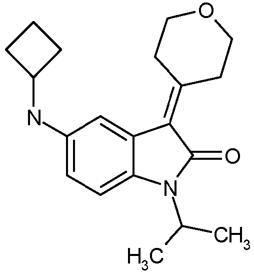 | 26 | 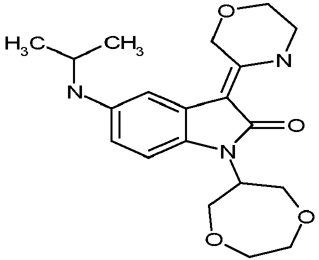 |
| 27 | 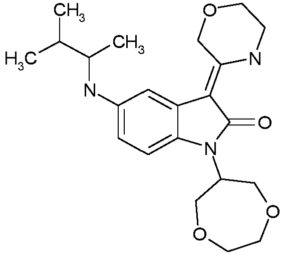 | 28 | 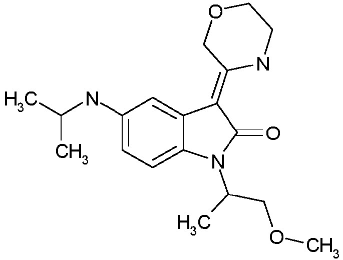 |
| 29 | 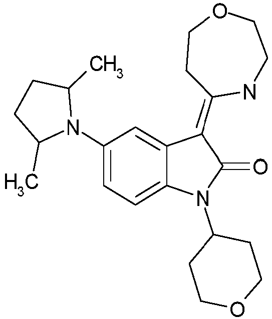 | 30 | 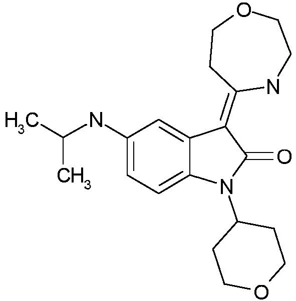 |
| 31 | 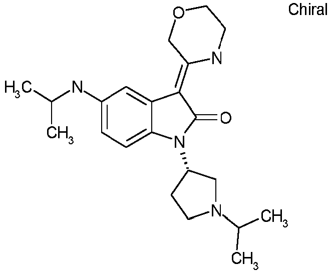 | 32 | 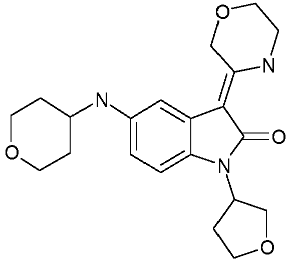 |
| 33 | 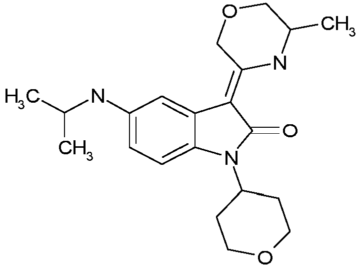 | 34 | 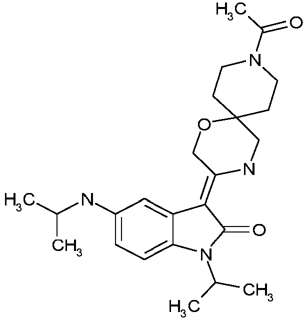 |
| 35 | 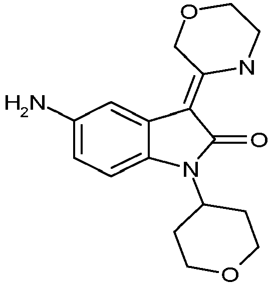 | 36 | 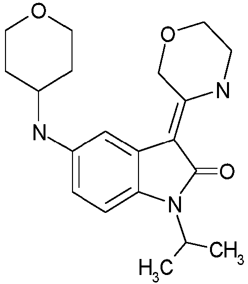 |
| 37 | 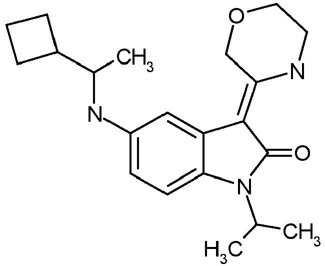 | 38 | 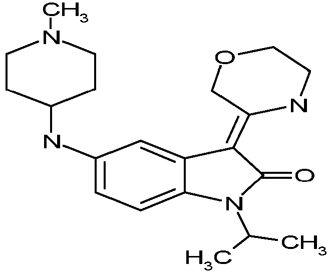 |
| 39 | 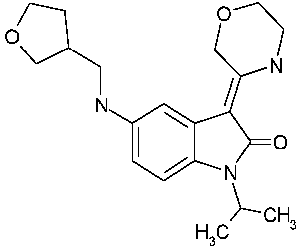 | 40 | 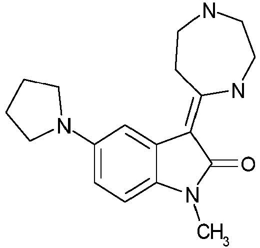 |
| 41 | 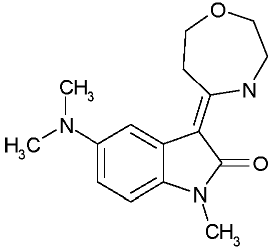 | 42 | 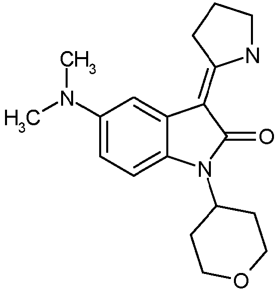 |
| 43 | 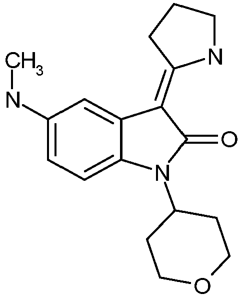 | 44 | 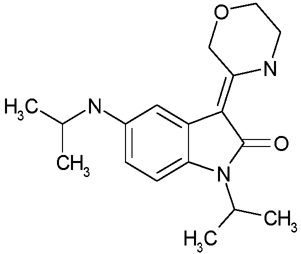 |
| 45 | 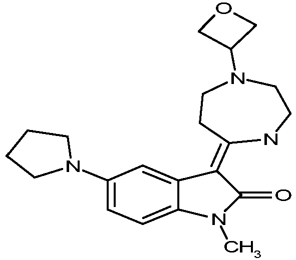 | 46 | 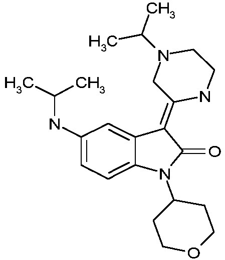 |
| 47 | 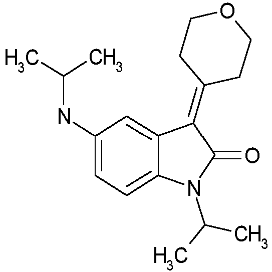 | 48 | 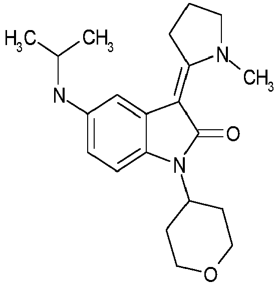 |
| 49 | 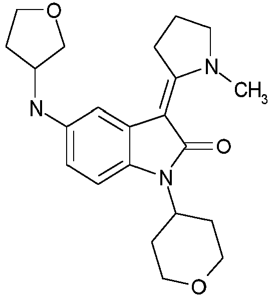 | 50 | 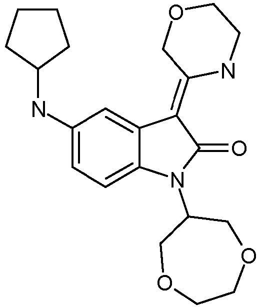 |
| 51 | 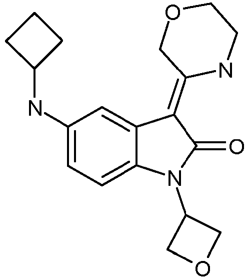 | 52 | 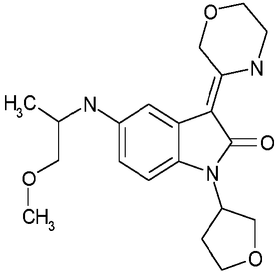 |
| 53 | 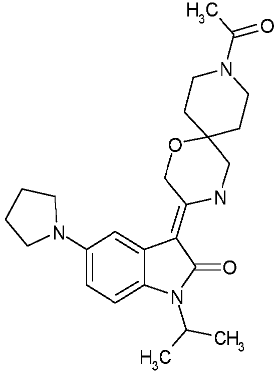 | 54 | 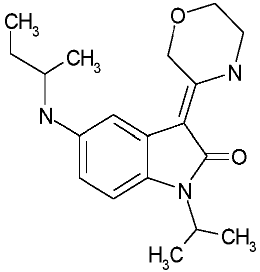 |
| 55 | 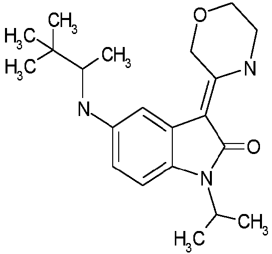 | 56 | 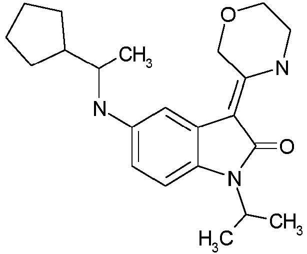 |
| 57 | 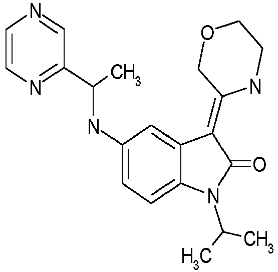 | 58 | 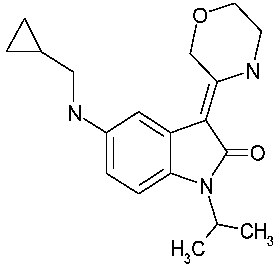 |
| 59 | 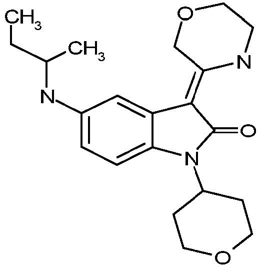 | 60 | 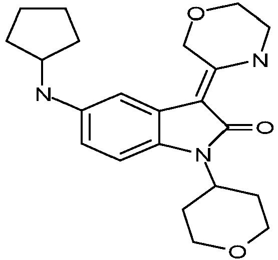 |
| 61 | 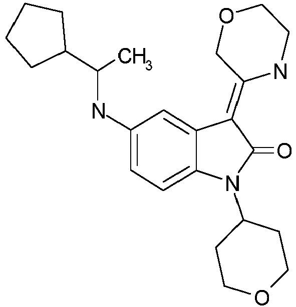 | 62 | 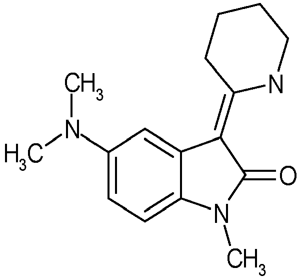 |
| 63 | 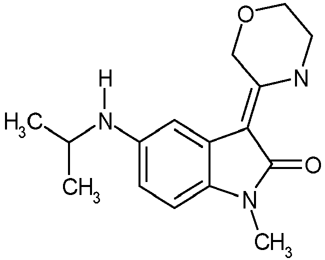 | 64 | 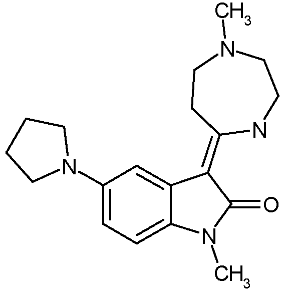 |
| 65 | 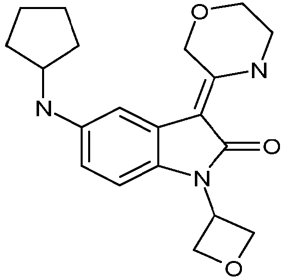 | 66 | 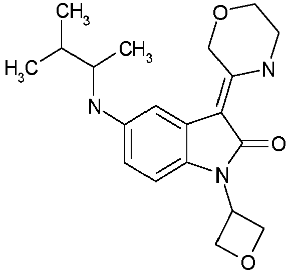 |
| 67 | 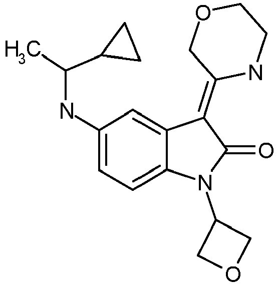 | 68 | 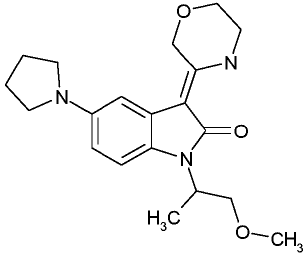 |
| 69 | 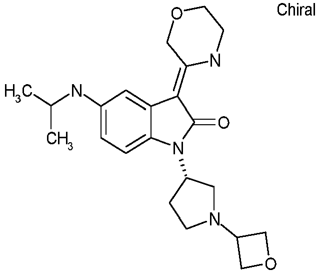 | 70 | 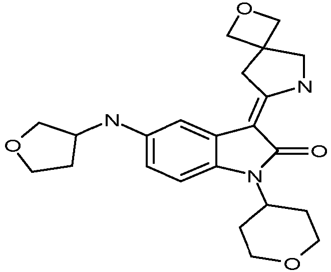 |
| 71 | 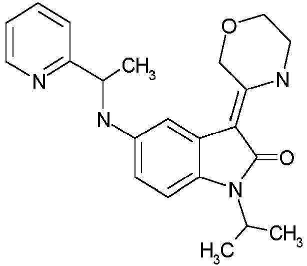 | 72 | 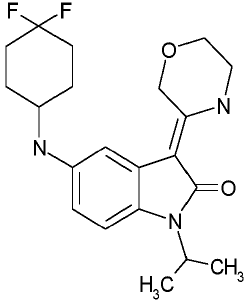 |
| 73 | 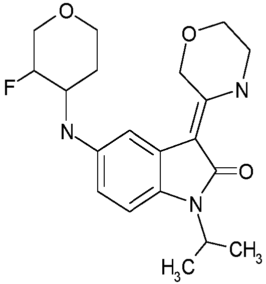 | 74 | 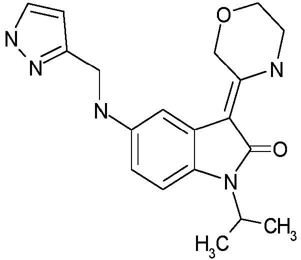 |
| 75 | 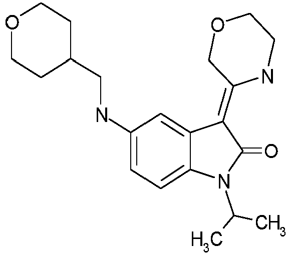 | 76 | 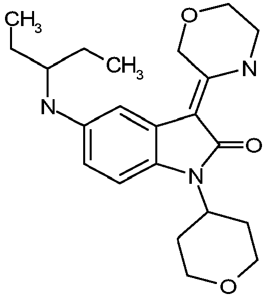 |
| 77 | 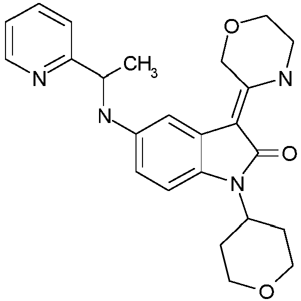 | 78 | 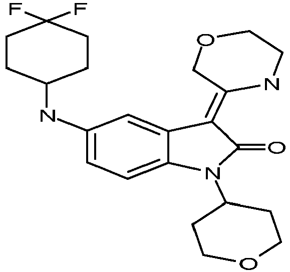 |
| 79 | 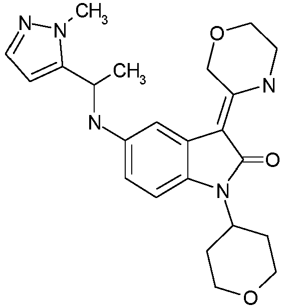 | 80 | 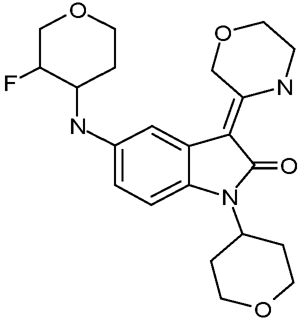 |
| 81 | 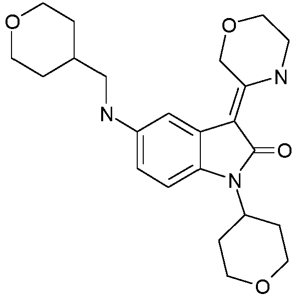 | 82 | 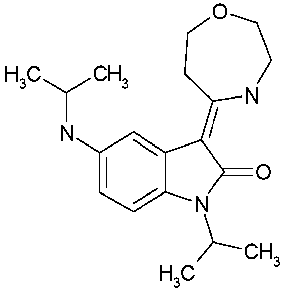 |
| 83 | 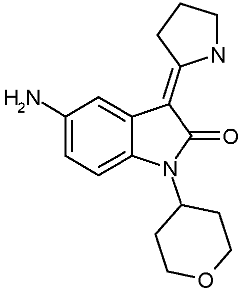 | 84 | 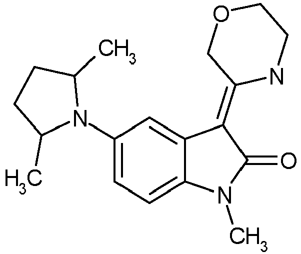 |
| 85 | 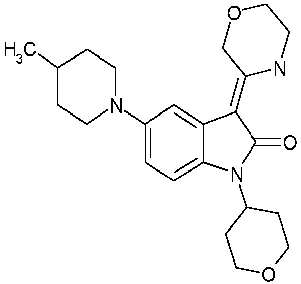 | 86 | 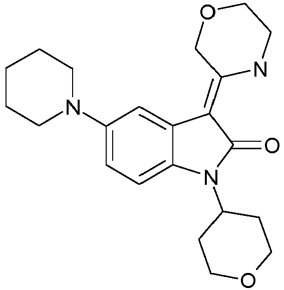 |
| 87 | 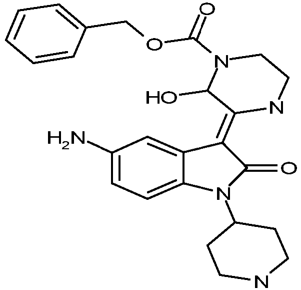 | 88 | 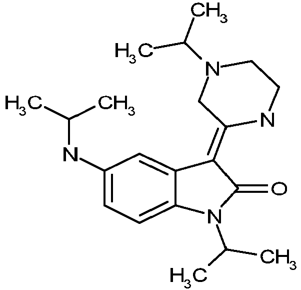 |
| 89 | 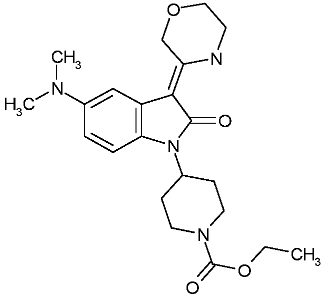 | 90 | 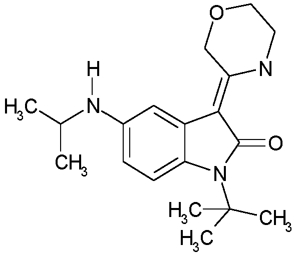 |
| 91 | 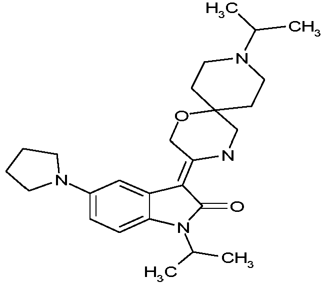 | 92 | 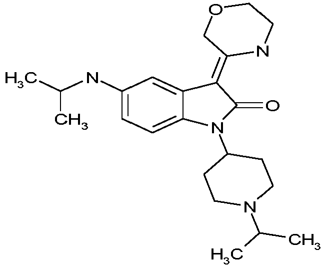 |
| 93 | 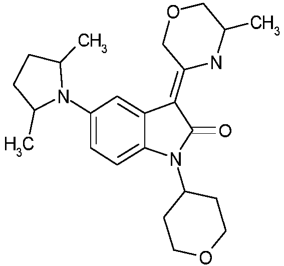 | 94 | 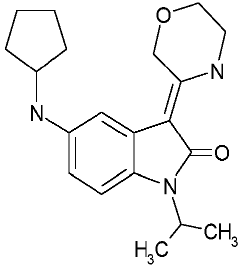 |
| 95 | 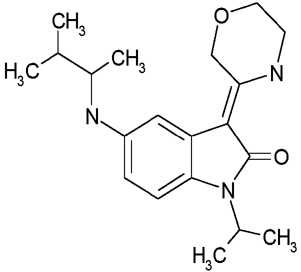 | 96 | 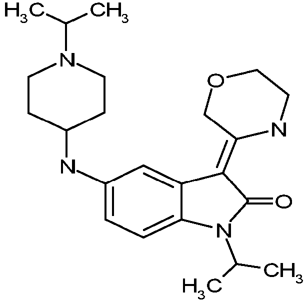 |
| 97 | 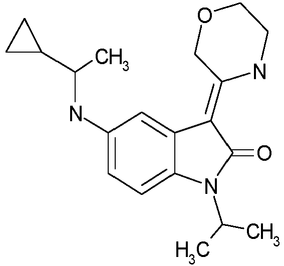 | 98 | 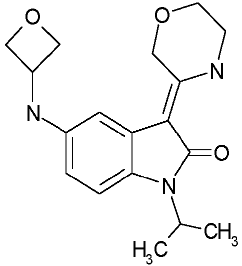 |
| 99 | 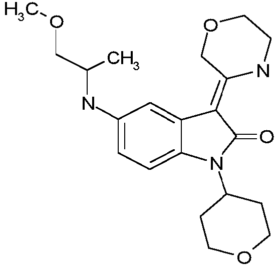 | 100 | 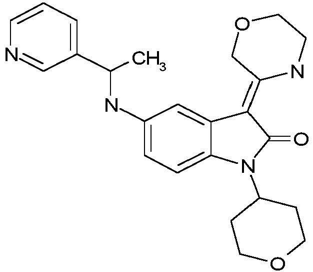 |
| 101 | 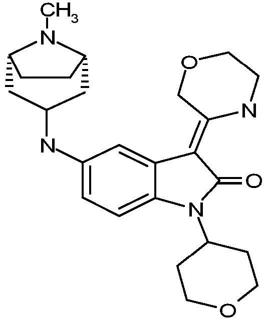 | 102 | 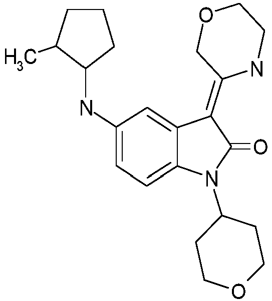 |
| 103 | 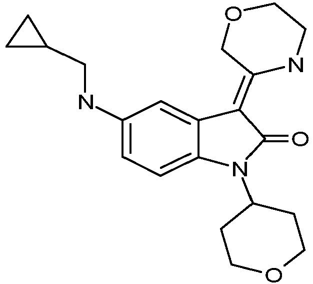 | 104 | 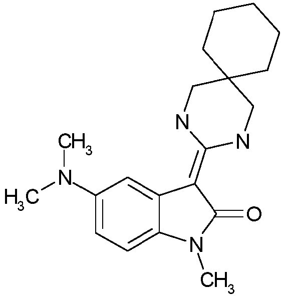 |
| 105 | 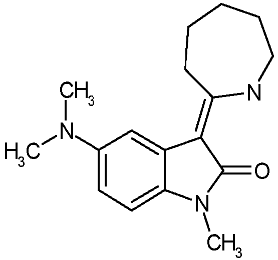 | 106 | 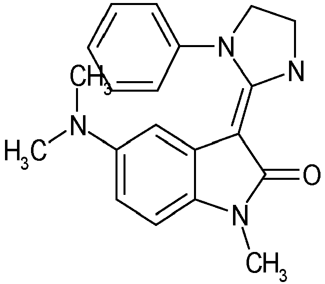 |
| 107 | 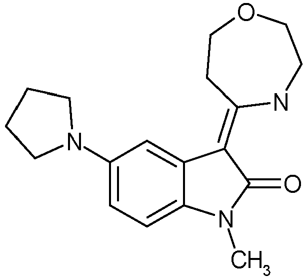 | 108 | 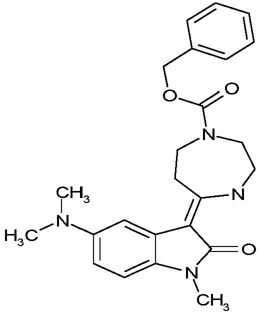 |
| 109 | 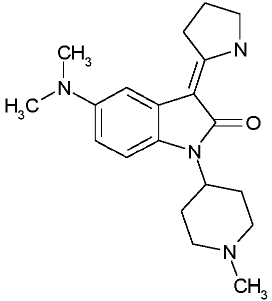 | 110 | 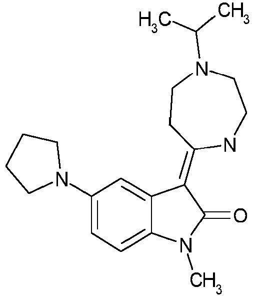 |
| 111 | 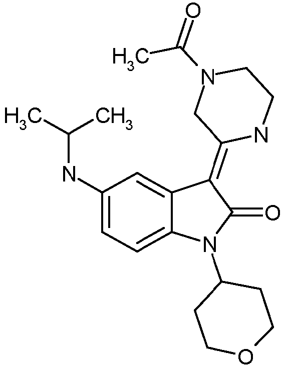 | 112 | 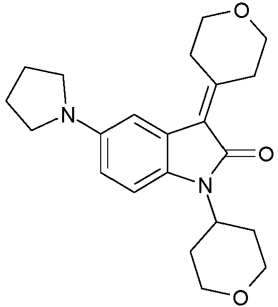 |
| 113 | 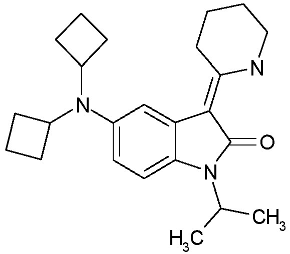 | 114 | 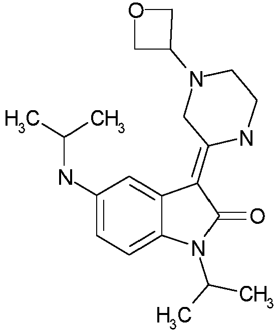 |
| 115 | 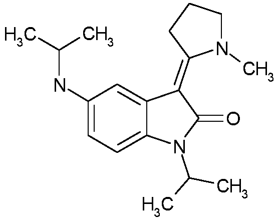 | 116 | 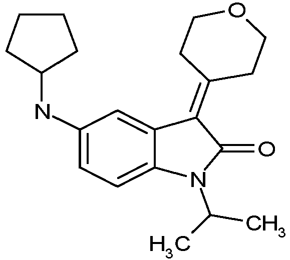 |
| 117 | 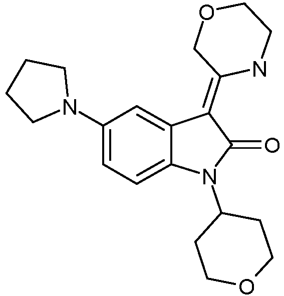 | 118 | 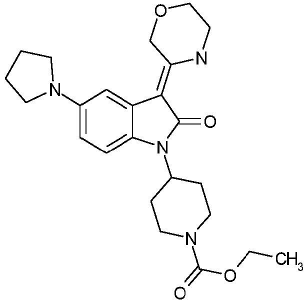 |
| 119 | 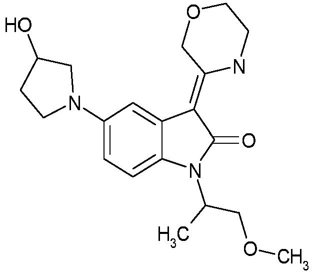 | 120 | 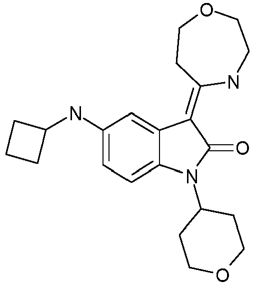 |
| 121 | 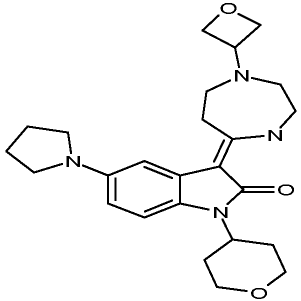 | 122 | 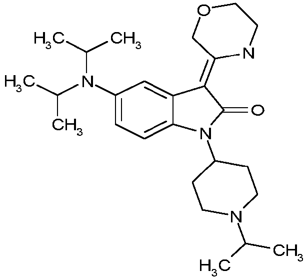 |
| 123 | 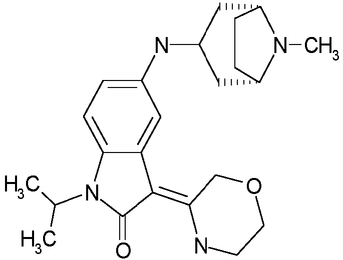 | 124 | 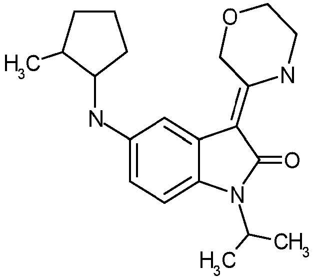 |
| 125 | 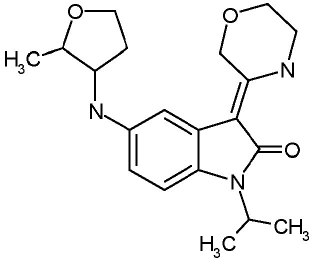 | 126 | 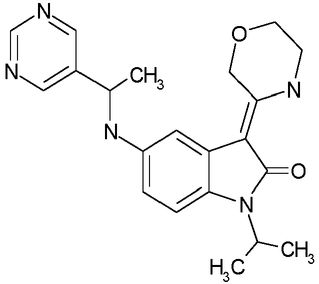 |
| 127 | 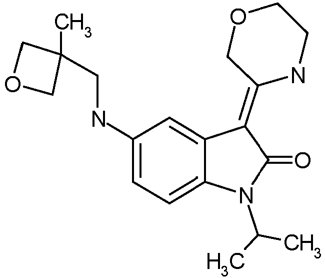 | 128 | 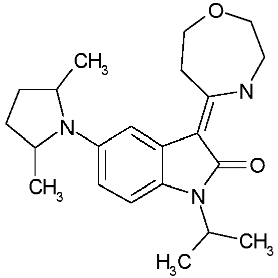 |
| 129 | 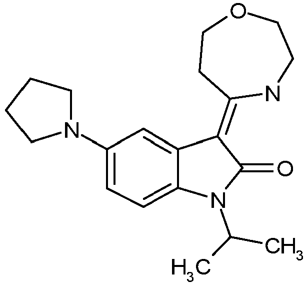 | 130 | 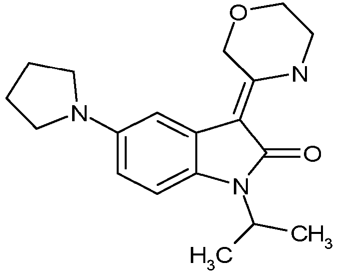 |
| 131 | 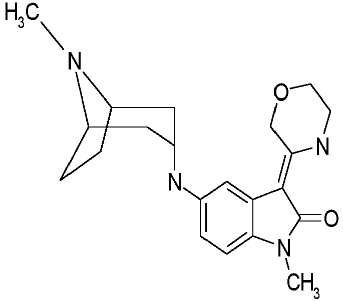 | 132 | 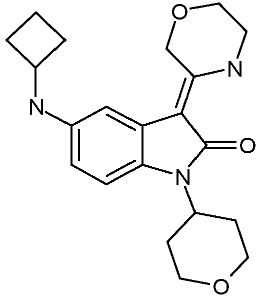 |
| 133 | 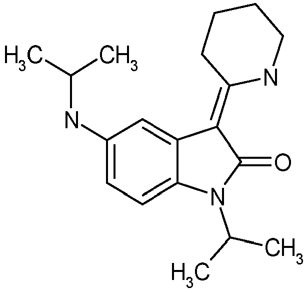 | 134 | 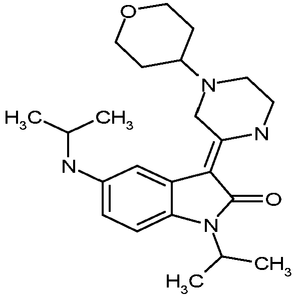 |
| 135 | 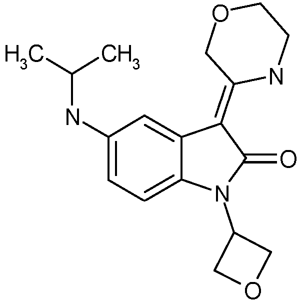 | 136 | 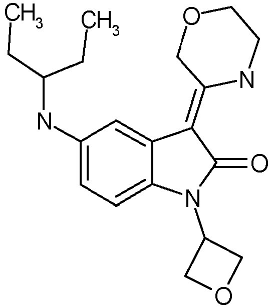 |
| 137 | 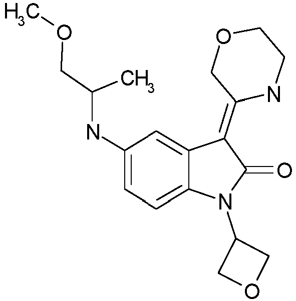 | 138 | 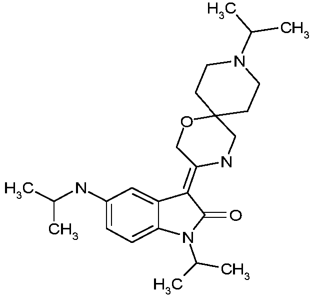 |
| 139 | 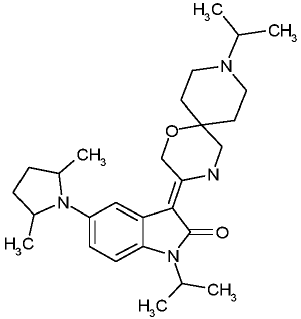 | 140 | 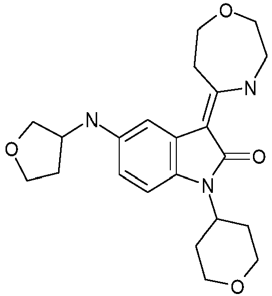 |
| 141 | 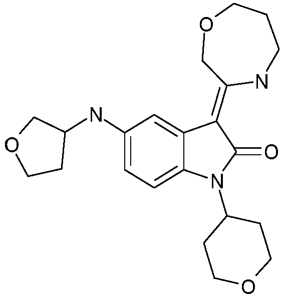 | 142 | 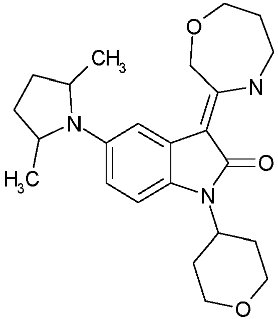 |
| 143 | 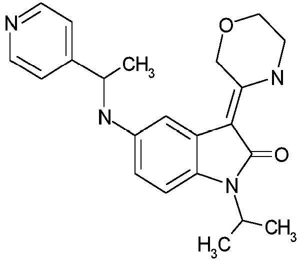 | 144 | 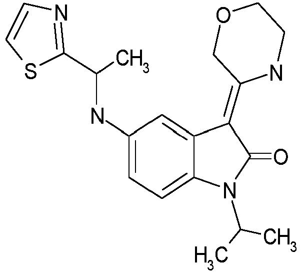 |
| 145 | 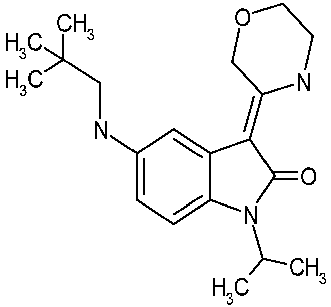 | 146 | 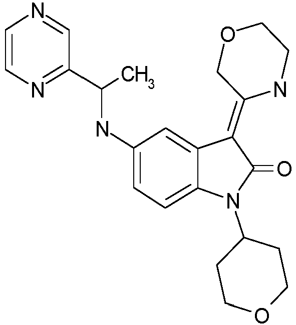 |
| 147 | 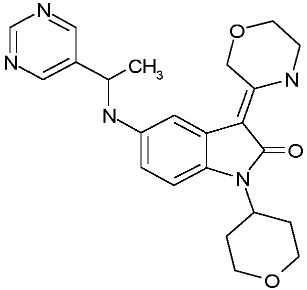 | 148 | 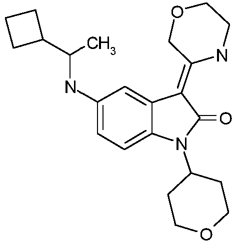 |
| 149 | 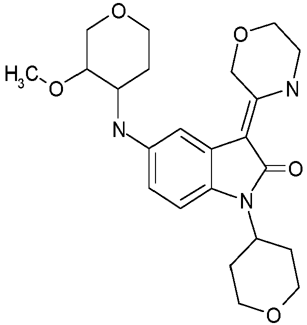 | 150 | 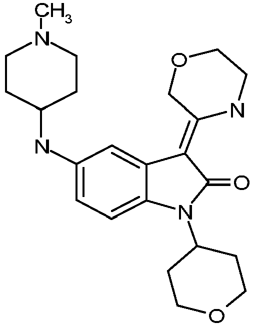 |
| 151 | 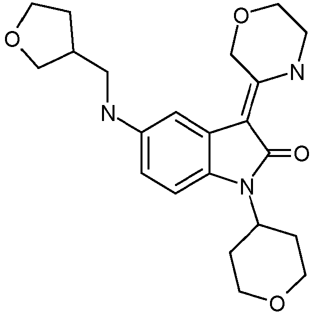 | 152 | 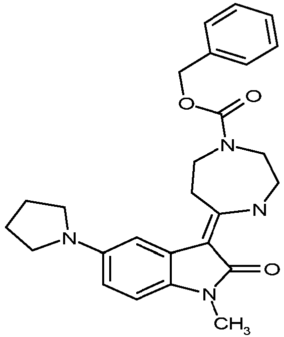 |
| 153 | 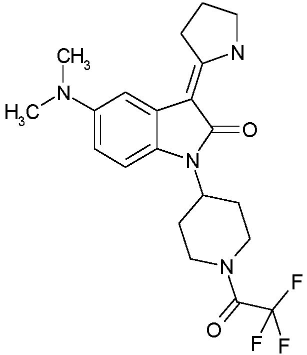 | 154 | 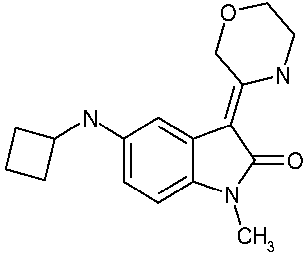 |
| 155 | 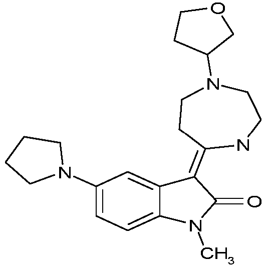 | 156 | 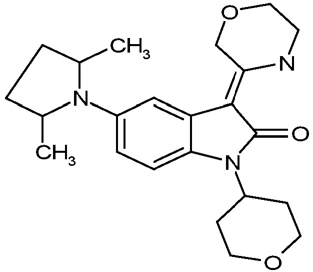 |
| 157 | 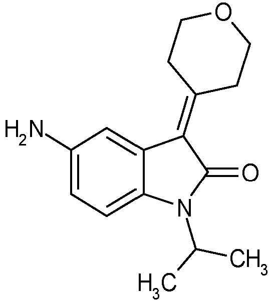 | 158 | 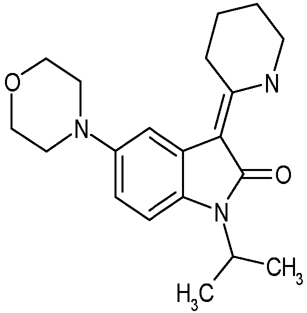 |
| 159 | 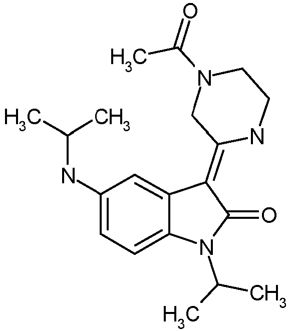 | 160 | 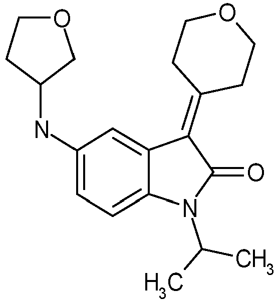 |
| 161 | 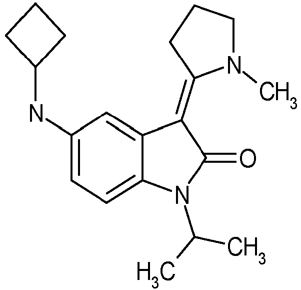 | 162 | 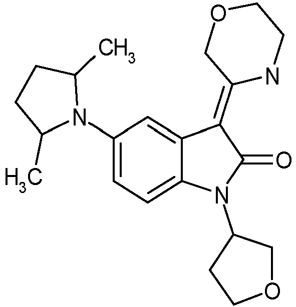 |
| 163 | 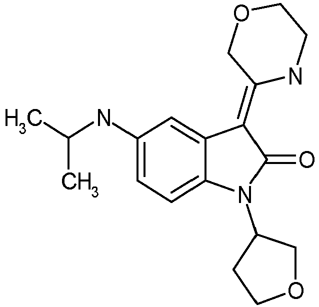 | 164 | 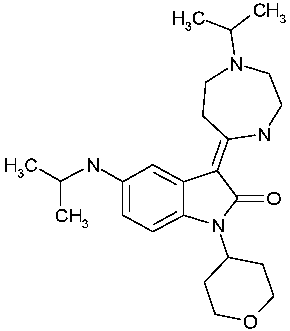 |
| 165 | 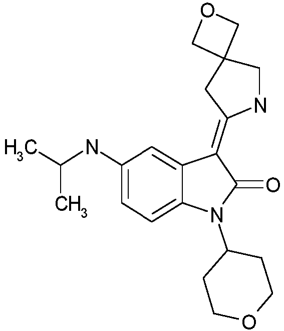 | 166 | 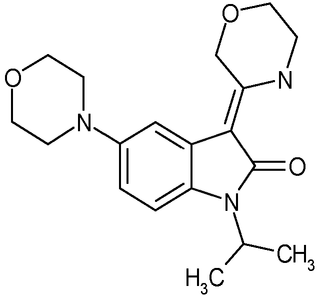 |
| 167 | 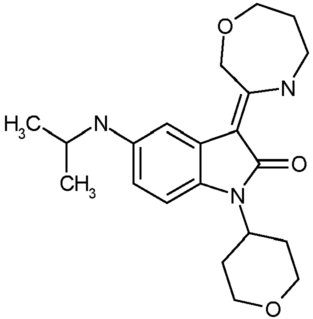 | 168 | 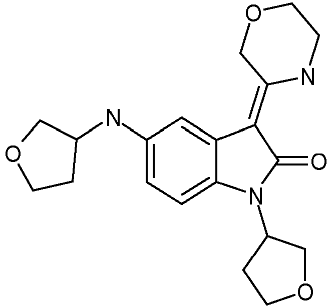 |
| 169 | 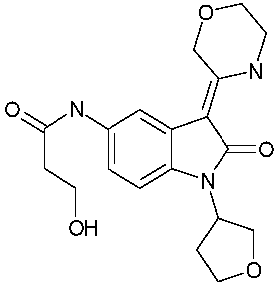 | 170 | 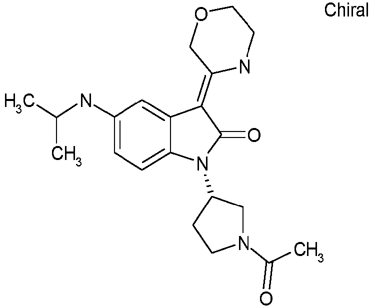 |
| 171 | 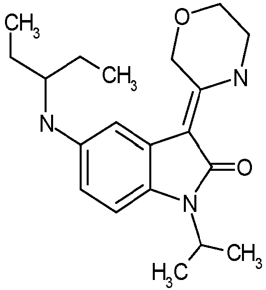 | 172 | 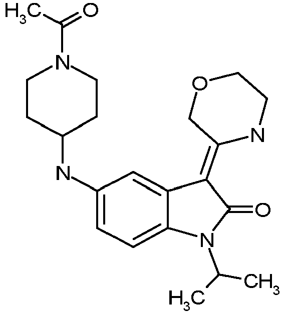 |
| 173 | 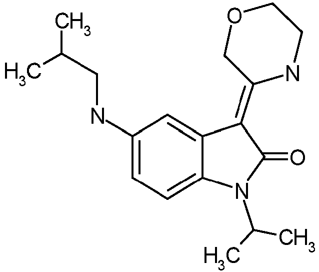 | 174 | 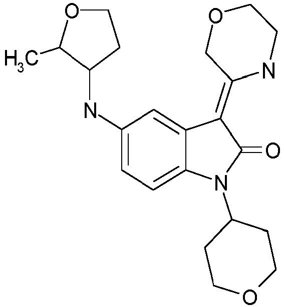 |
| 175 | 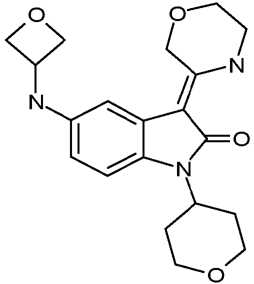 | 1001 | 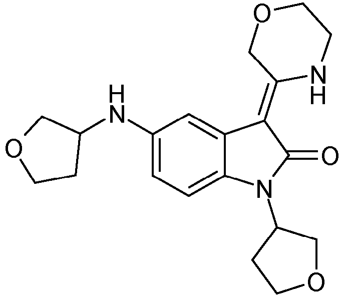 |
| 1002 | 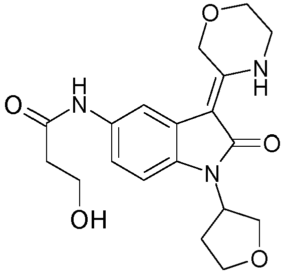 | 1003 | 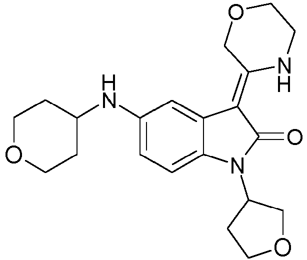 |
| 1004 | 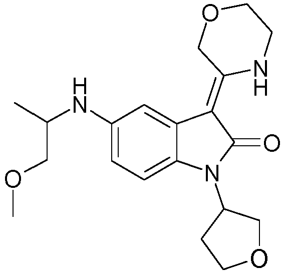 | 1005 | 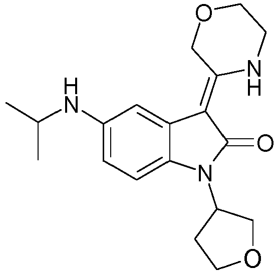 |
| 1006 | 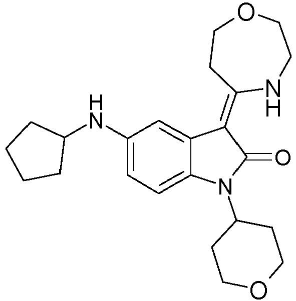 | 1007 | 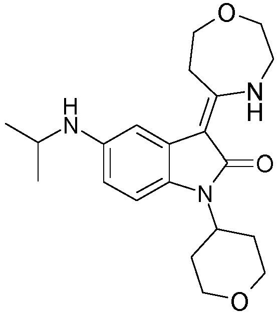 |
| 1008 | 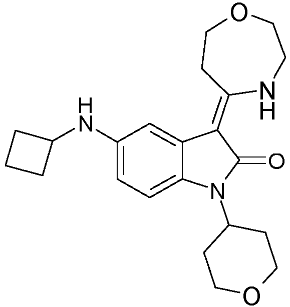 | 1009 | 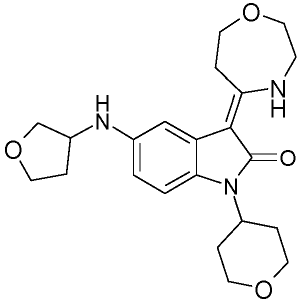 |
| 1010 | 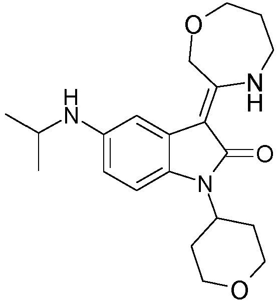 | 1011 | 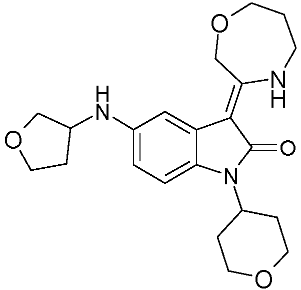 |
| 1012 | 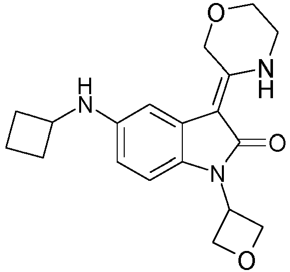 | 1013 | 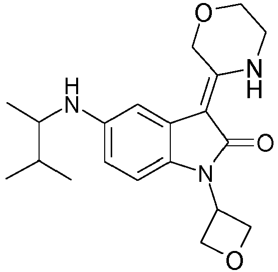 |
| 1014 | 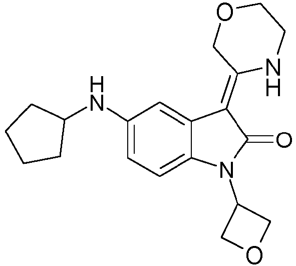 | 1015 | 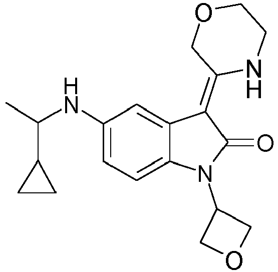 |
| 1016 | 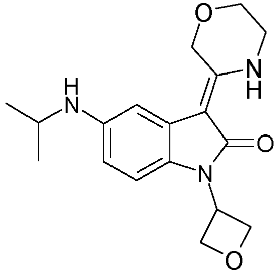 | 1017 | 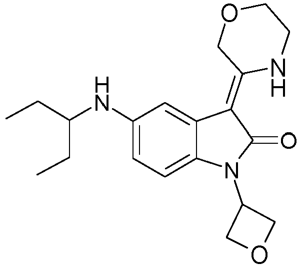 |
| 1018 | 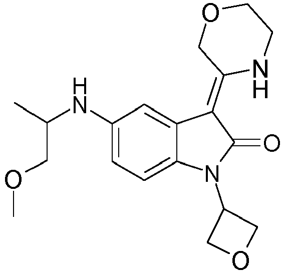 | 1019 | 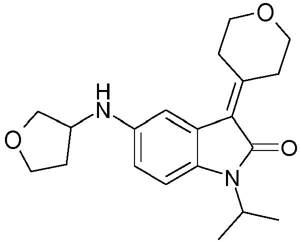 |
| 1020 | 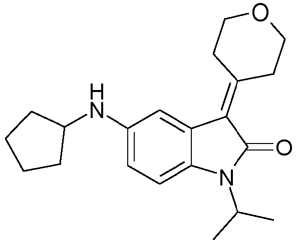 | 1021 | 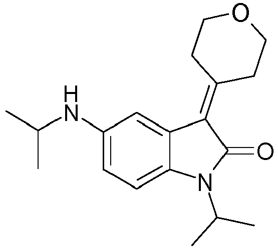 |
| 1022 | 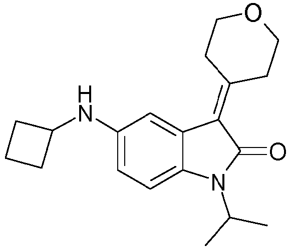 | 1023 | 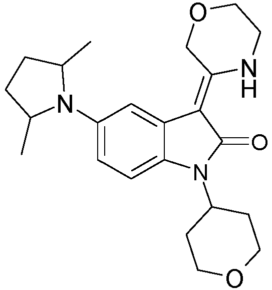 |
| 1024 | 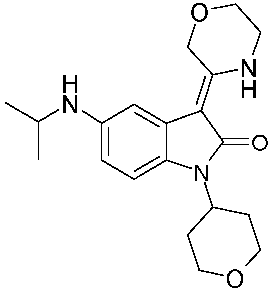 | 1025 | 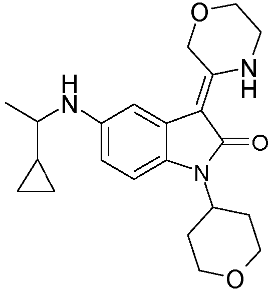 |
| 1026 | 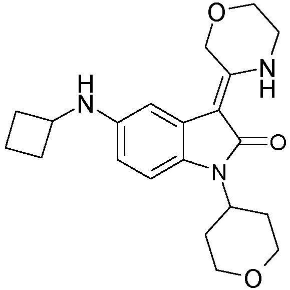 | 1027 | 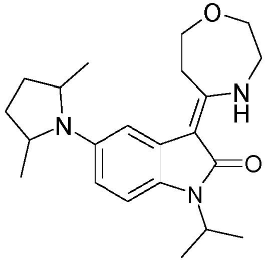 |
| 1028 | 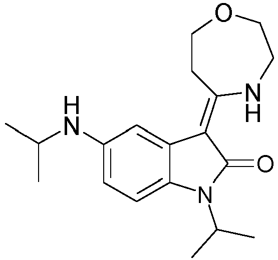 | 1029 | 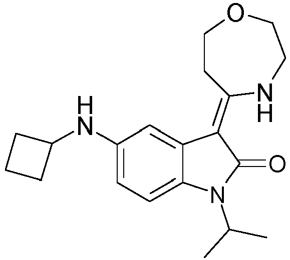 |
| 1030 | 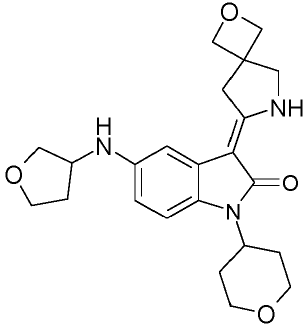 | 1031 | 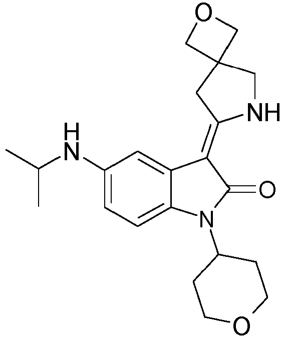 |
| 1032 | 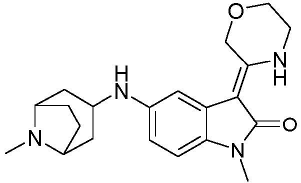 | 1033 | 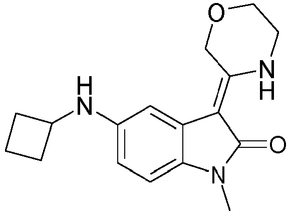 |
| 1034 | 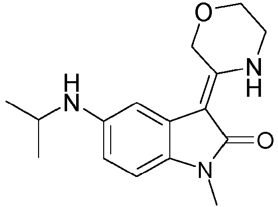 | 1035 | 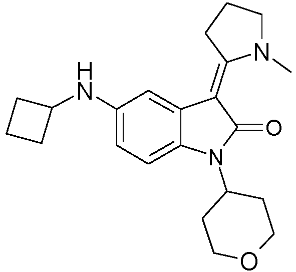 |
| 1036 | 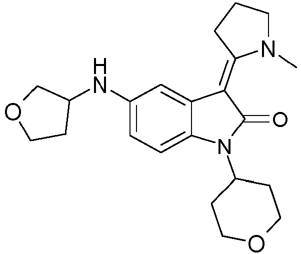 | 1037 | 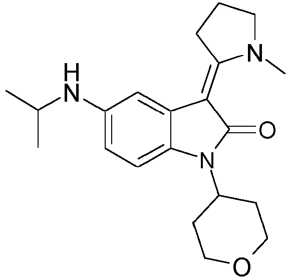 |
| 1038 | 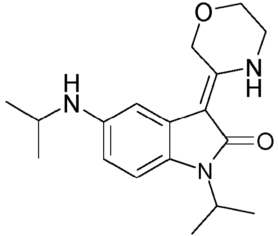 | 1039 | 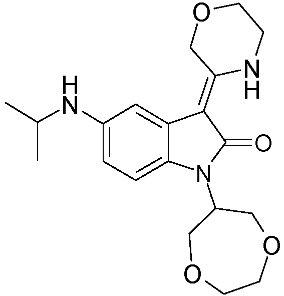 |
| 1040 | 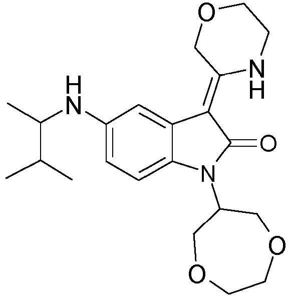 | 1041 | 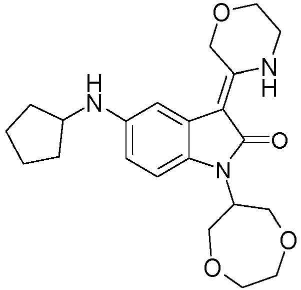 |
| 1042 | 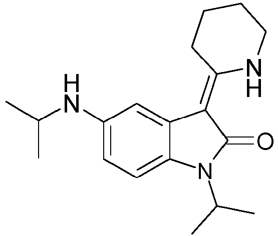 | 1043 | 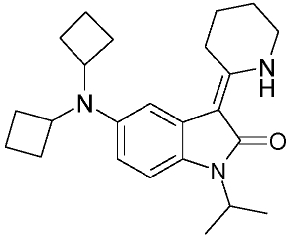 |
| 1044 | 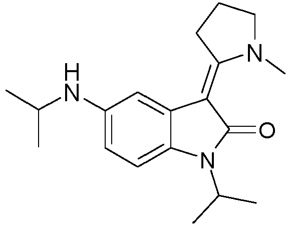 | 1045 | 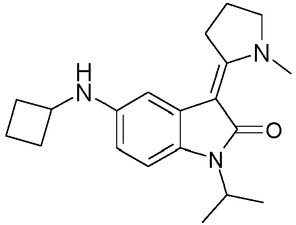 |
| 1046 | 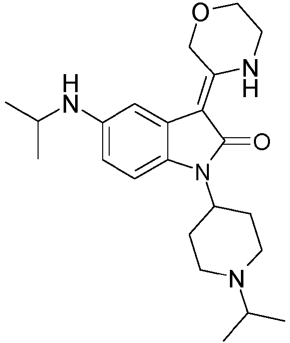 | 1047 | 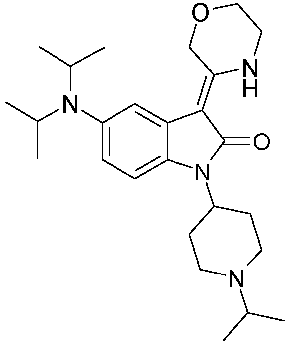 |
| 1048 | 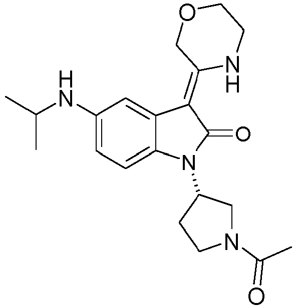 | 1049 | 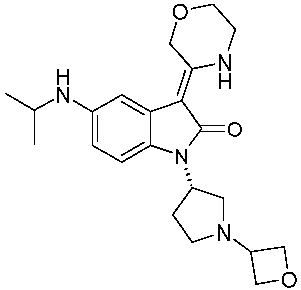 |
| 1050 | 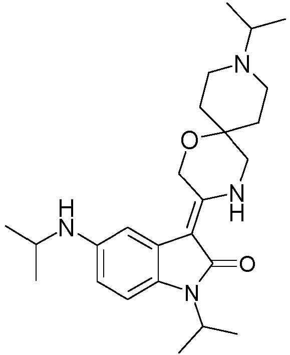 | 1051 | 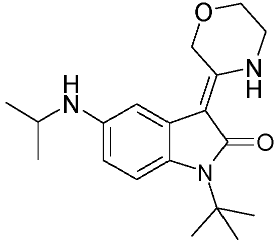 |
| 1052 | 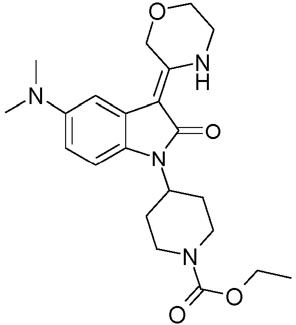 | 1053 | 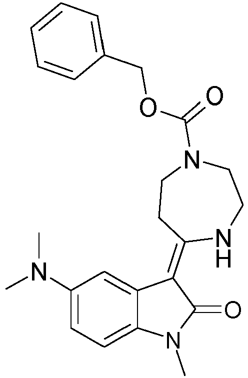 |
| 1054 | 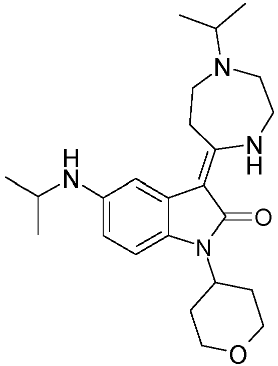 | 1055 | 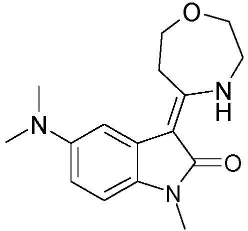 |
| 1056 | 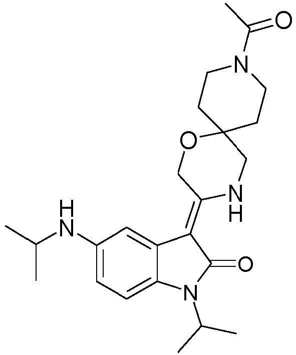 | 1057 | 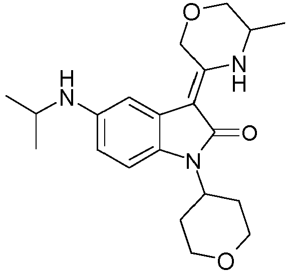 |
| 1058 | 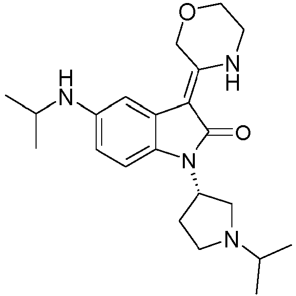 | 1059 | 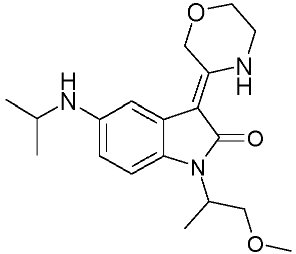 |
| 1060 | 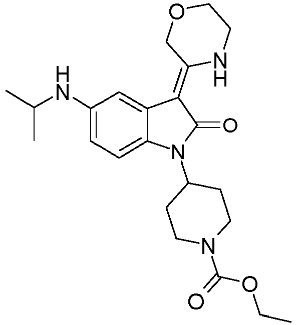 | 1061 | 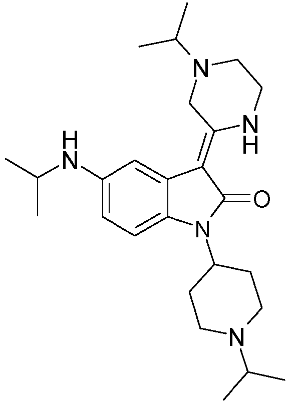 |
| 1062 | 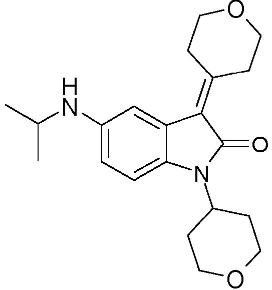 | 1063 | 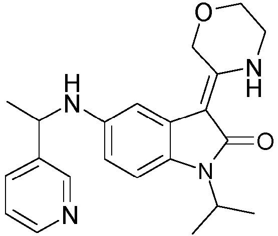 |
| 1064 | 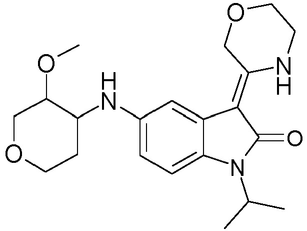 | 1065 | 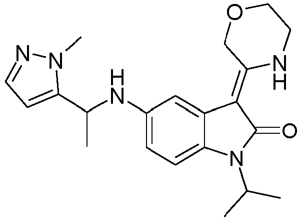 |
| 1066 | 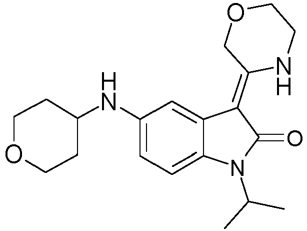 | 1067 | 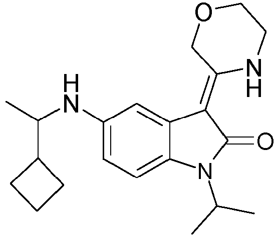 |
| 1068 | 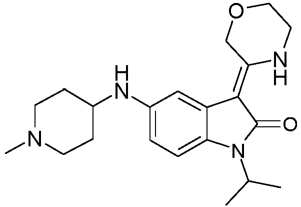 | 1069 | 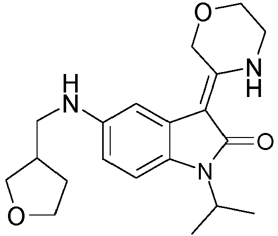 |
| 1070 | 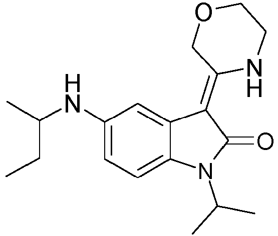 | 1071 | 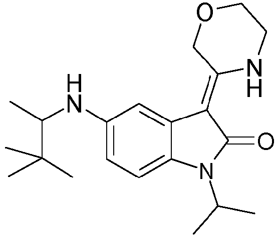 |
| 1072 | 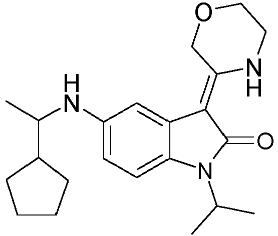 | 1073 | 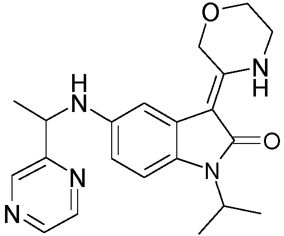 |
| 1074 | 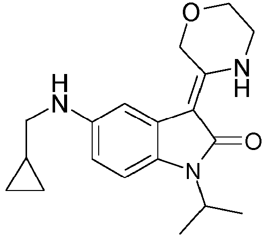 | 1075 | 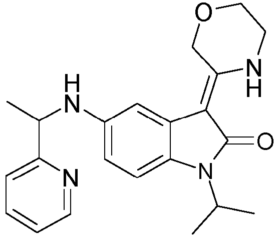 |
| 1076 | 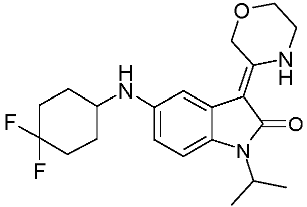 | 1077 | 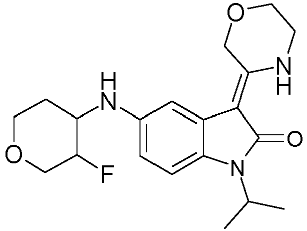 |
| 1078 | 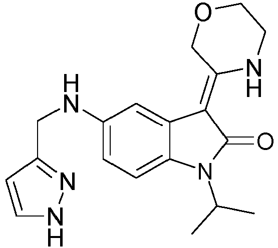 | 1079 | 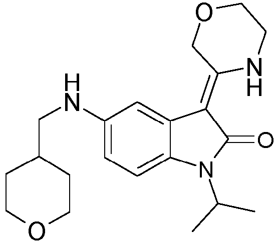 |
| 1080 | 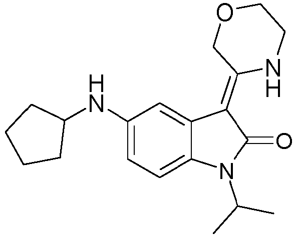 | 1081 | 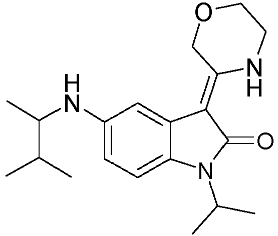 |
| 1082 | 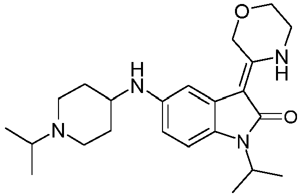 | 1083 | 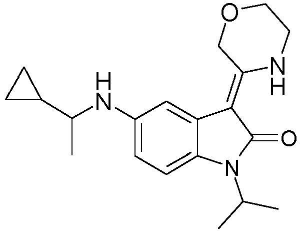 |
| 1084 | 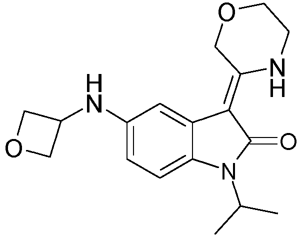 | 1085 | 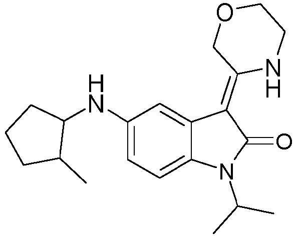 |
| 1086 | 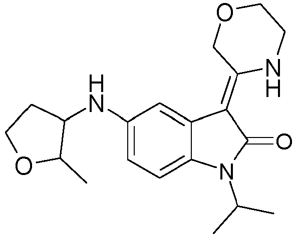 | 1087 | 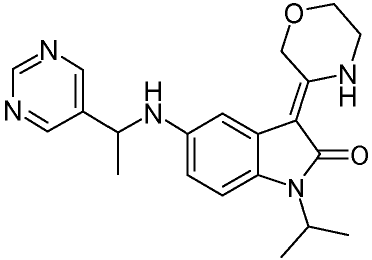 |
| 1088 | 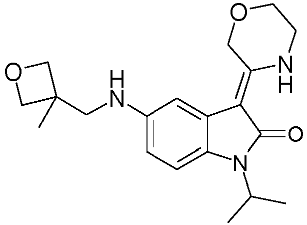 | 1089 | 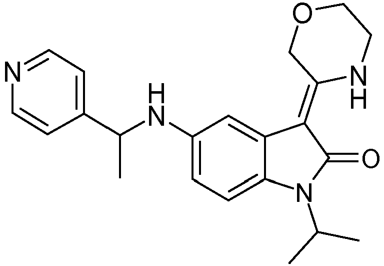 |
| 1090 | 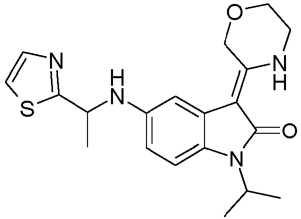 | 1091 | 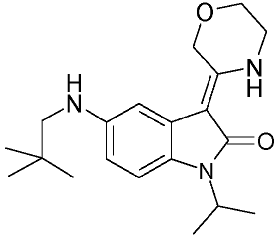 |
| 1092 | 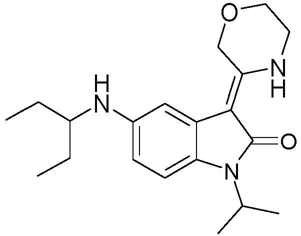 | 1093 | 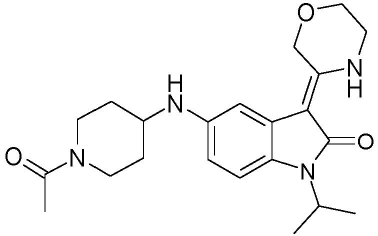 |
| 1094 | 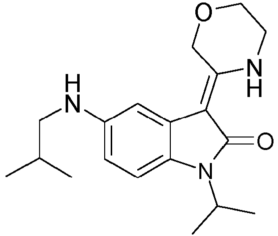 | 1095 | 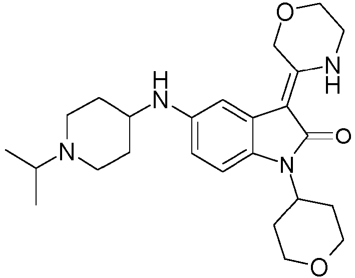 |
| 1096 | 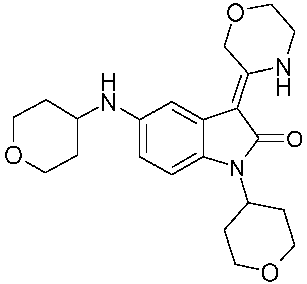 | 1097 | 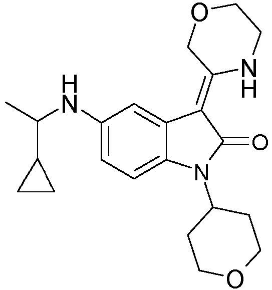 |
| 1098 | 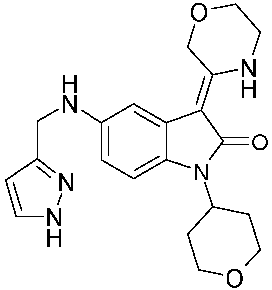 | 1099 | 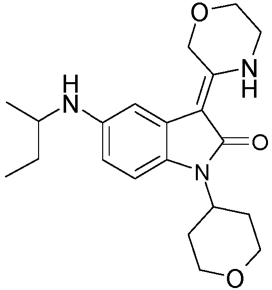 |
| 1100 | 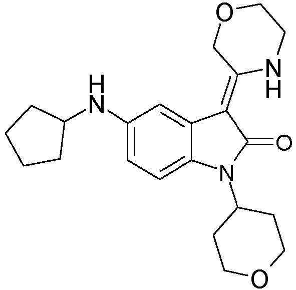 | 1101 | 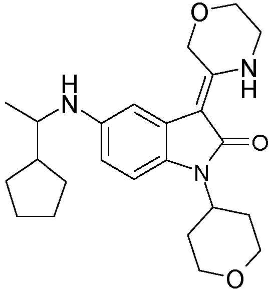 |
| 1102 | 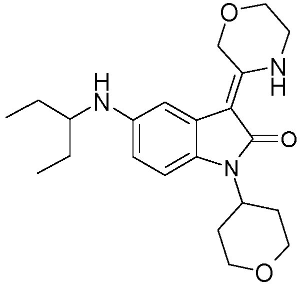 | 1103 | 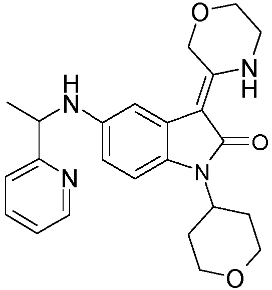 |
| 1104 | 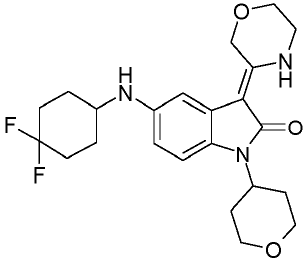 | 1105 | 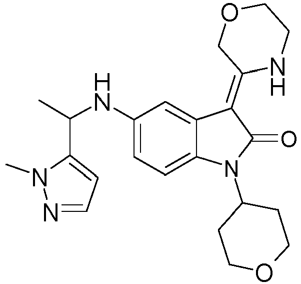 |
| 1106 | 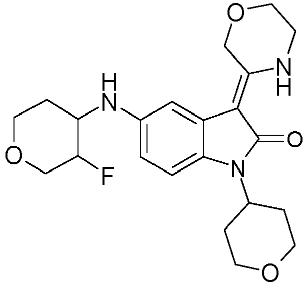 | 1107 | 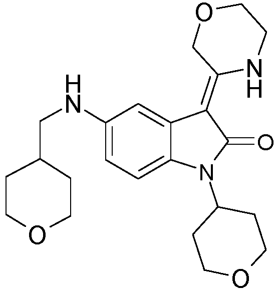 |
| 1108 | 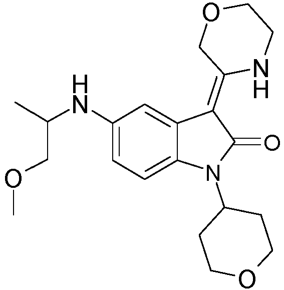 | 1109 | 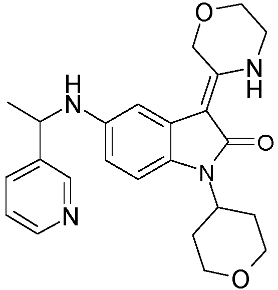 |
| 1110 | 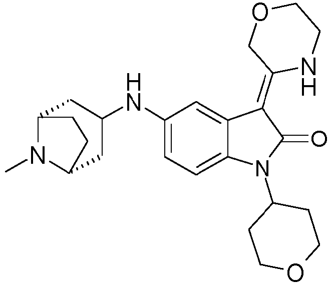 | 1111 | 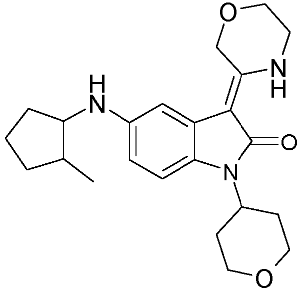 |
| 1112 | 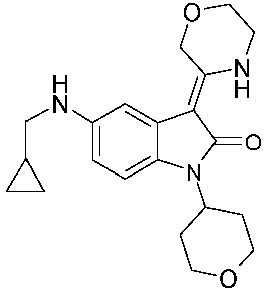 | 1113 | 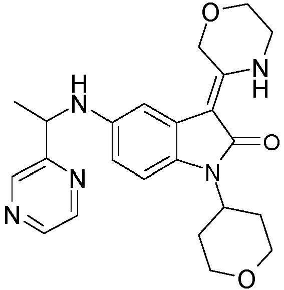 |
| 1114 | 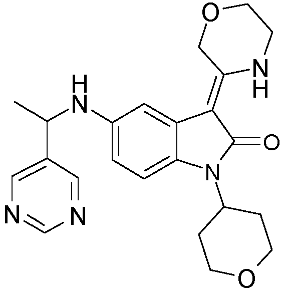 | 1115 | 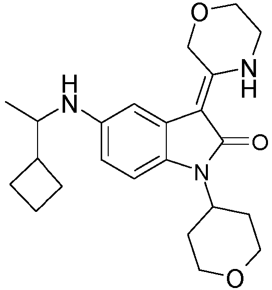 |
| 1116 | 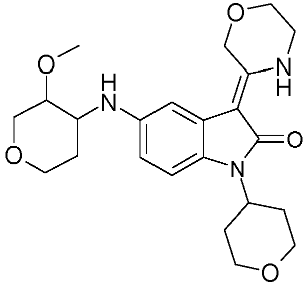 | 1117 | 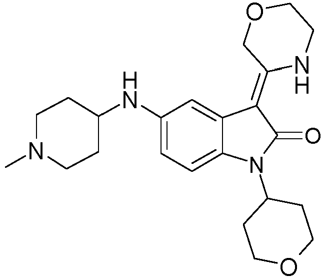 |
| 1118 | 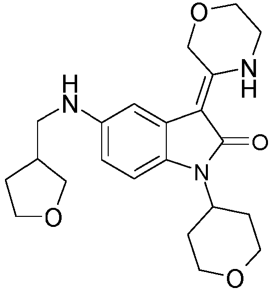 | 1119 | 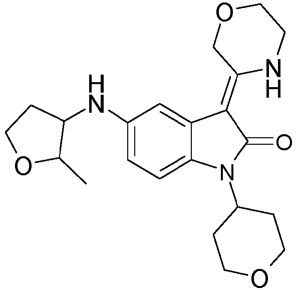 |
| 1120 | 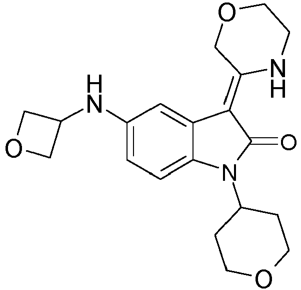 | 1121 | 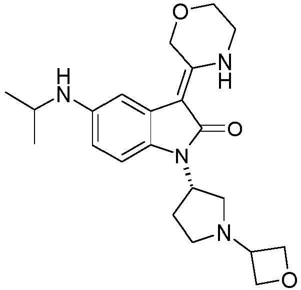 |
| 1122 | 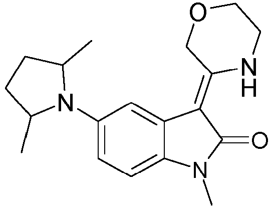 | 1123 | 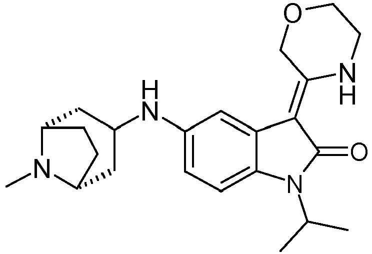 |
| 1124 | 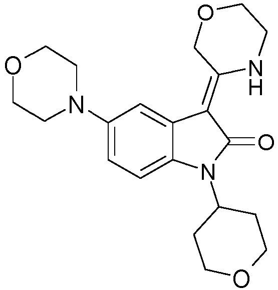 | 1125 | 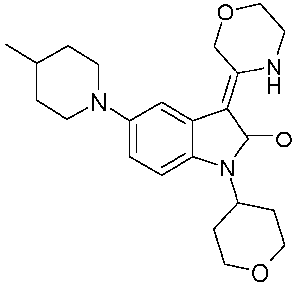 |
| 1126 | 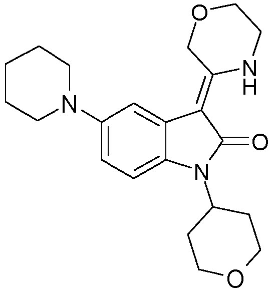 | 1127 | 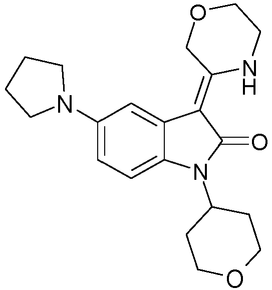 |
| 1128 | 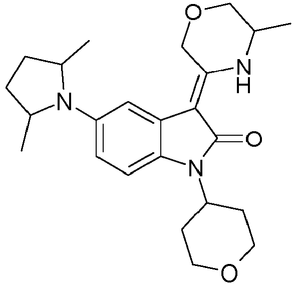 | 1129 | 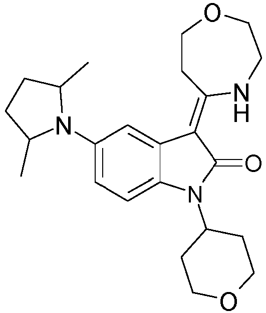 |
| 1130 | 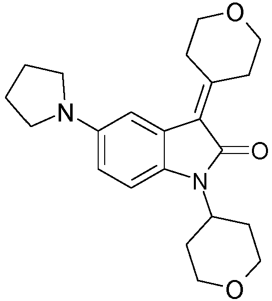 | 1131 | 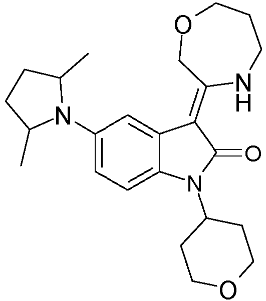 |
| 1132 | 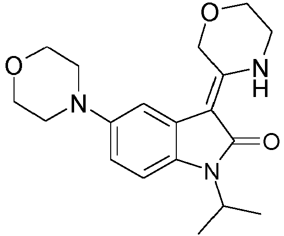 | 1133 | 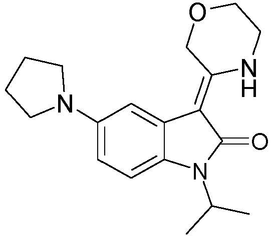 |
| 1134 | 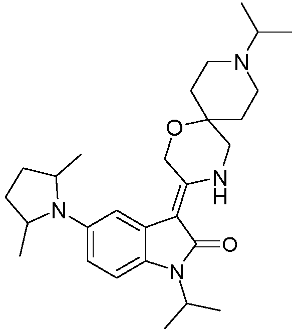 | 1135 | 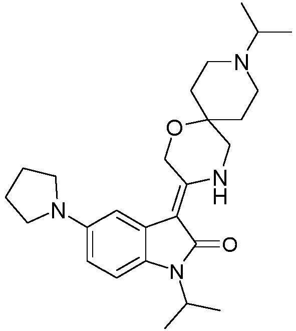 |
| 1136 | 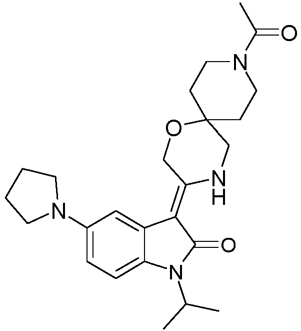 | 1137 | 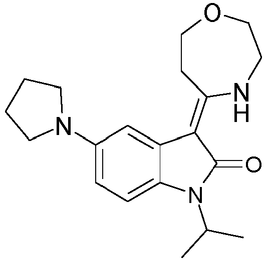 |
| 1138 | 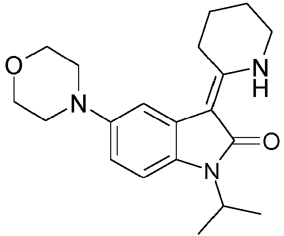 | 1139 | 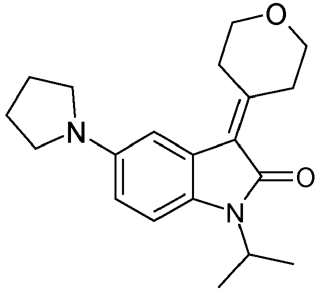 |
| 1140 | 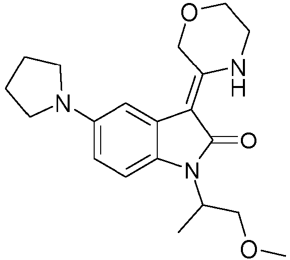 | 1141 | 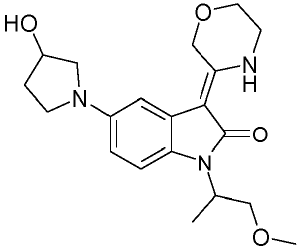 |
| 1142 | 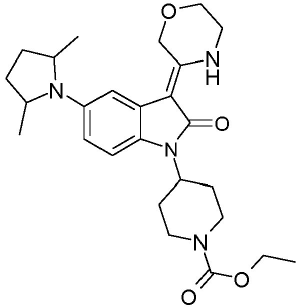 | 1143 | 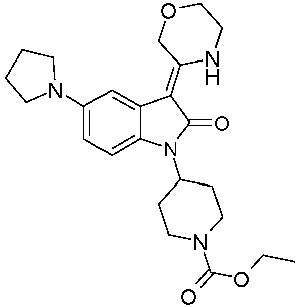 |
| 1144 | 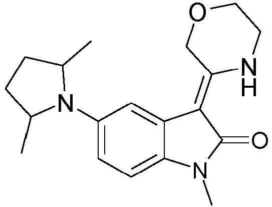 | 1145 | 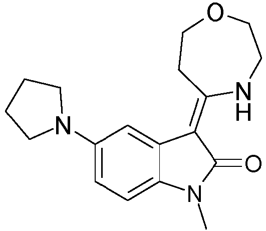 |
| 1146 | 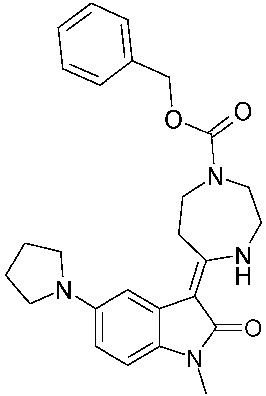 | 1147 | 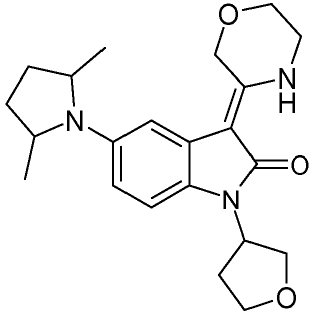 |
| 1148 | 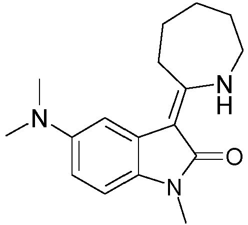 | 1149 | 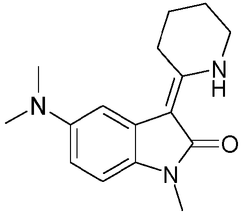 |
| 1150 | 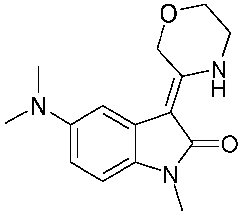 | 1151 | 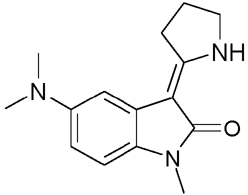 |
| 1152 | 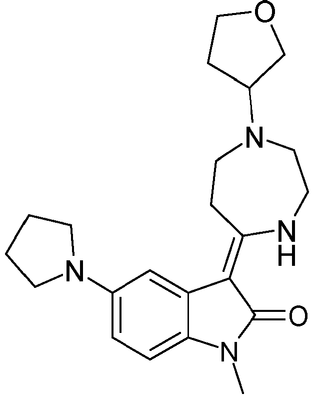 | 1153 | 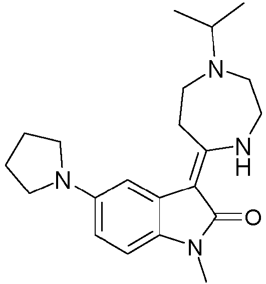 |
| 1154 | 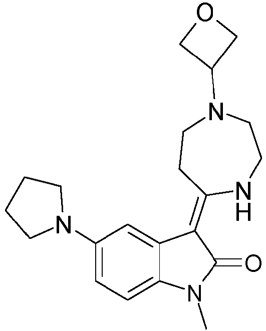 | 1155 | 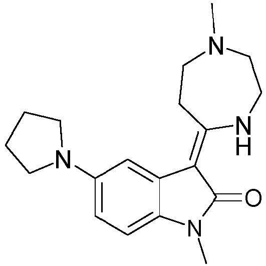 |
| 1156 | 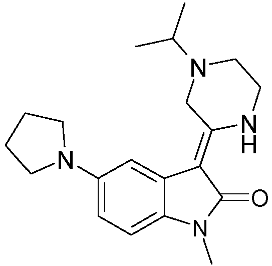 | 1157 | 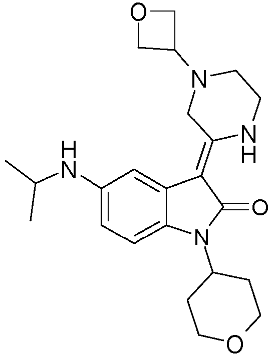 |
| 1158 | 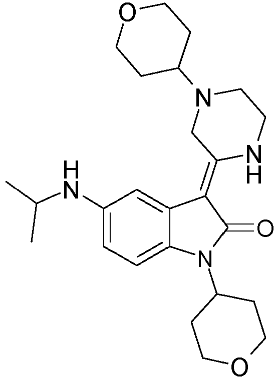 | 1159 | 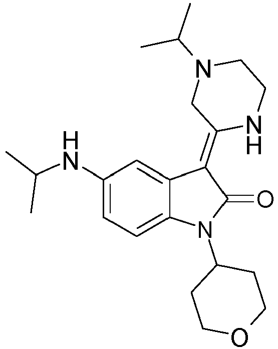 |
| 1160 | 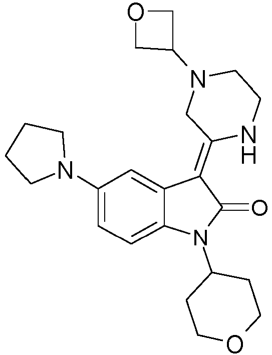 | 1161 | 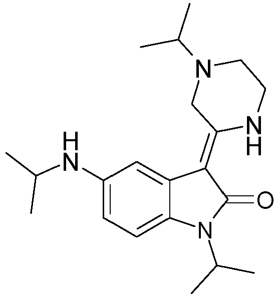 |
| 1162 | 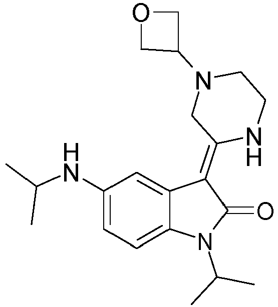 | 1163 | 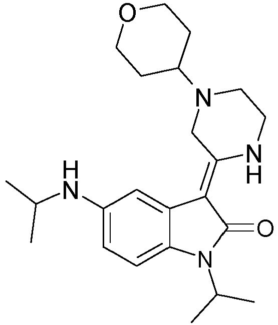 |
| 1164 | 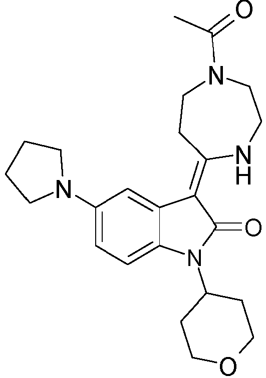 | 1165 | 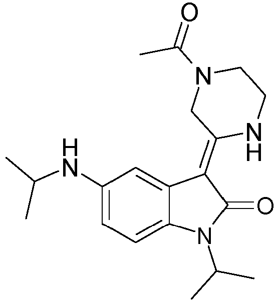 |
| 1166 | 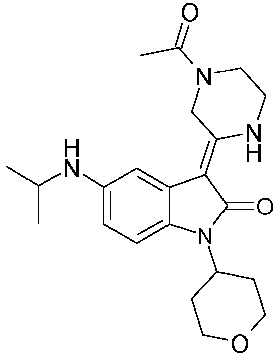 | 1167 | 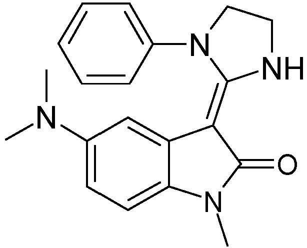 |
| 1168 | 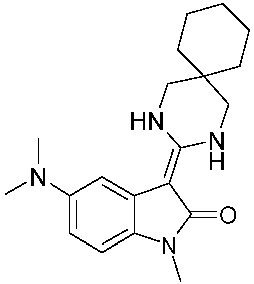 | 1169 | 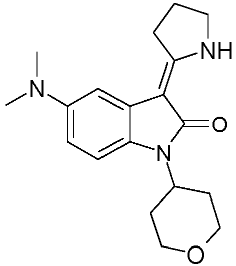 |
| 1170 | 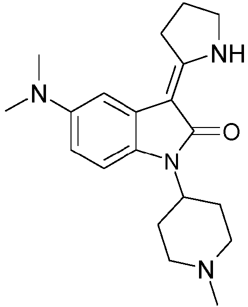 | 1171 | 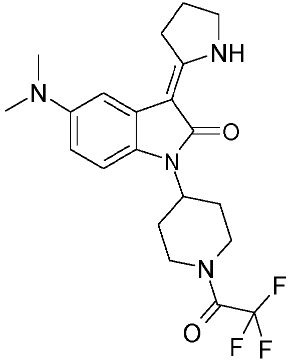 |
| 1172 | 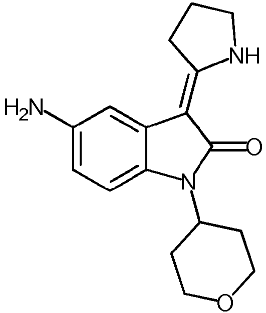 | 1173 | 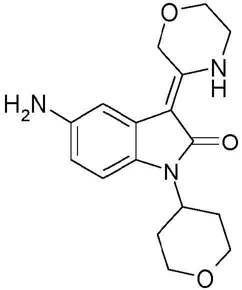 |
| 1174 | 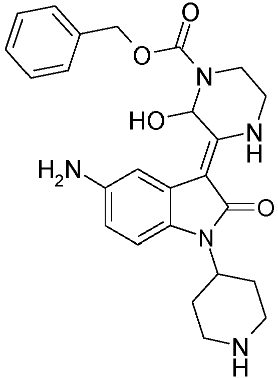 | 1175 | 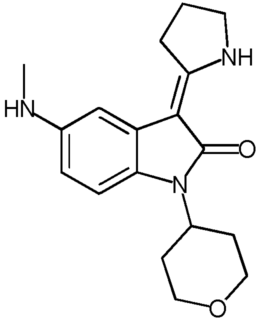 |
| 1176 | 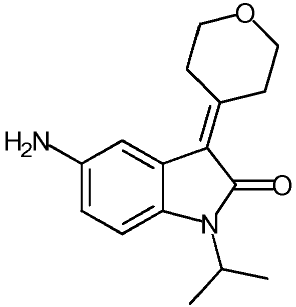 | 1177 | 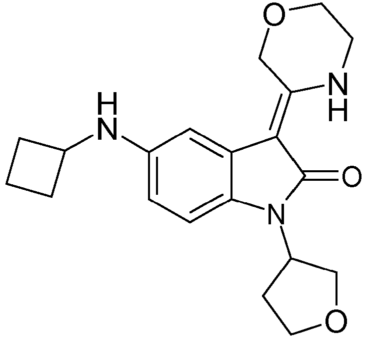 |
| 1178 | 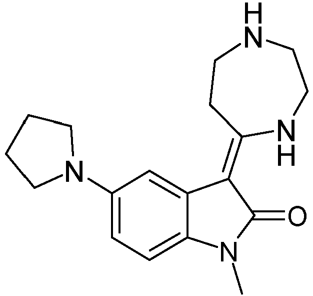 | | |

12. A medicament comprising a compound or salt thereof according to any one of claims 1 to 11.

13. A method for the preparation of the compound of claim 1, comprising
(a) reacting (2-Fluoro-5-nitro-phenyl)-acetic acid (compound II) 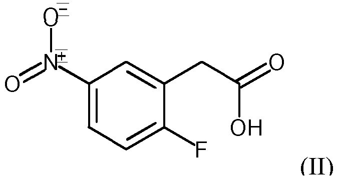 with an amine R1-NH₂ using an appropriate solvent to form a 5-nitro-2,3-dihydro-1H-indol-2-one (compound III) 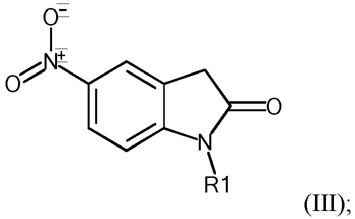
(b) condensing the 5-nitro-2,3-dihydro-1H-indol-2-one (compound III) at elevated temperature in a microwave either without or in a suitable solvent like peridine with an electrophile suitable to result in a 5-nitro-3-ylidene-2,3-dihydro-1H-indol-2-one (compound IV) 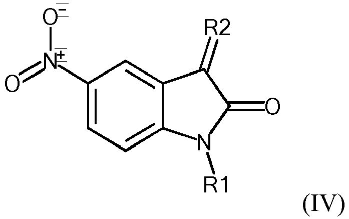
(c) reducing the 5-nitro-3-ylidene-2,3-dihydro-1H-indol-2-ones (compound IV) to an amino-substituted indol-2-one (compound V) 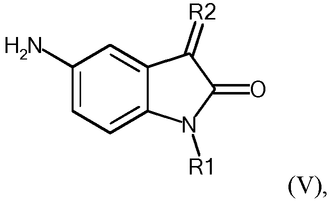
(d1) either reacting the compound of formula (V) with an aldehyde or ketone and a reducing agent suitable to obtain a compound of formula (I), or
(d2)reacting an amine of formula (V) in a substitution reaction with electrophiles suitable to carry a leaving group in the presence of a suitable base.

14. A pharmaceutical composition for use in the treatment or prevention of a neurological or neurodegenerative or psychiatric condition in a human being comprising a compound according to any one of claims 1 to 11, or a pharmaceutically acceptable salt thereof, and a pharmaceutically acceptable carrier.

15. The pharmaceutical composition according to claim 14 comprising a therapeutically effective amount of 0.1 to 2000 mg of the compound according to any one of claims 1 to 11, or a pharmaceutically acceptable salt thereof.

## Patentansprüche

1. Verbindung der Formel (I) oder deren Salz, Hydrat oder Solvat 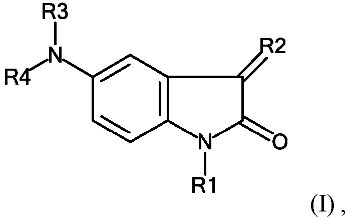 wobei:
**R1** C₁₋₄-Alkyl darstellt, unsubstituiert oder substituiert mit MeO; Tetrahydropyranyl, Tetrahydrofuranyl, Oxetanyl, Dioxepanyl, Pyrrolidinyl oder Piperidinyl; oder Pyrrolidinyl oder Piperidinyl, wobei der Stickstoff mit Methyl, Isopropyl, Oxetanyl, Ethoxycarbonyl, Acetyl oder Trifluoracetyl substituiert ist;
**R2** einen 5-, 6- oder 7-gliedrigen unsubstituierten oder substituierten Ring darstellt, enthaltend 1 oder 2 Heteroatome, ausgewählt aus O oder N, oder eine unsubstituierte oder substituierte spirocyclische Heterocyclylgruppe, enthaltend 1 bis 3 Heteroatome, ausgewählt aus N oder O, bestehend aus 4 bis 11 Ringatomen, wobei der Ring oder die spirocyclische Heterocyclylgruppe in Formel (I) durch eine C=C-Doppelbindung verknüpft ist, und
wobei in dem Ring oder der spirocyclischen Heterocyclylgruppe ein N-Atom mit Methyl, Isopropyl, Acetyl, Benzyloxycarbonyl, Phenyl, Oxetanyl oder Tetrahydropyranyl substituiert sein kann, und
in denen ein oder mehrere C-Atome mit -Methyl oder -OH substituiert sein können;
**R3** oder **R4** sind unabhängig voneinander
Wasserstoff; C₁₋₆-Alkyl, unsubstituiert oder substituiert mit einem oder mehreren F, Methoxy, C₃₋₈-Cycloalkyl, unsubstituiert oder substituiert mit einem oder mehreren F; Aryl; Heteroaryl, bestehend aus 5 bis 6 Ringatomen; oder Heterocyclyl, ausgewählt aus der Gruppe, bestehend aus Oxetanyl, Tetrahydropyranyl und Pyrrolidinyl, wobei das Heterocyclyl unsubstituiert oder mit C₁₋₆-Alkyl, Acetyl, Tetrahydrofuranyl, Oxetanyl oder Hydroxyethylacetyl substituiert ist;
oder **R3** und **R4** bilden zusammen mit dem verknüpften N einen Heterocyclylring, ausgewählt aus der Gruppe, bestehend aus Morpholinyl und Pyrrolidinyl, beide unsubstituiert oder mit C₁₋₆-Alkyl, F oder Hydroxyl substituiert.

2. Verbindung nach Anspruch 1 oder deren Salz, wobei **R1** darstellt: 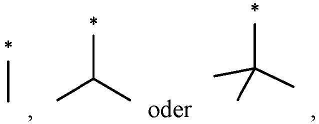 unsubstituiert oder substituiert mit MeO; 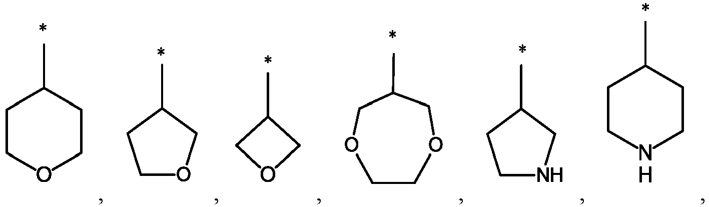 unsubstituiert oder der Stickstoff substituiert mit

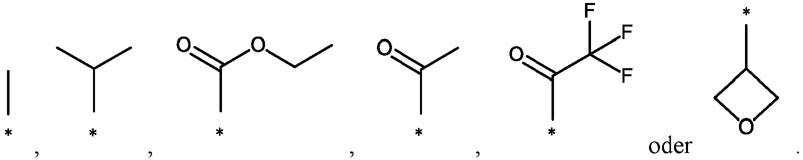

3. Verbindung nach Anspruch 1 oder deren Salz, wobei **R1** darstellt:

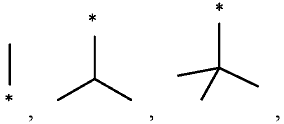

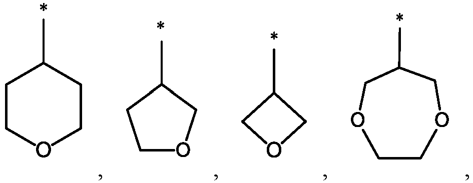

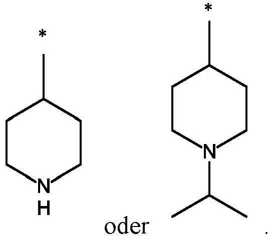

4. Verbindung nach Anspruch 2 oder deren Salz, wobei **R1** Tetrahydropyranyl oder Dioxepanyl darstellt.

5. Verbindung nach Anspruch 1 oder deren Salz, wobei **R2** einen 5-, 6- oder 7-gliedrigen Ring darstellt, enthaltend 1 oder 2 Heteroatome, ausgewählt aus O oder N, der in Formel (1) durch eine C=C-Doppelbindung verknüpft ist,
in dem eines oder beide N-Atome mit Methyl, Isopropyl, Acetyl, Benzyloxycarbonyl, Phenyl, Oxetanyl oder Tetrahydropyranyl substituiert sein können, und
in denen ein oder mehrere C-Atome mit -Methyl oder -OH substituiert sein können.

6. Verbindung nach Anspruch 1 oder deren Salz, wobei das Strukturelement 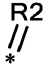 darstellt:

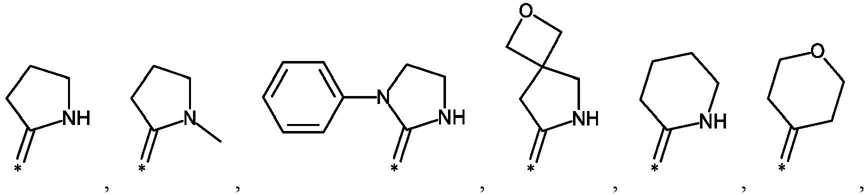

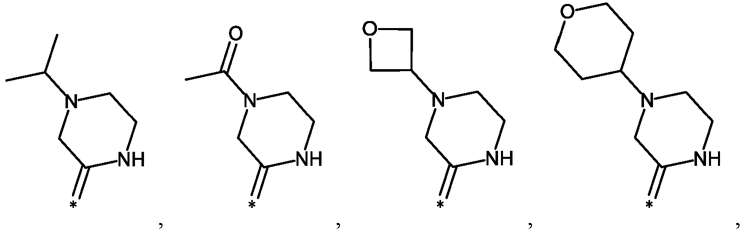

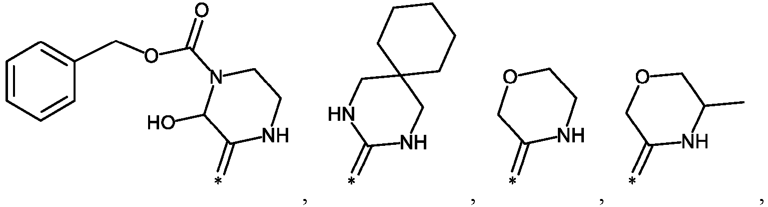

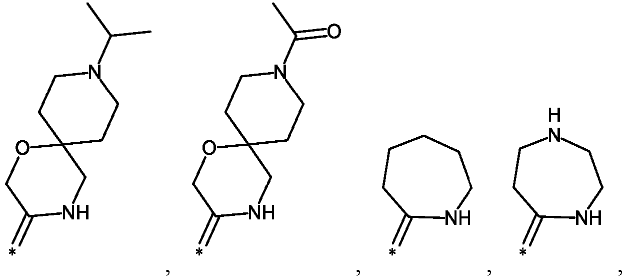

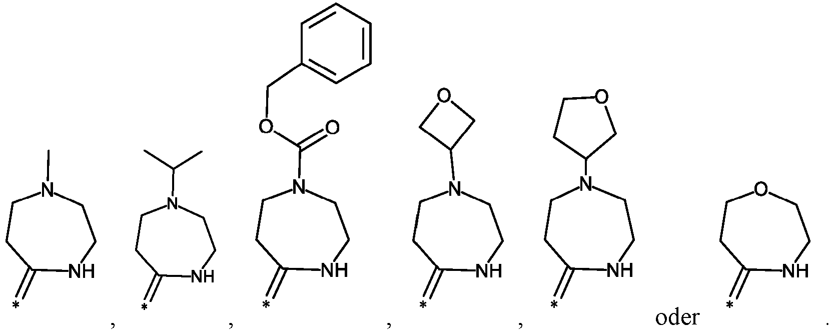

7. Verbindung nach Anspruch 1 oder deren Salz, wobei die Aminogruppe, die **R3** und **R4** in Formel (I) enthält, darstellt:

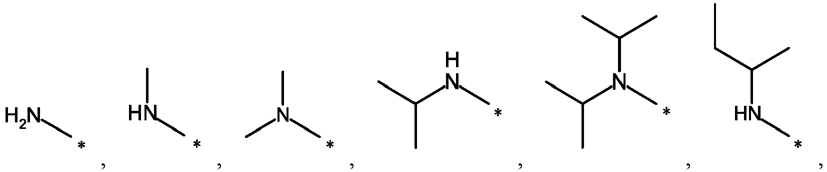

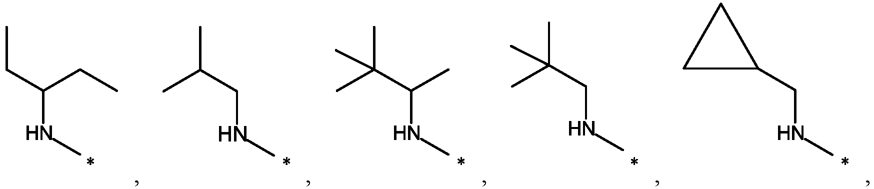

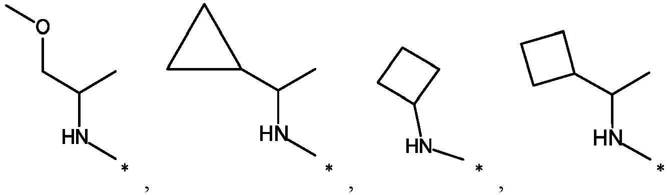

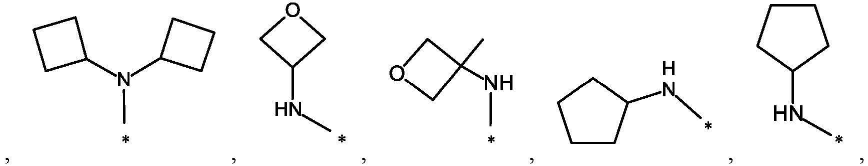

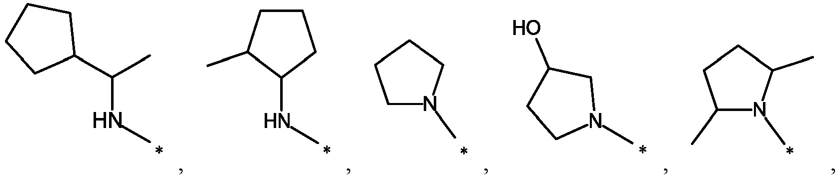

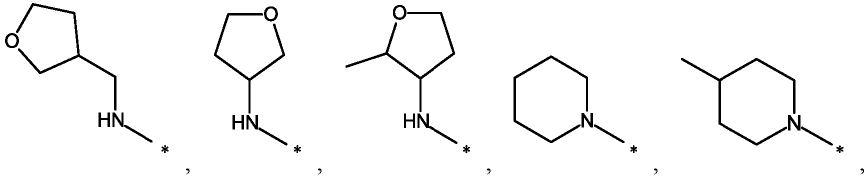

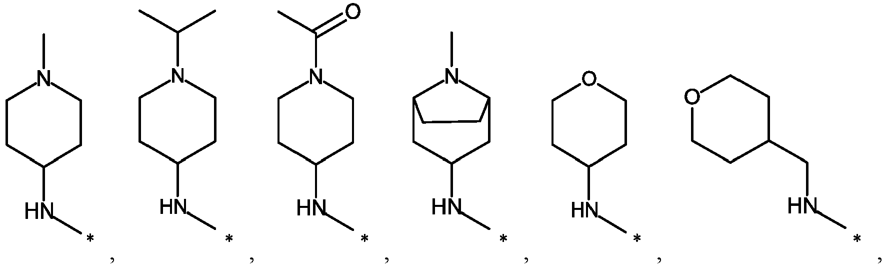

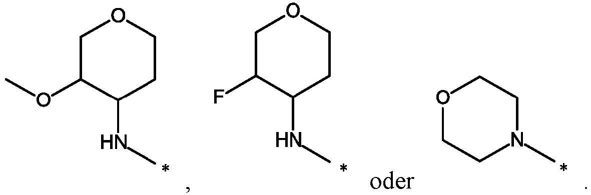

8. Verbindung nach Anspruch 1 oder deren Salz, wobei eine Aminogruppe, die **R3** und **R4** enthält, in Formel (I) Wasserstoff als **R3** umfasst, während **R4** Isopropyl, Cyclobutyl oder Cyclopentyl darstellt, die unsubstituiert oder mit Fluor (F) substituiert sind

9. Verbindung nach Anspruch 1 oder deren Salz, wobei eine Aminogruppe, die **R3** und **R4** enthält, in Formel (I) darstellt: 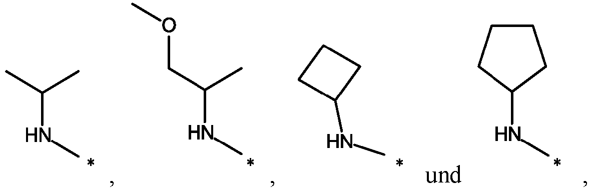 die unsubstituiert oder mit Fluor (F) substituiert sind.

10. Salz einer Verbindung nach einem der Ansprüche 1-9.

11. Verbindung nach Anspruch 1 oder deren Salz, wobei Formel (I) die Formel einer der Verbindungen 1 bis 175 und 1001-1178 darstellt:
| **Nr.** | **Formel** | **Nr.** | **Formel** |
|---|---|---|---|
| 1 | 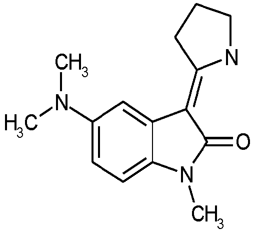 | 2 | 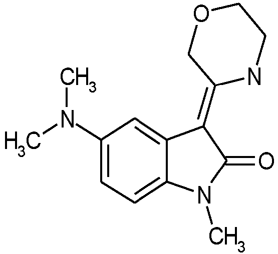 |
| 3 | 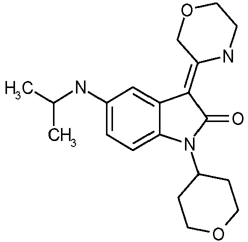 | 4 | 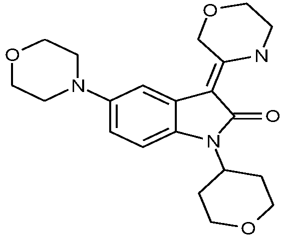 |
| 5 | 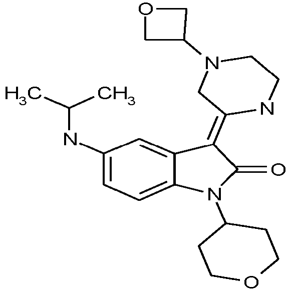 | 6 | 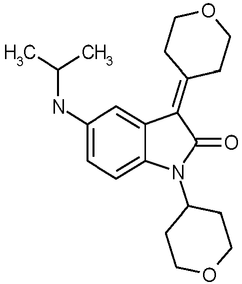 |
| 7 | 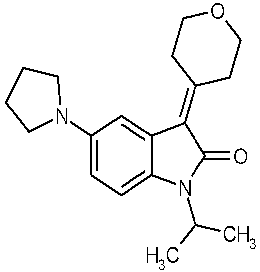 | 8 | 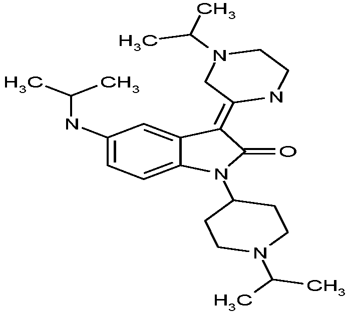 |
| 9 | 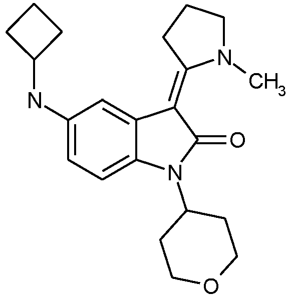 | 10 | 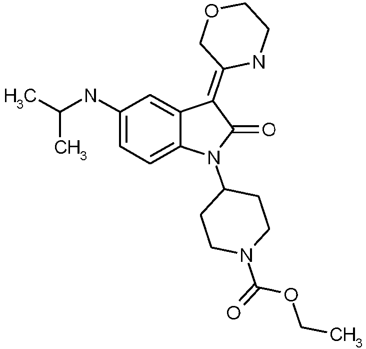 |
| 11 | 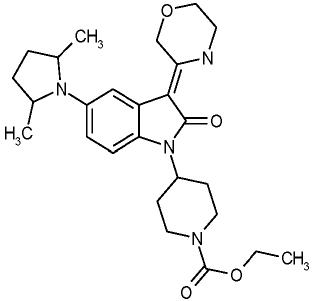 | 12 | 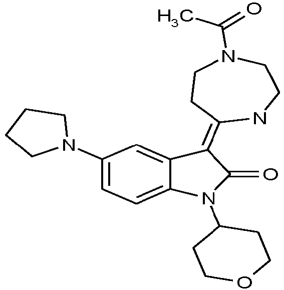 |
| 13 | 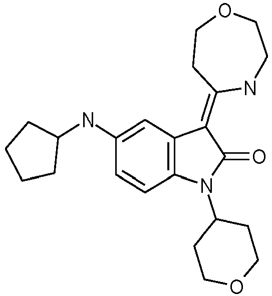 | 14 | 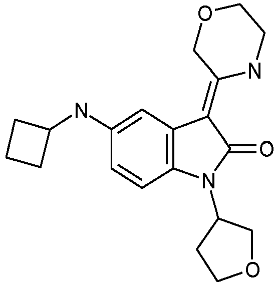 |
| 15 | 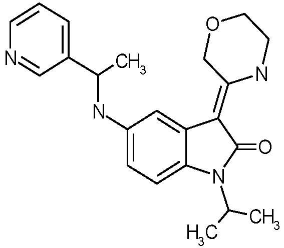 | 16 | 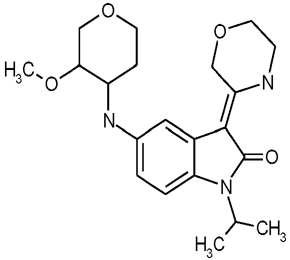 |
| 17 | 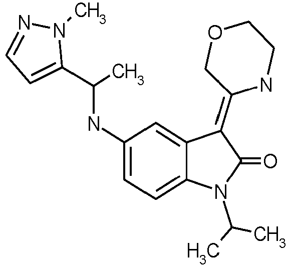 | 18 | 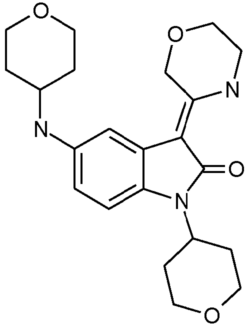 |
| 19 | 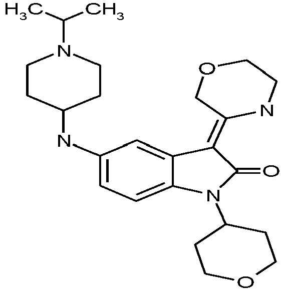 | 20 | 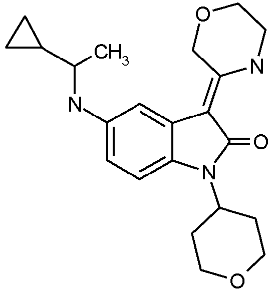 |
| 21 | 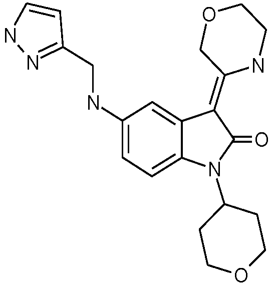 | 22 | 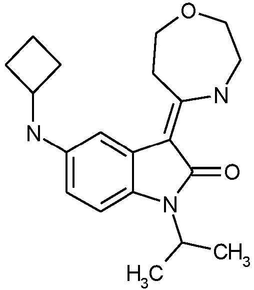 |
| 23 | 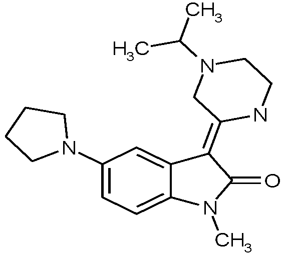 | 24 | 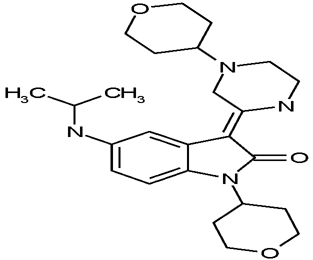 |
| 25 | 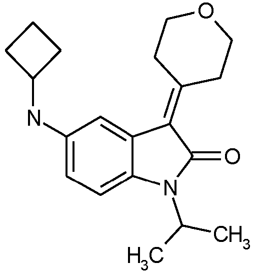 | 26 | 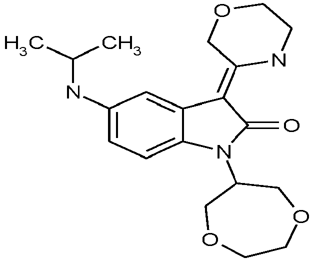 |
| 27 | 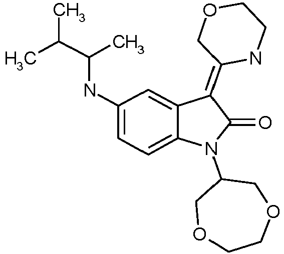 | 28 | 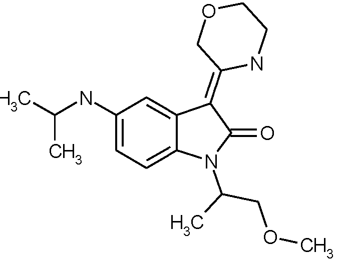 |
| 29 | 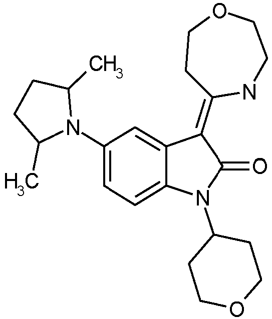 | 30 | 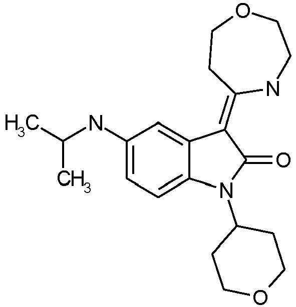 |
| 31 | 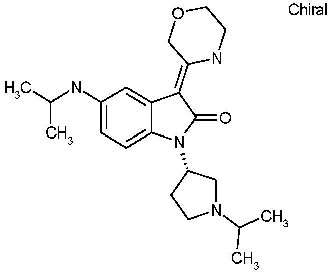 | 32 | 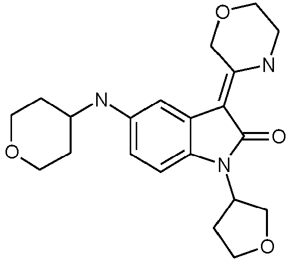 |
| 33 | 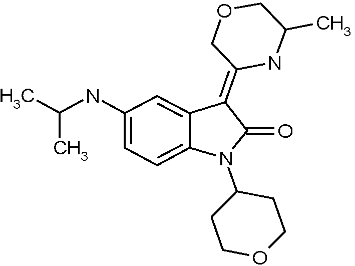 | 34 | 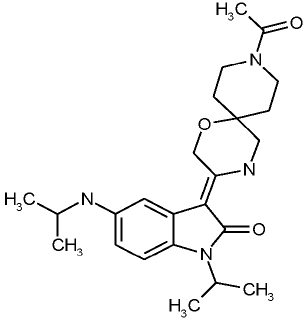 |
| 35 | 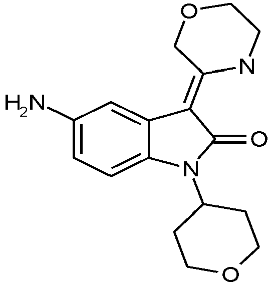 | 36 | 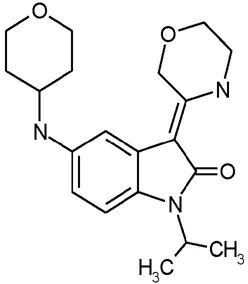 |
| 37 | 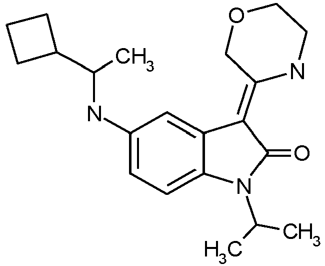 | 38 | 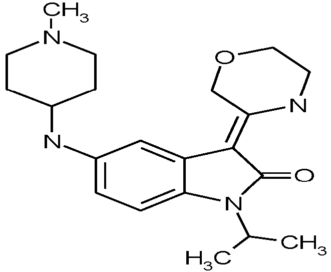 |
| 39 | 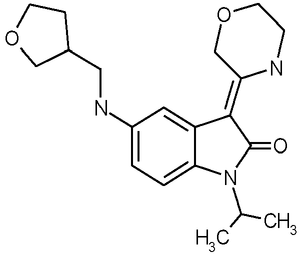 | 40 | 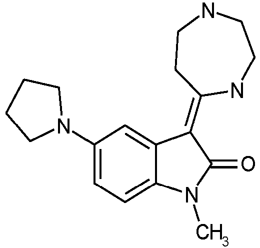 |
| 41 | 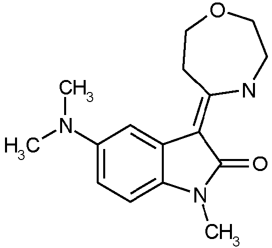 | 42 | 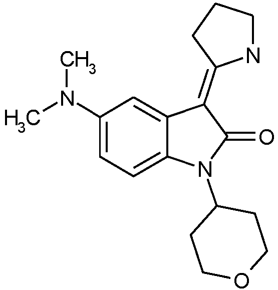 |
| 43 | 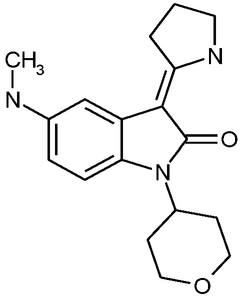 | 44 | 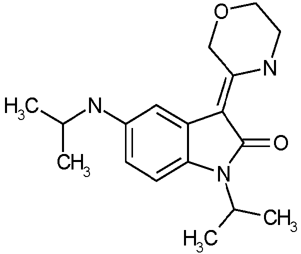 |
| 45 | 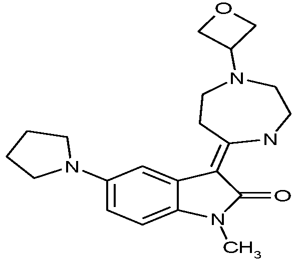 | 46 | 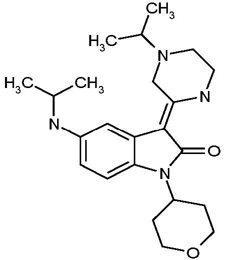 |
| 47 | 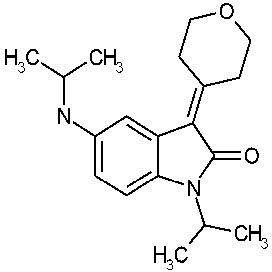 | 48 | 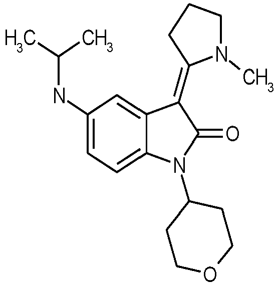 |
| 49 | 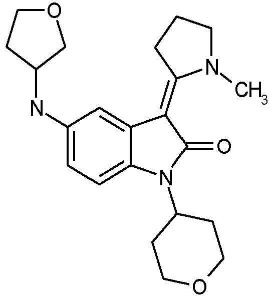 | 50 | 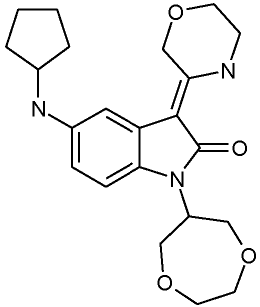 |
| 51 | 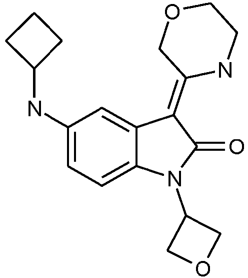 | 52 | 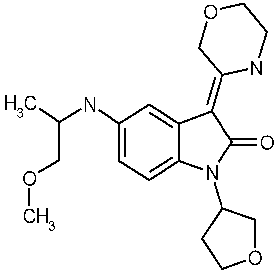 |
| 53 | 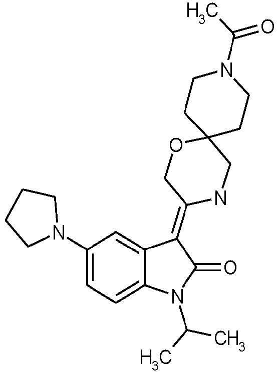 | 54 | 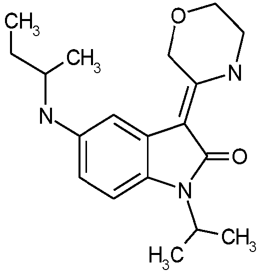 |
| 55 | 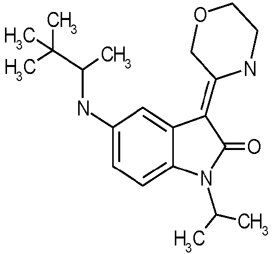 | 56 | 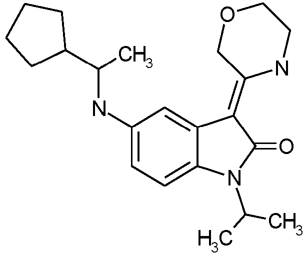 |
| 57 | 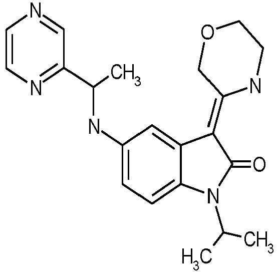 | 58 | 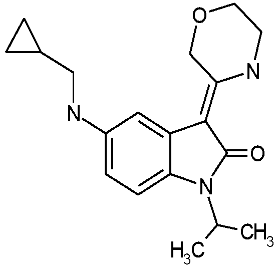 |
| 59 | 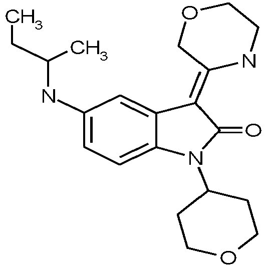 | 60 | 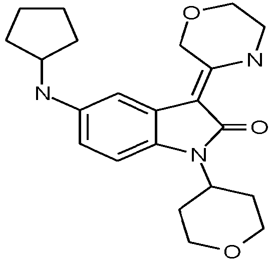 |
| 61 | 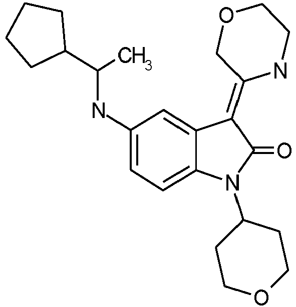 | 62 | 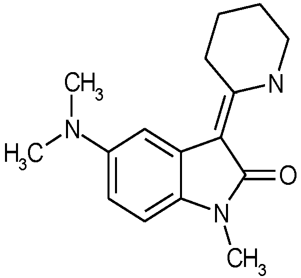 |
| 63 | 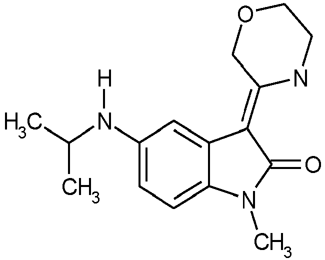 | 64 | 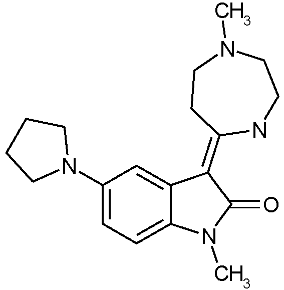 |
| 65 | 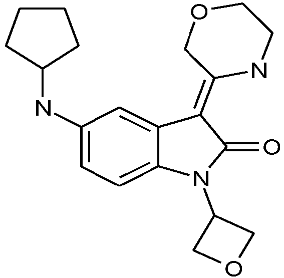 | 66 | 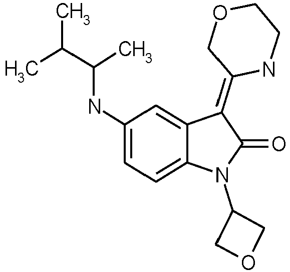 |
| 67 | 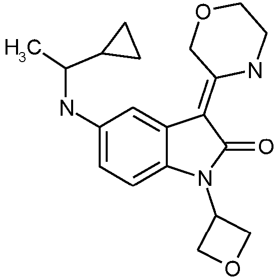 | 68 | 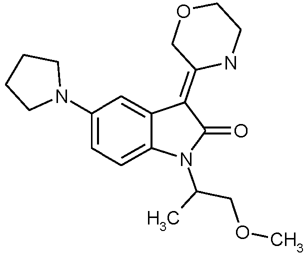 |
| 69 | 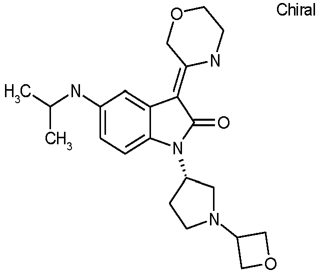 | 70 | 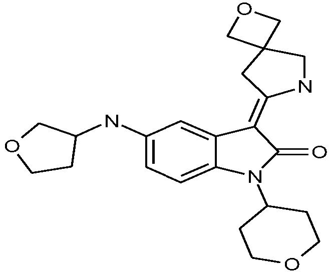 |
| 71 | 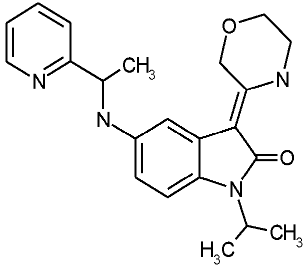 | 72 | 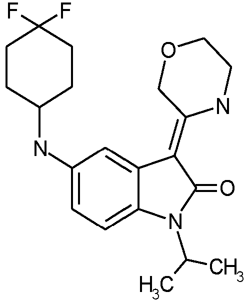 |
| 73 | 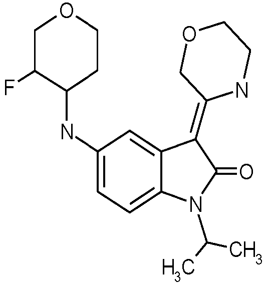 | 74 | 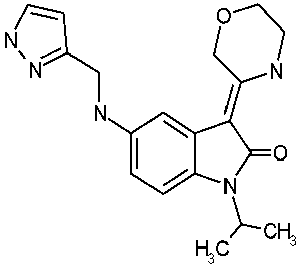 |
| 75 | 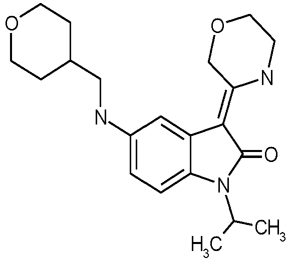 | 76 | 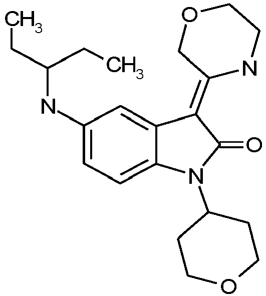 |
| 77 | 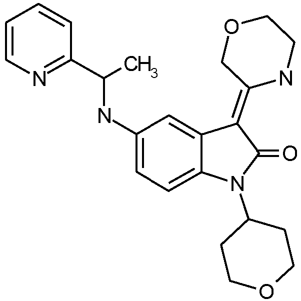 | 78 | 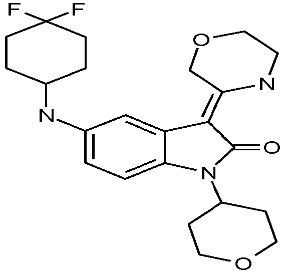 |
| 79 | 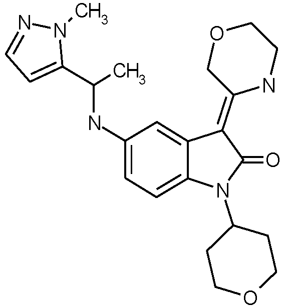 | 80 | 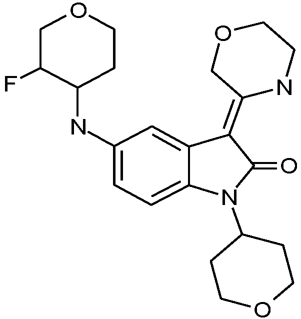 |
| 81 | 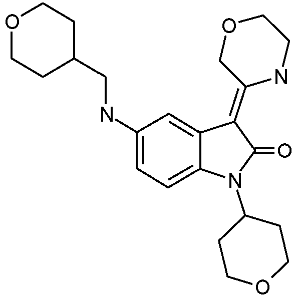 | 82 | 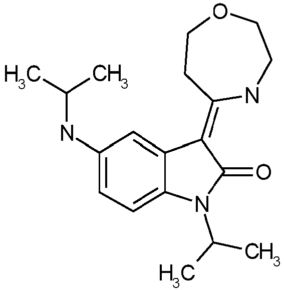 |
| 83 | 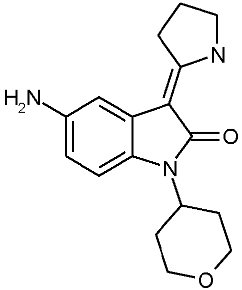 | 84 | 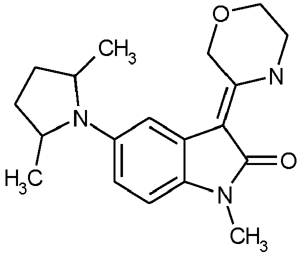 |
| 85 | 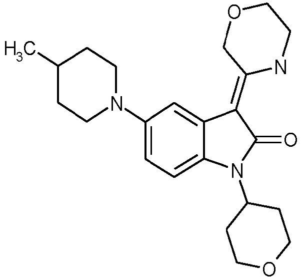 | 86 | 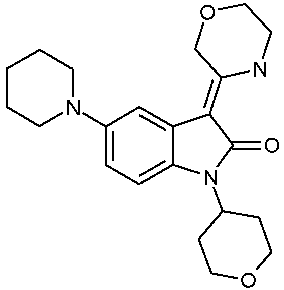 |
| 87 | 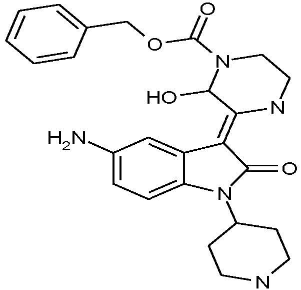 | 88 | 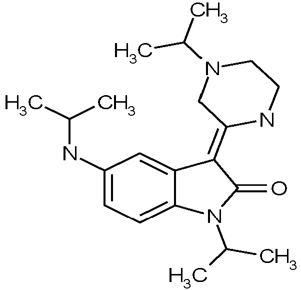 |
| 89 | 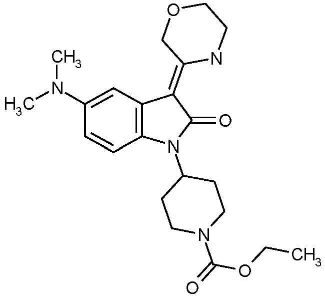 | 90 | 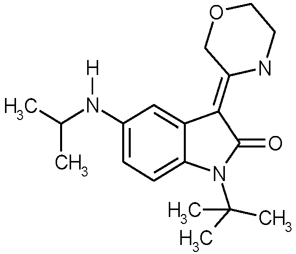 |
| 91 | 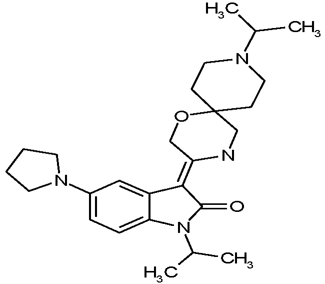 | 92 | 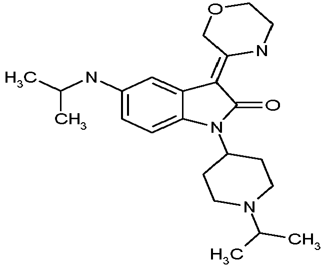 |
| 93 | 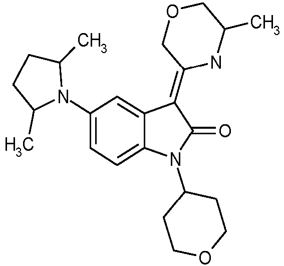 | 94 | 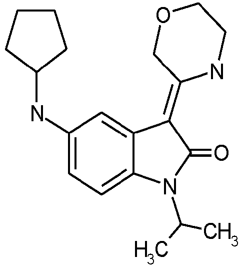 |
| 95 | 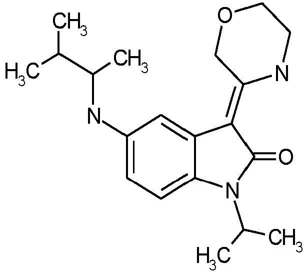 | 96 | 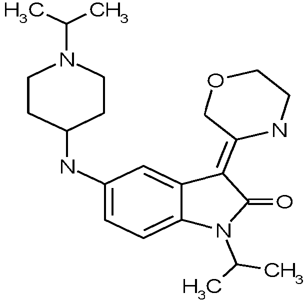 |
| 97 | 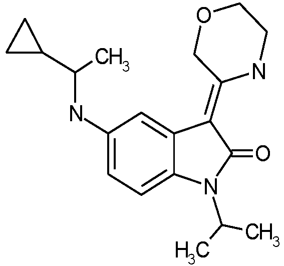 | 98 | 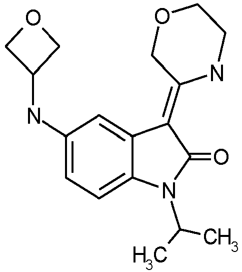 |
| 99 | 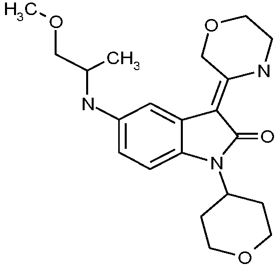 | 100 | 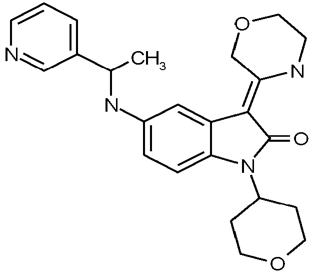 |
| 101 | 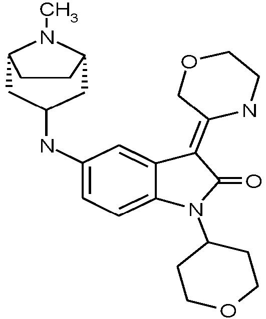 | 102 | 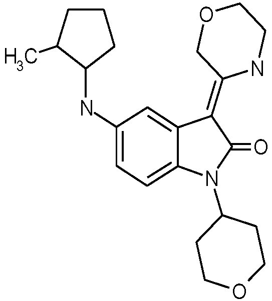 |
| 103 | 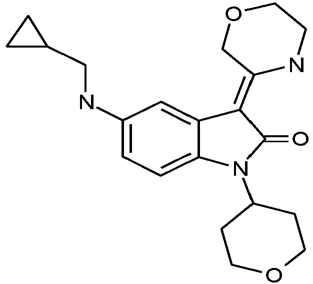 | 104 | 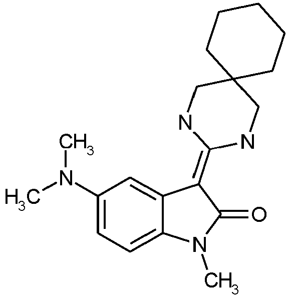 |
| 105 | 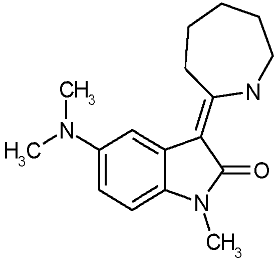 | 106 | 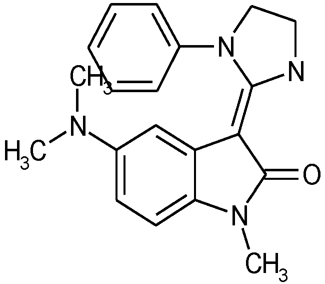 |
| 107 | 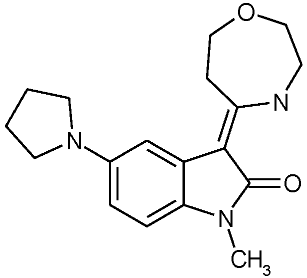 | 108 | 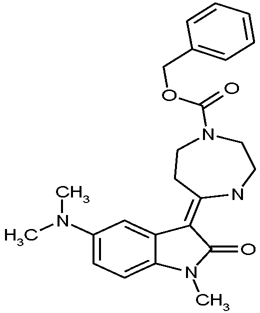 |
| 109 | 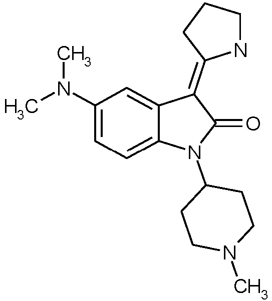 | 110 | 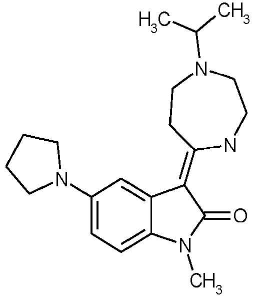 |
| 111 | 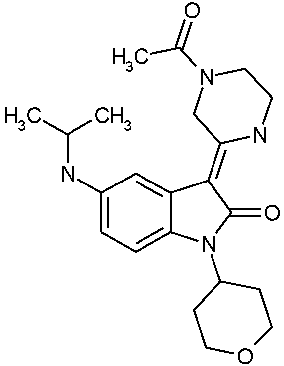 | 112 | 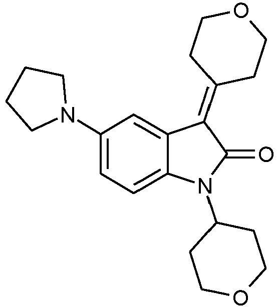 |
| 113 | 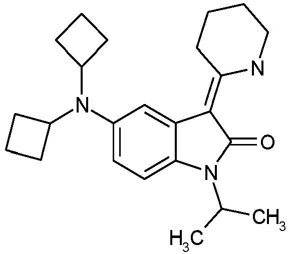 | 114 | 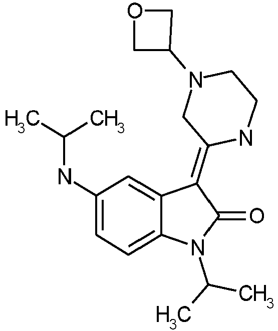 |
| 115 | 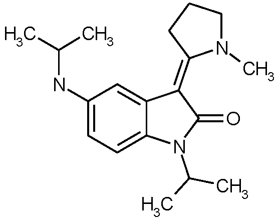 | 116 | 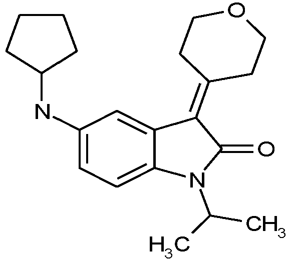 |
| 117 | 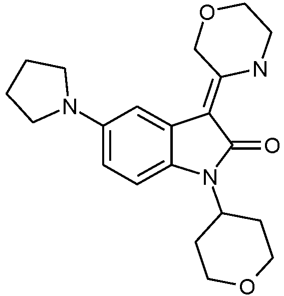 | 118 | 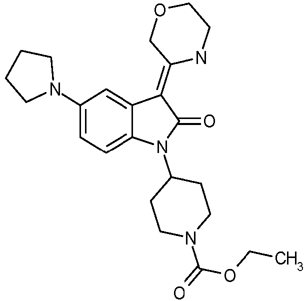 |
| 119 | 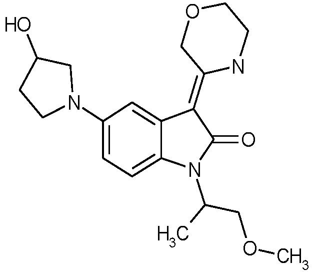 | 120 | 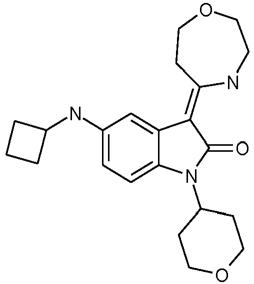 |
| 121 | 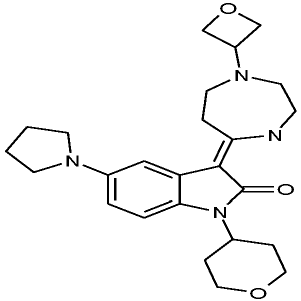 | 122 | 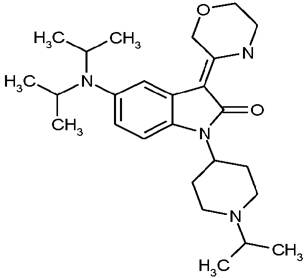 |
| 123 | 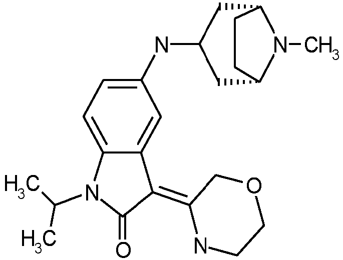 | 124 | 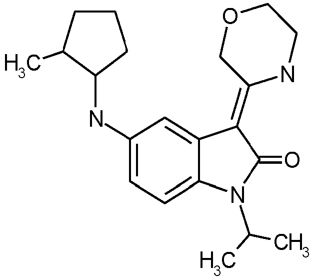 |
| 125 | 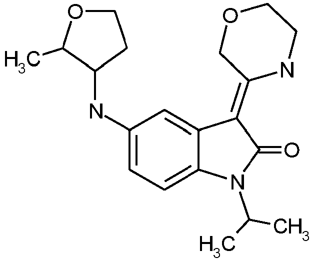 | 126 | 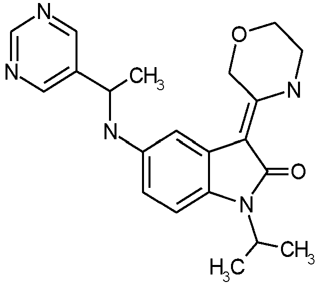 |
| 127 | 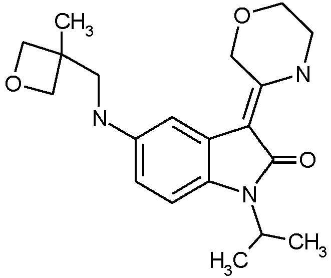 | 128 | 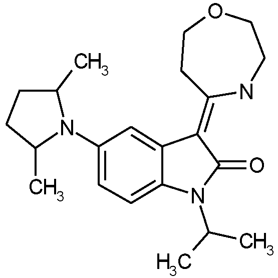 |
| 129 | 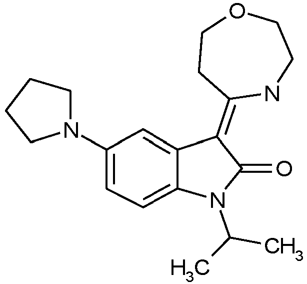 | 130 | 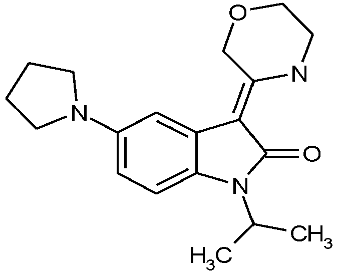 |
| 131 | 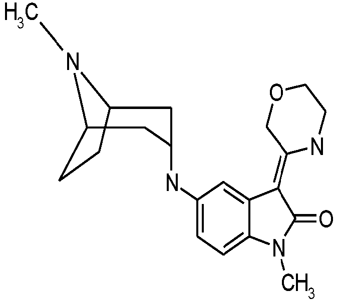 | 132 | 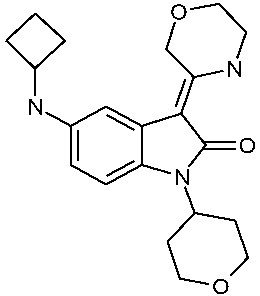 |
| 133 | 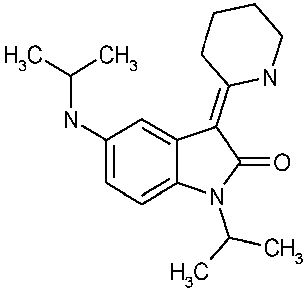 | 134 | 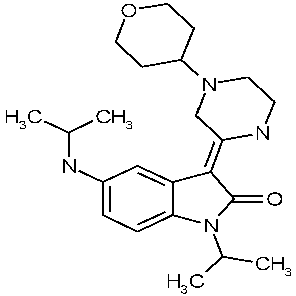 |
| 135 | 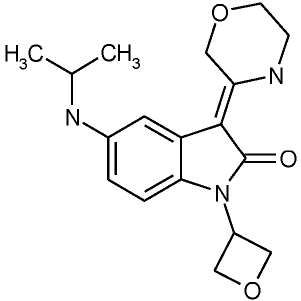 | 136 | 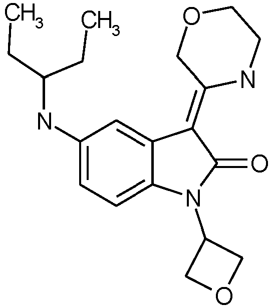 |
| 137 | 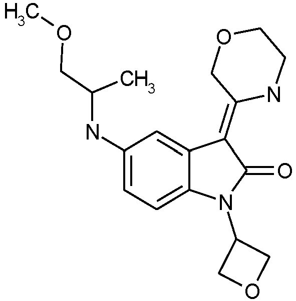 | 138 | 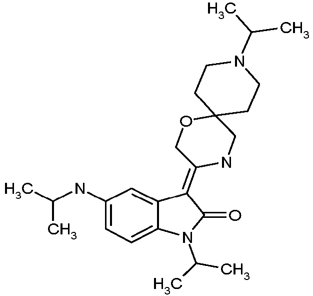 |
| 139 | 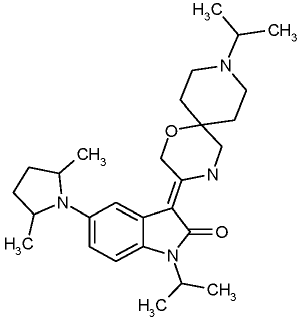 | 140 | 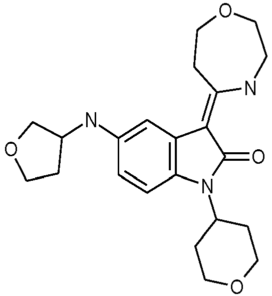 |
| 141 | 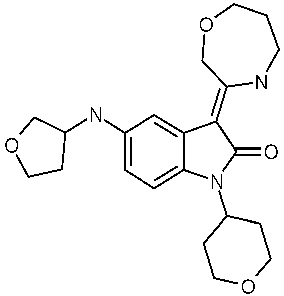 | 142 | 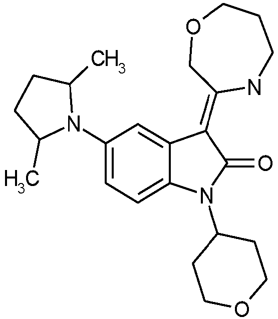 |
| 143 | 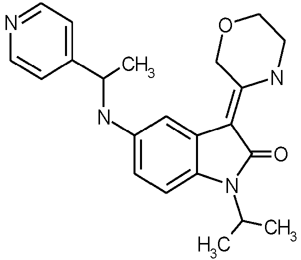 | 144 | 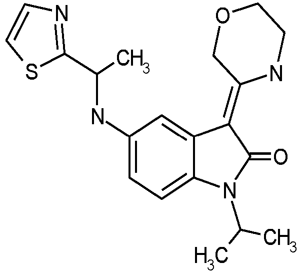 |
| 145 | 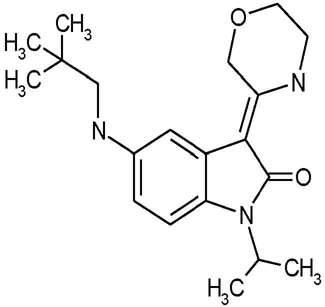 | 146 | 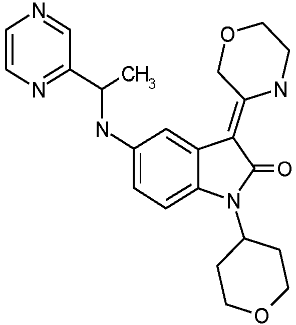 |
| 147 | 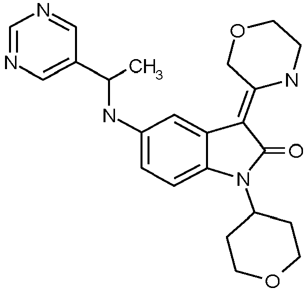 | 148 | 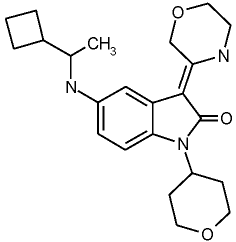 |
| 149 | 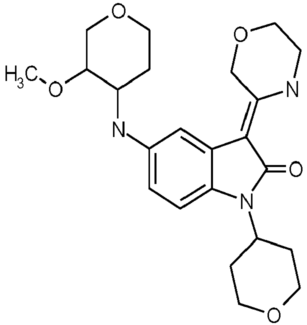 | 150 | 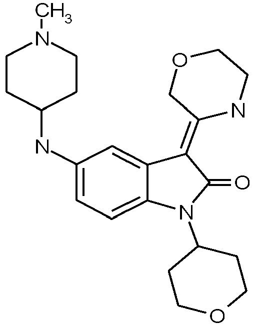 |
| 151 | 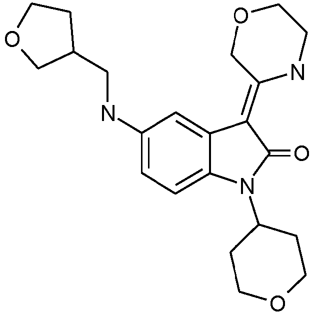 | 152 | 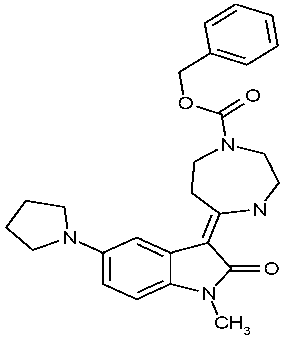 |
| 153 | 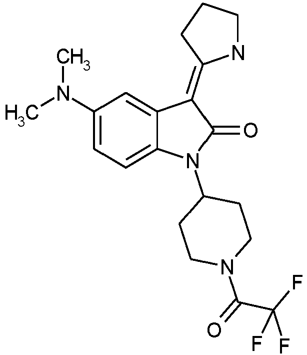 | 154 | 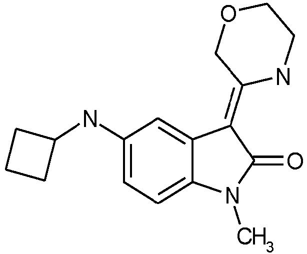 |
| 155 | 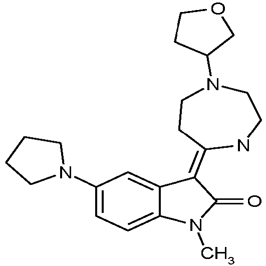 | 156 | 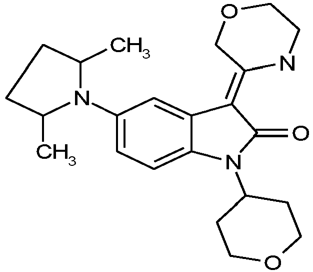 |
| 157 | 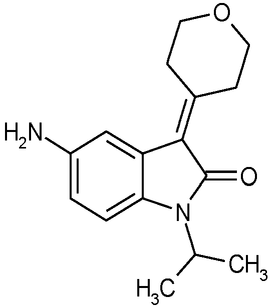 | 158 | 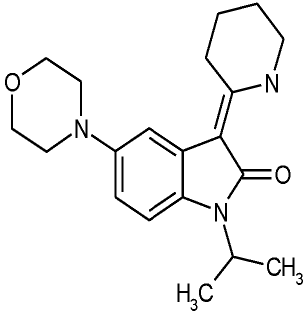 |
| 159 | 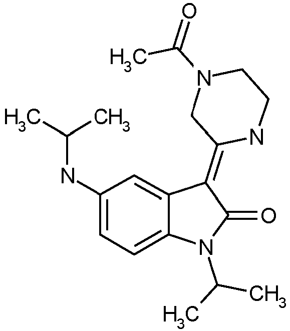 | 160 | 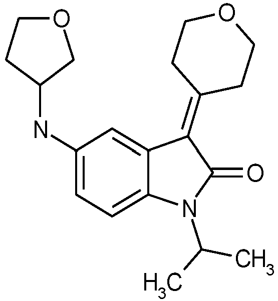 |
| 161 | 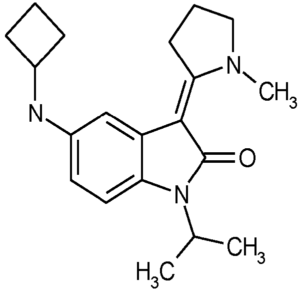 | 162 | 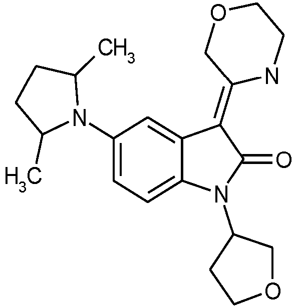 |
| 163 | 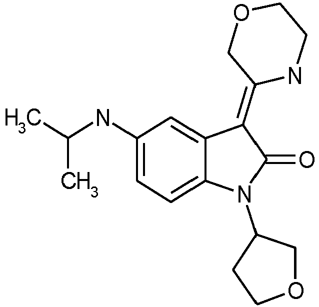 | 164 | 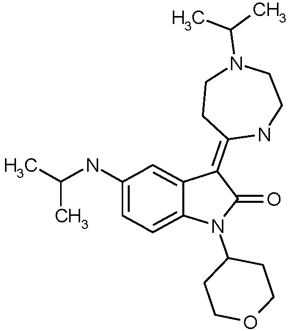 |
| 165 | 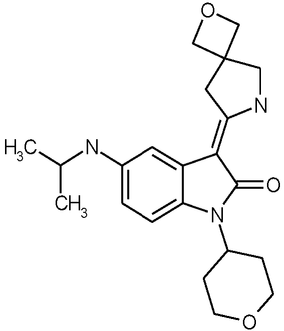 | 166 | 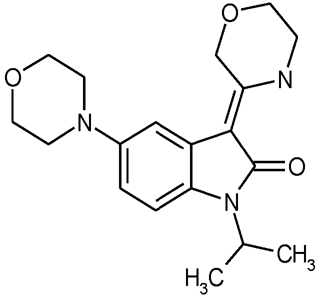 |
| 167 | 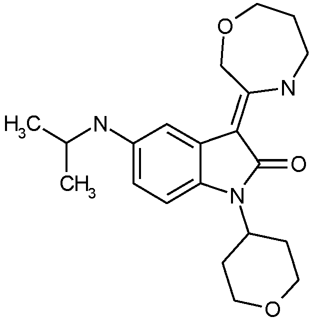 | 168 | 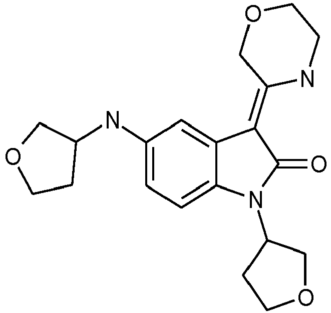 |
| 169 | 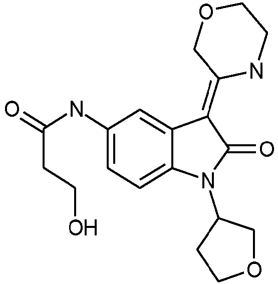 | 170 | 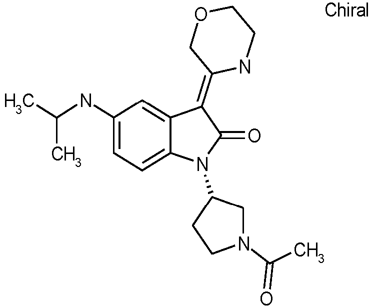 |
| 171 | 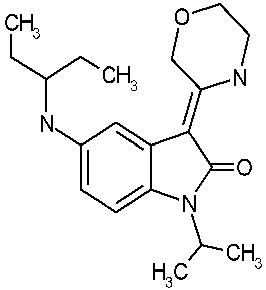 | 172 | 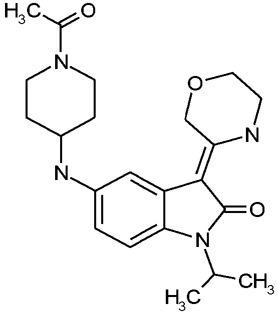 |
| 173 | 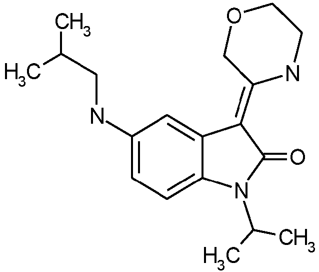 | 174 | 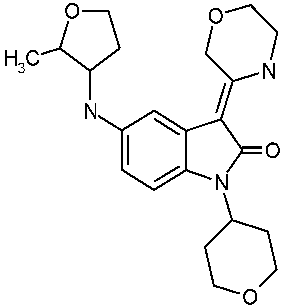 |
| 175 | 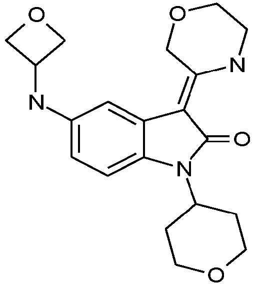 | 1001 | 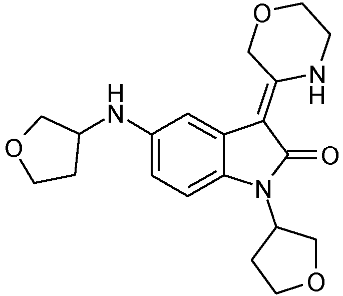 |
| 1002 | 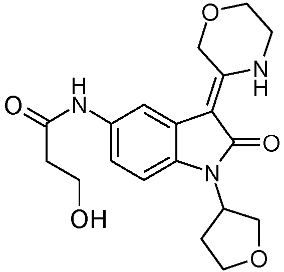 | 1003 | 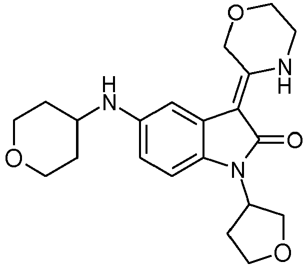 |
| 1004 | 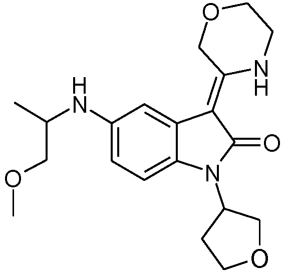 | 1005 | 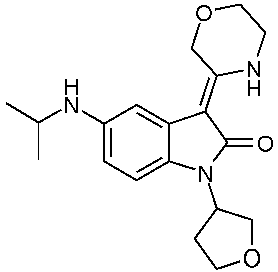 |
| 1006 | 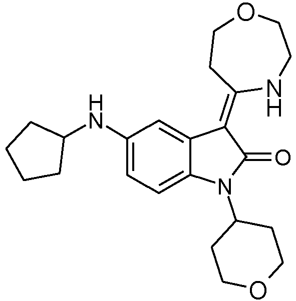 | 1007 | 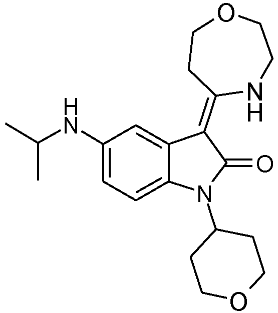 |
| 1008 | 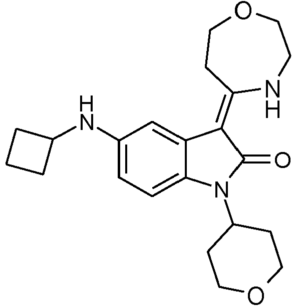 | 1009 | 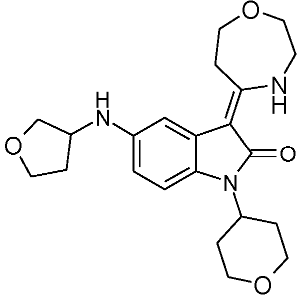 |
| 1010 | 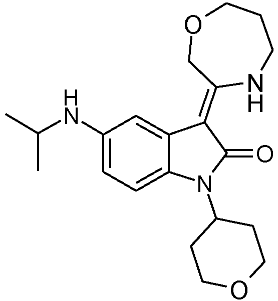 | 1011 | 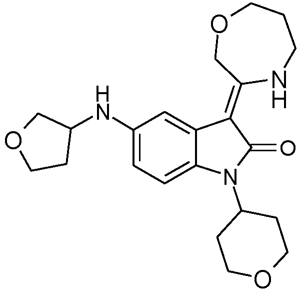 |
| 1012 | 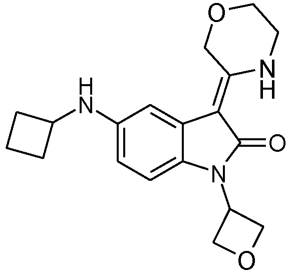 | 1013 | 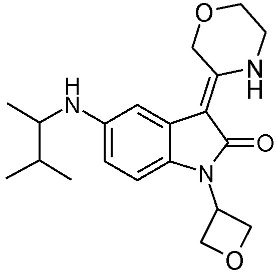 |
| 1014 | 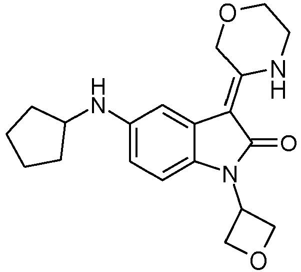 | 1015 | 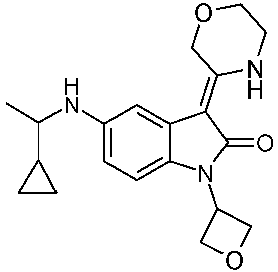 |
| 1016 | 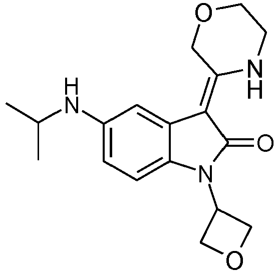 | 1017 | 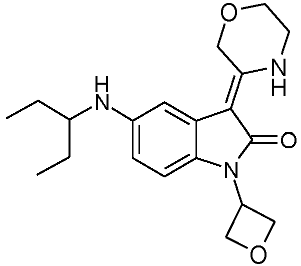 |
| 1018 | 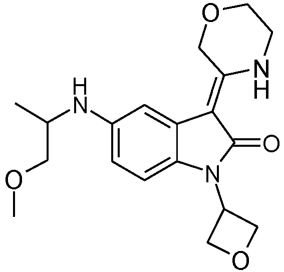 | 1019 | 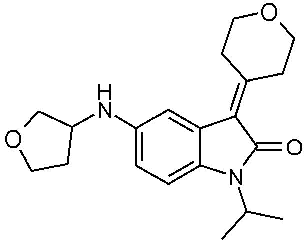 |
| 1020 | 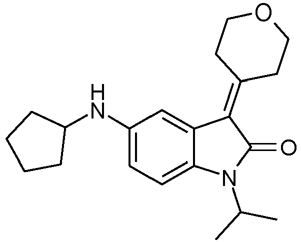 | 1021 | 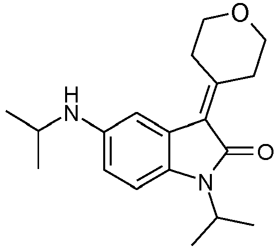 |
| 1022 | 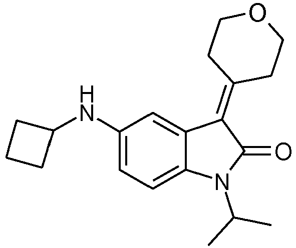 | 1023 | 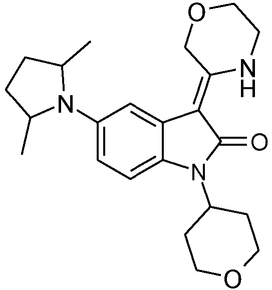 |
| 1024 | 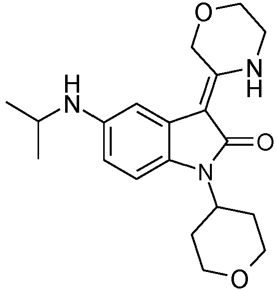 | 1025 | 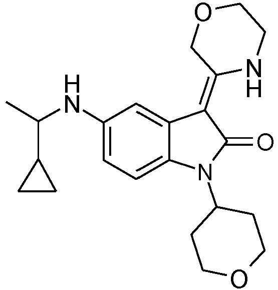 |
| 1026 | 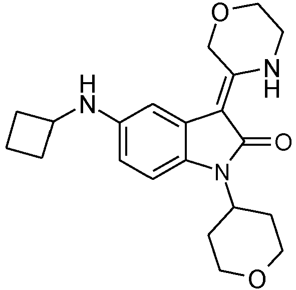 | 1027 | 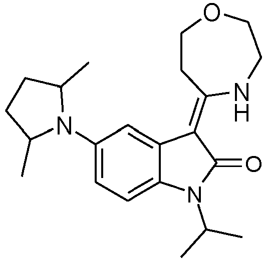 |
| 1028 | 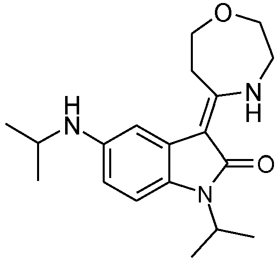 | 1029 | 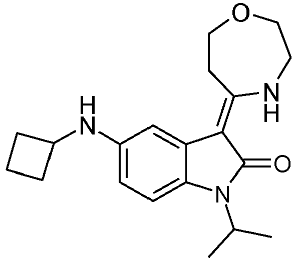 |
| 1030 | 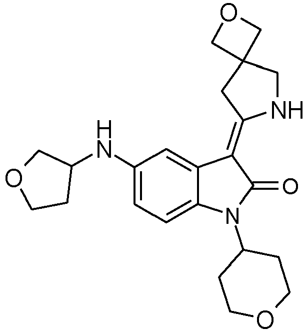 | 1031 | 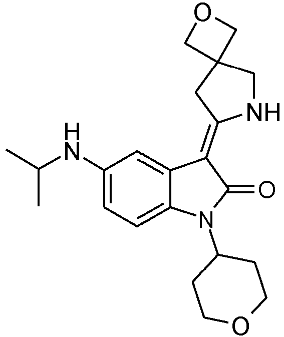 |
| 1032 | 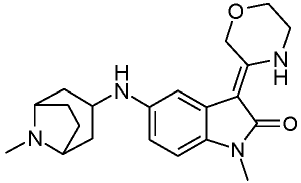 | 1033 | 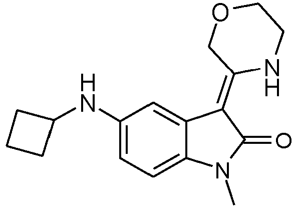 |
| 1034 | 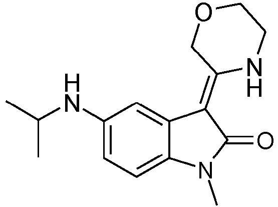 | 1035 | 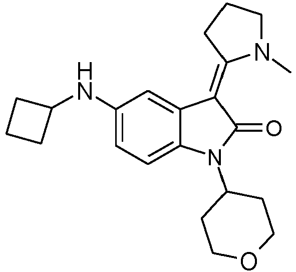 |
| 1036 | 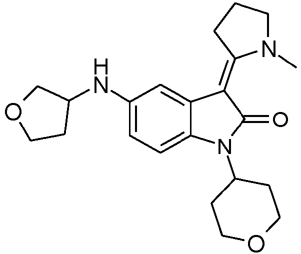 | 1037 | 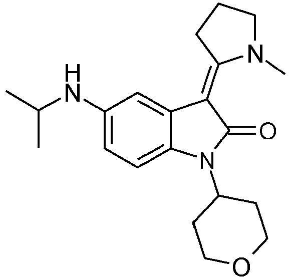 |
| 1038 | 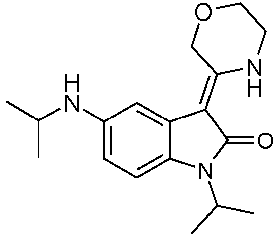 | 1039 | 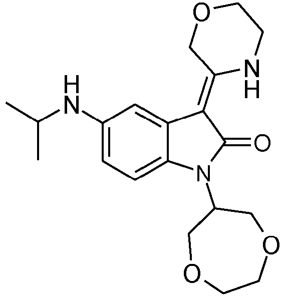 |
| 1040 | 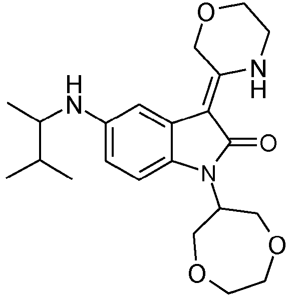 | 1041 | 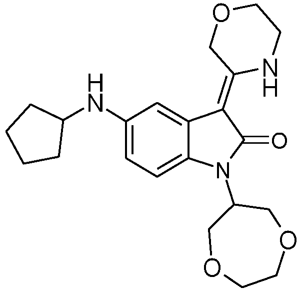 |
| 1042 | 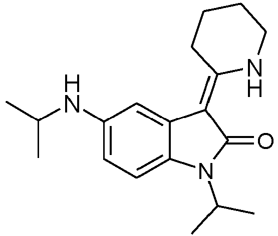 | 1043 | 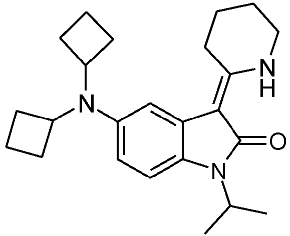 |
| 1044 | 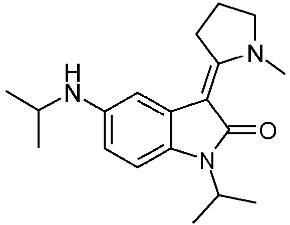 | 1045 | 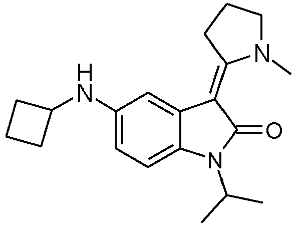 |
| 1046 | 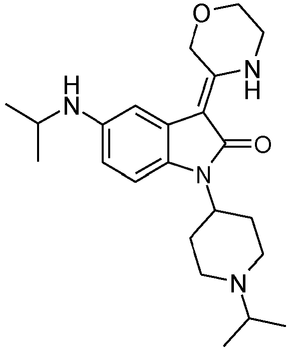 | 1047 | 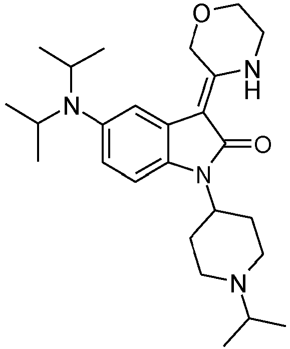 |
| 1048 | 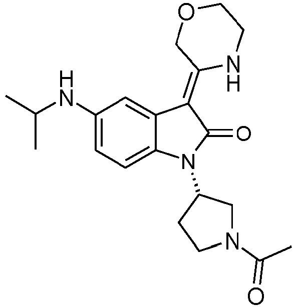 | 1049 | 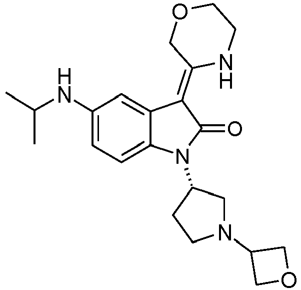 |
| 1050 | 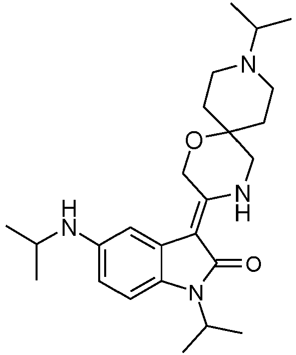 | 1051 | 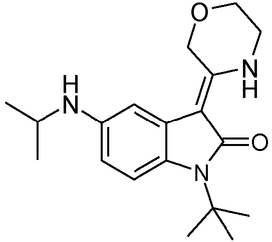 |
| 1052 | 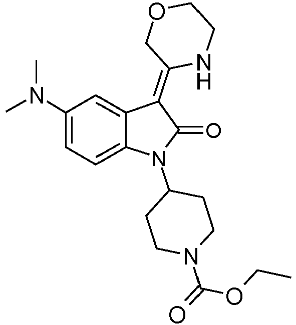 | 1053 | 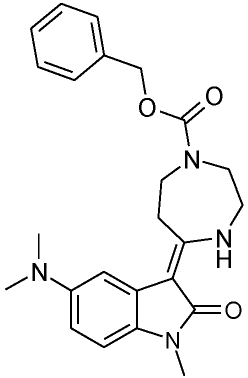 |
| 1054 | 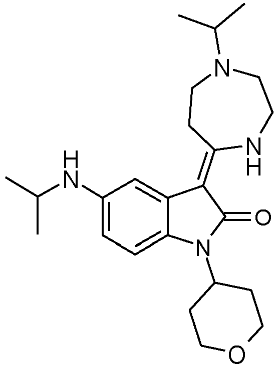 | 1055 | 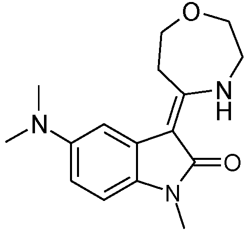 |
| 1056 | 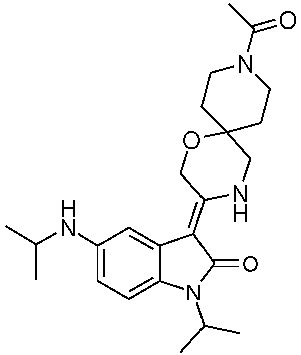 | 1057 | 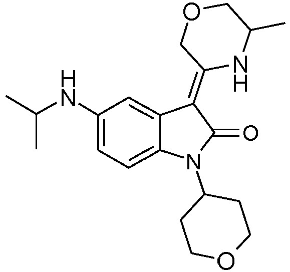 |
| 1058 | 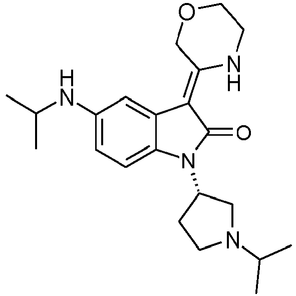 | 1059 | 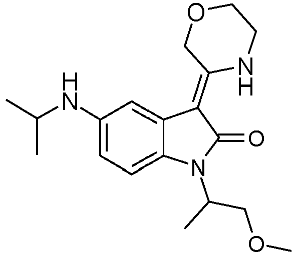 |
| 1060 | 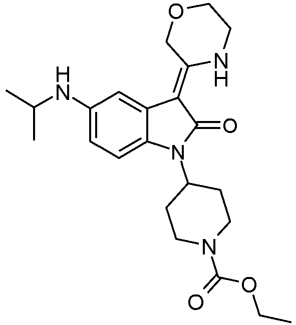 | 1061 | 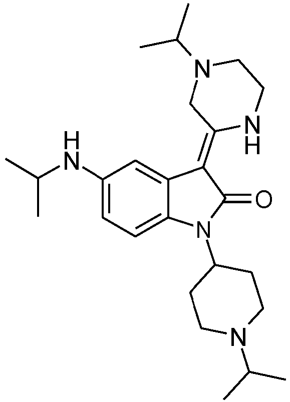 |
| 1062 | 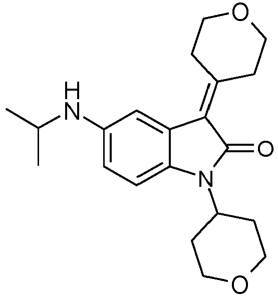 | 1063 | 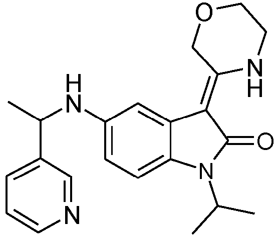 |
| 1064 | 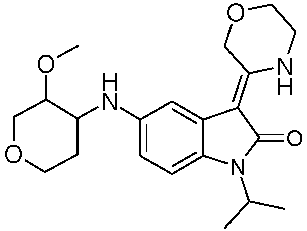 | 1065 | 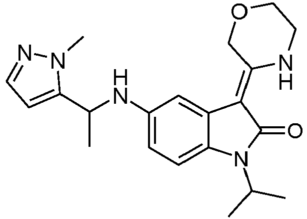 |
| 1066 | 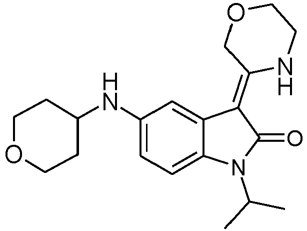 | 1067 | 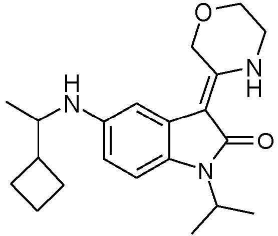 |
| 1068 | 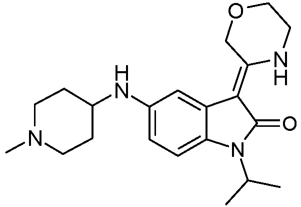 | 1069 | 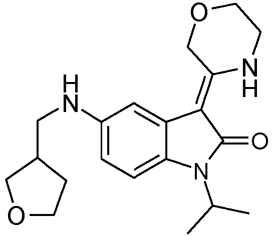 |
| 1070 | 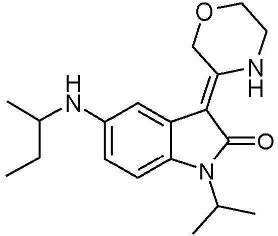 | 1071 | 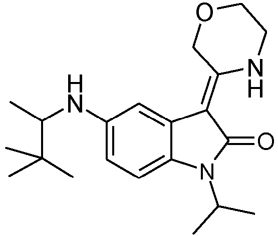 |
| 1072 | 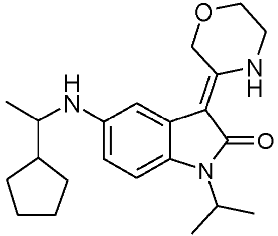 | 1073 | 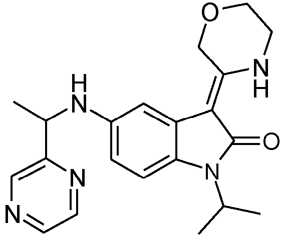 |
| 1074 | 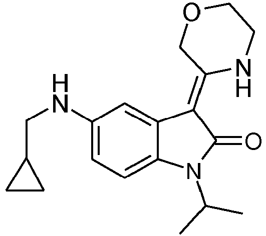 | 1075 | 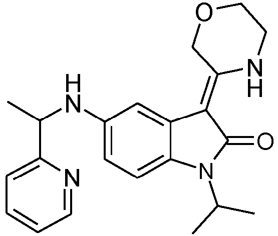 |
| 1076 | 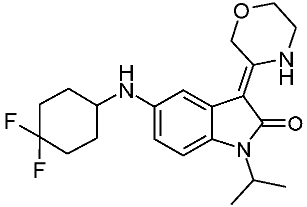 | 1077 | 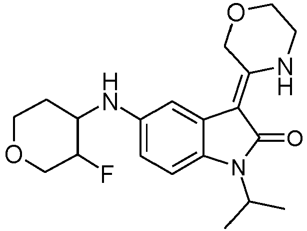 |
| 1078 | 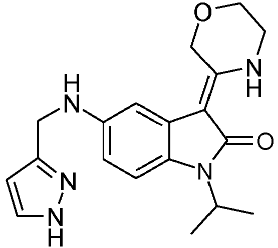 | 1079 | 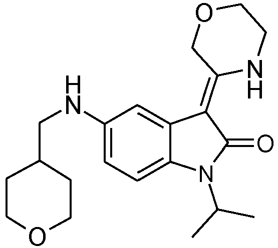 |
| 1080 | 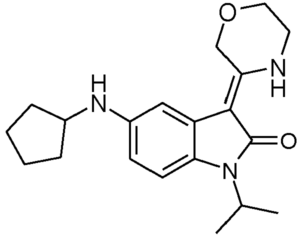 | 1081 | 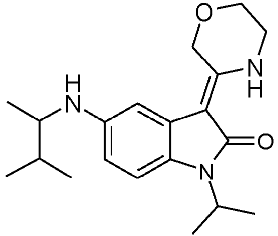 |
| 1082 | 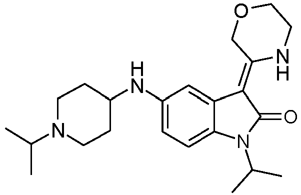 | 1083 | 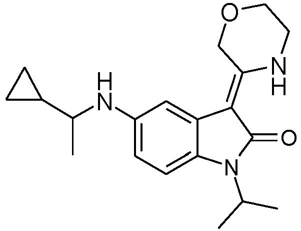 |
| 1084 | 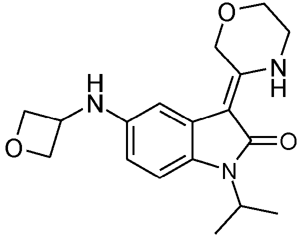 | 1085 | 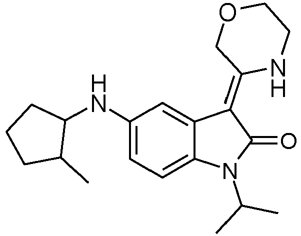 |
| 1086 | 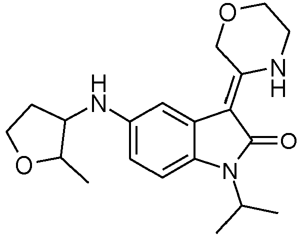 | 1087 | 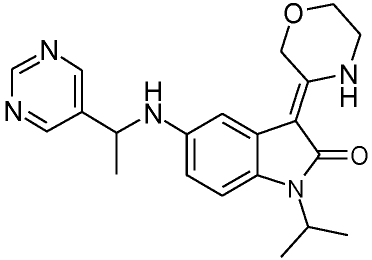 |
| 1088 | 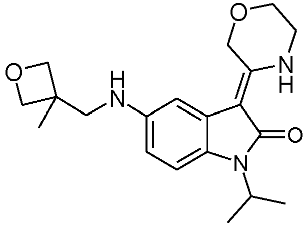 | 1089 | 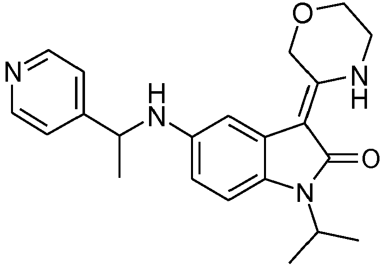 |
| 1090 | 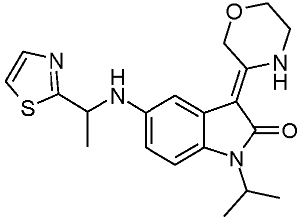 | 1091 | 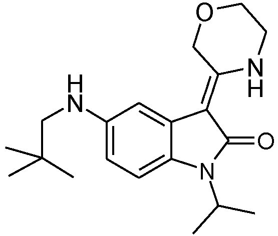 |
| 1092 | 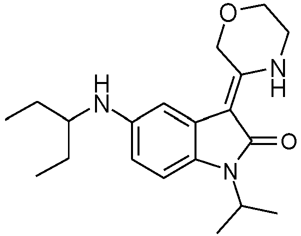 | 1093 | 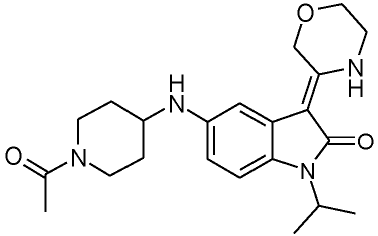 |
| 1094 | 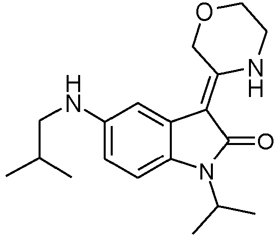 | 1095 | 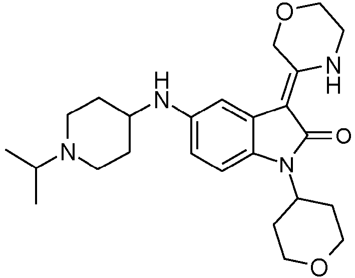 |
| 1096 | 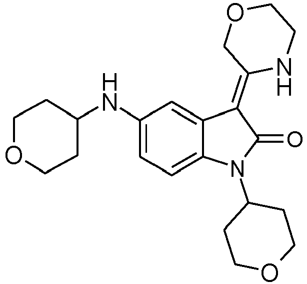 | 1097 | 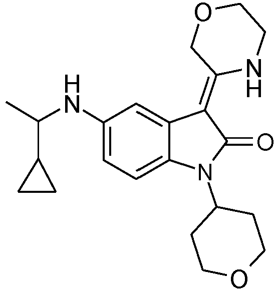 |
| 1098 | 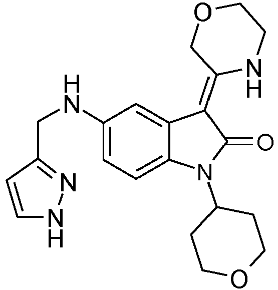 | 1099 | 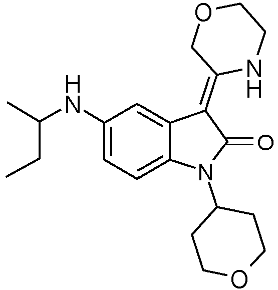 |
| 1100 | 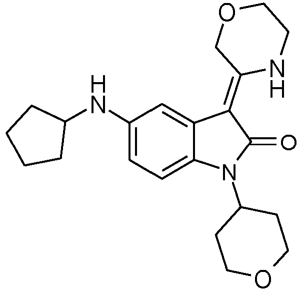 | 1101 | 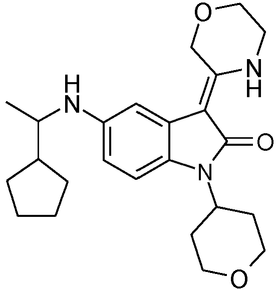 |
| 1102 | 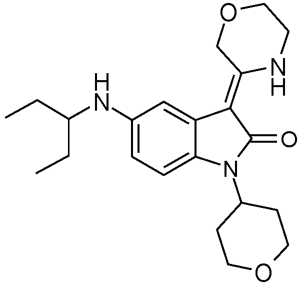 | 1103 | 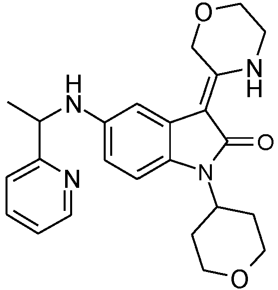 |
| 1104 | 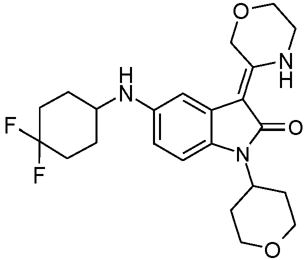 | 1105 | 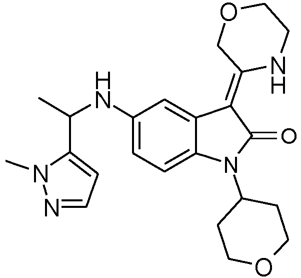 |
| 1106 | 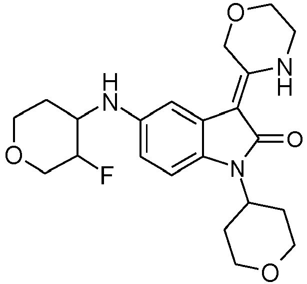 | 1107 | 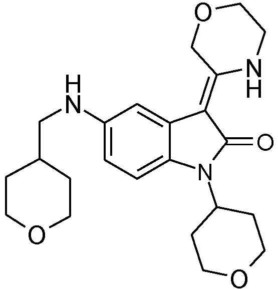 |
| 1108 | 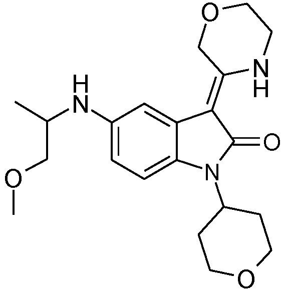 | 1109 | 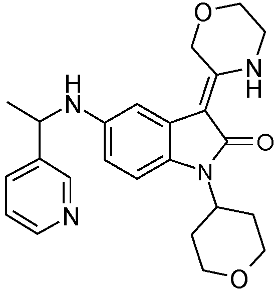 |
| 1110 | 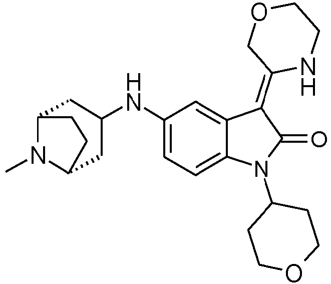 | 1111 | 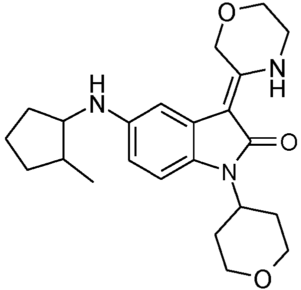 |
| 1112 | 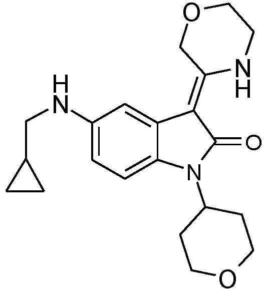 | 1113 | 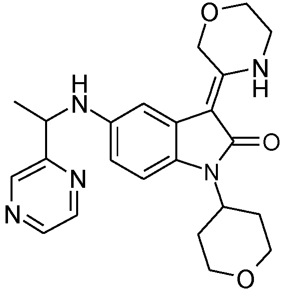 |
| 1114 | 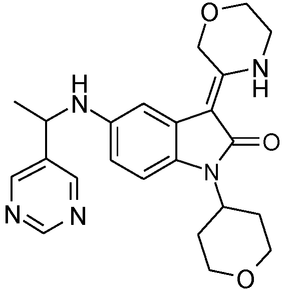 | 1115 | 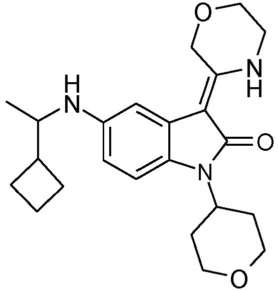 |
| 1116 | 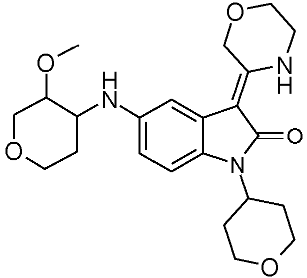 | 1117 | 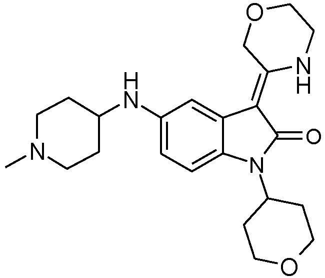 |
| 1118 | 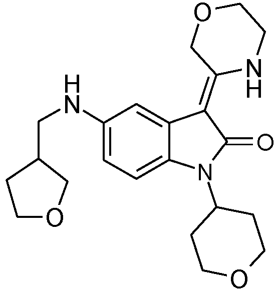 | 1119 | 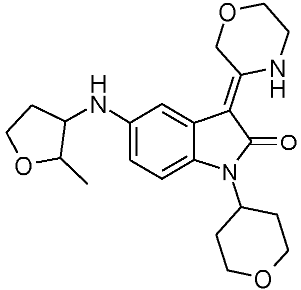 |
| 1120 | 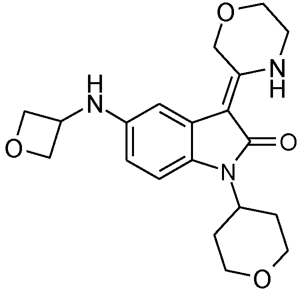 | 1121 | 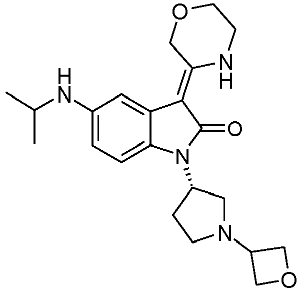 |
| 1122 | 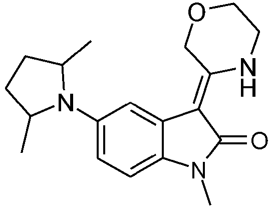 | 1123 | 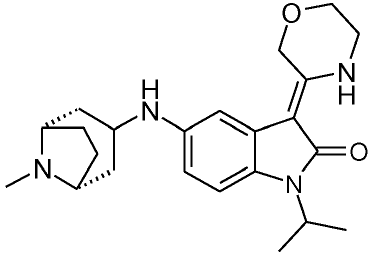 |
| 1124 | 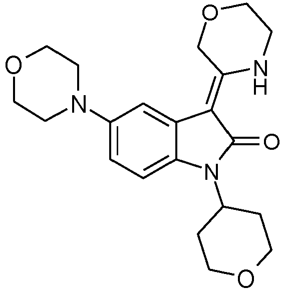 | 1125 | 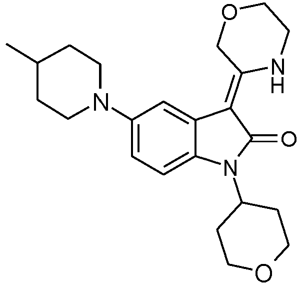 |
| 1126 | 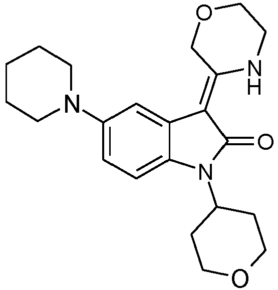 | 1127 | 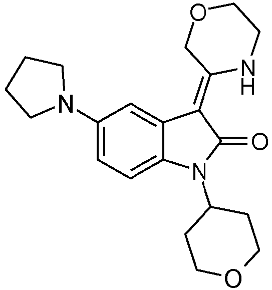 |
| 1128 | 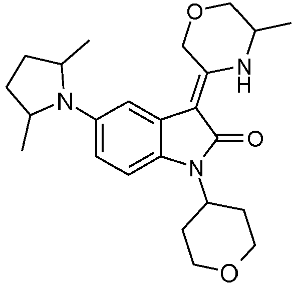 | 1129 | 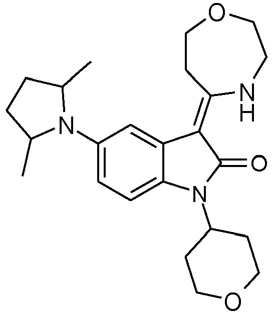 |
| 1130 | 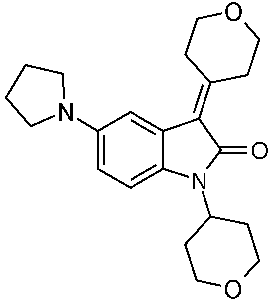 | 1131 | 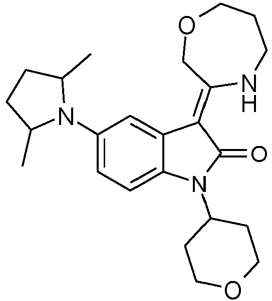 |
| 1132 | 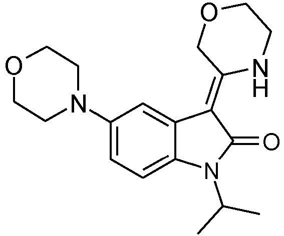 | 1133 | 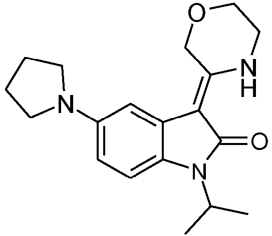 |
| 1134 | 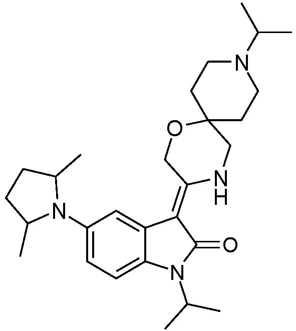 | 1135 | 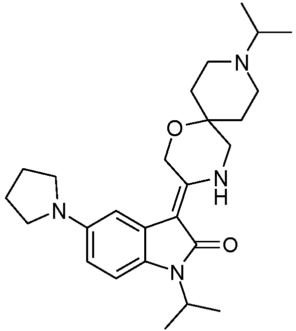 |
| 1136 | 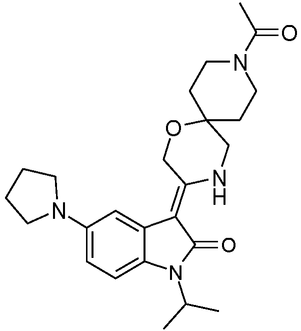 | 1137 | 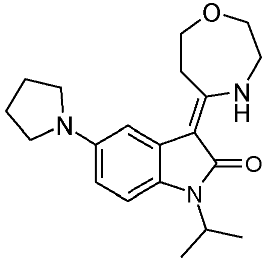 |
| 1138 | 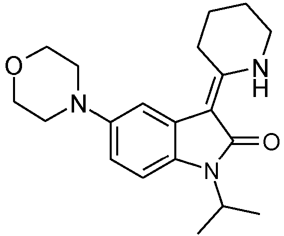 | 1139 | 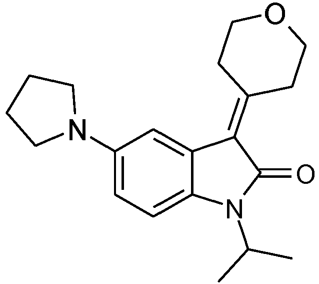 |
| 1140 | 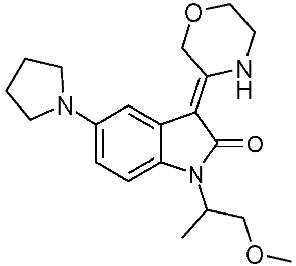 | 1141 | 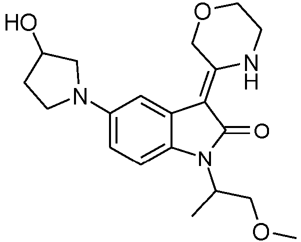 |
| 1142 | 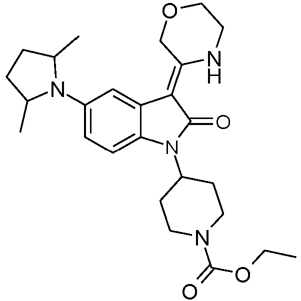 | 1143 | 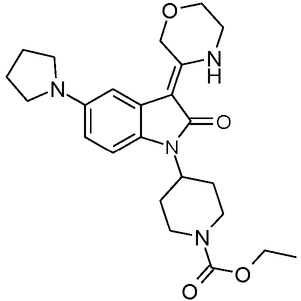 |
| 1144 | 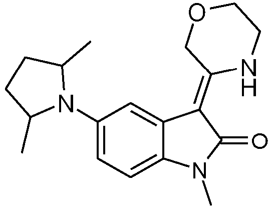 | 1145 | 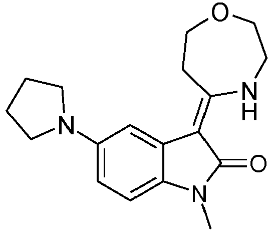 |
| 1146 | 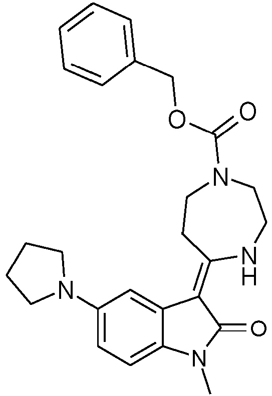 | 1147 | 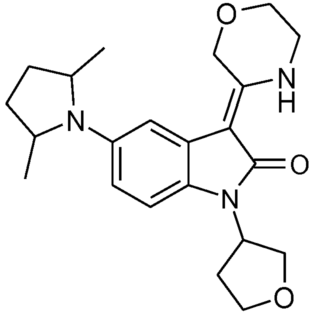 |
| 1148 | 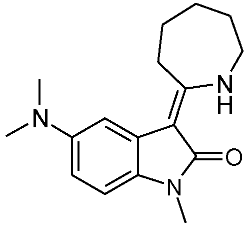 | 1149 | 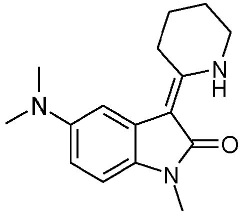 |
| 1150 | 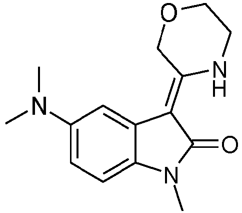 | 1151 | 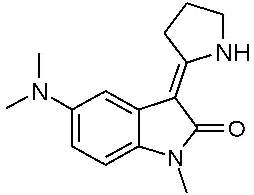 |
| 1152 | 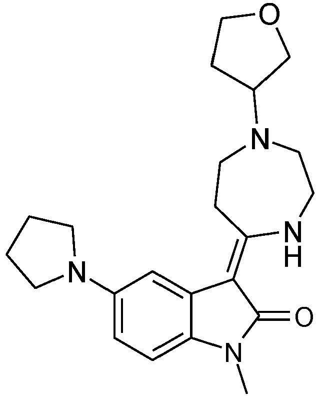 | 1153 | 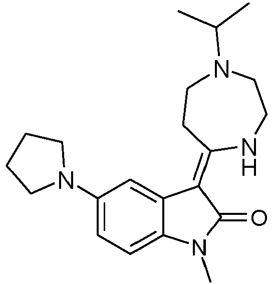 |
| 1154 | 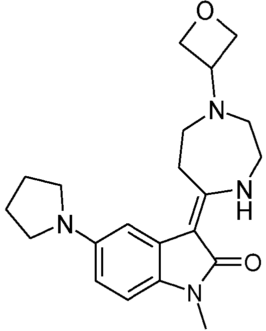 | 1155 | 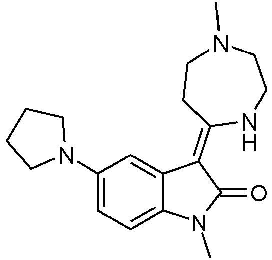 |
| 1156 | 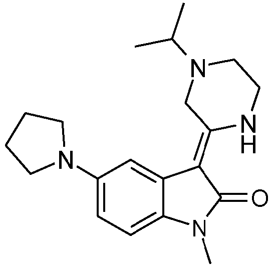 | 1157 | 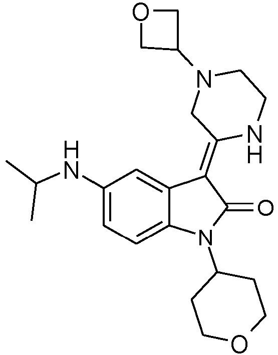 |
| 1158 | 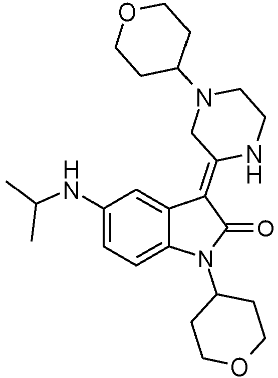 | 1159 | 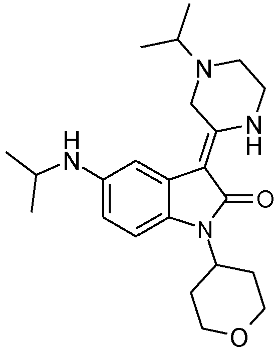 |
| 1160 | 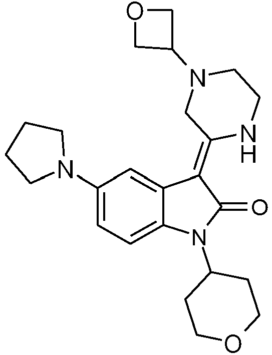 | 1161 | 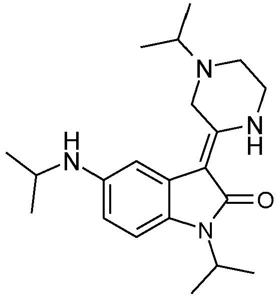 |
| 1162 | 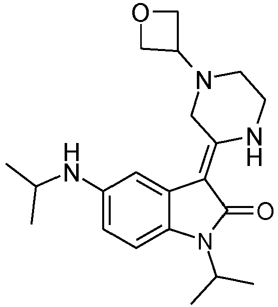 | 1163 | 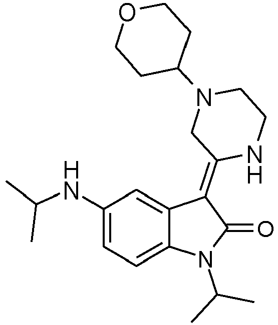 |
| 1164 | 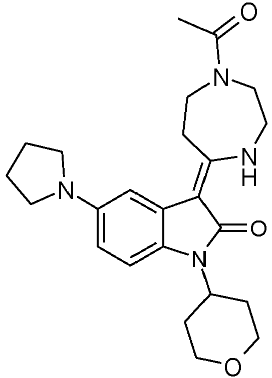 | 1165 | 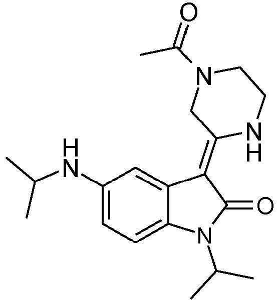 |
| 1166 | 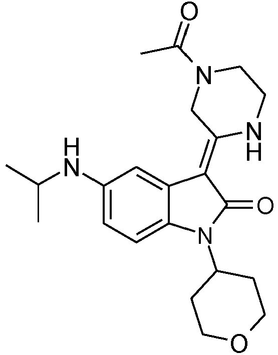 | 1167 | 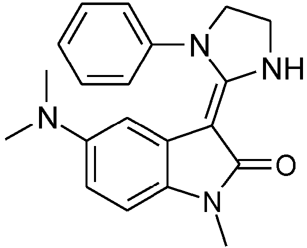 |
| 1168 | 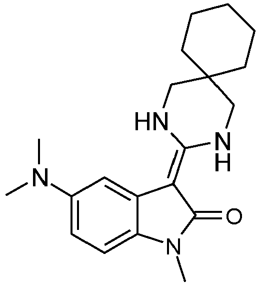 | 1169 | 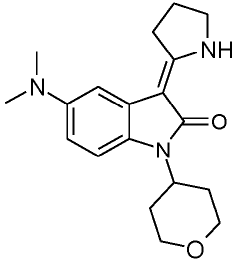 |
| 1170 | 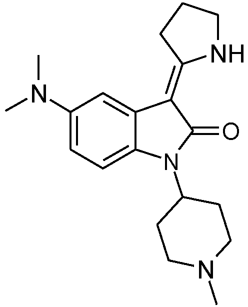 | 1171 | 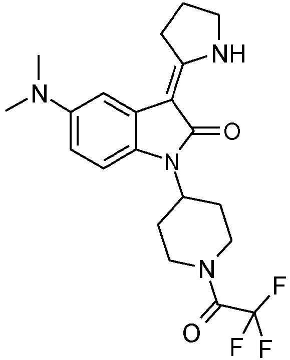 |
| 1172 | 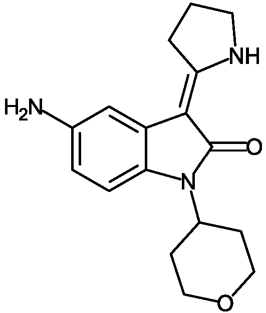 | 1173 | 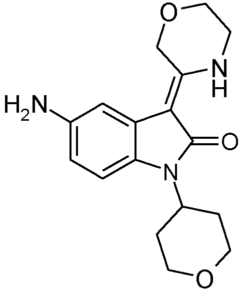 |
| 1174 | 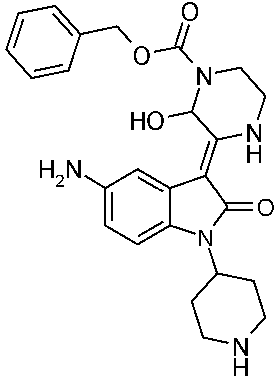 | 1175 | 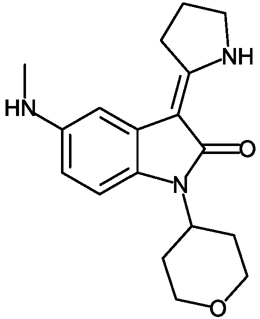 |
| 1176 | 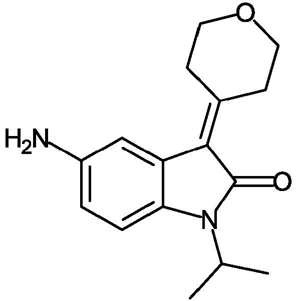 | 1177 | 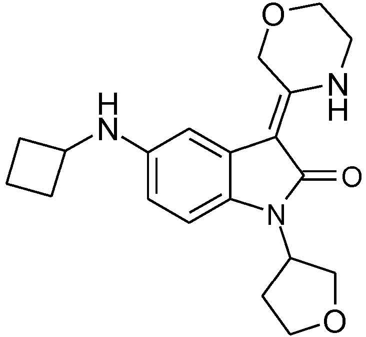 |
| 1178 | 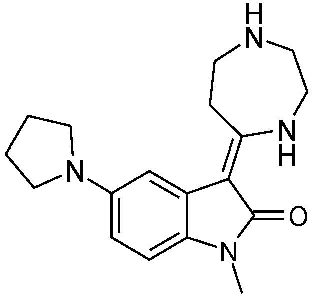 | | |

12. Medikament, umfassend eine Verbindung oder deren Salz nach einem der Ansprüche 1 bis 11.

13. Verfahren zur Herstellung der Verbindung nach Anspruch 1, umfassend
(a) Umsetzen von (2-Fluor-5-nitro-phenyl)-essigsäure (Verbindung II) 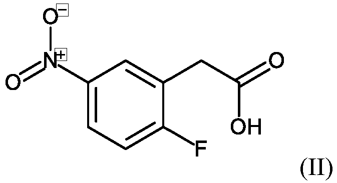 mit einem Amin R₁-NH₂ unter Verwendung eines geeigneten Lösungsmittels unter Bildung eines 5-Nitro-2,3-dihydro-1H-indol-2-ons (Verbindung III) 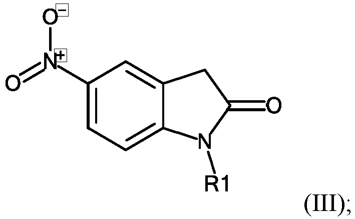
(b) Kondensieren des 5-Nitro-2,3-dihydro-1H-indol-2-ons (Verbindung III) bei erhöhter Temperatur in einer Mikrowelle, entweder ohne oder in einem geeigneten Lösungsmittel wie Peridin, mit einem Elektrophil, das geeignet ist, ein 5-Nitro-3-yliden-2,3-dihydro-1H-indol-2-on (Verbindung IV) zu ergeben 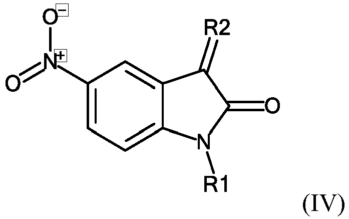
(c) Reduzieren des 5-Nitro-3-yliden-2,3-dihydro-1H-indol-2-ons (Verbindung IV) zu einem aminosubstituierten Indol-2-on (Verbindung V) 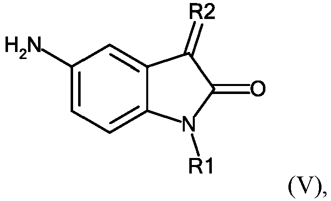
(d1) entweder Umsetzen der Verbindung der Formel (V) mit einem Aldehyd oder Keton und einem geeigneten Reduktionsmittel, um eine Verbindung der Formel (I) zu erhalten, oder
(d2)Umsetzen eines Amins der Formel (V) in einer Substitutionsreaktion mit Elektrophilen, die geeignet sind, eine Abgangsgruppe zu tragen, in Gegenwart einer geeigneten Base.

14. Pharmazeutische Zusammensetzung zur Verwendung in der Behandlung oder Vorbeugung eines neurologischen oder neurodegenerativen oder psychiatrischen Zustands bei einem Menschen, umfassend eine Verbindung nach einem der Ansprüche 1 bis 11 oder deren pharmazeutisch akzeptables bzw. annehmbares Salz und einen pharmazeutisch akzeptablen bzw. annehmbaren Träger.

15. Pharmazeutische Zusammensetzung nach Anspruch 14, umfassend eine therapeutisch wirksame Menge von 0,1 bis 2000 mg der Verbindung nach einem der Ansprüche 1 bis 11 oder deren pharmazeutisch akzeptables bzw. annehmbares Salz.

## Revendications

1. Composé ayant la formule (I) ou sel ou hydrate ou solvate de celui-ci 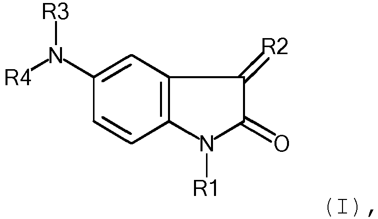 dans lequel :
**R1** est C₁₋₄-alkyle non substitué ou substitué par MeO ; tétrahydropyranyle, tétrahydrofuranyle, oxétanyle, dioxépanyle, pyrrolidinyle ou pipéridinyle ; ou pyrrolidinyle ou pipéridinyle avec l'azote substitué par méthyle, isopropyle, oxétanyle, éthoxycarbonyle, acétyle ou trifluoroacétyle ;
**R2** est un cycle non substitué ou substitué de 5, 6 ou 7 chaînons contenant 1 ou 2 hétéroatomes sélectionnés parmi O ou N ou un groupe hétérocyclyle spirocyclique non substitué ou substitué contenant 1 à 3 hétéroatomes sélectionnés parmi N ou O consistant en 4 à 11 atomes de cycle, le cycle ou groupe hétérocyclyle spirocyclique étant lié dans la formule (I) par une double liaison C=C, et
cycle ou groupe hétérocyclyle spirocyclique dans lequel un atome N peut être substitué par méthyle, isopropyle, acétyle, benzyloxycarbonyle, phényle, oxétanyle ou tétrahydropyranyle, et
dans lequel un ou plusieurs atomes C peuvent être substitués par -méthyle ou -OH ;
**R3** ou **R4** sont indépendamment l'un de l'autre hydrogène ; C₁₋₆-alkyle, non substitué ou substitué par un ou plusieurs F, méthoxy, C₃₋₈-cycloalkyle non substitué ou substitué par un ou plusieurs F ; aryle ; hétéroaryle consistant en 5 à 6 atomes de cycle ; ou hétérocyclyle sélectionné dans le groupe consistant en oxétanyle, tétrahydropyranyle et pyrrolidinyle, ledit hétérocyclyle étant non substitué ou substitué par C₁₋₆-alkyle, acétyle, tétrahydrofuranyle, oxétanyle ou hydroxyéthylacétyle ;
**ou R3** et **R4** conjointement avec l'atome N attaché forment un cycle hétérocyclyle sélectionné dans le groupe consistant en morpholinyle et pyrrolidinyle tous deux non substitués ou substitués par C₁₋₆-alkyle, F, ou hydroxyle.

2. Composé selon la revendication 1 ou sel de celui-ci, dans lequel **R1** est 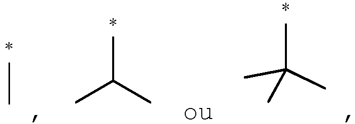 non substitué ou substitué par MeO ; 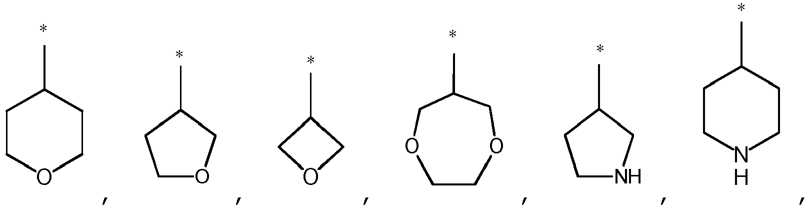 non substitué ou l'azote substitué par

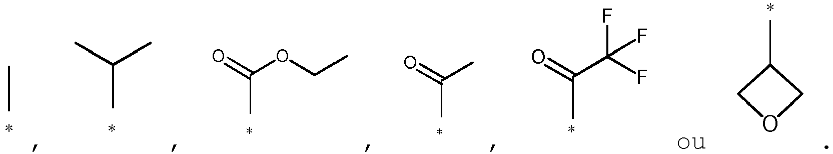

3. Composé selon la revendication 1 ou sel de celui-ci dans lequel **R1** est

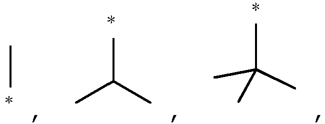

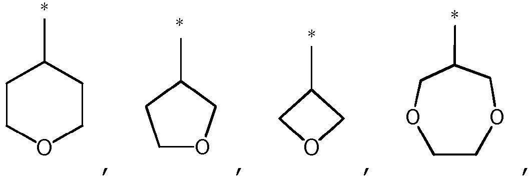

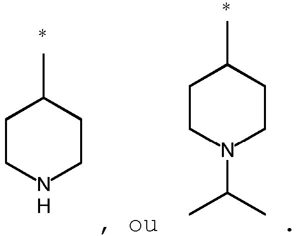

4. Composé selon la revendication 2 ou sel de celui-ci, dans lequel **R1** est tétrahydropyranyle ou dioxépanyle.

5. Composé selon la revendication 1 ou sel de celui-ci dans lequel **R2** est un cycle à 5, 6 ou 7 chaînons contenant 1 ou 2 hétéroatomes sélectionnés parmi O ou N liés dans la formule (1) par une double liaison C=C,
dans laquelle un ou les deux atomes N peuvent être substitués par méthyle, isopropyle, acétyle, benzyloxycarbonyle, phényle, oxétanyle ou tétrahydropyranyle, et
dans laquelle un ou plusieurs atomes C peuvent être substitués par -méthyle ou -OH.

6. Composé selon la revendication 1 ou sel de celui-ci, dans lequel l'élément structurel 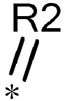 est

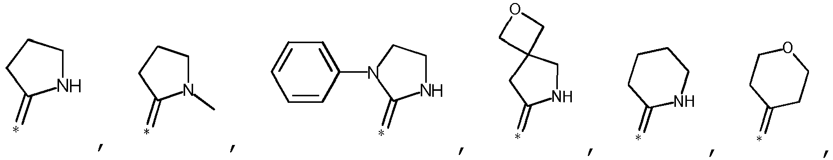

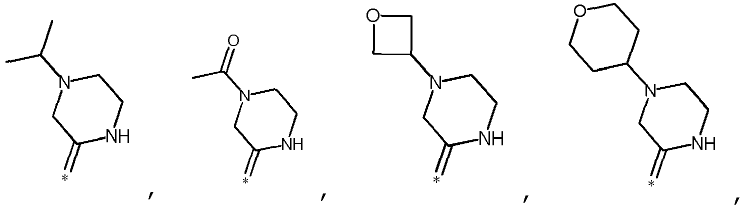

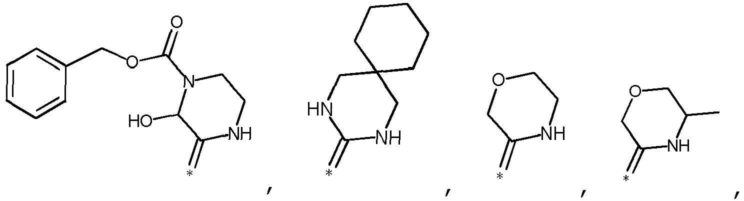

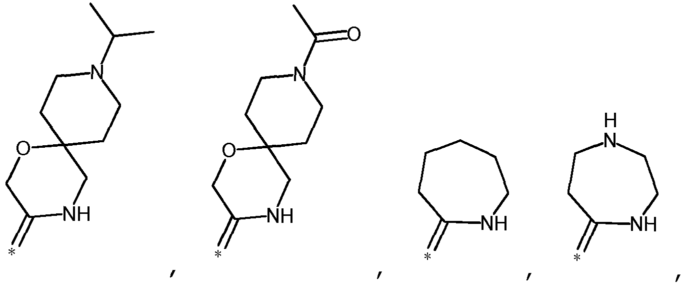

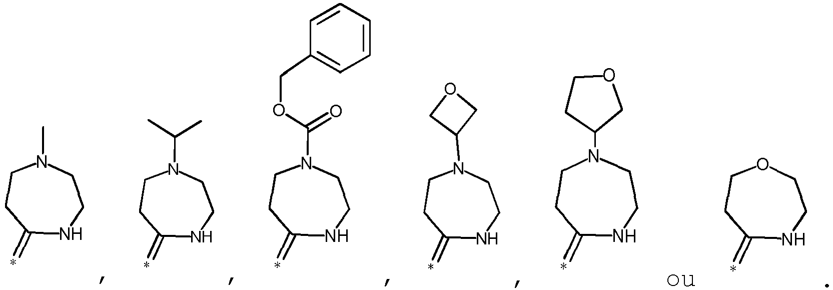

7. Composé selon la revendication 1 ou sel de celui-ci dans lequel le groupe amino contenant **R3** et **R4** dans la formule (I) est 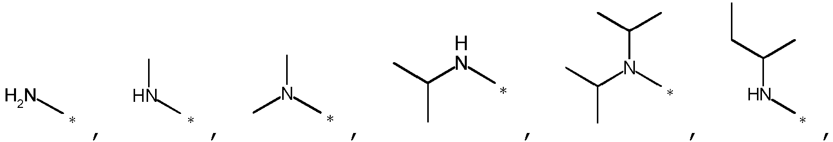 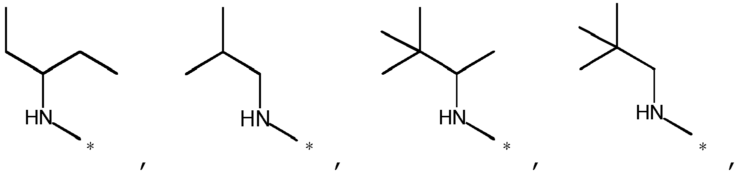 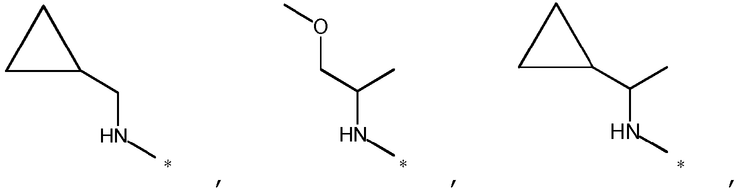 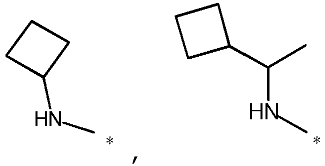 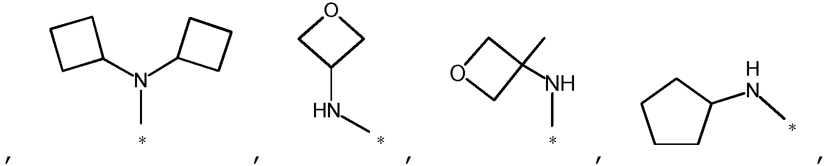 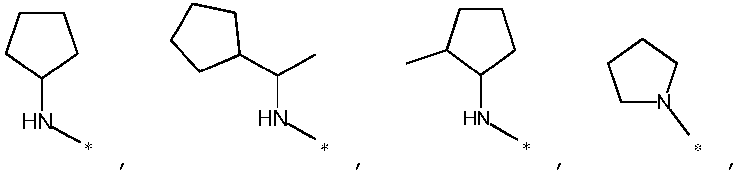 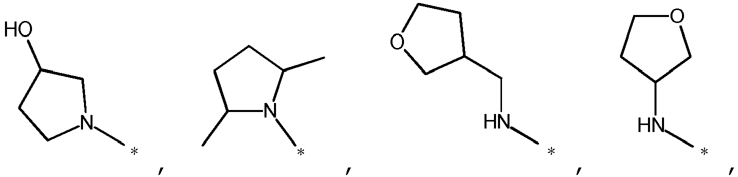 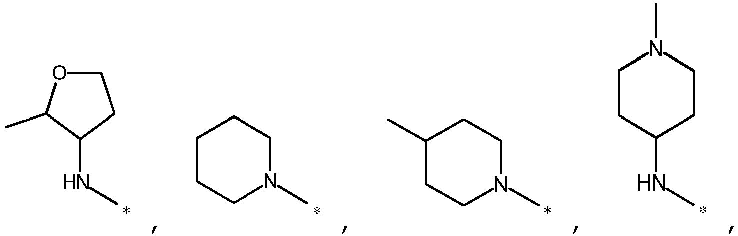 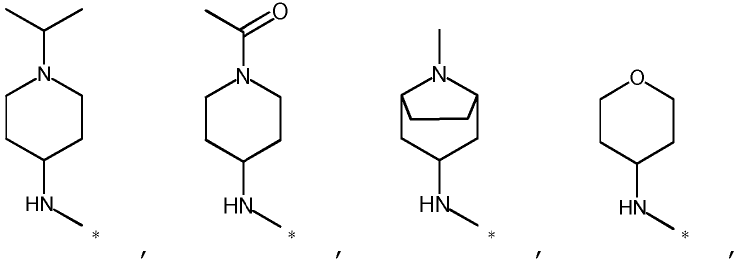 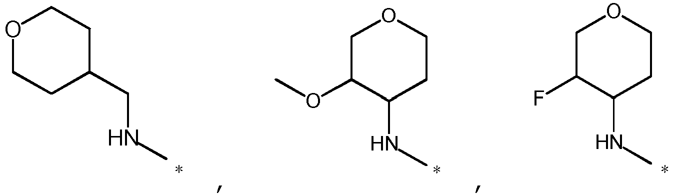 ou

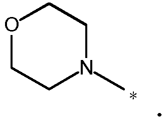

8. Composé selon la revendication 1 ou sel de celui-ci, dans lequel le groupe amino contenant **R3** et **R4** dans la formule (I) englobe hydrogène en tant que **R3** tandis que **R4** est iso-propyle, cyclobutyle ou cyclopentyle, non substitué ou substitué par fluor (F).

9. Composé selon la revendication 1 ou sel de celui-ci, dans lequel le groupe amino contenant **R3** et **R4** dans la formule (I) est 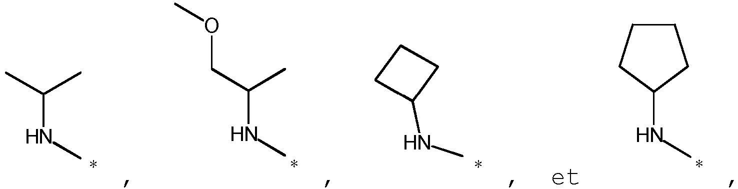 non substitué ou substitué par F.

10. Sel d'un composé de l'une quelconque des revendications 1 à 9.

11. Composé selon la revendication 1 ou sel de celui-ci dans lequel la formule (I) est la formule de l'un quelconque des composés 1 à 175 et 1001 à 1178.
| **#** | **Formule** | **#** | **Formule** |
|---|---|---|---|
| 1 | 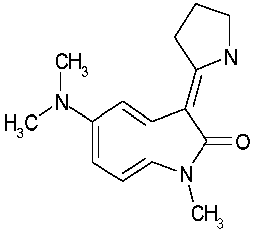 | 2 | 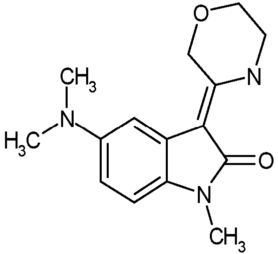 |
| 3 | 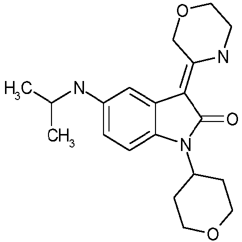 | 4 | 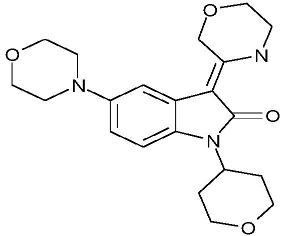 |
| 5 | 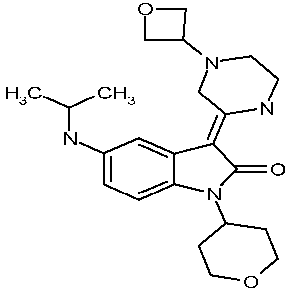 | 6 | 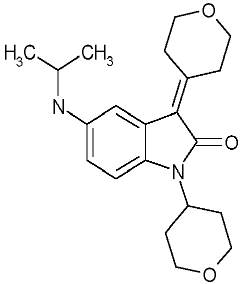 |
| 7 | 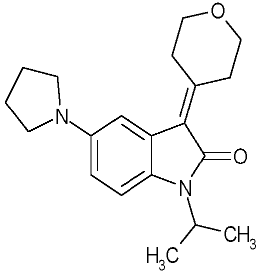 | 8 | 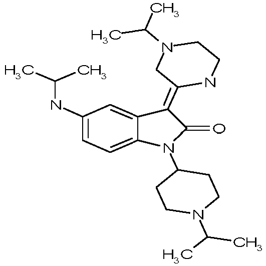 |
| 9 | 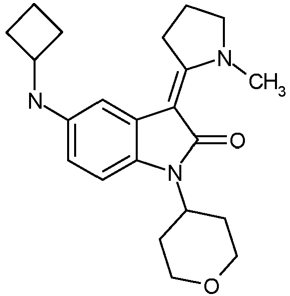 | 10 | 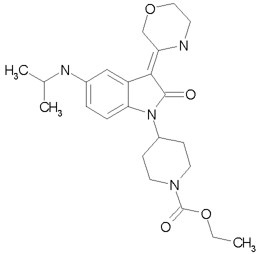 |
| 11 | 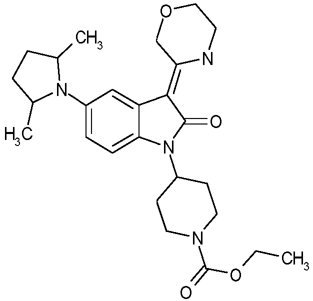 | 12 | 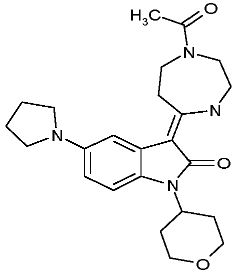 |
| 13 | 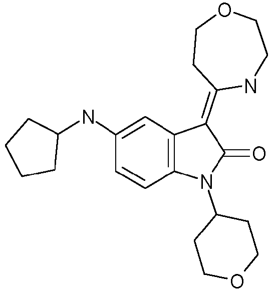 | 14 | 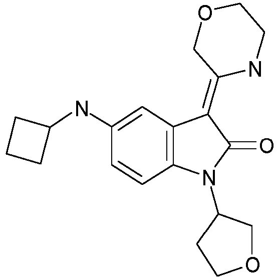 |
| 15 | 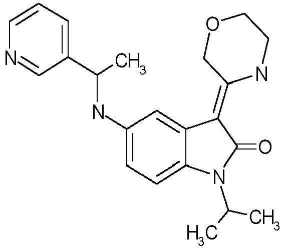 | 16 | 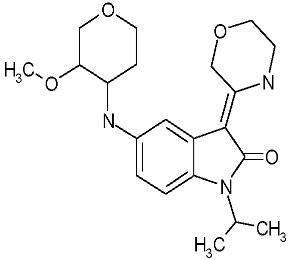 |
| 17 | 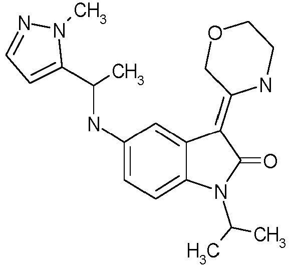 | 18 | 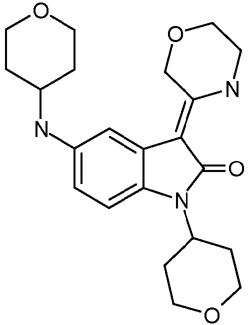 |
| 19 | 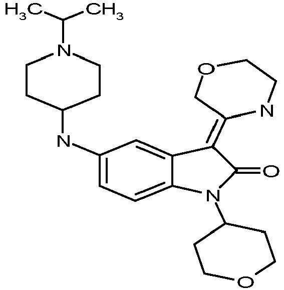 | 20 | 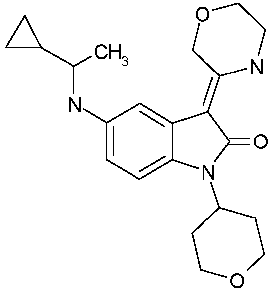 |
| 21 | 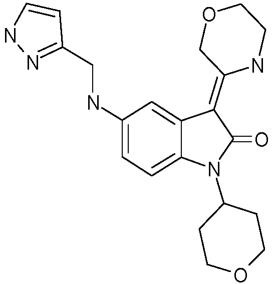 | 22 | 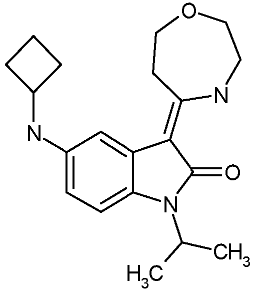 |
| 23 | 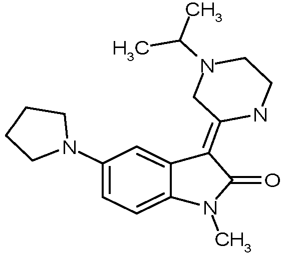 | 24 | 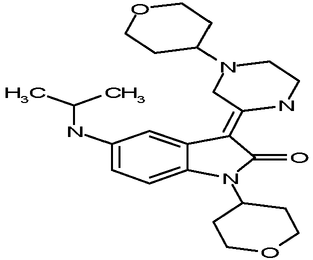 |
| 25 | 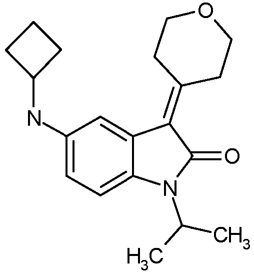 | 26 | 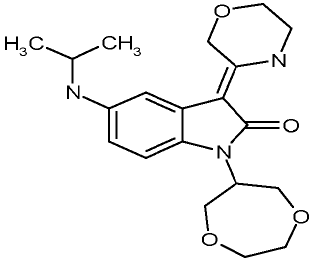 |
| 27 | 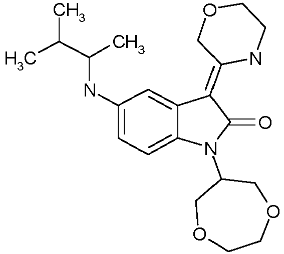 | 28 | 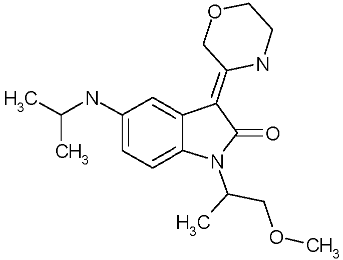 |
| 29 | 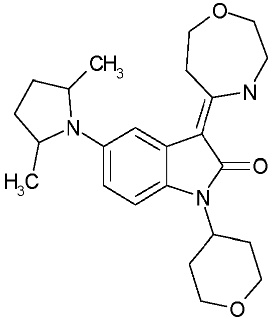 | 30 | 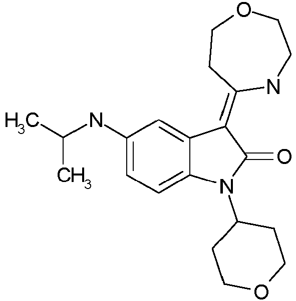 |
| 31 | 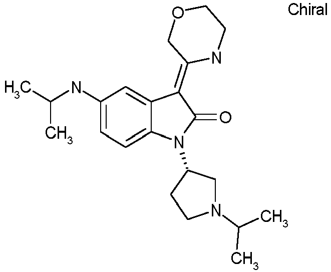 | 32 | 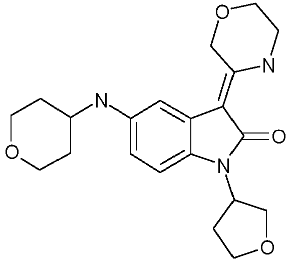 |
| 33 | 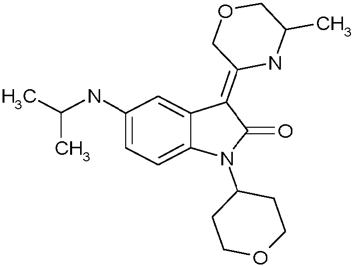 | 34 | 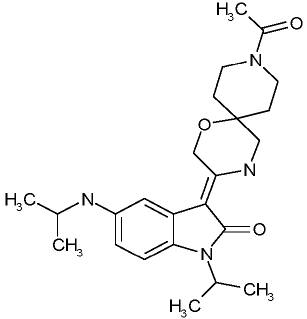 |
| 35 | 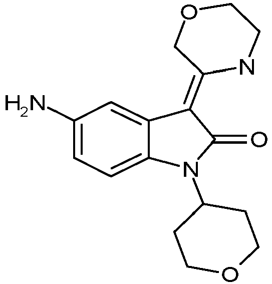 | 36 | 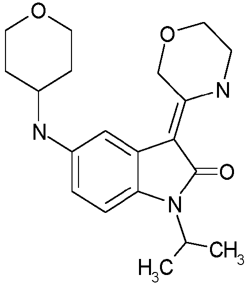 |
| 37 | 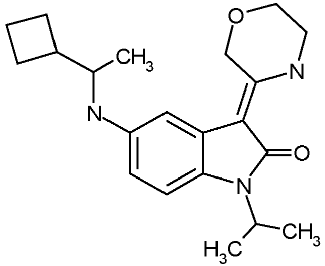 | 38 | 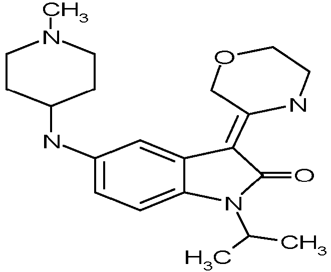 |
| 39 | 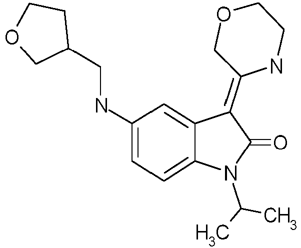 | 40 | 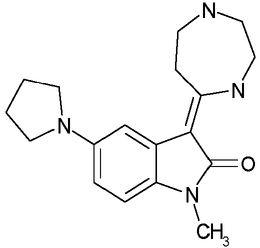 |
| 41 | 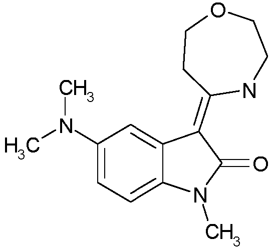 | 42 | 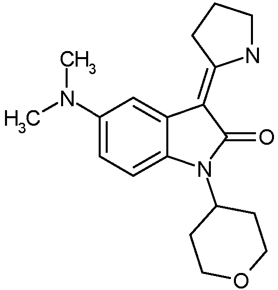 |
| 43 | 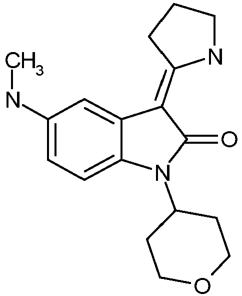 | 44 | 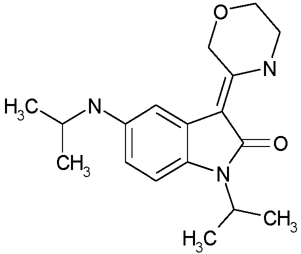 |
| 45 | 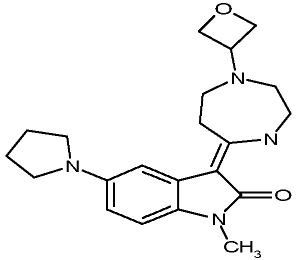 | 46 | 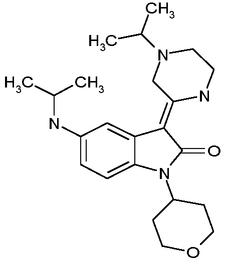 |
| 47 | 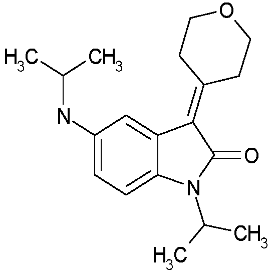 | 48 | 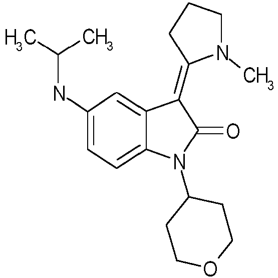 |
| 49 | 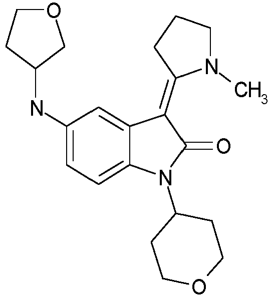 | 50 | 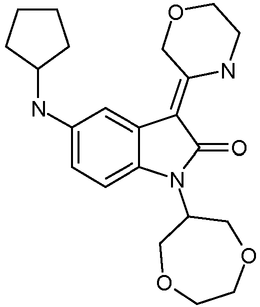 |
| 51 | 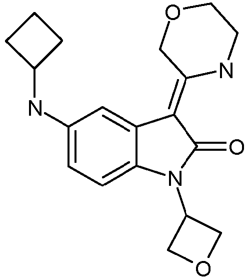 | 52 | 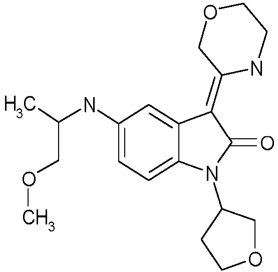 |
| 53 | 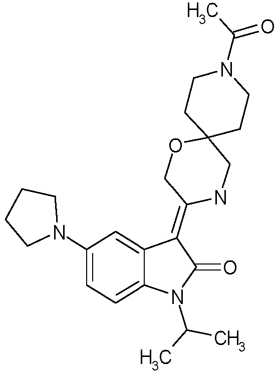 | 54 | 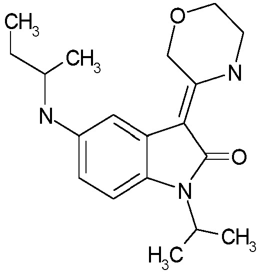 |
| 55 | 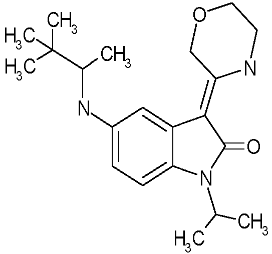 | 56 | 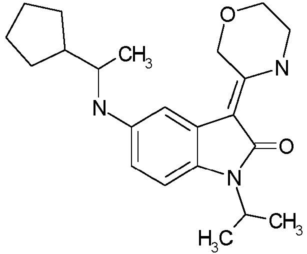 |
| 57 | 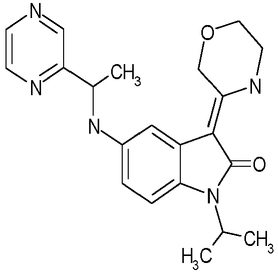 | 58 | 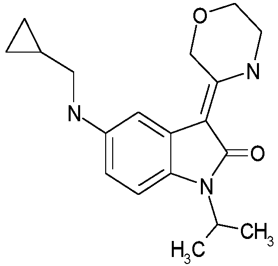 |
| 59 | 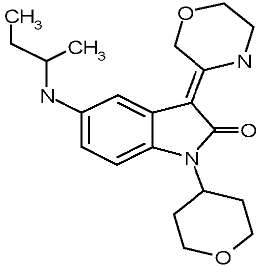 | 60 | 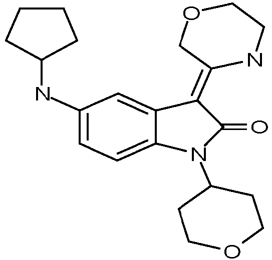 |
| 61 | 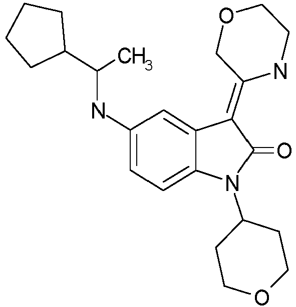 | 62 | 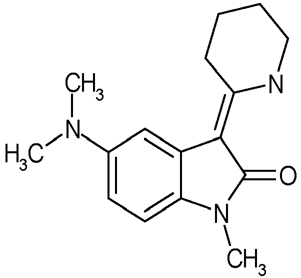 |
| 63 | 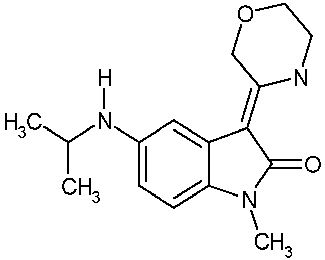 | 64 | 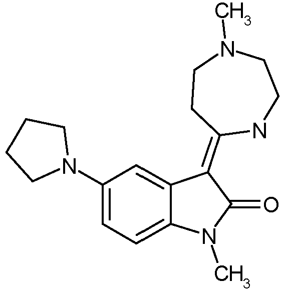 |
| 65 | 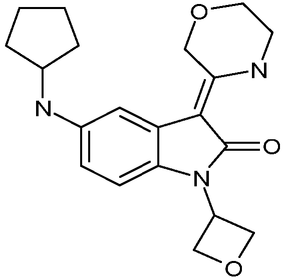 | 66 | 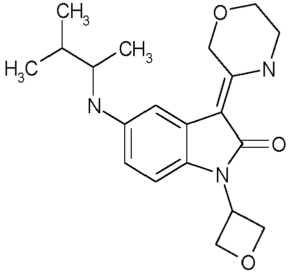 |
| 67 | 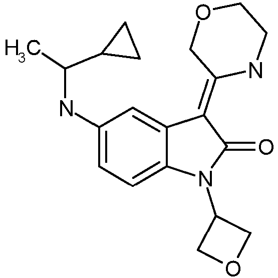 | 68 | 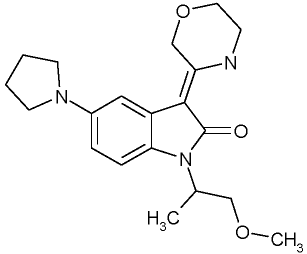 |
| 69 | 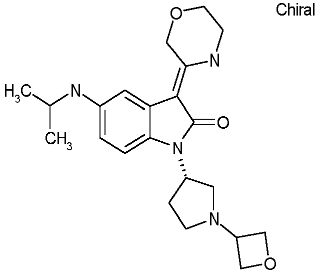 | 70 | 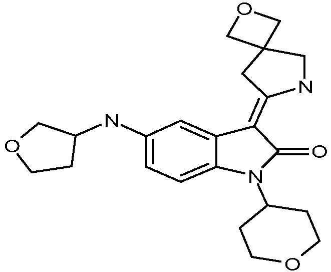 |
| 71 | 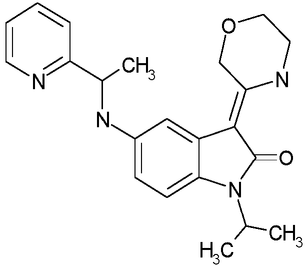 | 72 | 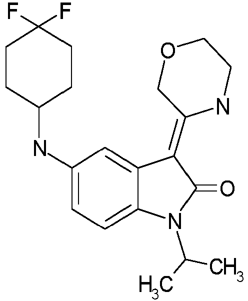 |
| 73 | 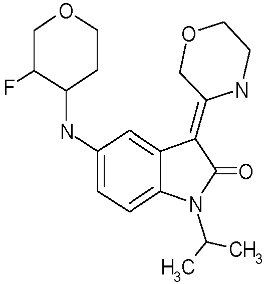 | 74 | 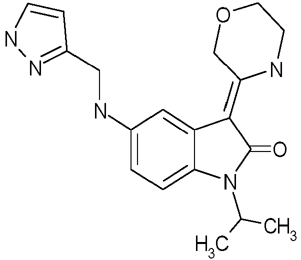 |
| 75 | 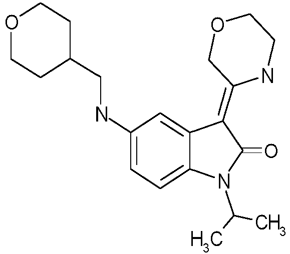 | 76 | 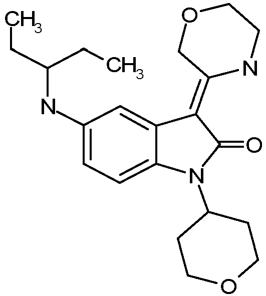 |
| 77 | 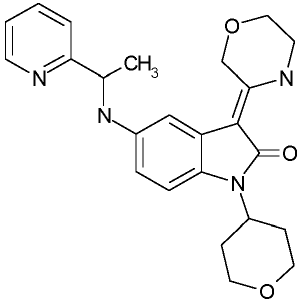 | 78 | 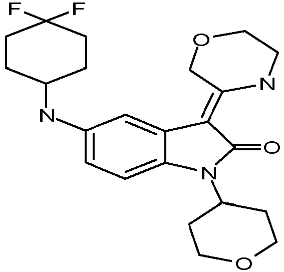 |
| 79 | 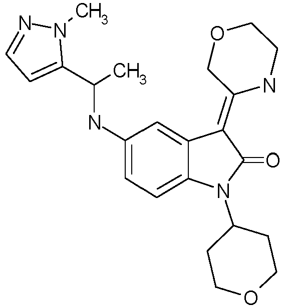 | 80 | 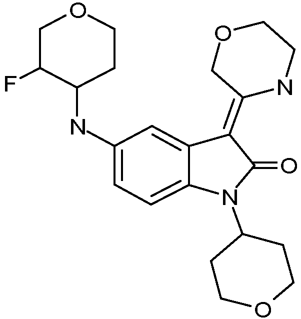 |
| 81 | 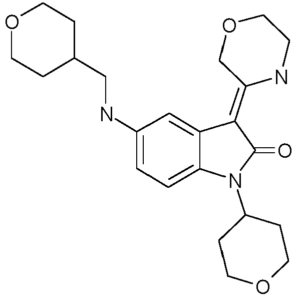 | 82 | 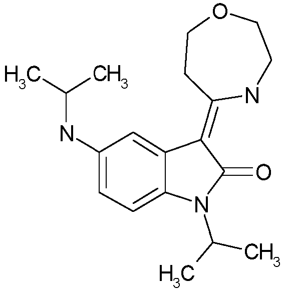 |
| 83 | 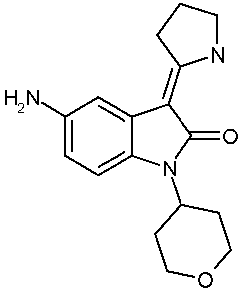 | 84 | 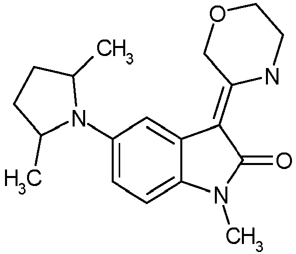 |
| 85 | 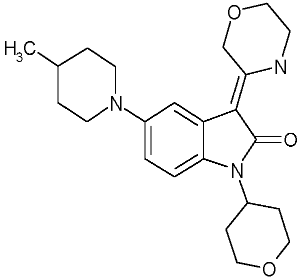 | 86 | 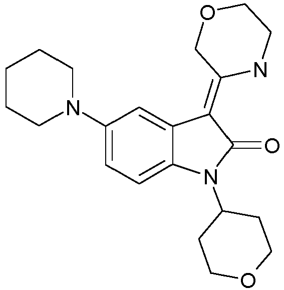 |
| 87 | 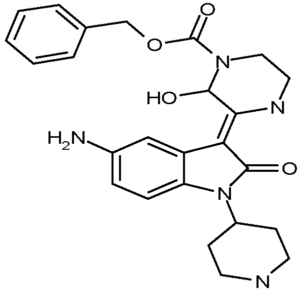 | 88 | 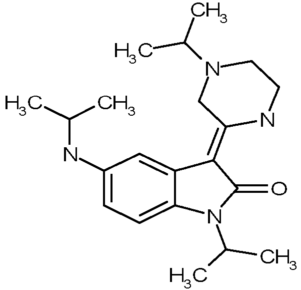 |
| 89 | 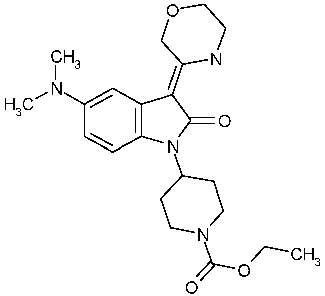 | 90 | 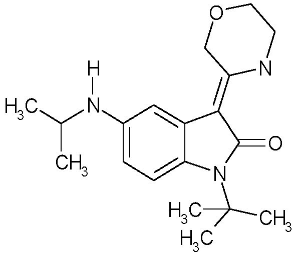 |
| 91 | 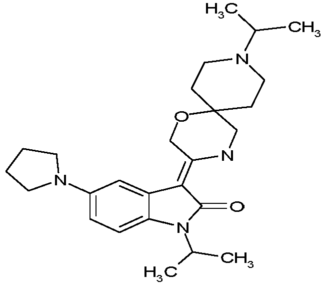 | 92 | 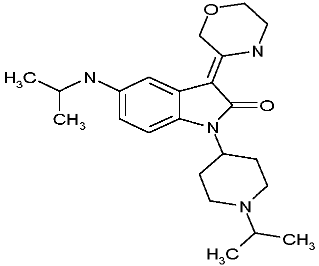 |
| 93 | 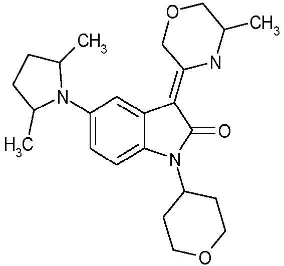 | 94 | 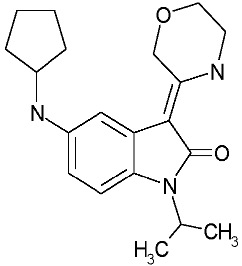 |
| 95 | 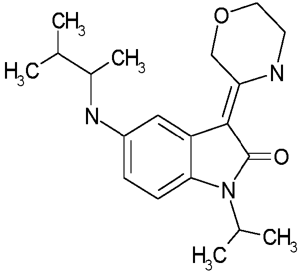 | 96 | 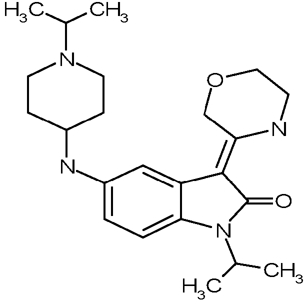 |
| 97 | 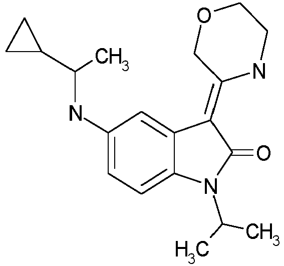 | 98 | 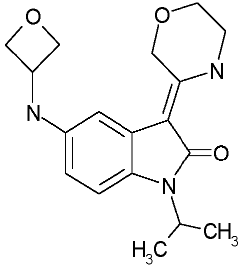 |
| 99 | 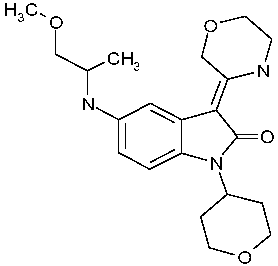 | 100 | 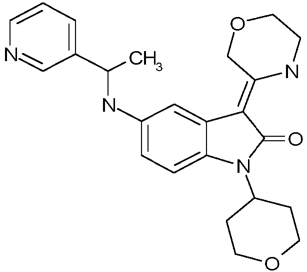 |
| 101 | 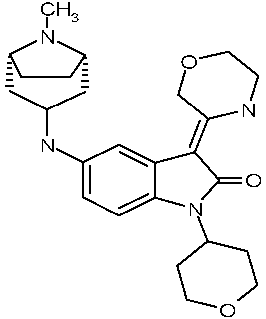 | 102 | 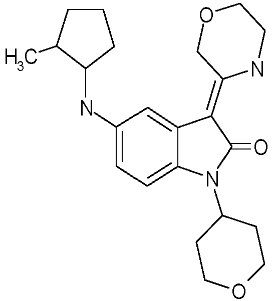 |
| 103 | 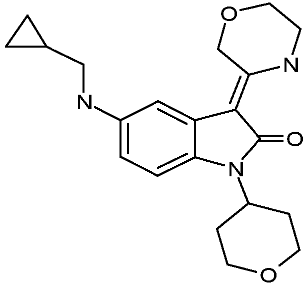 | 104 | 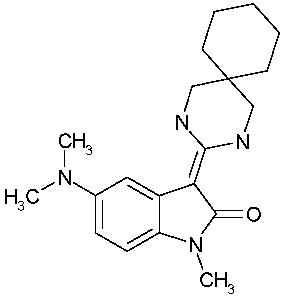 |
| 105 | 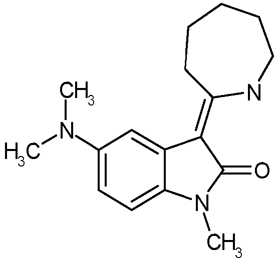 | 106 | 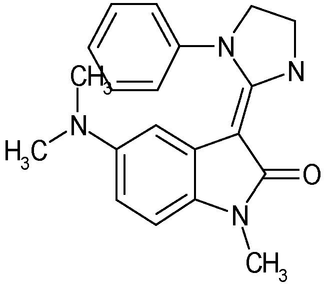 |
| 107 | 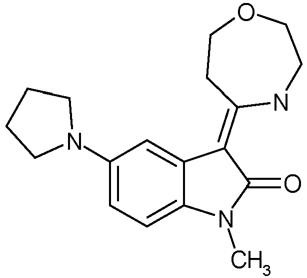 | 108 | 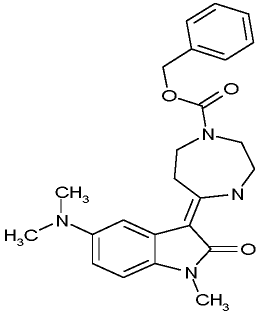 |
| 109 | 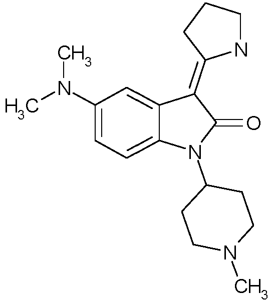 | 110 | 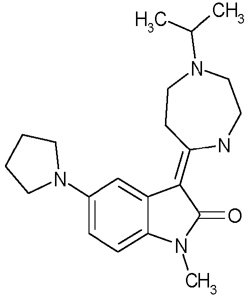 |
| 111 | 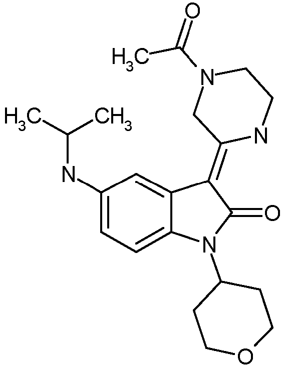 | 112 | 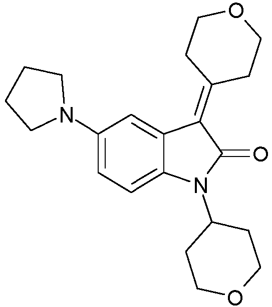 |
| 113 | 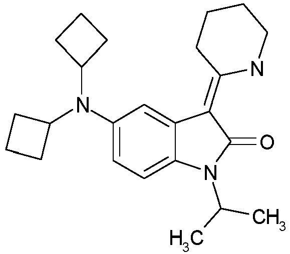 | 114 | 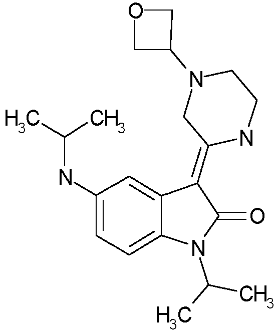 |
| 115 | 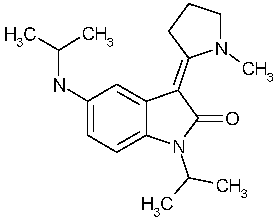 | 116 | 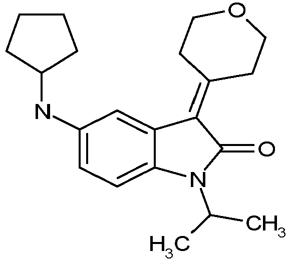 |
| 117 | 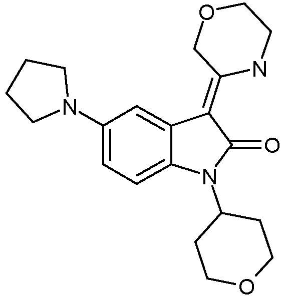 | 118 | 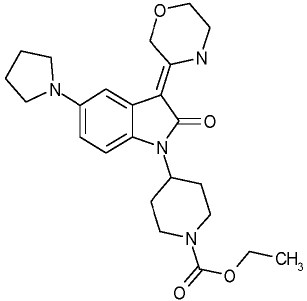 |
| 119 | 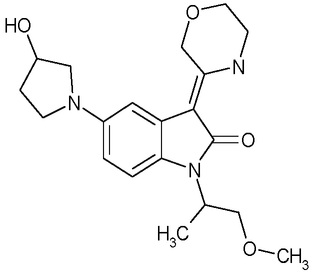 | 120 | 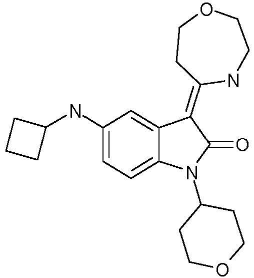 |
| 121 | 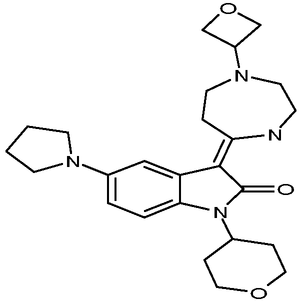 | 122 | 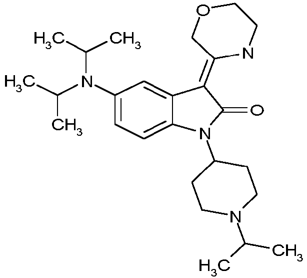 |
| 123 | 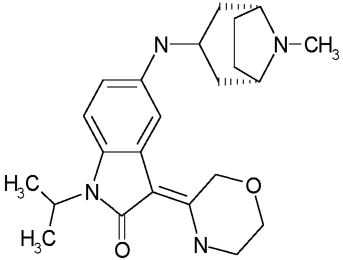 | 124 | 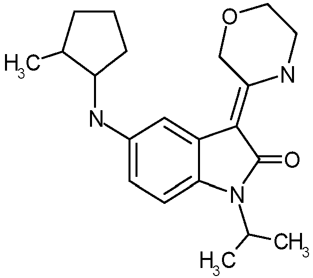 |
| 125 | 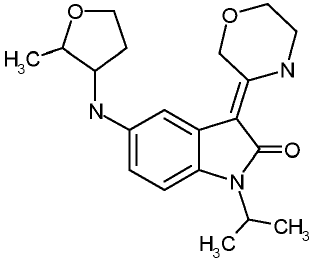 | 126 | 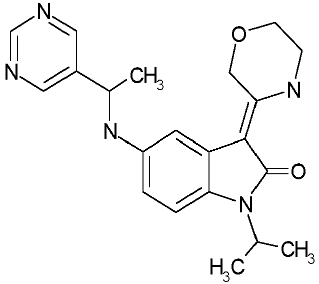 |
| 127 | 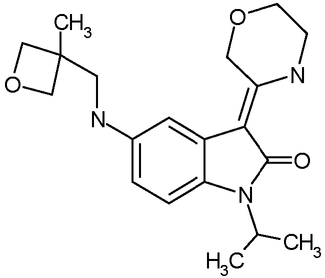 | 128 | 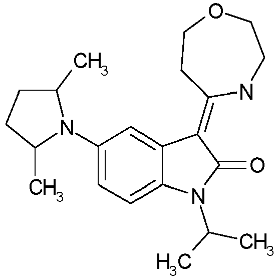 |
| 129 | 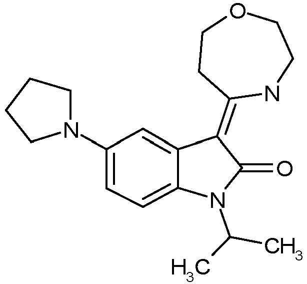 | 130 | 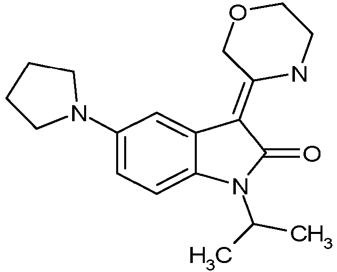 |
| 131 | 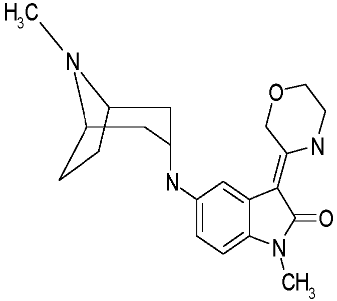 | 132 | 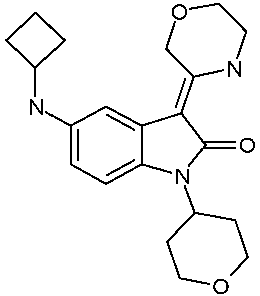 |
| 133 | 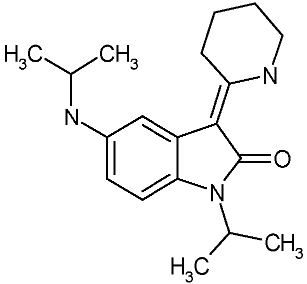 | 134 | 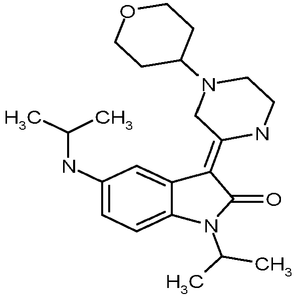 |
| 135 | 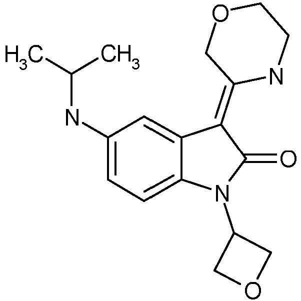 | 136 | 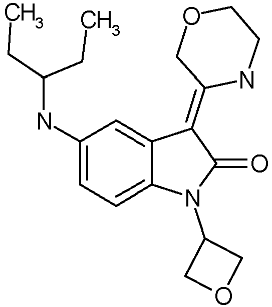 |
| 137 | 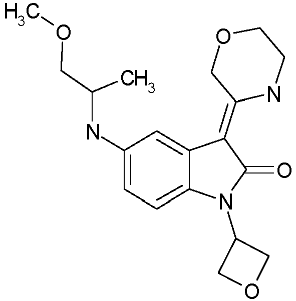 | 138 | 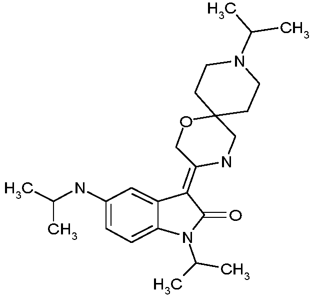 |
| 139 | 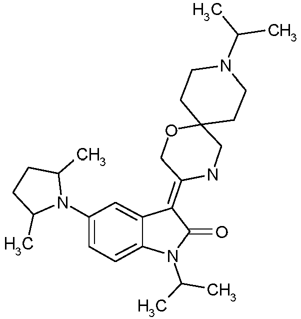 | 140 | 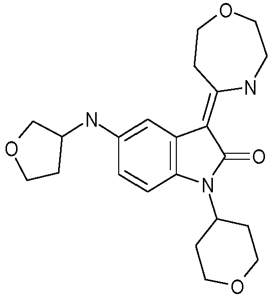 |
| 141 | 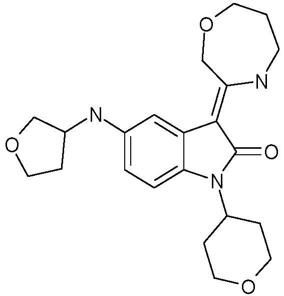 | 142 | 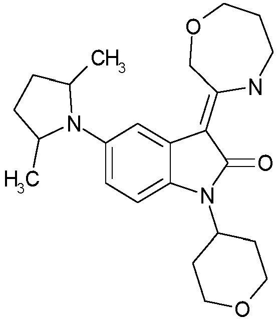 |
| 143 | 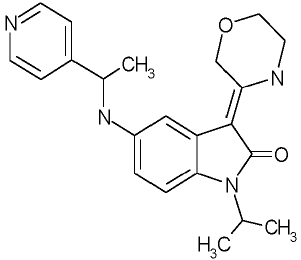 | 144 | 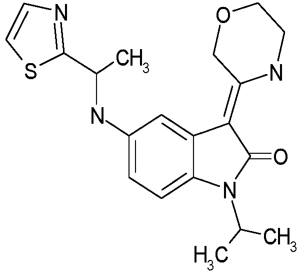 |
| 145 | 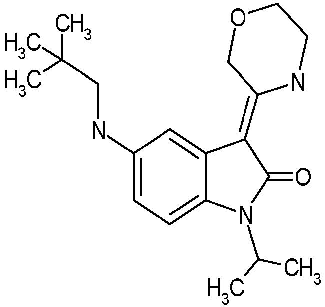 | 146 | 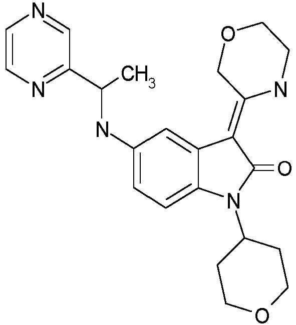 |
| 147 | 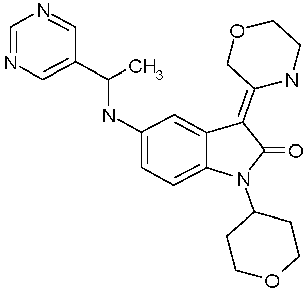 | 148 | 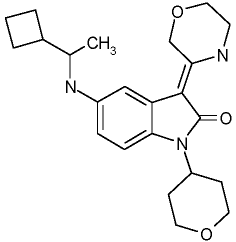 |
| 149 | 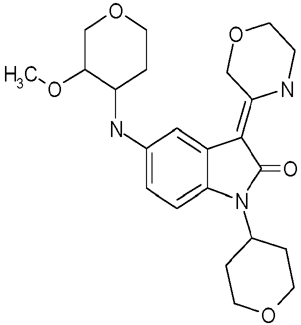 | 150 | 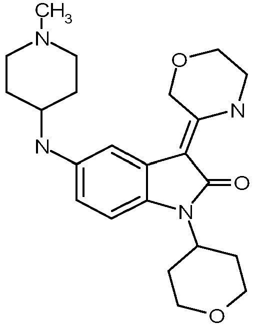 |
| 151 | 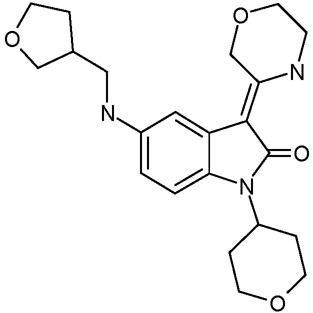 | 152 | 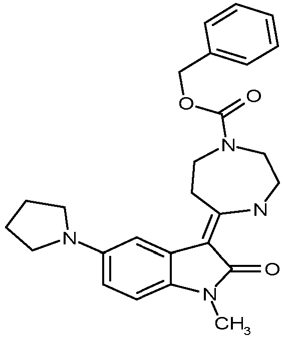 |
| 153 | 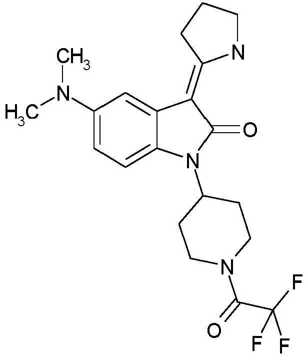 | 154 | 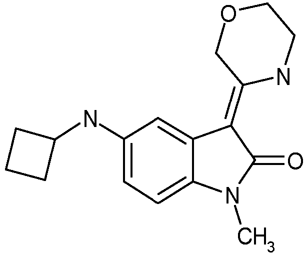 |
| 155 | 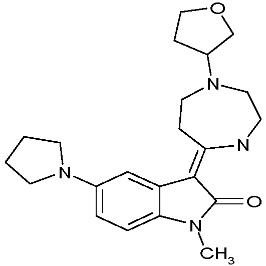 | 156 | 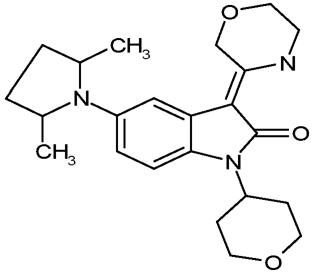 |
| 157 | 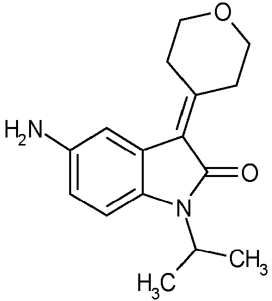 | 158 | 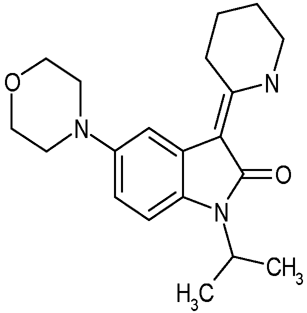 |
| 159 | 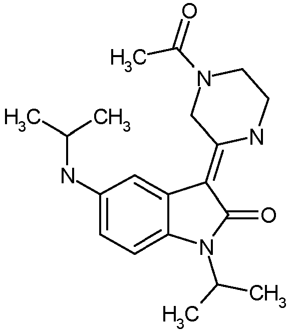 | 160 | 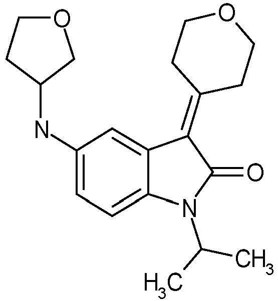 |
| 161 | 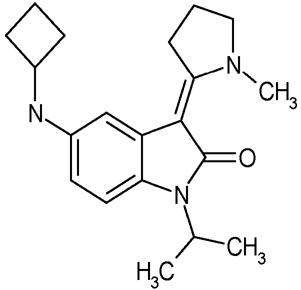 | 162 | 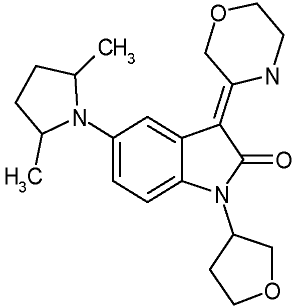 |
| 163 | 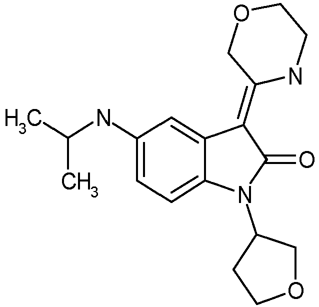 | 164 | 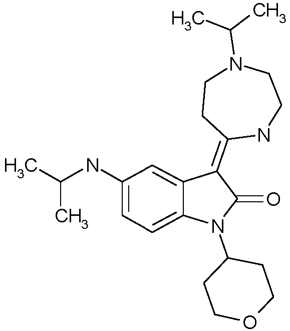 |
| 165 | 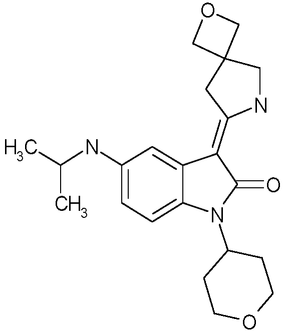 | 166 | 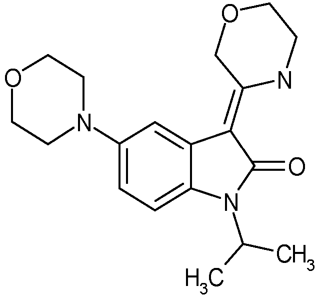 |
| 167 | 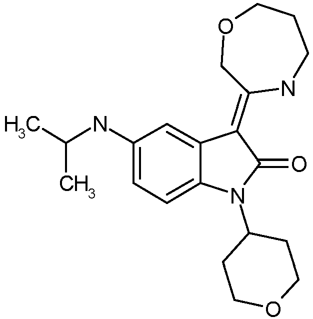 | 168 | 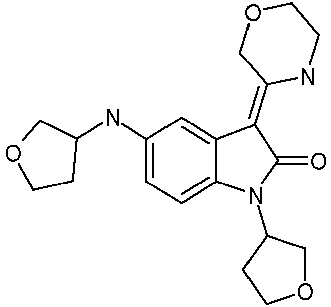 |
| 169 | 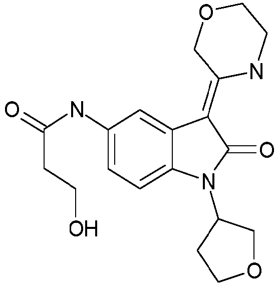 | 170 | 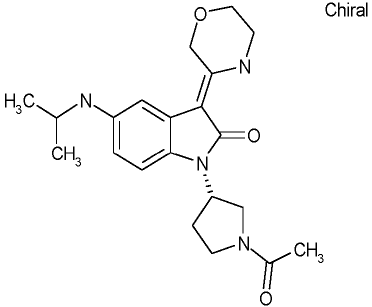 |
| 171 | 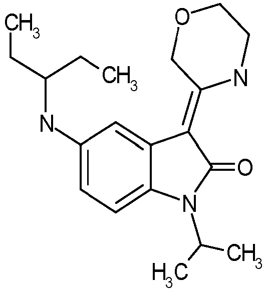 | 172 | 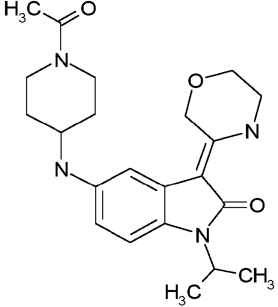 |
| 173 | 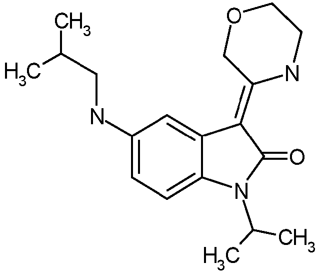 | 174 | 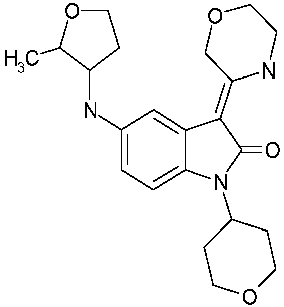 |
| 175 | 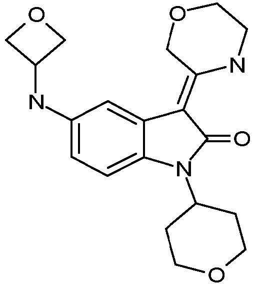 | 1001 | 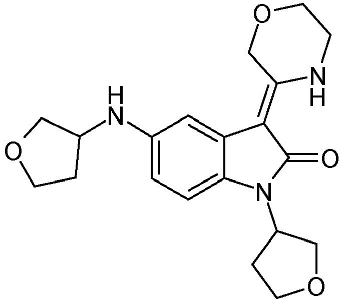 |
| 1002 | 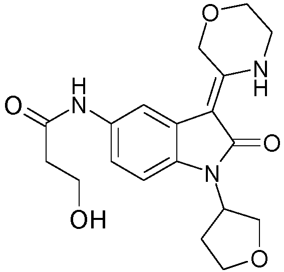 | 1003 | 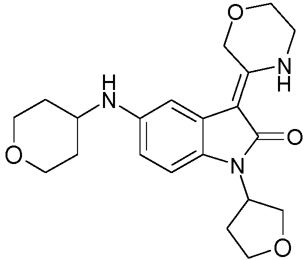 |
| 1004 | 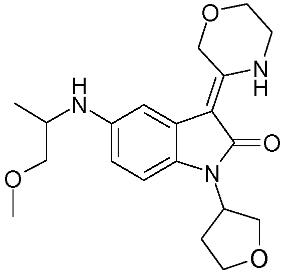 | 1005 | 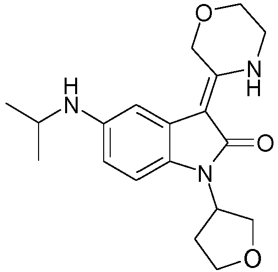 |
| 1006 | 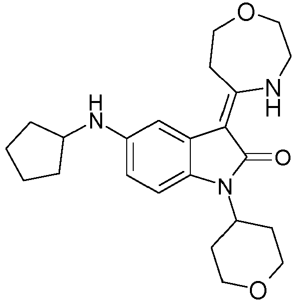 | 1007 | 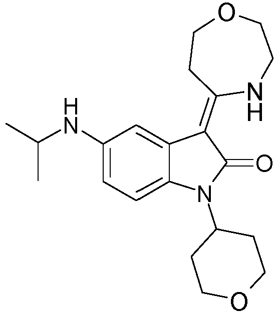 |
| 1008 | 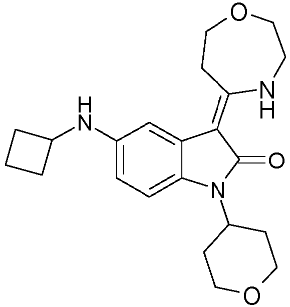 | 1009 | 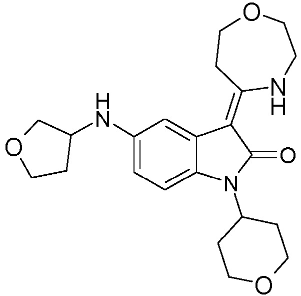 |
| 1010 | 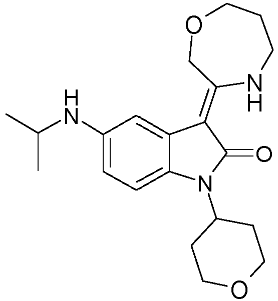 | 1011 | 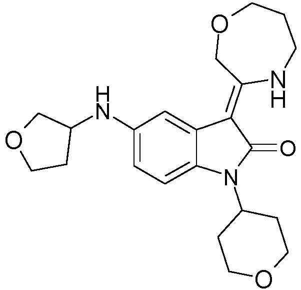 |
| 1012 | 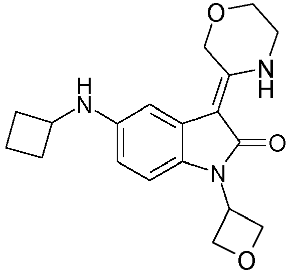 | 1013 | 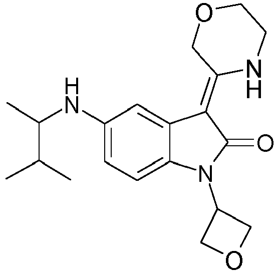 |
| 1014 | 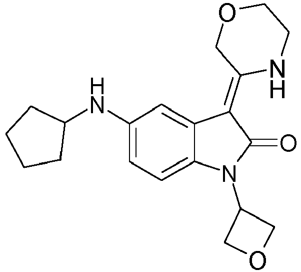 | 1015 | 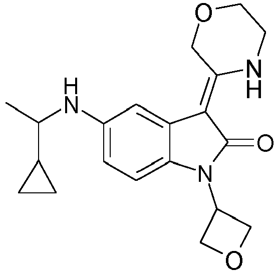 |
| 1016 | 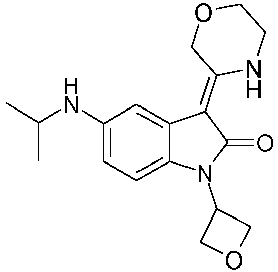 | 1017 | 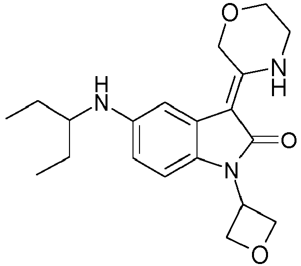 |
| 1018 | 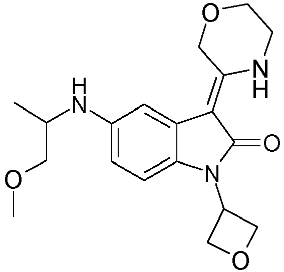 | 1019 | 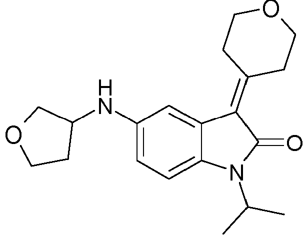 |
| 1020 | 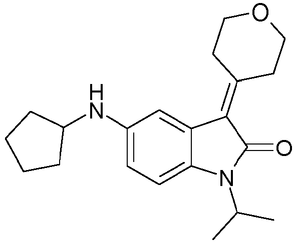 | 1021 | 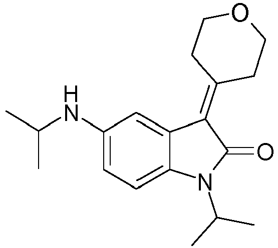 |
| 1022 | 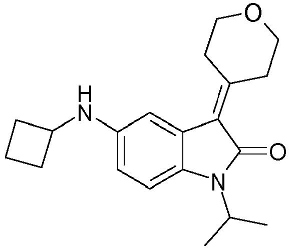 | 1023 | 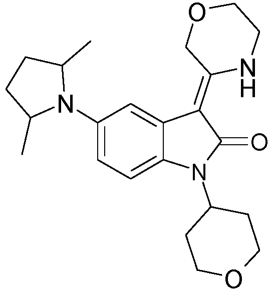 |
| 1024 | 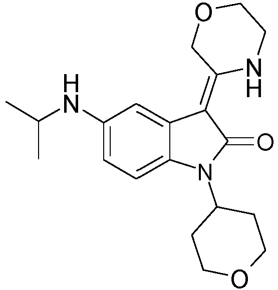 | 1025 | 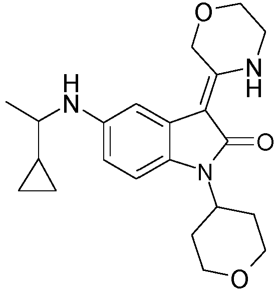 |
| 1026 | 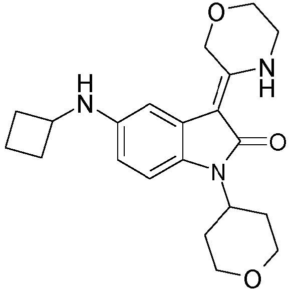 | 1027 | 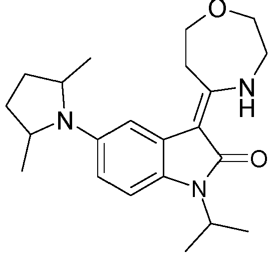 |
| 1028 | 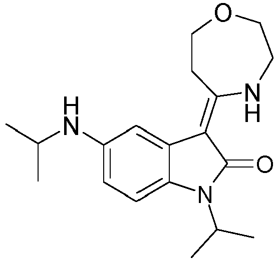 | 1029 | 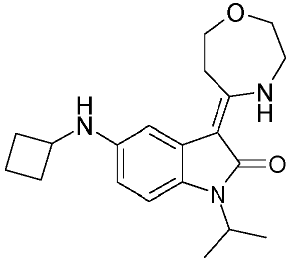 |
| 1030 | 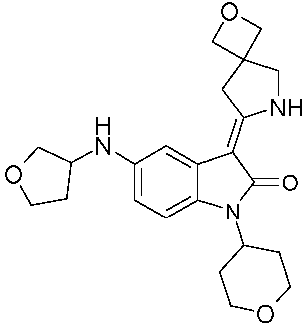 | 1031 | 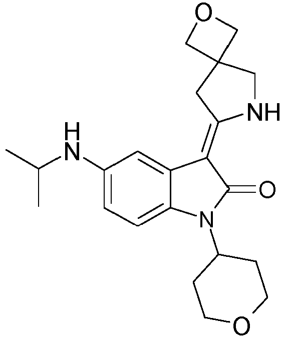 |
| 1032 | 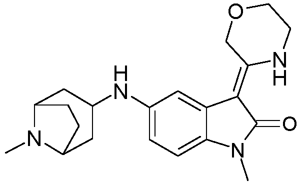 | 1033 | 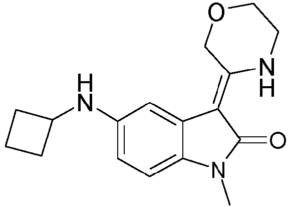 |
| 1034 | 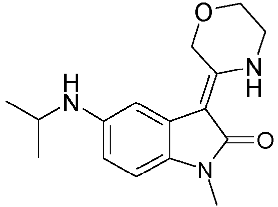 | 1035 | 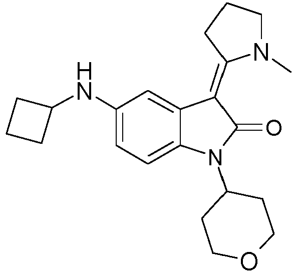 |
| 1036 | 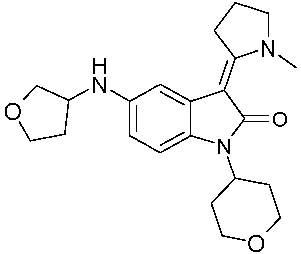 | 1037 | 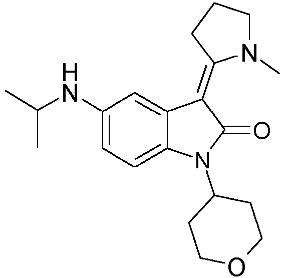 |
| 1038 | 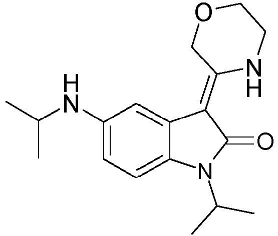 | 1039 | 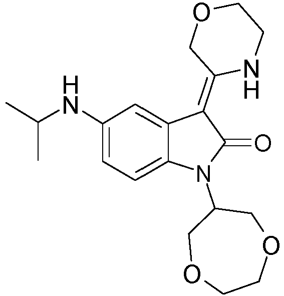 |
| 1040 | 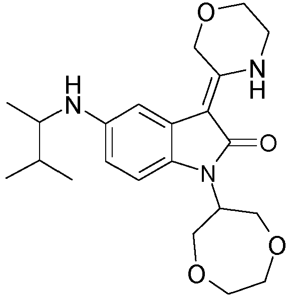 | 1041 | 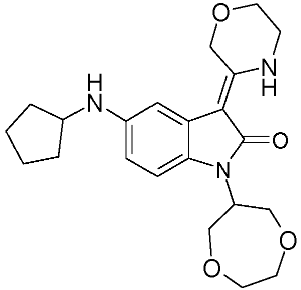 |
| 1042 | 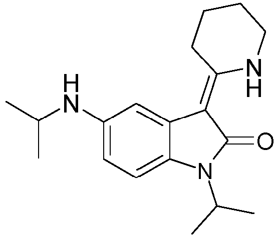 | 1043 | 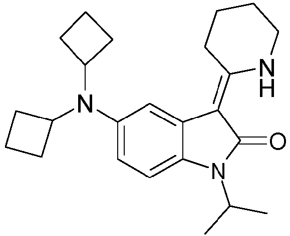 |
| 1044 | 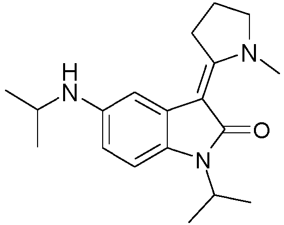 | 1045 | 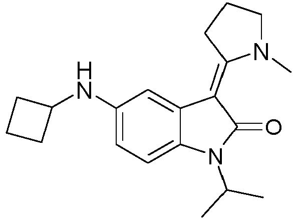 |
| 1046 | 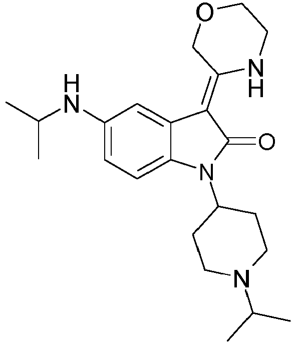 | 1047 | 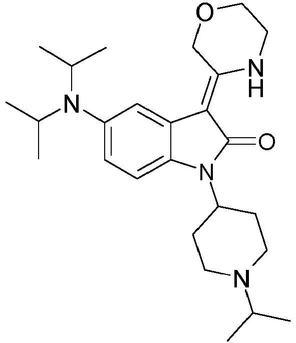 |
| 1048 | 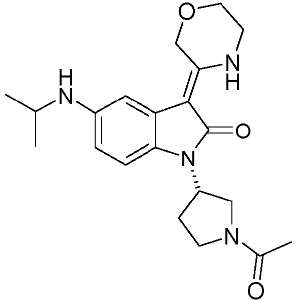 | 1049 | 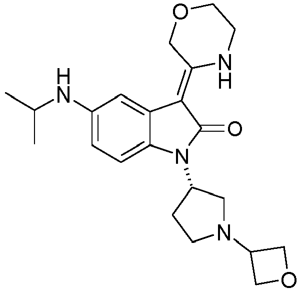 |
| 1050 | 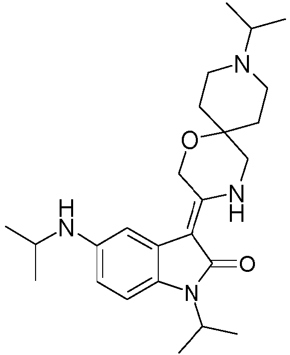 | 1051 | 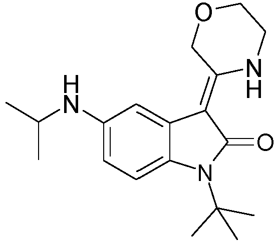 |
| 1052 | 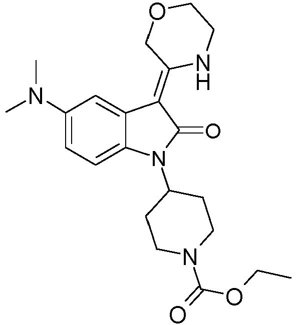 | 1053 | 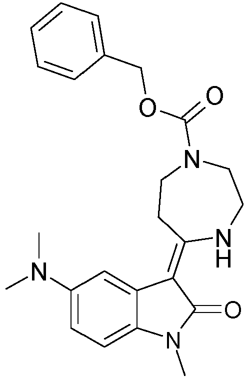 |
| 1054 | 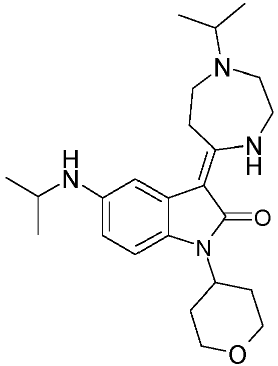 | 1055 | 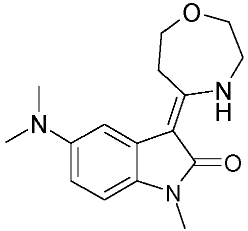 |
| 1056 | 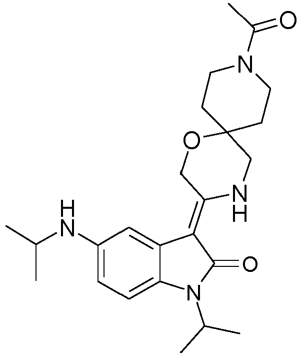 | 1057 | 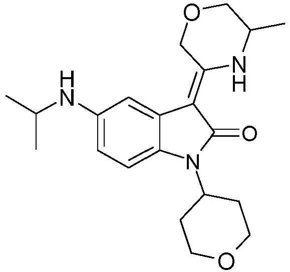 |
| 1058 | 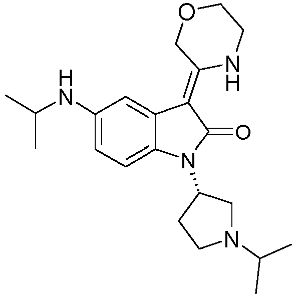 | 1059 | 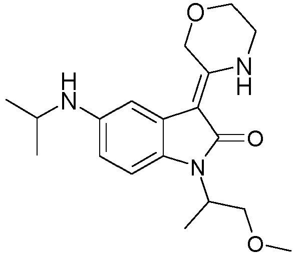 |
| 1060 | 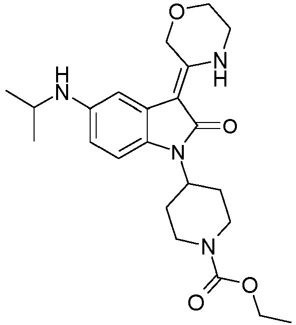 | 1061 | 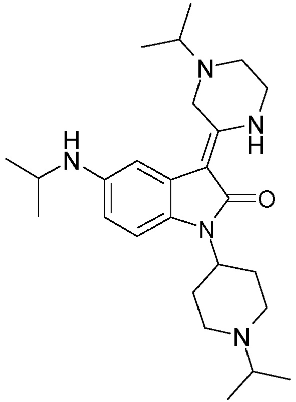 |
| 1062 | 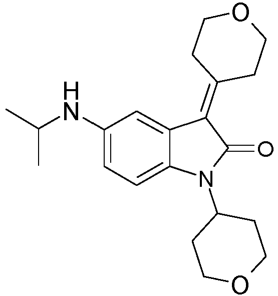 | 1063 | 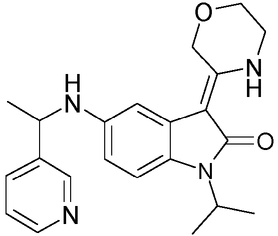 |
| 1064 | 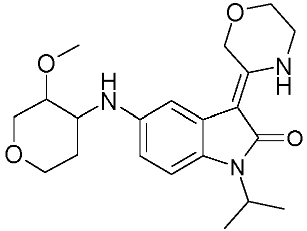 | 1065 | 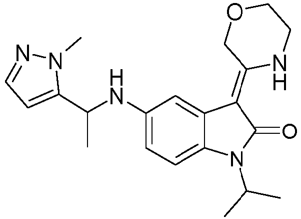 |
| 1066 | 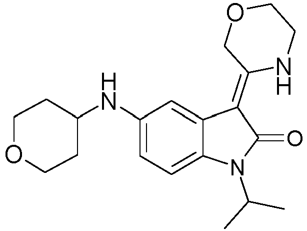 | 1067 | 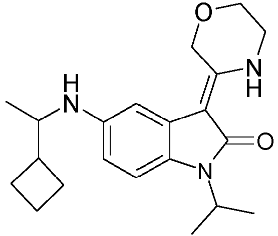 |
| 1068 | 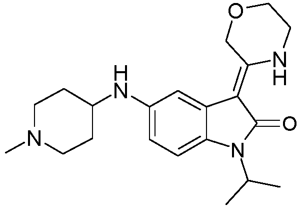 | 1069 | 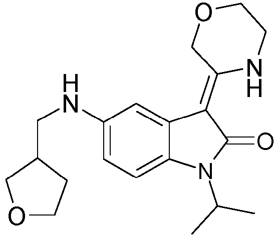 |
| 1070 | 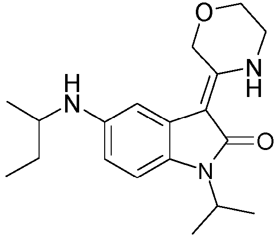 | 1071 | 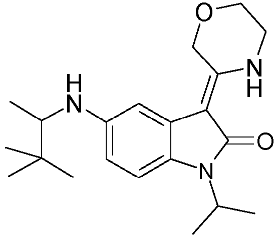 |
| 1072 | 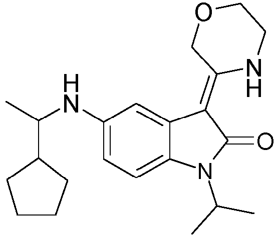 | 1073 | 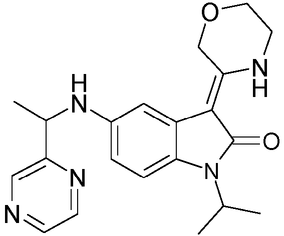 |
| 1074 | 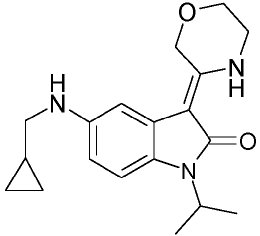 | 1075 | 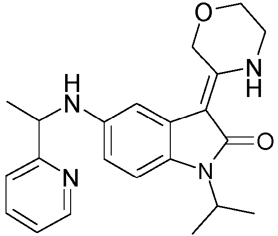 |
| 1076 | 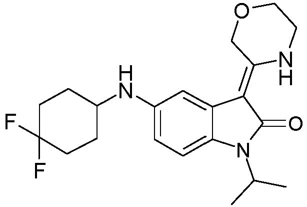 | 1077 | 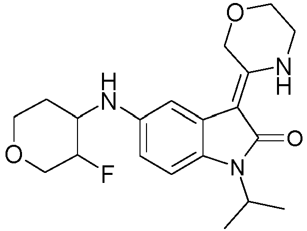 |
| 1078 | 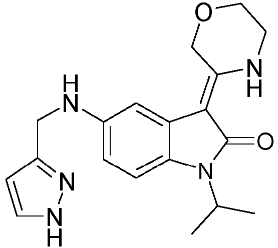 | 1079 | 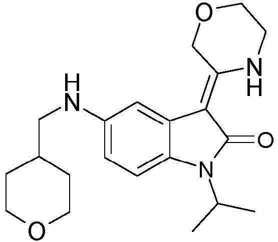 |
| 1080 | 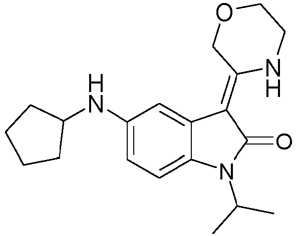 | 1081 | 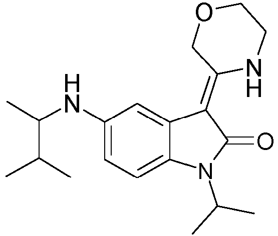 |
| 1082 | 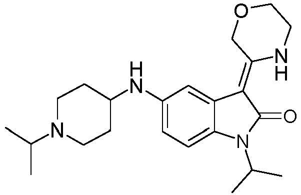 | 1083 | 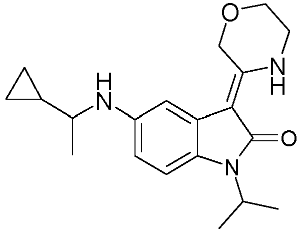 |
| 1084 | 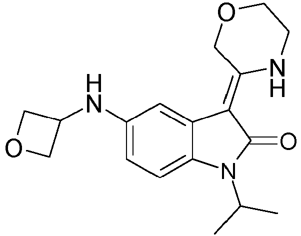 | 1085 | 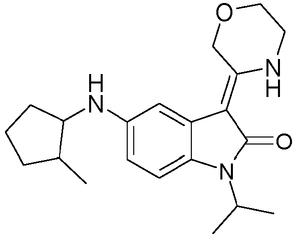 |
| 1086 | 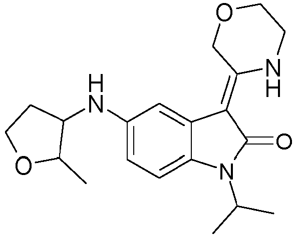 | 1087 | 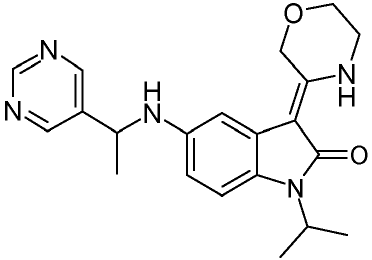 |
| 1088 | 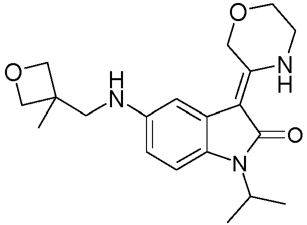 | 1089 | 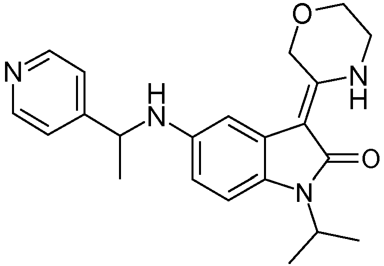 |
| 1090 | 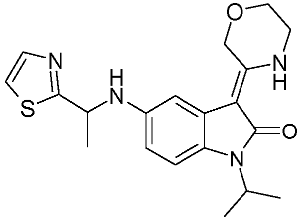 | 1091 | 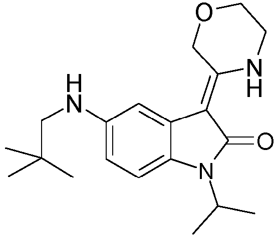 |
| 1092 | 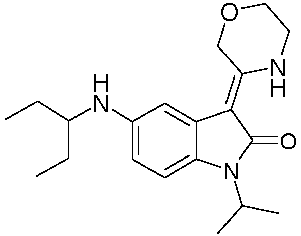 | 1093 | 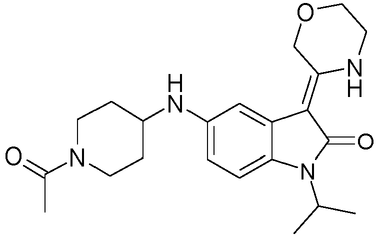 |
| 1094 | 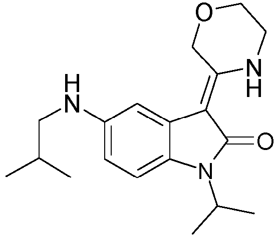 | 1095 | 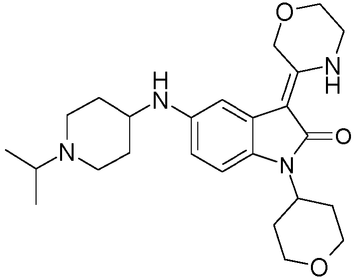 |
| 1096 | 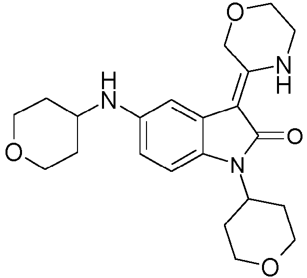 | 1097 | 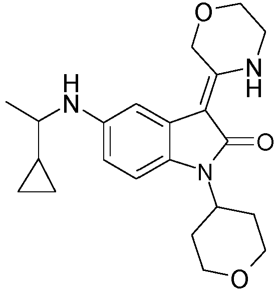 |
| 1098 | 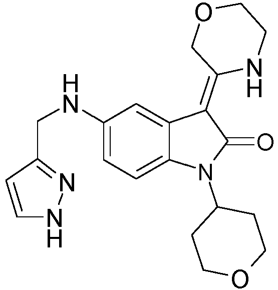 | 1099 | 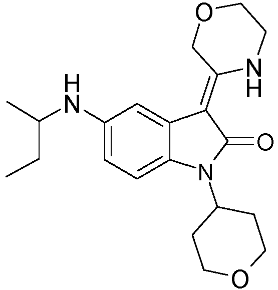 |
| 1100 | 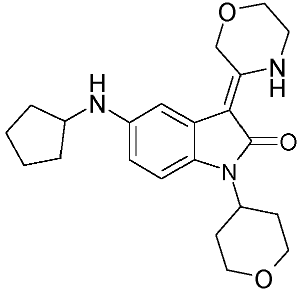 | 1101 | 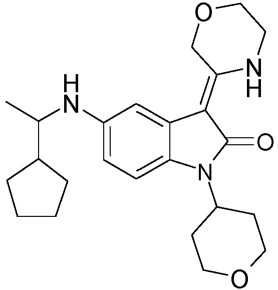 |
| 1102 | 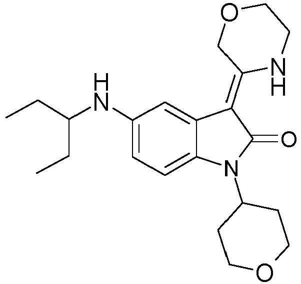 | 1103 | 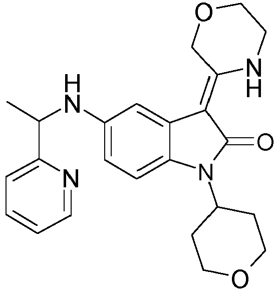 |
| 1104 | 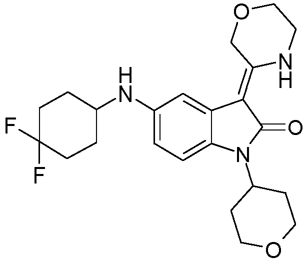 | 1105 | 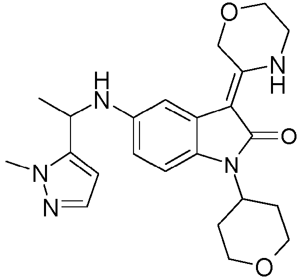 |
| 1106 | 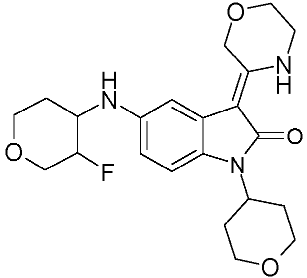 | 1107 | 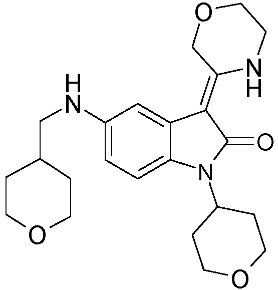 |
| 1108 | 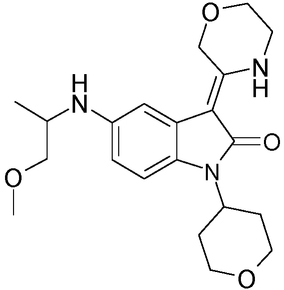 | 1109 | 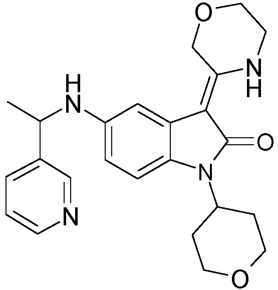 |
| 1110 | 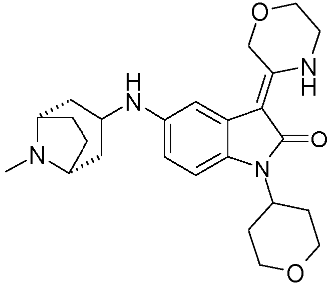 | 1111 | 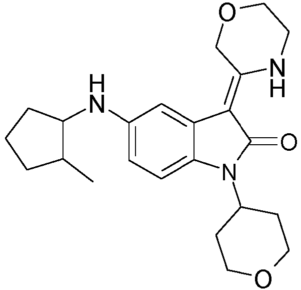 |
| 1112 | 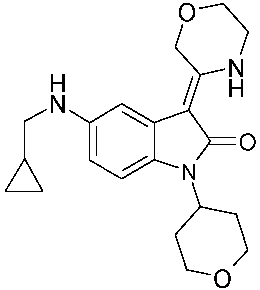 | 1113 | 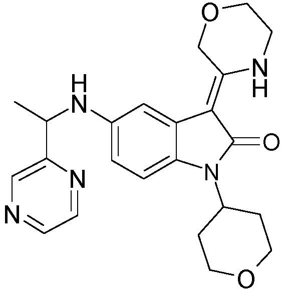 |
| 1114 | 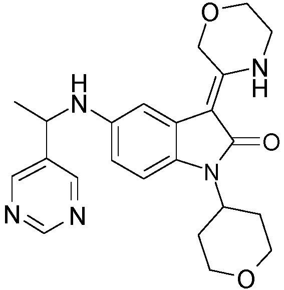 | 1115 | 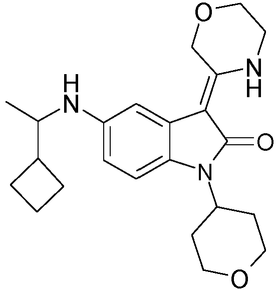 |
| 1116 | 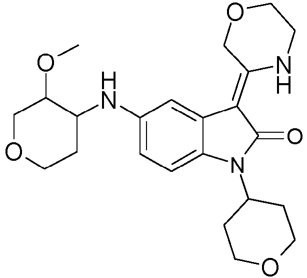 | 1117 | 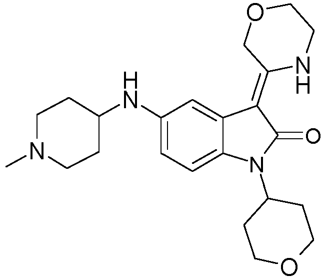 |
| 1118 | 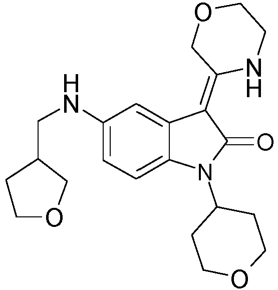 | 1119 | 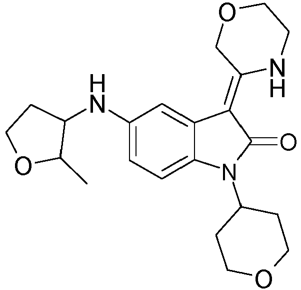 |
| 1120 | 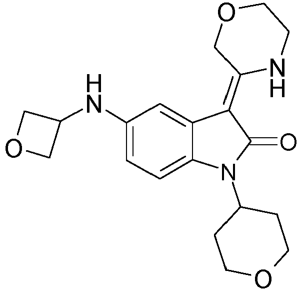 | 1121 | 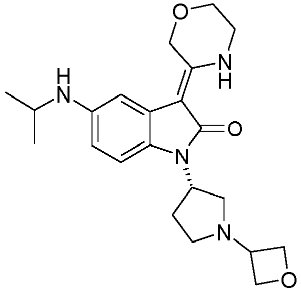 |
| 1122 | 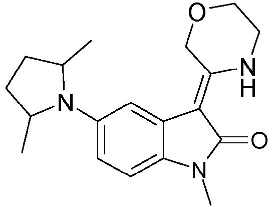 | 1123 | 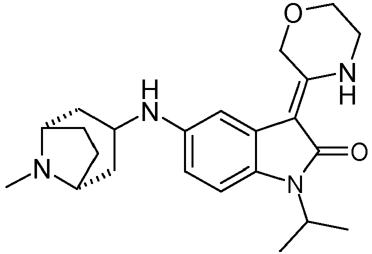 |
| 1124 | 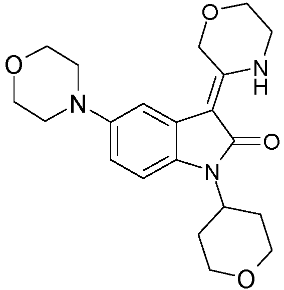 | 1125 | 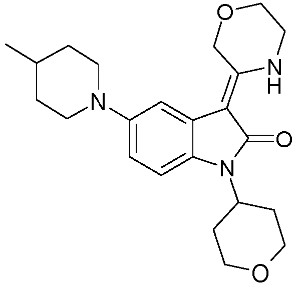 |
| 1126 | 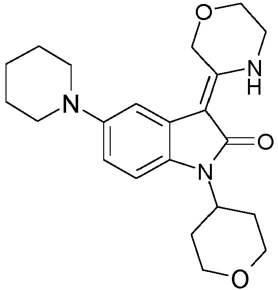 | 1127 | 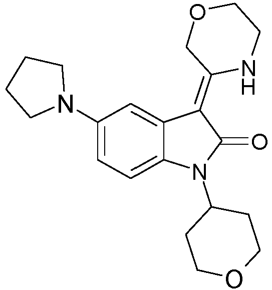 |
| 1128 | 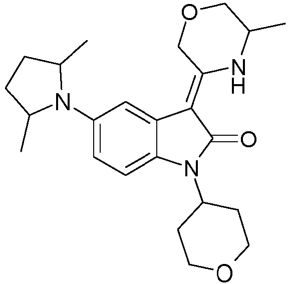 | 1129 | 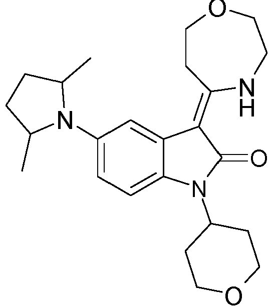 |
| 1130 | 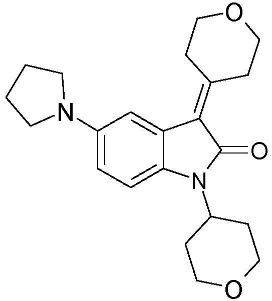 | 1131 | 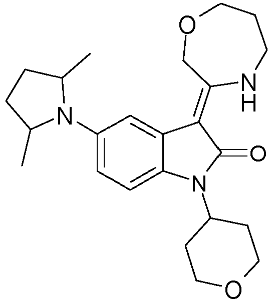 |
| 1132 | 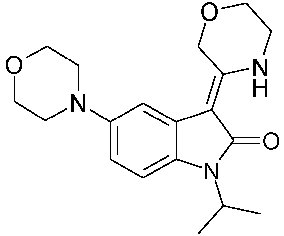 | 1133 | 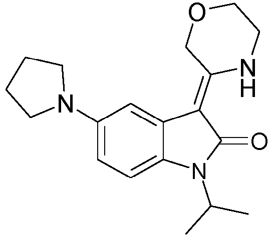 |
| 1134 | 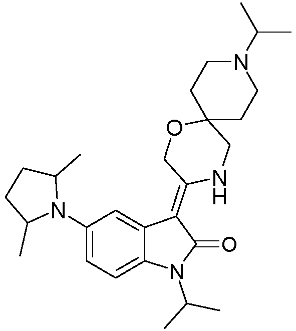 | 1135 | 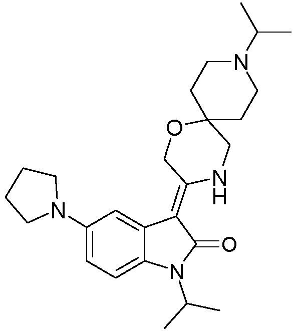 |
| 1136 | 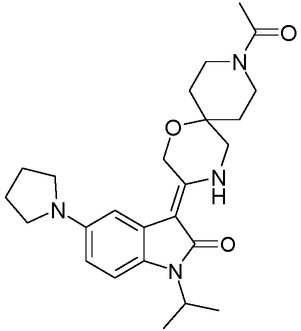 | 1137 | 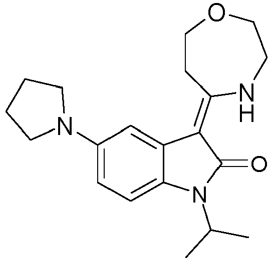 |
| 1138 | 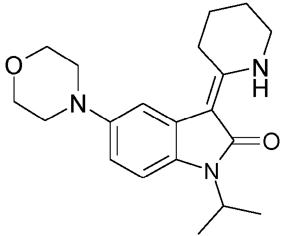 | 1139 | 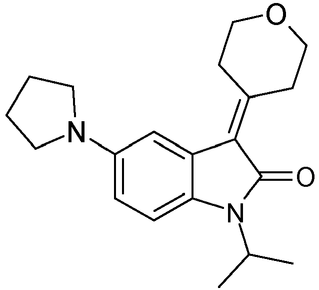 |
| 1140 | 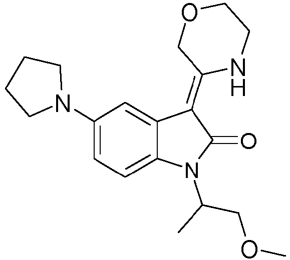 | 1141 | 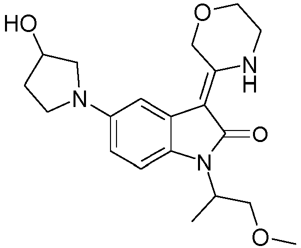 |
| 1142 | 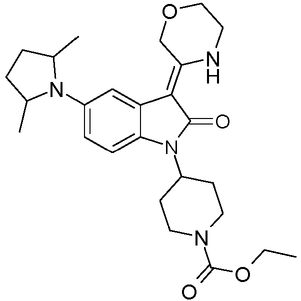 | 1143 | 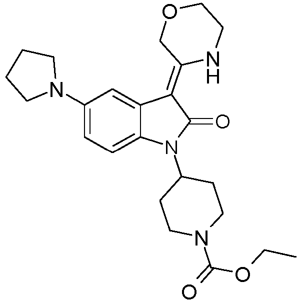 |
| 1144 | 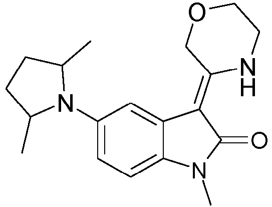 | 1145 | 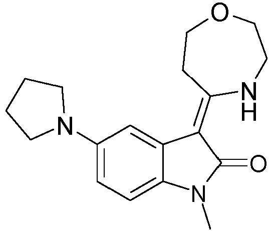 |
| 1146 | 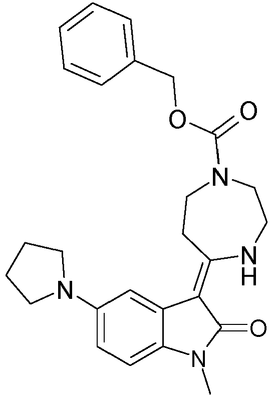 | 1147 | 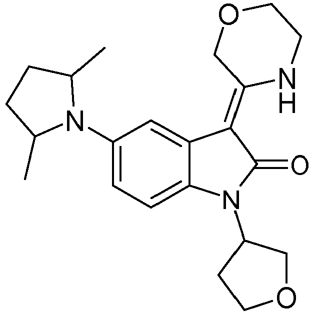 |
| 1148 | 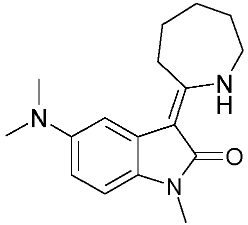 | 1149 | 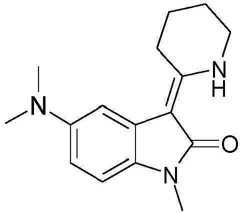 |
| 1150 | 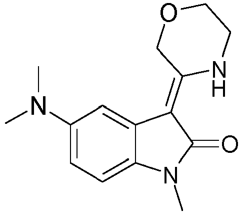 | 1151 | 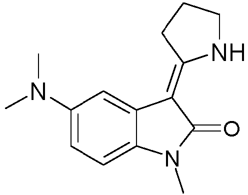 |
| 1152 | 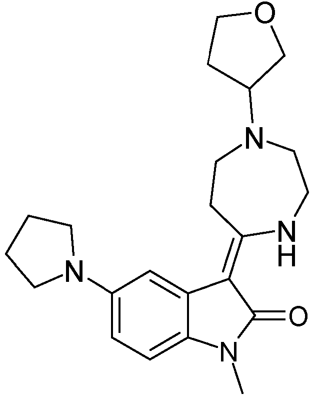 | 1153 | 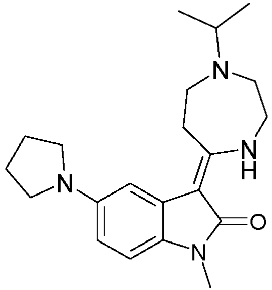 |
| 1154 | 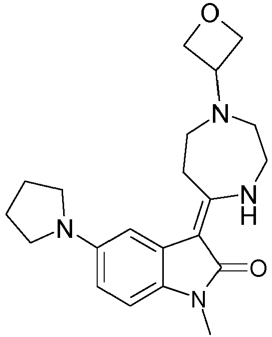 | 1155 | 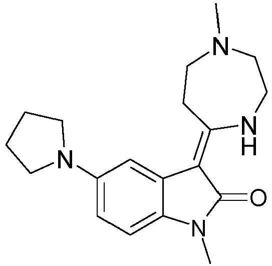 |
| 1156 | 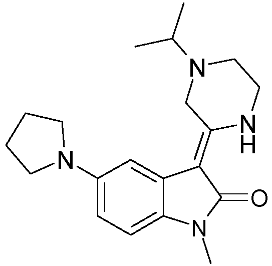 | 1157 | 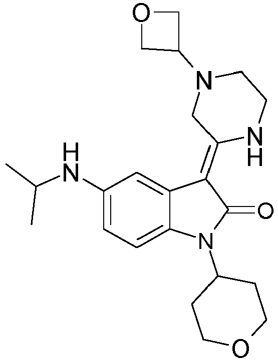 |
| 1158 | 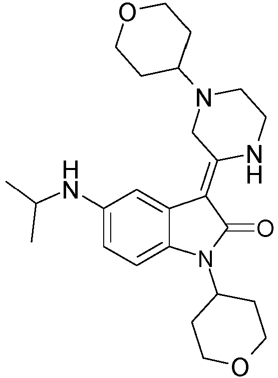 | 1159 | 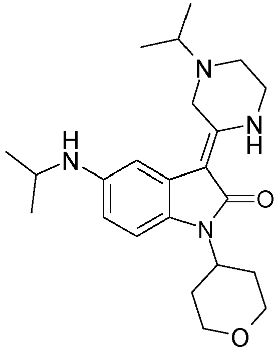 |
| 1160 | 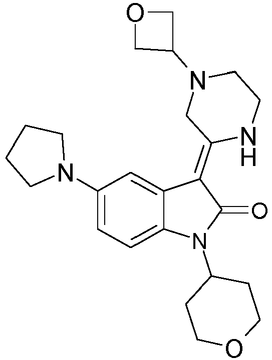 | 1161 | 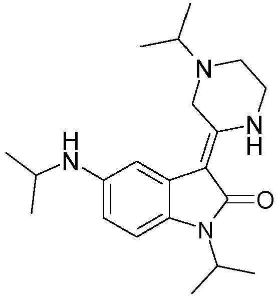 |
| 1162 | 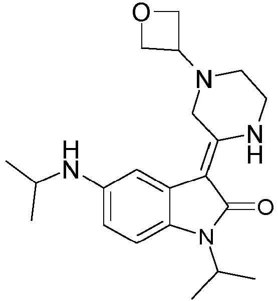 | 1163 | 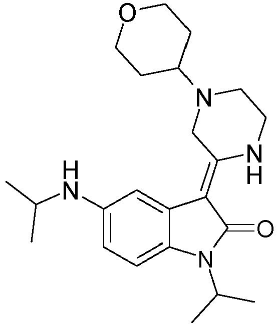 |
| 1164 | 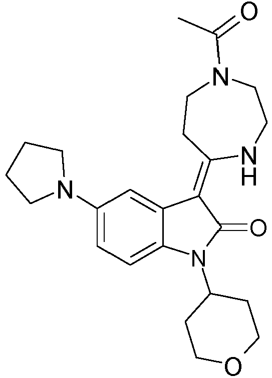 | 1165 | 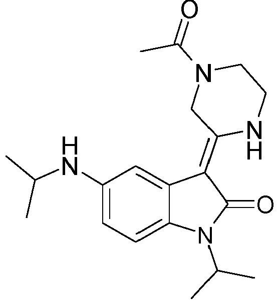 |
| 1166 | 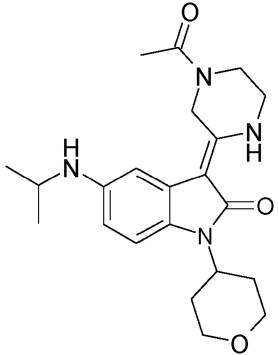 | 1167 | 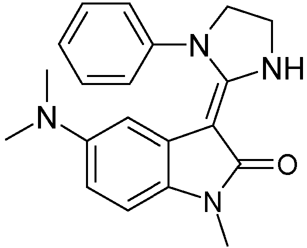 |
| 1168 | 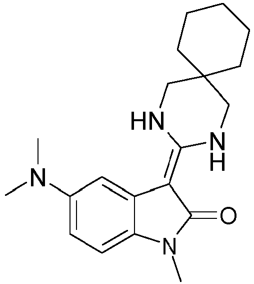 | 1169 | 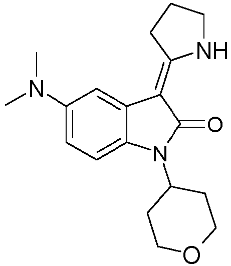 |
| 1170 | 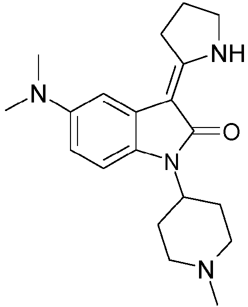 | 1171 | 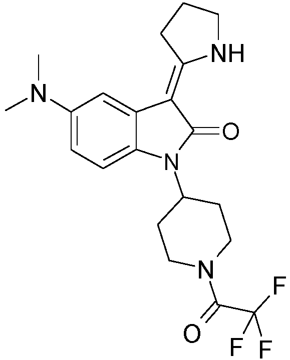 |
| 1172 | 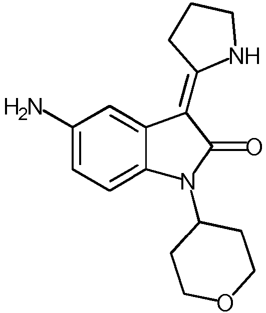 | 1173 | 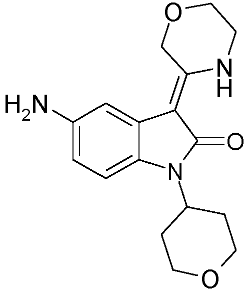 |
| 1174 | 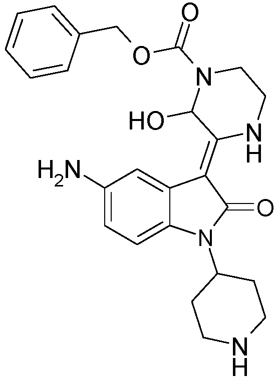 | 1175 | 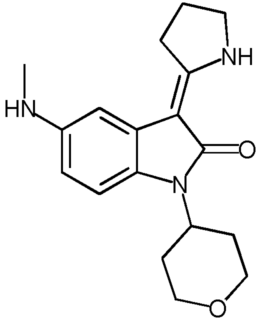 |
| 1176 | 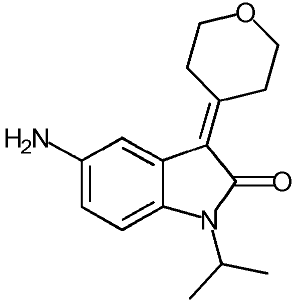 | 1177 | 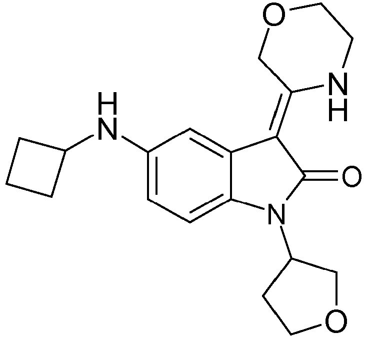 |
| 1178 | 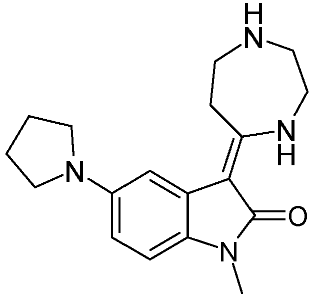 | | |

12. Médicament comprenant un composé ou un sel de celui-ci selon l'une quelconque des revendications 1 à 11.

13. Méthode pour la préparation du composé de la revendication 1, comprenant
(a) la mise en réaction d'acide (2-fluoro-5-nitro-phényl)-acétique (composé II) 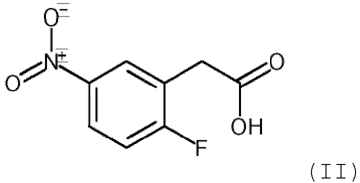 avec une amine R1-NH₂ à l'aide d'un solvant approprié pour former une 5-nitro-2, 3-dihydro-1H-indol-2-one (composé III) 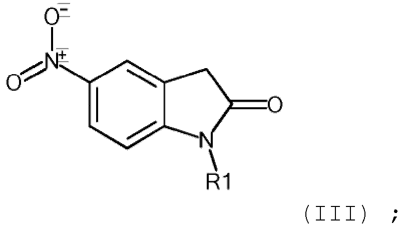
(b) la condensation de la 5-nitro-2,3-dihydro-1H-indol-2-one (composé III) à une température élevée dans un four à micro-ondes soit sans soit dans un solvant convenable comme la péridine avec un électrophile convenable pour donner une 5-nitro-3-ylidène-2, 3-dihydro-1H-indol-2-one (composé IV) 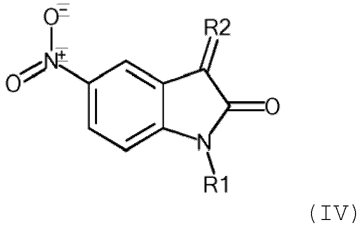
(c) la réduction des 5-nitro-3-ylidène-2,3-dihydro-1H-indol-2-ones (composé IV) en une amino-substituée indol-2-one (composé V) 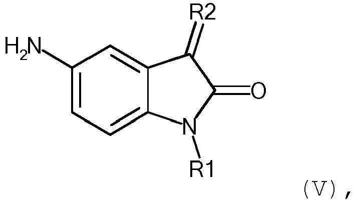
(d1) la mise en réaction du composé de formule **(V)** avec un aldéhyde ou une cétone et un agent de réduction convenable pour obtenir un composé de formule **(I),** ou
(d2) la mise en réaction d'une amine de formule (V) dans une réaction de substitution avec des électrophiles convenables pour porter un groupe partant en présence d'une base convenable.

14. Composition pharmaceutique pour son utilisation dans le traitement ou la prévention d'une affection neurologique ou neurodégénérative ou psychiatrique chez un être humain, comprenant un composé selon l'une quelconque des revendications 1 à 11, ou un sel pharmaceutiquement acceptable de celui-ci, et un support pharmaceutiquement acceptable.

15. Composition pharmaceutique selon la revendication 14, comprenant une quantité thérapeutiquement efficace de 0,1 à 2000 mg du composé selon l'une quelconque des revendications 1 à 11, ou d'un sel pharmaceutiquement acceptable de celui-ci.
